# EUROPEAN PATENT APPLICATION

(11) **EP 2 426 122 A1**
(43) Date of publication of application: **07.03.2012**
(21) Application number: 11009407.5
(22) Date of filing: 08.10.2003
(51) Int. Cl.: C07D 401/12, C07D 409/12, C07D 413/12, C07D 417/12, C07D 213/68, C07D 213/75, A61K 31/44, A61K 31/4427, A61P 29/00

(54) **Methylene urea derivative as RAF kinasse inhibitors**

(30) Priority: 24.10.2002 EP 02023906; 28.07.2003 US 490285 P
(62) Divisional of application: 03750697.9
(71) Applicant: Merck Patent GmbH, 64293 Darmstadt (DE)
(72) Inventor: Buchstaller, Hans-Peter, 64347 Griesheim (DE); Wiesner, Matthias, 64342 Seeheim-Jugenheim (DE); Schadt, Oliver, 63517 Rodenbach (DE); Amendt, Christiane, 64367 Muehltal/Trautheim (DE); Zenke, Frank, 64291 Darmstadt (DE); Sirrenberg, Christian, 64285 Darmstadt (DE); Grell, Matthias, 64291 Darmstat (DE); Finsinger, Dirk, 64291 Darmstadt (DE)

(57) **Abstract**

The present invention relates to methylene urea derivatives of formula I, the use of the compounds of formula I as inhibitors of raf-kinase, the use of the compounds of formula I for the manufacture of a pharmaceutical composition and a method of treatment, comprising administering said pharmaceutical composition to a patient.

## Description

The present invention relates to methylene urea derivatives, methylene urea derivatives as medicaments, methylene urea derivatives as inhibitors of raf-kinase, the use of methylene urea derivatives for the manufacture of a pharmaceutical, a method for producing a pharmaceutical composition containing said methylene urea derivatives, the pharmaceutical composition obtainable by said method and a method of treatment, comprising administering said pharmaceutical composition.

Protein phosphorylation is a fundamental process for the regulation of cellular functions. The coordinated action of both protein kinases and phosphatases controls the levels of phosphorylation and, hence, the activity of specific target proteins. One of the predominant roles of protein phosphorylation is in signal transduction, where extracellular signals are amplified and propagated by a cascade of protein phosphorylation and dephosphorylation events, e.g. in the p21^{ras}/raf pathway.

The p21^{ras} gene was discovered as an oncogene of the Harvey (rasH) and Kirsten (rasK) rat sarcoma viruses. In humans, characteristic mutations in the cellular ras gene (c-ras) have been associated with many different types of cancers. These mutant alleles, which render Ras constitutively active, have been shown to transform cells, such as the murine cell line NIH 3T3, in culture.

The p21^{ras} oncogene is a major contributor to the development and progression of human solid cancers and is mutated in 30 % of all human cancers (Bolton et al. (1994) Ann. Rep. Med. Chem., 29, 165-74; Bos. (1989) Cancer Res., 49, 4682-9). Oncogenic Ras mutations have been identified for example in lung cancer, colorectal cancer, pancreas, thyroid cancer, melanoma, bladder tumors, liver tumor, kidney tumor, dermatological tumors and haematological tumors (Ddjei et al. (2001), J. Natl. Cancer Inst. 93(14), 1062-74; Midgley, R.S. and Kerr, D.J. (2002) Critical Rev. Onc/ hematol 44, 109-120; Downward, J. (2003), Nature reviews 3, 11-22). In its normal, unmutated form, the ras protein is a key element of the signal transduction cascade directed by growth factor preceptors in almost all tissues (Avruch et al. (1994) Trends Biochem. Sci., 19, 279-83).

Biochemically, ras is a guanine nucleotide binding protein, and cycling between a GTP-bound activated and a GDP-bound resting form is strictly controlled by ras endogenous GTPase activity and other regulatory proteins. The ras gene product binds to guanine triphosphate (GTP) and guanine diphosphate (GDP) and hydrolyzes GTP to GDP. It is the GTP-bound state of Ras that is active. In the ras mutants in cancer cells, the endogenous GTPase activity is alleviated and, therefore, the protein delivers constitutive growth signals to downstream effectors such as the enzyme raf kinase. This leads to the cancerous growth of the cells which carry these mutants (Magnuson et al. (1994) Semin. Cancer Biol., 5, 247-53). The ras proto-oncogene requires a functionally intact c-raf1 proto-oncogene in order to transduce growth and differentiation signals initiated by receptor and non-receptor tyrosine kinases in higher eukaryotes.

Activated Ras is necessary for the activation of the c-raf-1 proto-oncogene, but the biochemical steps through which Ras activates the Raf-1 protein (Ser/Thr) kinase are now well characterized. It has been shown that inhibiting the effect of active ras by inhibiting the raf kinase signaling pathway by administration of deactivating antibodies to raf kinase or by co-expression of dominant negative raf kinase or dominant negative MEK also called ERK, the substrate of raf kinase, leads to the reversion of transformed cells to the normal growth phenotype see: Daum et al. (1994) Trends Biochem. Sci., 19, 474-80; Fridman et al. (1994) J Biol. Chem., 269, 30105-8. Kolch et al. (1991) Nature, 349, 426-28) and for review Weinstein-Oppenheimer et al. Pharm. & Therap. (2000), 88, 229-279. Similarly, inhibition of raf kinase (by antisense oligodeoxynucleotides) has been correlated in vitro and in vivo with inhibition of the growth of a variety of human tumor types (Monia et al., Nat. Med. 1996, 2, 668-75; Geiger et al. (1997), Clin. Cancer Res. 3(7): 1179-85; Lau et al. (2002), Antisense Nucl. Acid. Drug Dev. 12(1): 11-20 ; McPhillips et al. (2001), Br. J. Cancer 85(11): 1753-8).

Raf serine- and threonine-specific protein kinases are cytosolic enzymes that stimulate cell growth in a variety of cell systems (Rapp, U.R., et al. (1988) in The oncogene handbook; T. Curran, E.P. Reddy, and A. Skalka (ed.) Elsevier Science Publishers; The Netherlands, pp. 213-253; Rapp, U.R., et al. (1988) Cold Spring Harbor Sym. Quant. Biol. 53:173-184; Rapp, U.R., et al. (1990) Inv Curr. Top. Microbiol. Amunol. Potter and Melchers (eds), Berlin, Springer-Verlag 166:129-139).

Three isozymes have been characterized:
c-Raf (also named Raf-1, c-raf 1 or c-raf1) (Bonner, T.I., et al. (1986) Nucleic Acids Res. 14:1009-1015). A-Raf (Beck, T.W., et al. (1987) Nucleic Acids Res. 15:595-609), and B-Raf (Qkawa, S., et al. (1998) Mol. Cell. Biol. 8:2651-2654; Sithanandam, G. et a. (1990) Oncogene:1775). These enzymes differ in their expression in various tissues. Raf-1 is expressed in all organs and in all cell lines that have been examined, and A- and B-Raf are expressed in urogenital and brain tissues, respectively (Storm, S.M. (1990) Oncogene 5:345-351).
Raf genes are proto-oncogenes: they can initiate malignant transformation of cells when expressed in specifically altered forms. Genetic changes that lead to oncogenic activation generate a constitutively active protein kinase by removal or interference with an N-terminal negative regulatory domain of the protein (Heidecker, G., et al. (1990) Mol. Cell. Biol. 10:2503-2512; Rapp, U.R., et al. (1987) in Oncogenes and cancer S. A. Aaronson, J. Bishop, T. Sugimura, M. Terada, K. Toyoshima, and P. K. Vogt (ed). Japan Scientific Press, Tokyo). Microinjection into NIH 3T3 cells of oncogenically activated but not wild-type versions of the Raf-protein prepared with Escherichia coli expression vectors results in morphological transformation and stimulates DNA synthesis (Rapp, U.R., et al. (1987) in Oncogenes and cancer; S. A. Aaronson, J. Bishop, T. Sugimura, M. Terada, K. Toyoshima, and P. K. Vogt (ed.) Japan Scientific Press, Tokyo; Smith, M. R., et al (1990) Mol. Cell. Biol. 10:3828-3833). Activating mutants of B-Raf have been identified in a wide range of human cancers e.g. colon, ovarien, melanomas and sarcomas (Davies, H., et al. (2002), Nature 417 949-945. Published online June 9, 2002, 10.1038/nature00766). The preponderant mutation is a single phosphomimetic substitution in the kinase activation domain (V599E), leading to constitutive kinase activity and transformation of NIH3T3 cells.

Thus, activated Raf-1 is an intracellular activator of cell growth. Raf-1 protein serine kinase in a candidate downstream effector of mitogen signal transduction, since Raf oncogenes overcome growth arrest resulting from a block of cellular ras activity due either to a cellular mutation (ras revertant cells) or microinjection of anti-ras antibodies (Rapp, U.R., et al. (1988) in The Oncogene Handbook, T. Curran, E.P. Reddy, and A. Skalka (ed.), Elsevier Science Publishers; The Netherlands, pp. 213-253; Smith, M.R., et al. (1986) Nature (London) 320:540-543).

c-Raf function is required for transformation by a variety of membrane-bound oncogenes and for growth stimulation by mitogens contained in serums (Smith, M.R., et al. (1986) Nature (London) 320:540-543). Raf-1 protein serine kinase activity is regulated by mitogens via phosphorylation (Morrison, D.K., et al. (1989) Cell 58:648-657), which also effects sub cellular distribution (Olah, Z., et al. (1991) Exp. Brain Res. 84:403; Rapp, U.R., et al. (1988) Cold Spring Harbor Sym. Quant. Biol. 53:173-184. Raf-1 activating growth factors include platelet-derived growth factor (PDGF) (Morrison, D.K., et al. (1988) Proc. Natl. Acad. Sci. USA 85:8855-8859), colony-stimulating factor (Baccarini, M., et al. (1990) EMBO J. 9:3649-3657), insulin (Blackshear, P.J., et al. (1990) J. Biol. Chem. 265:12115-12118), epidermal growth factor (EGF) (Morrison, R.K., et al. (1988) Proc. Natl. Acad. Sci. USA 85:8855-8859), interleukin 2 (Turner, B.C., et al (1991) Proc. Natl. Acad. Sci. USA 88:1227), and interleukin 3 and granulocytemacrophage colony-stimulating factor (Carroll, M.P., et al (1990) J. Biol. Chem. 265:19812-19817).

Upon mitogen treatment of cells, the transiently activated Raf-1 protein serine kinase translocates to the perinuclear area and the nucleus (Olah, Z., et al. (1991) Exp. Brain Res. 84:403; Rapp, U.R., et al. (1988) Cold Spring Habor Sym. Quant. Biol. 53:173-184). Cells containing activated Raf are altered in their pattern of gene expression (Heidecker, G., et al. (1989) in Genes and signal transduction in multistage carcinogenesis, N. Colburn (ed.), Marcel Dekker, Inc., New York, pp. 339-374), and Raf oncogenes activate transcription from Ap-I/PEA3-dependent promoters in transient transfection assays (Jamal, S., et al (1990) Science 344:463-466; Kaibuchi, K., et al (1989) J. Biol. Chem. 264:20855-20858; Wasylyk, C., et al. (1989) Mol. Cell. Biol. 9:2247-2250).

There are at least two independent pathways for Raf-1 activation by extracellular mitogens: one involving protein kinase C (KC) and a second initiated by protein tyrosine kinases (Blackshear, P.J., et al. (1990) J. Biol. Chem. 265:12131-12134; Kovacina, K.S., et al (1990) J. Biol. Chem. 265:12115-12118; Morrison, D.K., et al. (1988) Proc. Natl. Acad. Sci. USA 85:8855-8859; Siegel, J.N., et al (1990) J. Biol. Chem. 265:18472-18480; Turner, B.C., et al (1991) Proc. Natl. Acad. Sci. USA 88:1227). In either case, activation involves Raf-1 protein phosphorylation. Raf-1 phosphorylation may be a consequence of a kinase cascade amplified by autophosphorylation or may be caused entirely by autophosphorylation initiated by binding of a putative activating ligand to the Raf-1 regulatory domain, analogous to PKC activation by diacylglycerol (Nishizuka, Y. (1986) Science 233:305-312).

The process of angiogenesis is the development of new blood vessels, generally capillaries, from pre-existing vasculature. Angiogenesis is defined as involving (i) activation of endothelial cells; (ii) increased vascular permeability; (iii) subsequent dissolution of the basement membrane and extravisation of plasma components leading to formation of a provisional fibrin gel extracellular matrix; (iv) proliferation and mobilization of endothelial cells; (v) reorganization of mobilized endothelial cells to form functional capillaries; (vi) capillary loop formation; and (vii) deposition of basement membrane and recruitment of perivascular cells to newly formed vessels.

Normal angiogenesis is activated during tissue growth, from embryonic development through maturity, and then enters a period of relative quiescence during adulthood.

Normal angiogensesis is also activated during wound healing, and at certain stages of the female reproductive cycle. Inappropriate or pathological angiogenesis has been associated with several disease states including various retinopathies; ischemic disease; atherosclerosis; chronic inflammatory disorders; rheumatoid arthritis, and cancer. The role of angiogenesis in disease states is discussed, for instance, in Fan et al, Trends in Pharmacol Sci. 16:54 66; Shawver et al, DOT Vol. 2, No. 2 February 1997; Folkmann, 1995, Nature Medicine 1:27-31.

In cancer the growth of solid tumors has been shown to be angiogenesis dependent. (See Folkmann, J., J. Nat'l. Cancer Inst., 1990, 82, 4-6.) Consequently, the targeting of pro-angiogenic pathways is a strategy being widely pursued in order to provide new therapeutics in these areas of great, unmet medical need.

Raf is involved in angiogenic processes. Endothelial growth factors (e.g. vascular endothelial growth factor VEGF or basic fibroblast growth factor bFGF) activates receptor tyrosine kinases (e.g. VEGFR-2) and signal through the Ras/Raf/Mek/Erk kinase cascade and protects endothelial cells from apoptosis (Alavi et al. (2003), Science 301, 94-96; Hood, J.D. et al. (2002), Science 296, 2404; Mikula, M. et al. (2001), EMBO J. 20, 1952; Hauser, M. et al. (2001), EMBO J. 20, 1940; Wojnowski et al. (1997), Nature Genet. 16, 293). Activation of VEGFR-2 by VEGF is a critical step in the signal transduction pathway that initiates tumor angiogenesis. VEGF expression may be constitutive to tumor cells and can also be upregulated in response to certain stimuli. One such stimuli is hypoxia, where VEGF expression is upregulated in both tumor and associated host tissues. The VEGF ligand activates VEGFR-2 by binding with its extracellular VEGF binding site. This leads to receptor dimerization of VEGFRs and autophosphorylation of tyrosine residues at the intracellular kinase domain of VEGFR- 2. The kinase domain operates to transfer a phosphate from ATP to the tyrosine residues, thus providing binding sites for signaling proteins downstream of VEGFR-2 leading ultimately to initiation of angiogenesis (McMahon, G., The Oncologist, Vol. 5, No. 90001, 3-10, April 2000).

Mice with a targeted disruption in the Braf gene die of vascular defects during development (Wojnowski, L. et al. 1997, Nature genetics 16, page 293- 296). These mice show defects in the formation of the vascular system and in angiogenesis e.g. enlarged blood vessels and increased apoptotic death of differentiated endothelial cells.

For the identification of a signal transduction pathway and the detection of cross talks with other signaling pathways suitable models or model systems have been generated by various scientists, for example cell culture models (e.g. Khwaja et al., EMBO, 1997, 16, 2783-93) and transgenic animal models (e.g. White et al., Oncogene, 2001, 20, 7064-7072). For the examintion of particular steps in the signal transduction cascade, interfering compounds can be used for signal modulation (e.g. Stephens et al., Biochemical J., 2000, 351, 95-105). The compounds according to the invention may also be useful as reagents for the examination of kinase dependent signal transduction pathways in animal and/or cell culture models or any of the clinical disorders listed throughout this application.

The measurement of kinase activity is a well known technique feasible for each person skilled in the art. Generic test systems for kinase activity detection with substrates, for example histone (e.g. Alessi et al., FEBS Lett. 1996, 399, 3, page 333-8) or myelin basic protein are well described in the literature (e.g. Campos-González, R. and Glenney, Jr., J.R. 1992 J. Biol. Chem. 267, Page 14535).

For the identification of kinase inhibitors various assay systems are available (see for example Walters et al., Nature Drug Discovery 2003, 2; page 259-266). For example, in scintillation proximity assays (e.g. Sorg et al., J. of. Biomolecular Screening, 2002, 7, 11-19) or flashplate assays the radioactive phosphorylation of a protein or peptide as substrate with □ATP can be measured. In the presence of an inhibitory compound no signal or a decreased radioactive signal is detectable. Furthermore homogeneous time-resolved fluorescence resonance energy transfer (HTR-FRET), and fluorescence polarization (FP) technologies are useful for assay methods (for example Sills et al., J. of Biomolecular Screening, 2002, 191-214).

Other non-radioactive ELISA based assay methods use specific phosphoantibodies (AB). The phospho-AB binds only the phosphorylated substrate. This binding is detectable with a secondary peroxidase conjugated antibody, measured for example by chemiluminescence (for exaple Ross et al., Biochem. J., 2002, 366, 977-981).

The present invention provides compounds generally described as methylene urea derivatives, including both aryl and/or heteroaryl derivatives which are preferably kinase inhibitors and more preferably inhibitors of the enzyme raf kinase. Since the enzyme is a downstream effector of p21^{ras}, the inhibitors are useful in pharmaceutical compositions for human or veterinary use where inhibition of the raf kinase pathway is indicated, e.g., in the treatment of tumors and/or cancerous cell growth mediated by raf kinase. In particular, the compounds are useful in the treatment of human or animal solid cancers, e.g. murine cancer, since the progression of these cancers is dependent upon the ras protein signal transduction cascade and therefore susceptible to treatment by interruption of the cascade, i.e., by inhibiting raf kinase. Accordingly, the compound of Formula I or a pharmaceutically acceptable salt thereof is administered for the treatment of diseases mediated by the raf kinase pathway especially cancers, including solid cancers, such as, for example, carcinomas (e.g., of the lungs, pancreas, thyroid, bladder or colon), myeloid disorders (e.g., myeloid leukemia) or adenomas (e.g., villous colon adenoma), pathological angiogenesis and metastatic cell migration. Furthermore the compounds are useful in the treatment of complement activation dependent chronic inflammation (Niculescu et al. (2002) Immunol. Res., 24:191-199) and HIV-1 (human immunodeficiency virus type1) induced immunodeficiency (Popik et al. (1998)J Virol, 72: 6406-6413) and infection disease, Influenza A virus (Pleschka, S. et al. (2001), Nat. Cell. Biol, 3(3):301-5) and Helicobacter pylori infection (Wessler, S. et al. (2002), FASEB J., 16(3): 417-9).

Therefore, subject of the present invention are methylene urea derivatives of formula I

A-D-B (I)

wherein
- D: is a bivalent methylene urea moiety which is directly bonded to A and B, preferably to one bonding partner via the carbon atom of the N-methylene moiety and to the other bonding partner via the N'-nitrogen atom, wherein the carbon atom of the N-methylene moiety is unsubstituted or substituted with one or more substituents, wherein said substituents are preferably selected from the group consisting of alkyl, alkylene, halogen, haloalkyl, C₃-C₇-cycloalkyl, C₃-C₇-cycloalkylene, heterocyclyl, aryl, aralkyl, heteroaryl, hydroxy, alkoxy, haloalkoxy, aralkoxy, aryloxy, mercapto, alkylsulfanyl, haloalkylsulfanyl, arylsuffanyl, heteroarylsulfanyl, alkylsulfenyl, haloalkylsulfenyl, arylsulfenyl, heteroarylsulfenyl, alkylsulfonyl, haloalkylsulfonyl, arylsulfonyl, heteroarylsulfonyl, carboxy, cyano, cyanoalkyl, aminosulfonyl, acyl, acyloxy, carbamoyl, aroyl, heteroaryl, heteroaroyloxy, unsubstituted amino groups and substituted amino groups, and wherein the carbonyl group of said methylene urea moiety can be derivatized, preferably to a C=S, C=NR⁵, C=C(R⁵)-NO₂, C=C(R⁵-CN or C= C(CN)₂ group
- A: is a unsubstituted or preferably substituted moiety of up to 40 carbon atoms of the formula: -L-(M-L')_{α}, where L is a 5, 6 or 7 membered cyclic structure, preferably selected from the group consisting of aryl, heteroaryl, arylene and heteroarylene, bound directly to D, L' comprises an optionally substituted cyclic moiety having at least 5 members, preferably selected from the group consisting of aryl, heteroaryl, aralkyl, cycloalkyl and heterocyclyl, M is a bond or a bridging group having at least to one atom, α is an integer of from 1-4; and each cyclic structure of L and L' contains 0-4 members of the group consisting of nitrogen, oxygen and sulfur, wherein L' is preferably substituted by at least one substituent selected from the group consisting of -SO_{β}Rₓ, -C(O)Rₓ and -C(NR_{y})R_{z},
- B: is a substituted or unsubstituted, up to tricyclic aryl or heteroaryl moiety of up to 30 carbon atoms, preferably of up to 20 carbon atoms, comprising at least one 5-, 6-, or 7-membered cyclic structure, preferably a 5- or 6-membered cyclic structure, bound directly to D containing 0-4 members of the group consisting of nitrogen, oxygen and sulfur, wherein said cyclic structure directly bound to D is preferably selected from the group consisting of aryl, heteroaryl and heterocyclyl, R_{y} is hydrogen or a carbon based moiety of up to 24 carbon atoms optionally containing heteroatoms selected from N, S and O and optionally halosubstituted, up to per halo,
- R_{z}: is hydrogen or a carbon based moiety of up to 30 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen;
- Rₓ: is R_{z} or NRₐR_{b}, where Rₐ and R_{b} are
a) independently hydrogen, a carbon based moiety of up to 30 carbon atoms optionally containing heteroatoms selected from N, S and O and optionally substituted by halogen, hydroxy and carbon based substituents of up to 24 carbon atoms, which optionally contain heteroatoms, selected from N, S and O, and are optionally substituted by halogen, or -OSi(R_{f})₃ where R_{f} is hydrogen or a carbon based moiety of up to 24 carbon atoms optionally containing heteroatoms selected from N, S and O and optionally substituted by halogen, hydroxy and carbon based substituents of up to 24 carbon atoms, which optionally contain heteroatoms selected from N, S and O, and are optionally substituted by halogen; or
b) Rₐ and R_{b} together from a 5-7 member heterocyclic structure of 1-3 heteroatoms selected from N, S and O, or a substituted 5-7 member heterocyclic structure of 1-3 heteroatoms selected from N, S and O substituted by halogen, hydroxy or carbon based substituents of up to 24 carbon atoms, which optionally contain heteroatoms selected from N, S and O and are optionally substituted by halogen; or
c) one of Rₐ or R_{b} is -C(O)-, a C₁-C₅ divalent alkylene group or a substituted C₁-C₅ divalent alkylene group bound to the moiety L to form a cyclic structure with at least 5 members, wherein the substituents of the substituted C₁-C₅ divalent alkylene group are selected from the group consisting of halogen, hydroxy, and carbon based substituents of up to 24 carbon atoms, which optionally contain heteroatoms selected from N, S and O and are optionally substituted by halogen; where B is substituted, L is substituted or L' is additionally substituted, the substituents are selected from the group consisting of halogen, up to per-halo and Wγ, where γ is 0-3;
   wherein each W is independently selected from the group consisting of -CN, -CO₂R, -C(O)NR⁵R⁵, -C(O)-R⁵, -NO₂, -OR⁵, -SR⁵, -NR⁵R⁵, -NR5C(O)OR⁵, -NR⁵C(O)R⁵, -Q-Ar, and carbon based moieties of up to 24 carbon atoms, optionally containing heteroatoms selected from N, S and O and optionally substituted by one or more substituents independently selected from the group consisting of -CN, -CO₂R, -C(O)NR⁵R⁵, -C(O)-R⁵, -NO₂, -OR⁵, -SR⁵, -NR⁵R⁵, -NR⁵C(O)OR⁵, -NR⁵C(O)R⁵ and halogen up to per-halo; with each R⁵ independently selected from H or a carbon based moiety of up to 24 carbon atoms, optionally containing heteroatoms selected from N, S
d) and O and optionally substituted by halogen;
   wherein Q is -O-, -S-, -N(R⁵)-, -(CH₂)_{β}, -C(O)-, -CH(OH)-, -(CH₂)_{β}-, -(CH₂)_{β}S-, -(CH₂)_{β}N(R⁵)-, -O(CH₂)_{β}-CHHal-, -CHal₂-, -S-(CH₂)- and -N(R⁵)(CH₂)_{β}- where β = 1-3, and Hal is halogen;
   and
   Ar is a 5- or 6-member aromatic structure containing 0-2 members selected from the group consisting of nitrogen, oxygen and sulfur, which is optionally substituted by halogen, up to per-halo, and optionally substituted by Z_{δ1} wherein δ1 is 0 to 3 and each Z is independently selected from the group consisting -CN, -CO₂R⁵, -C(O)NR⁵R⁵, -C(O)-R⁵, -NO₂, -OR⁵, -SR⁵, -NR⁵R⁵, -NR⁵C(O)OR⁵, -NR⁵C(O)R⁵, and a carbon based moiety of up to 24 carbon atoms, optionally containing heteroatoms selected from N, A and O and optionally substituted by one or more substituents selected from the group consisting of -CN, -CO₂R⁵, -C(O)NR⁵R⁵, -C(O)-R⁵, -NO₂, -OR⁵, -SR⁵, -NR⁵R⁵, -NR⁵C(O)OR⁵, -NR⁵C(O)R⁵, and with R⁵ as defined above,
and the pharmaceutically acceptable derivatives, solvates, salts and stereoisomers thereof, including mixtures thereof in all ratios, and more preferred the salts and/or solvates thereof, and especially preferred the physiologically acceptable salts and/or solvates thereof.

More preferred, in the compound of formula I,
- R_{y}: is hydrogen, C₁₋₁₀ alkyl, C₁₋₁₀ alkoxy, C₃₋₁₀ cycloalkyl having 0-3 heteroatoms, C₂₋₁₀ alkenyl, C₁₋₁₀ alkenoyl, C₆₋₁₂ arfy, C₃₋₁₂ hetaryl having 1-3 heteroatoms selected from N, S and O, C₇₋₂₄ aralkyl, C₇₋₂₄ alkaryl, substituted C₁₋₁₀ alkyl, substituted C₁₋₁₀ alkoxy, substituted C₃₋₁₀ cycloalkyl having 0-3 heteroatoms selected from N, S and O, substituted C₆-C₁₄ aryl, substituted C₃₋₁₂ hetaryl having 1-3 heteroatoms selected from N, S and O, substituted C₇₋₂₄ alkaryl or substituted C₇₋₂₄ aralkyl, where Ry is a substituted group, it is substituted by halogen up to per halo,
- R_{z}: is hydrogen, C₁₋₁₀ alkyl, C₁₋₁₀ alkoxy, C₃₋₁₀ cycloalkyl having 0-3 heteroatoms, C₂₋₁₀ alkenyl, C₁₋₁₀ alkenoyl, C₆₋₁₂ aryl, C₃-C₁₂ hetaryl having 1-3 heteroatoms selected form S, N and O, C₇₋₂₄ alkaryl, C₇₋₂₄ aralkyl, substituted C₃-C₁₀ cycloalkyl having 0-3 heteroatoms selected from S, N and O, substituted C₃₋₁₂ hetaryl having 1-3 heteroatoms selected from S, N and O, substituted C₇₋₂₄ alkaryl or substituted C₇-C₂₄ aralkyl, where R_{z} is a substituted group, it is substituted by halogen up to per halo, hydroxy, C₁₋₁₀ alkyl, C₃₋₁₂ cycloalkyl having 0-3 heteroatoms selected from N, S and O, substituted C₃-C₁₂ hetaryl having 1-3 heteroatoms selected from N, S and O, C₁₋₁₀ alkoxy, C₆₋₁₂ aryl, C₁₋₆ halo substituted alkyl up to per halo alkyl, C₆-C₁₂ halo substituted aryl up to per halo aryl, C₃-C₁₂ halo substituted cycloalkyl up to per halo cycloalkyl having 0-3 heteroatoms selected from N, S and O, halo substituted C₃-C₁₂ hetaryl up to per halo, hetaryl having 1-3 heteroatoms selected from O, N and S, halo substituted C₇-C₂₄ aralkyl up to per halo aralkyl, halo substituted C₇-C₂₄ alkaryl up to per halo alkaryl, and -C(O)R_{g},
- Rₐ and R_{b}: are,
a) independently hydrogen, a carbon based moiety selected from the group consisting of C₁-C₁₀ alkyl, C₁-C₁₀ alkoxy, C₃₋₁₀ cycloalkyl, C₂₋₁₀ alkenyl, C₁₋₁₀ alkenoyl, C₆₋₁₂ aryl, C₃₋₁₂ hetaryl having 1-3 heteroatoms selected from O, N and S, C₃₋₁₂ cycloalkyl having 0-3 heteroatoms selected from N, S and O, C₇₋₂₄ aralkyl, C₇-C₂₄ alkaryl, substituted C₁₋₁₀ alkyl, substituted C₁₋₁₀ alkoxy, substituted C₃₋₁₀ cycloalkyl, having 0-3 heteroatoms selected from N, S and O, substituted C₆₋₁₂ aryl, substituted C₃₋₁₂ hetaryl having 1-3 heteroatoms selected from N, S and O, substituted C₇₋₂₄ aralkyl, substituted C₇₋₂₄ alkaryl, where Rₐ and R_{b} are a substituted group, they are substituted by halogen up to per halo, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O C₁₋₁₀ alkoxy, C₆₋₁₂ aryl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g}; or
   -OSi(R_{f})₃ where R_{f} is hydrogen, C₁-C₁₀ alkyl, C₁-C₁₀ alkoxy, C₃₋₁₀ cycloalkyl, C₂₋₁₀ alkenyl, C₁₋₁₀ alkenoyl, C₆₋₁₂ aryl, C₃₋₁₂ hetaryl having 1-3 heteroatoms selected from O, N and S, C₃₋₁₂ cycloalkyl having 0-3 heteroatoms selected from N, S and O, C₇₋₂₄ aralkyl, C₇-C₂₄ alkaryl, substituted C₁₋₁₀ alkyl, substituted C₁₋₁₀ alkoxy, substituted C₃₋₁₀ cycloalkyl, having 0-3 heteroatoms selected from N, S and O, substituted C₆₋₁₂ aryl, substituted C₃₋₁₂ hetaryl having 1-3 heteroatoms selected from N, S and O, substituted C₇₋₂₄ aralkyl, substituted C₇₋₂₄ alkaryl, where Rₐ and R_{b} are a substituted group, they are substituted by halogen up to per halo, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O, C₁₋₁₀ alkoxy, C₆₋₁₂ aryl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g},
   or
b) Rₐ and R_{b} together form a 5-7 member heterocyclic structure of 1-3 heteroatoms selected from N, S and O, or a substituted 5-7 member heterocyclic structure of 1-3 heteroatoms selected from N, S and O with substituents selected from the group consisting of halogen up to per halo, hydroxy, C₁-C₁₀ alkyl, C₁-C₁₀ alkoxy, C₃₋₁₀ cycloalkyl, C₂₋₁₀ alkenyl, C₁₋₁₀ alkenoyl, C₆₋₁₂ aryl, C₃₋₁₂ hetaryl having 1-3 heteroatoms selected from O, N and S, C₃₋₁₂ cycloalkyl having 0-3 heteroatoms selected from N, S and O, C₇₋₂₄ aralkyl, C₇-C₂₄ alkaryl, substituted C₁₋₁₀ alkyl, substituted C₁₋₁₀ alkoxy, substituted C₃₋₁₀ cycloalkyl, having 0-3 heteroatoms selected from N, S and O, substituted C₆₋₁₂ aryl, substituted C₃₋₁₂ hetaryl having 1-3 heteroatoms selected from N, S and O, substituted C₇₋₂₄ aralkyl, substituted C₇₋₂₄ alkaryl, where Rₐ and R_{b} are a substituted group, they are substituted by halogen up
c) to per halo, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O, C₁₋₁₀ alkoxy, C₆₋₁₂ aryl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g},
   or
d) one of Rₐ or R_{b} is -C(O)-, a C₁-C₅ divalent alkylene group or a substituted C₁-C₅ divalent alkylene group bound to the moiety L to form a cyclic structure with at least 5 members, wherein the substituents of the substituted C₁-C₅ divalent alkylene group are selected from the group consisting of halogen, hydroxy, C₁₋₁₀ alkyl, C₃₋₁₂ cycloalkyl having 0-3 heteroatoms selected from, S and N, C₃₋₁₂ hetaryl having 1-3 heteroatoms selected from N, S and O, C₁₋₁₀ alkoxy, C₆₋₁₂ aryl, C₇-C₂₄ alkaryl, C₇-C₂₄ aralkyl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g},
   where R_{g} is C₁₋₁₀ alkyl; -CN, -CO₂R_{d}, -OR_{d}, -SR_{d}, -NO₂, -C(O)Rₑ, -NR_{d}Rₑ, -NR_{d}C(O)ORₑ and -NR_{d}(CO)Rₑ and R_{d} and Rₑ are independently selected from the group consisting of hydrogen, C₁₋₁₀ alkyl, C₁₋₁₀ alkoxy, C₃₋₁₀ cycloalkyl having 0-3 heteroatoms selected from O, N and S, C₆₋₁₂ aryl, C₃-C₁₂ hetaryl with 1-3 heteroatoms selected from O, N and S and C₇-C₂₄ aralkyl, C₇-C₂₄ alkaryl, up to per halo substituted C₁-C₁₀ alkyl, up to per halo substituted C₃-C₁₀ cycloalkyl having 0-3 heteroatoms selected from O, N and S, up to per halo substituted C₆-C₁₄ aryl, up to per halo substituted C₃-C₁₂ hetaryl having 1-3 heteroatoms selected from O, N and S, halo substituted C₇-C₂₄ alkaryl up to per halo alkaryl, and up to per halo substituted C₇-C₂₄ aralkyl,
- W: is independently selected from the group consisting -CN, -CO₂R⁵, -C(O)NR⁵R⁵, -C(O)-R⁵, -NO₂, -OR⁵, -SR⁹, -NR⁵R⁵, -NR⁵C(O)OR⁵, -NR⁵C(O)R⁵. C₁-C₁₀ alkyl, C₁-C₁₀ alkoxy, C₂-C₁₀ alkenyl, C₁-C₁₀ alkenoyl, C₃-C₁₀ cycloalkyl having 0-3 heteroatoms selected from O, S and N, C₆-C₁₄ aryl, C_{T}-C₂₄ alkaryl, C₇-C₂₄ aralkyl, C₃-C₁₂ heteroaryl having 1-3 heteroatoms selected form O, N and S, C₄-C₂₃ alkheteroaryl having 1-3 heteroatoms selected from O, N and S, substituted C₁-C₁₀ alkyl, substituted C₁-C₁₀ alkoxy, substituted C₂-C₁₀ alkenyl, substituted C₁-C₁₀ alkenoyl, substituted C₃-C₁₀ cycloalkyl having 0-3 heteroatoms selected from O, N and S, substituted C₆-C₁₂ aryl, substituted C₃-C₁₂ hetaryl having 1-3 heteroatoms selected from O, N and S, substituted C₇-C₂₄ aralkyl, substituted C₇-C₂₄ alkaryl, substituted C₄-C₂₃ alkheteroaryl having 1-3 heteroatoms selected from O, N and S, and -Q-Ar;
- R⁵: is independently selected from H, C₁-C₁₀ alkyl, C₁-C₁₀ alkoxy, C₂-C₁₀ alkenyl, C₁-C₁₀ alkenoyl, C₃-C₁₀ cycloalkyl having 0-3 heteroatoms selected from O, C and N, C₆-C₁₄ aryl, C₃-C₁₃ hetaryl having 1-3 heteroatoms selected from O, N and S, C₇-C₁₄ alkaryl, C₇-C₂₄ aralkyl, C₄-C₂₃ alkheteroaryl having 1-3 heteroatoms selected from O, N and S, up to per-halosubstituted C₁-C₁₀ alkyl, up to per-halosubstituted C₃-C₁₀ cycloalkyl having 0-3 heteroatoms selected from O, N and S, up to per-halosubstituted C₆-C₁₄ aryl, up to per-halosubstituted C₃-C₁₃ hetaryl having 1-3 heteroatoms selected from O, N and S, up to per-halosubstituted C₇-C₂₄ aralkyl, up to per-halosubstituted C₇-C₂₄ alkaryl, and up to per-halosubstituted C₄-C₂₃ alkheteroaryl; and each
- Z: is independently selected from the group consisting -CN, -CO₂R⁵, -C(O)NR⁵R⁵, -C(O)-R⁵, -NO₂, -OR⁵, -SR⁵, -NR⁵R⁵, -NR⁵C(O)OR⁵, -NR⁵C(O)R⁵, C₁-C₁₀ alkyl, C₁-C₁₀ alkoxy, C₂-C₁₀ alkenyl, C₁-C₁₀ alkenoyl, C₃-C₁₀ cycloalkyl having 0-3 heteroatoms selected from O, S and N, C₆-C₁₄ aryl, C₇-C₂₄ alkaryl, C₇-C₂₄ aralkyl, C₃-C₁₂ heteroaryl having 1-3 heteroatoms selected from O, N and S, C₄-C₂₃ alkheteroaryl having 1-3 heteroatoms selected from O, N and S, substituted C₁-C₁₀ alkyl, substituted C1-C10 alkoxy, substituted C₂-C₁₀ alkenyl, substituted C₁-C₁₀ alkenoyl, substituted C₃-C₁₀ cycloalkyl having 0-3 heteroatoms selected from O, N and S, substituted C₆-C₁₂ aryl, substituted C₃-C₁₂ hetaryl having 1-3 heteroatoms selected from O, N and S; wherein if Z is a substituted group, the one or more substituents are selected from the group consisting of -CN, -CO₂R⁵, -C(O)NR⁵R⁵, -C(O)-R⁵, -NO₂, -OR⁵, -SR⁵, -NR⁵R⁵, -NR⁵C(O)OR⁵, -NR⁵C(O)R⁵.

According to the invention, each M independently from one another represents a bond OR is a bridging group, selected from the group consisting of (CR⁵R⁵)ₕ, or (CHR⁵)ₕ-Q-(CHR⁵)ᵢ, wherein
- Q: is selected from a group consisting of O, S, N-R⁵, (CHal₂)ⱼ, (O-CHR⁵)ⱼ, (CHR⁵-O)ⱼ, CR⁵=CR⁵, (O-CHR⁵CHR⁵)ⱼ, (CHR⁵CHR⁵-O)ⱼ, C=O, C=S, C=NR⁵, CH(OR⁵), C(OR⁵)(OR⁵), C(=O)O, OC(=O), OC(=O)O, C(=O)N(R⁵), N(R⁵)C(=O), OC(=O)N(R⁵), N(R⁵)C(=O)O, CH=N-O, CH=N-NR⁵, OC(O)NR⁵, NR⁵C(O)O, S=O, SO₂, SO₂NR⁵ and NR⁵S₂O, wherein
- R⁵: is in each case independently selected from the meanings given above, preferably from hydrogen, halogen, alkyl, aryl, aralkyl,
- h, i: are independently from each other 0, 1, 2, 3, 4, 5 or 6, preferably 0, 1, 2, or 3, and
- j: is 1, 2, 3, 4, 5 or 6, preferably 0, 1, 2 or 3.

More preferred, each M independently from one another represents a bond or is a bridging group, selected from the group consisting of -O-, -S-, -N(R⁵)-, -(CH₂)_{β}-, -C(O)-, -CH(OH)-, -(CH_{2)β}O-, -(CH₂)_{β}S-, -(CH₂)_{β}N(R⁵)-, -O(CH₂)_{β}, -CHHal-, -CHal₂-, -S-(CH₂)_{β}- and -N(R⁵)(CH₂)_{β}, where β is 1 to 6 and especially preferred 1 to 3, Hal is halogen and R⁵ is as defined above. More preferred, the group B of Formula I is a substituted or unsubstituted six member aryl moiety or six member hetaryl moiety, said hetaryl moiety having 1 to 4 members selected from the group of hetaryl atoms consisting of nitrogen, oxygen and sulfur with the balance of the hetaryl moiety being carbon.

Even more preferred, the group B of Formula I is
a) an unsubstituted phenyl group, an unsubstituted pyridyl group, an unsubstituted pyrimidinyl, a phenyl group substituted by a substituent selected from the group consisting of halogen and Wγ wherein W and γ are as defined in claim 1, a pyrimidinyl group substituted by a substituent selected from the group constituting of halogen and Wγ, whereas W and γ are as defined above, or a substituted pyridyl group, substituted by a substituent selected from the group consisting of halogen and Wγ wherein W and γ are as defined above; or a substituted phenyl group, a substituted pyrimidinyl group, or substituted pyridyl group substituted 1 to 3 times by 1 or more substituents selected from the group consisting of -CN, halogen, C₁-C₁₀ alkyl, C₁-C₁₀ alkyl alkoxy, -OH, up to per halo substituted C₁-C₁₀ alkyl, up to per halo substituted C₁-C₁₀ alkoxy or phenyl substituted by halogen up to per halo; or
b) a substituted phenyl group, a substituted pyrimidinyl group, or substituted pyridyl group substituted 1 to 3 times b 1 or more substituents selected from the group consisting of -CN, halogen, alkyl, especially C₁-C₄ alkyl, alkoxy, especially C₁-C₄ alkoxy, -OH, up to per halo substituted alkyl, especially up to per halo substituted C₁-C₄ alkyl, up to per halo substituted alkoxy, especially up to per halo substituted C₁-C₄ alkoxy or phenyl substituted by halogen up to per halo.

In the formula I, the group L which is directly bound to D is preferably a substituted or unsubstituted 6 member aryl moiety or a substituted or unsubstituted 6 member hetaryl moiety, wherein said hetaryl moiety has 1 to 4 members selected from the group of heteroatoms consisting of nitrogen, oxygen and sulfur with the balance of said hetaryl moiety being carbon, wherein the one or more substituents are selected from the group consisting of halogen and Wγ wherein W and y are as defined above.

More preferred, the group L is a substituted phenyl, unsubstituted phenyl, substituted pyrimidinyl, unsubstituted pyrimidinyl, substituted pyridyl or unsubstituted pyridyl group.

In the formula I, the group L' preferably comprises a 5 to 6 membered aryl moiety or hetaryl moiety, wherein said heteraryl moiety comprises 1 to 4 members selected from the group of heteroatoms consisting of nitrogen, oxygen and sulfur.

More preferred, the group L' is phenyl, pyridinyl or pyrimidinyl.

According to the invention, a methylene moiety is a bivalent radical of formula -CRR-, where R and R are selected independently from one another from hydrogen or suitable substituents other than hydrogen. Suitable substituents are preferably selected from the group consisting of alkyl, alkylene, halogen, haloalkyl, C₃-C₇-cycloalkyl, C₃-C₇-cycloalkylene, heterocyclyl, aryl, aralkyl, heteroaryl, hydroxy, alkoxy, haloalkoxy, aralkoxy, aryloxy, mercapto, alkylsulfanyl, haloalkylsulfanyl, arylsulfanyl, heteroarylsulfanyl, alkylsulfenyl, haloalkylsulfenyl, arylsulfenyl, heteroarylsulfenyl, alkylsulfonyl, haloalkylsulfonyl, arylsulfonyl, heteroarylsulfonyl, carboxy, cyano, cyanoalkyl, aminosulfonyl, acyl, acyloxy, carbamoyl, aroyl, heteroaryl, heteroaroyloxy, unsubstituted amino groups and substituted amino groups. A methylene moiety wherein R and R both are hydrogen is also referred to as an unsubstituted methylene moiety. A methylene moiety wherein R and/or R are other than hydrogen is referred to as a substituted methylene moiety.

Thus, a methylene urea moiety according to the invention is a bivalent radical wherein one of the nitrogen atoms of the urea moiety is substituted by a methylene moiety. Preferably, A and B are bonded to the resulting methylene urea moiety via the nitrogen atom of the urea moiety that is not substituted by the methylene moiety, and to the carbon atom of the methylene moiety, respectively.
The hydrogen atoms of one or both nitrogen atoms of the methylene urea moiety can be substituted by suitable substituents, preferably selected from the group consisting of alkyl, alkylene, haloalkyl, C₃-C₇-cycloalkyl, C₃-C₇-cycloalkylene, heterocyclyl, aryl, aralkyl, heteroaryl, carboxy, cyanoalkyl, acyl and heteroaryl. Preferably, both nitrogen atoms of the methylene urea moiety are unsubstituted. In this respect, one or both of the nitrogen atoms of D can, independently from one another, optionally be deprotonated, protonated and/or quarternized. The resulting ions or salts are also subject of the present invention.

Accordingly, preferred compounds of formula I are of formula la

A-NH-CO-NH-B la

wherein A and B are as defined above/below, and wherein the carbonyl moiety in formula Ia can be derivatized as described above/below, and the salts or solvates thereof. Especially preferred are compounds of formula la, wherein the carbonyl moiety is not derivatized.

Preferably, A or B is substituted by one or more substituents as described above/below. More preferably, A and B each are substituted by one or more substituents as described above/below. Even more preferably, A is substituted by two or more substituents as described above/below.

Preferably, subject of the present invention are the optically active forms or stereo isomers of the compounds according to the invention, such as the enantiomers, the diastereomers and/or mixtures thereof in all ratios, such as, for example, stereochemically uniform compounds or racemates. Preferably, further subject of the present invention are the solvates and hydrates of the compounds according to the invention. Preferably, further subject of the present invention are the pharmaceutically acceptable derivatives or physiologically functional derivatives of the compounds according to the invention. More preferably, further subject of the present invention are the salts of the compounds according to the invention, especially the pharmaceutically and/or physiologically acceptable salts of compounds according to the invention.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As used herein, the term "alkyl" preferably refers to a straight or branched chain hydrocarbon having from one to twelve carbon atoms, optionally substituted with substituents selected from the group consisting of C₁-C₆ alkyl, C₁-C₆ alkoxy, C₁-C₆ alkylsulfanyl, C₁-C₆ alkylsulfenyl, C₁-C₆ alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or C₁-C₆ perfluoroalkyl, multiple degrees of substitution being allowed. Examples of "alkyl" as used herein include, but are not limited to, methyl, ethyl, n-propyl, isopropyl, n-butyl, isobutyl, t-butyl, n-pentyl, isopentyl, and the like.

As used herein, the term "C₁-C₆ alkyl" preferably refers to an alkyl group as defined abovecontaining at least 1, and at most 6, carbon atoms. Examples of branched or straight chained "C₁-C₆ alkyl" groups useful in the present invention include, but are not limited to, methyl, ethyl, n-propyl, isopropyl, isobutyl, n-butyl, t-butyl, n-pentyl and isopentyl.
As used herein, the term "alkylene" preferably refers to a straight or branched chain divalent hydrocarbon radical having from one to ten carbon atoms, optionally substituted with substituents selected from the group which includes lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl, optionally substituted by alkyl, nitro, cyano, halogen and lower perfluoroalkyl, multiple degrees of substitution being allowed. Examples of "alkylene" as used herein include, but are not limited to, methylene, ethylene, n-propylene, n-butylene and the like.

As used herein, the term "C₁-C₆ alkylene" preferably refers to an alkylene group, as defined above, which contains at least 1, and at most 6, carbon atoms respectively. Examples of "C₁-C₆ alkylene" groups useful in the present invention include, but are not limited to, methylene, ethylene and n-Propylene.

As used herein, the term "halogen" or "hal" preferably refers to fluorine (F), chlorine (Cl), bromine (Br) or iodine (I).

As used herein, the term "C₁-C₆ haloalkyl" preferably refers to an alkyl group as defined above containing at least 1, and at most 6, carbon atoms substituted with at least one halogen, halogen being as defined herein. Examples of branched or straight chained "C₁-C₆ haloalkyl" groups useful in the present invention include, but are not limited to, methyl, ethyl, propyl, isopropyl, isobutyl and n-butyl substituted independently with one or more halogens, e.g., fluoro, chloro, bromo and iodo.

As used herein, the term "C₃-C₇ cycloalkyl" preferably refers to a nonaromatic cyclic hydrocarbon ring having from three to seven carbon atoms and which optionally includes a C₁-C₆ alkyl linker through which it may be attached. The C₁-C₆ alkyl group is as defined above. Exemplary "C₃-C₇ cycloalkyl" groups include, but are not limited to, cyclopropyl, cyclobutyl, cyclopentyl, cyclohexyl and cycloheptyl. The term "cycloalkyl", as used herein preferably also includes saturated heterocyclic groups, which are preferably selected from the cycloalkyl-groups as defined above, wherein one or two carbon atoms are replaced by hetero atoms, selected from the group consisting of O, N and S.

As used herein, the term "C₃-C₇ cycloalkylene" preferably refers to a nonaromatic alicyclic divalent hydrocarbon radical having from three to seven carbon atoms, optionally substituted with substituents selected from the group which includes lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, lower perfluoroalkyl, multiple degrees of substitution being allowed. Examples of "cycloalkylene" as used herein include, but are not limited to, cyclopropyl-1,1-diyl, cyclopropyl-1,2-diyl, cyclobutyl-1,2-diyl, cyclopentyl-1,3-diyl, cyclohexyl-1,4-diyl, cycloheptyl-1,4-diyl, or cyclooctyl-1,5-diyl, and the like.

As used herein, the term "heterocyclic" or the term "heterocyclyl" preferably refers to a three to twelve-membered heterocyclic ring having one or more degrees of unsaturation containing one or more heteroatomic substitutions selected from S, SO, SO₂, O or N, optionally substituted with substituents selected from the group consisting of C₁-C₆ alkyl, C₁-C₆ haloalkyl, C₁-C₆ alkoxy, C₁-C₆ alkylsulfanyl, C₁-C₆ haloalkylsulfanyl, C₁-C₆ alkylsulfenyl, C₁-C₆ alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, or C₁-C₆ perfluoroalkyl, multiple degrees of substitution being allowed. Such a ring may be optionally fused to one or more other "heterocyclic" ring(s) or cycloalkyl ring(s). Examples of "heterocyclic" moieties include, but are not limited to, tetrahydrofuran, pyran, 1,4-dioxane, 1,3-dioxane, pyrrolidine, piperidine, morpholine, tetrahydrothiopyran, tetrahydrothiophene, and the like.

As used herein, the term "heterocyclylene" preferably refers to a three to twelve-membered heterocyclic ring diradical having one or more degrees of unsaturation containing one or more heteroatoms selected from S, SO, SO₂, O or N, optionally substituted with substituents selected from the group which includes lower alkyl, lower alkoxy, lower alkylsulfanyl, lower alkylsulfenyl, lower alkylsulfonyl, oxo, hydroxy, mercapto, amino optionally substituted by alkyl, carboxy, carbamoyl optionally substituted by alkyl, aminosulfonyl optionally substituted by alkyl, nitro, cyano, halogen, lower perfluoroalkyl, multiple degrees of substitution being allowed. Such a ring may be optionally fused to one or more benzene rings or to one or more of another "heterocyclic" rings or cycloalkyl rings. Examples of "heterocyclylene" include, but are not limited to, tetrahydrofuran-2,5-diyl, morpholine-2,3-diyl, pyran-2,4-diyl, 1,4-dioxane-2,3-diyl, 1,3-dioxane-2,4-diyl, piperidine-2,4-diyl, piperidine-1,4-diyl, pyrrolidine-1,3-diyl, morpholine-2,4-diyl, and the like.

As used herein, the term "aryl" preferably refers to an optionally substituted benzene ring or to an optionally substituted benzene ring system fused to one or more optionally substituted benzene rings to form, for example, anthracene, phenanthrene, or napthalene ring systems. Exemplary optional substituents include C₁-C₆ alkyl, C₁-C₆ alkoxy, C₁-C₆ alkylsulfanyl, C₁-C₆ alkylsulfenyl, C₁-C₆ alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, C₁-C₆ perfluoroalkyl, heteroaryl, or aryl, multiple degrees of substitution being allowed. Examples of "aryl" groups include, but are not limited to Phenyl, 2-naphthyl, 1-naphthyl, biphenyl, as well as substituted derivatives thereof. As used herein, the term "arylene" preferably refers to a benzene ring diradical or to a benzene ring system diradical fused to one or more optionally substituted benzene rings, optionally substituted with substituents selected from the group which includes lower alkyl, lower alkoxy, lower alkylsulfanyl, lower alkylsulfenyl, lower alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, lower perfluoroalkyl, heteroaryl and aryl, multiple degrees of substitution being allowed. Examples of "arylene" include, but are not limited to benzene-1,4-diyl, naphthalene-1,8-diyl, anthracene-1,4-diyl, and the like.

As used herein, the term "aralkyl" preferably refers to an aryl or heteroaryl group, as defined herein, attached through a C₁-C₆ alkyl linker, wherein C₁-C₆ alkyl is as defined herein. Examples of "aralkyl" include, but are not limited to, benzyl, phenylpropyl, 2-pyridylmethyl, 3-isoxazolylmethyl, 5-methyl-3-isoxazolylmethyl and 2-imidazolylethyl.

As used herein, the term "heteroaryl" preferably refers to a monocyclic five to seven-membered aromatic ring, or to a fused bicyclic aromatic ring system comprising two of such monocyclic five to seven-membered aromatic rings. These hetroaryl rings contain one or more nitrogen, sulfur and/or oxygen heteroatoms, where N-Oxides and sulfur Oxides and dioxides are permissible heteroatom substitutions and may be optionally substituted with up to three members selected from a group consisting of C₁-C₆ alkyl, C₁-C₆ haloalkyl, C₁-C₆ alkoxy, C₁-C₆ alkylsulfanyl, C₁-C₆ haloalkylsulfanyl, C₁-C₆ alkylsulfenyl, C₁-C₆ alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, C₁-C₆ perfluoroalkyl, heteroaryl or aryl, multiple degrees of substitution being allowed. Examples of "heteroaryl" groups used herein include furanyl, thiophenyl, pyrrolyl, imidazolyl, pyrazolyl, triazolyl, tetrazolyl, thiazolyl, oxazolyl, isoxazolyl, oxadiazolyl, oxo-pyridyl, thiadiazolyl, isothiazolyl, pyridyl, pyridazyl, pyrazinyl, pyrimidyl, quinolinyl, isoquinolinyl, benzofuranyl, benzothiophenyl, indolyl, indazolyl, and substituted versions thereof.

As used herein, the term "heteroarylene" preferably refers to a five - to seven -membered aromatic ring diradical, or to a polycyclic heterocyclic aromatic ring diradical, containing one or more nitrogen, oxygen, or sulfur heteroatoms, where N-Oxides and sulfur monoxides and sulfur dioxides are permissible heteroaromatic substitutions, optionally substituted with substituents selected from the group consisting of lower alkyl, lower alkoxy, lower alkylsulfanyl, lower alkylsulfenyl, lower alkylsulfonyl, oxo, hydroxy, mercapto, amino optionally substituted by alkyl, carboxy, tetrazolyl, carbamoyl optionally substituted by alkyl, aminosulfonyl optionally substituted by alkyl, acyl, aroyl, heteroaroyl, acyloxy, aroyloxy, heteroaroyloxy, alkoxycarbonyl, nitro, cyano, halogen, lower perfluoroalkyl, heteroaryl, or aryl, multiple degrees of substitution being allowed. For polycyclic aromatic ring system diradicals, one or more of the rings may contain one or more heteroatoms. Examples of "heteroarylene" used herein are furan-2,5-diyl, thiophene-2,4-diyl, 1,3,4-oxadiazole-2,5-diyl, 1,3,4-thiadiazole-2,5-diyl, 1,3-thiazole-2,5-diyl, pyridine-2,4-diyl, pyridine-2,3-diyl, pyridine-2,5-diyl, pyrimidine-2,4-diyl, quinoline-2,3-diyl, and the like.

As used herein, the term "alkoxy" preferably refers to the group RₐO-, where Rₐ is alkyl as defined above and the term "C₁-C₆ alkoxy" preferably refers to an alkoxy group as defined herein wherein the alkyl moiety contains at least 1 and at most 6 carbon atoms. Exemplary C₁-C₆ alkoxy groups useful in the present invention include, but are not limited to methoxy, ethoxy, n-propoxy, isopropoxy, n-butoxy and t-butoxy.

As used herein, the term "haloalkoxy" preferably refers to the group RₐO-, where Rₐ is haloalkyl as defined above and the term "C₁-C₆ haloalkoxy" preferably refers to an haloalkoxy group as defined herein wherein the haloalkyl moiety contains at least 1 and at most 6 carbon atoms. Exemplary C₁-C₆ haloalkoxy groups useful in the present invention include, but are not limited to, methoxy, ethoxy, n-propoxy, isopropoxy, n-butoxy and t-butoxy substituted with one or more halo groups, for instance trifluoromethoxy.

As used herein the term "aralkoxy" preferably refers to the group R_{C}R_{B}O-, where R_{B} is alkyl and R_{C} is aryl as defined above.

As used herein the term "aryloxy" preferably refers to the group R_{C}O-, where R_{C} is aryl as defined above.

As used herein, the term "alkylsulfanyl" preferably refers to the group R_{A}S-, where R_{A} is alkyl as defined above and the term "C₁-C₆ alkylsulfanyl" preferably refers to an alkylsulfanyl group as defined herein wherein the alkyl moiety contains at least 1 and at most 6 carbon atoms.

As used herein, the term "haloalkylsulfanyl" preferably refers to the group R_{D}S-, where R_{D} is haloalkyl as defined above and the term "C₁-C6 haloalkylsulfanyl" preferably refers to a haloalkylsulfanyl group as defined herein wherein the alkyl moiety contains at least 1 and at most 6 carbon atoms.

As used herein, the term "alkylsulfenyl" preferably refers to the group R_{A}S(O)-, where R_{A} is alkyl as defined above and the term "C₁-C₆ alkylsulfenyl" preferably refers to an alkylsulfenyl group as defined herein wherein the alkyl moiety contains at least 1 and at most 6 carbon atoms.

As used herein, the term "alkylsulfonyl" preferably refers to the group R_{A}SO₂-, where R_{A} is alkyl as defined above and the term "C₁-C₆ alkylsulfonyl" preferably refers to an alkylsulfonyl group as defined herein wherein the alkyl moiety contains at least 1 and at most 6 carbon atoms.

As used herein, the term "oxo" preferably refers to the group =O.

As used herein, the term "mercapto" preferably refers to the group -SH.

As used herein, the term "carboxy" preferably refers to the group -COOH.

As used herein, the term "cyano" preferably refers to the group -CN.

As used herein, the term "cyanoalkyl" preferably refers to the group - R_{B}CN, wherein R_{B} is alkylen as defined above. Exemplary "cyanoalkyl" groups useful in the present invention include, but are not limited to, cyanomethyl, cyanoethyl and cyanoisopropyl.

As used herein, the term "aminosulfonyl" preferably refers to the group - SO₂NH₂.

As used herein, the term "carbamoyl" preferably refers to the group - C(O)NH₂.

As used herein, the term "sulfanyl" shall refer to the group -S-.

As used herein, the term "sulfenyl" shall refer to the group -S(O)-.

As used herein, the term "sulfonyl" shall refer to the group -S(O)₂- or -SO₂-.

As used herein, the term "acyl" preferably refers to the group R_{F}C(O)-, where R_{F} is alkyl, cycloalkyl or heterocyclyl as defined herein.

As used herein, the term "aroyl" preferably refers to the group R_{C}C(O)-, where R_{C} is aryl as defined herein.

As used herein, the term "heteroaroyl" preferably refers to the group R_{E}C(O)-, where R_{E} is heteroaryl as defined herein.

As used herein, the term "alkoxycarbonyl" preferably refers to the group R_{A}OC(O)-, where R_{A} is alkyl as defined herein.

As used herein, the term "acyloxy" preferably refers to the group R_{F}C(O)O-, where R_{F} is alkyl, cycloalkyl, or heterocyclyl as defined herein.

As used herein, the term "aroyloxy" preferably refers to the group R_{C}C(O)O-, where R_{C} is aryl as defined herein.

As used herein, the term "heteroaroyloxy" preferably refers to the group R_{E}C(O)O-, where R_{E} is heteroaryl as defined herein.

As used herein, the term "carbonyl" or "carbonyl moiety" preferably refers to the group C=O.

As used herein, the term "thiocarbonyl" or "thiocarbonyl moiety" preferably refers to the group C=S.

As used herein, the term "amino", "amino group" or "imino moiety" preferably refers to the group NR_{G}R_{G'}, wherein R_{G} and R_{G'}, are preferably selected, independently from one another, from the group consisting of hydrogen, halogen, alkyl, haloalkyl, alkenyl, cycloalkyl, alkylenecycloalkyl, cyanoalkyl, aryl, aralkyl, heteroaryl, acyl and aroyl. If both R_{G} and R_{G'} are hydrogen, NR_{G}R_{G'} is also referred to as "unsubstituted amino moiety" or "unsubstituted amino group". If R_{G} and/or R_{G'} are other than hydrogen, NR_{G}R_{G'} is also referred to as "substituted amino moiety" or "substituted amino group":

As used herein, the term "imino" or "imino moiety" preferably refers to the group C=NR_{G}, wherein R_{G} is preferably selected from the group consisting of hydrogen, halogen, alkyl, haloalkyl, alkenyl, cycloalkyl, alkylenecycloalkyl, cyanoalkyl, aryl, aralkyl, heteroaryl, acyl and aroyl. If R_{G} is hydrogen, C=NR_{G} is also referred to as "unsubstituted imino moiety". If R_{G} is a residue other than hydrogen, C=NR_{G} is also referred to as "substituted imino moiety".

As used herein, the term "ethene-1,1-diyl moiety" preferably refers to the group C=CR_{K}R_{L}, wherein R_{K} and R_{L} are preferably selected, independently from one another, from the group consisting of hydrogen, halogen, alkyl, haloalkyl, alkenyl, cycloalkyl, nitro, alkylenecycloalkyl, cyanoalkyl, aryl, aralkyl, heteroaryl, acyl and aroyl. If both hydrogen R_{K} and R_{L} are hydrogen, C=CR_{K}R_{L} is also referred to as "unsubstituted ethene-1,1-diyl moiety". If one of R_{K} and R_{L} or both are a residue other than hydrogen, C=CR_{K}R_{L} is also referred to as "substituted ethene-1,1-diyl moiety".

As used herein, the terms "group", "residue" and "radical" or "groups", "residues" and "radicals" are usually used as synonyms, respectively, as it is common practice in the art.

As used herein, the term "optionally" means that the subsequently described event(s) may or may not occur, and includes both event(s), which occur, and events that do not occur.

As used herein, the term "pharmaceutically acceptable derivative" preferably refers to any physiologically functional derivative of a compound of the present invention, for example, an ester or an amide, which upon administration to a mammal is capable of providing (directly or indirectly) a compound of the present invention or an active metabolite thereof. Such derivatives are clear to those skilled in the art, without undue experimentation, and with reference to the teaching of Burger's Medicinal Chemistry And Drug Discovery, 5th Edition, Vol 1: Principles and Practice, which is incorporated herein by reference to the extent that it teaches physiologically functional derivatives. Such derivatives preferably include so-called prodrug-compounds, for example compounds according to the invention that are derivatized with alkyl groups, acyl groups, sugars or peptides, such as oligopeptides, and that are easily degraded or metabolized to the active compounds according to the invention. Such derivatives preferably include biodegradable polymer derivatives of the compounds according to the invention. Suitable polymers and methods for producing biodegradable polymeric derivatives are known in the art, for example from Int. J. Pharm. 115, 61-67 (1995).

As used herein, the term "solvate" preferably refers to a complex of variable stoichiometry formed by a solute (in this invention, a compound of formula I or formula II or a salt or physiologically functional derivative thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water. Examples for suitable solvates are the mono- or dihydrates or alcoholates of the compounds according to the invention.

As used herein, the term "substituted" preferably refers to substitution with the named substituent or substituents, multiple degrees of substitution being allowed unless otherwise stated.

Certain of the compounds described herein may contain one or more chiral atoms, or may otherwise be capable of existing as two or more stereoisomers, which are usually enantiomers and/or diastereomers. Accordingly, the compounds of this invention include mixtures of stereoisomers, especially mixtures of enantiomers, as well as purified stereoisomers, especially purified enantiomers, or stereoisomerically enriched mixtures, especially enantiomerically enriched mixtures. Also included within the scope of the invention are the individual isomers of the compounds represented by formulae I and II above as well as any wholly or partially equilibrated mixtures thereof. The present invention also covers the individual isomers of the compounds represented by the formulas above as mixtures with isomers thereof in which one or more chiral Centers are inverted. Also, it is understood that all tautomers and mixtures of tautomers of the compounds of formulae (I) or (II) are included within the scope of the compounds of formulae (I) and (II) and preferably the formulae and subformulae corresponding thereto.

Racemates obtained can be resolved into the isomers mechanically or chemically by methods known per se. Diastereomers are preferably formed from the racemic mixture by reaction with an optically active resolving agent. Examples of suitable resolving agents are optically active acids, such as the D and L forms of tartaric acid, diacetyltartaric acid, dibenzoyltartaric acid, mandelic acid, malic acid, lactic acid or the various optically active camphorsulfonic acids, such as β-camphorsulfonic acid. Also advantageous is enantiomer resolution with the aid of a column filled with an optically active resolving agent (for example dinitrobenzoylphenyl-glycine); an example of a suitable eluent is a hexane/isopropanol/ acetonitrile mixture.

The diastereomer resolution can also be carried out by standard purification processes, such as, for example, chromatography or fractional crystallization.

It is of course also possible to obtain optically active compounds of the formula I or II by the methods described above by using starting materials which are already optically active.

Unless indicated otherwise, it is to be understood that reference to compounds of formula I preferably includes the reference to the compounds of formula II. Unless indicated otherwise, it is to be understood that reference to the compounds of formula II preferably includes the reference to the sub formulae corresponding thereto, for example the sub formulae II.1 to II.20 and preferably formulae IIa to IIx. It is also understood that the following embodiments, including uses and compositions, although recited with respect to formula I are preferably also applicable to formulae II, sub formulae II1 to II.20 and preferably formulae IIa to IIx.

Especially preferred compounds according to the invention are compounds of formula II 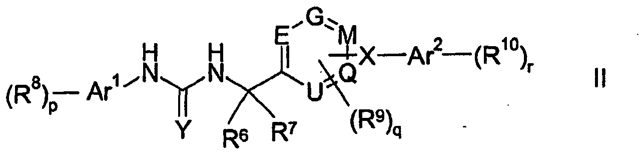 wherein
- Ar¹, Ar²: are selected independently from one another from aromatic hydrocarbons containing 6 to 14 carbon atoms and ethylenical unsaturated or aromatic heterocyclic residues containing 3 to 10 carbon atoms and one or two heteroatoms, independently selected from N, O and S,
- R⁶, R⁷: are independently selected from the meanings given for R⁸, R⁹ and R¹⁰, or R⁶ and R⁷ together form a carbocyclic residue comprising 3 to 7 carbon atoms or a heterocyclic residue comprising 1, 2 or 3 hetero atoms, selected from the group consisting of O, N and S, and 2 to 6 carbon atoms, said carbocyclic or heterocyclic residue being unsubstituted or comprising 1, 2 or 3 substituents, selected from the meanings given for R⁸, R⁹ and R¹⁰,
- E, G, M, Q and U: are selected, independently from one another, from carbon atoms and nitrogen atoms, with the proviso that one or more of E, G, M, Q and U are carbon atoms and that X is bonded to a carbon atom,
- R⁸, R⁹ and R¹⁰: are independently selected from a group consisting of H, A, cycloalkyl comprising 3 to 7 carbon atoms, Hal, CH₂Hal, CH(Hal)₂, C(Hal)₃, NO₂, (CH₂)ₙCN, (CH₂)nNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOOR¹³, (CH₂)ₙCOR¹³, (CH2)ₙCONR¹¹R¹², (CH₂)ₙNR¹¹COR¹³, (CH₂)ₙNR¹¹CONR¹¹R¹², (CH₂)ₙNR¹¹SO₂A, (CH₂)ₙSO₂NR¹¹R¹², (CH₂)ₙS(O)ᵤR¹³, (CH₂)ₙOC(O)R¹³, (CH₂)ₙCOR¹³, (CH₂)ₙSR¹¹, CH=N-OA, CH₂CH=N-OA, (CH₂)nNHOA, (CH₂)ₙCH=N-R¹¹, (CH2)ₙOC(O)NR¹¹R¹², (CH₂)ₙNR¹¹COOR¹³, (CH₂)ₙN(R¹¹)CH₂CH₂OR¹³, (CH₂)nN(R¹¹)CH₂CH₂OCF₃, (CH₂)nN(R¹¹)C(R¹³)HCOOR¹², (CH₂)ₙN(R¹¹)C(R¹³)HCOR¹¹, (CH₂)ₙN(R¹¹)CH₂CH₂N(R¹²)CH₂COOR¹¹, (CH₂)ₙN(R¹¹)CH₂CH₂NR¹¹R¹², CH=CHCOOR¹³, CH=CHCH₂NR¹¹R¹², CH=CHCH₂NR¹¹R¹², CH=CHCH₂OR¹³, (CH₂)ₙN(COOR¹³)COOR¹⁴, (CH₂)ₙN(CONH₂)COOR¹³, (CH₂)ₙN(CONH₂)CONH₂, (CH₂)ₙN(CH2COOR¹³)COOR¹⁴, (CH₂)ₙN(CH₂CONH₂)COOR¹³, (CH₂)ₙN(CH²CONH²)CONH², (CH₂)ₙCHR¹³COR¹⁴, (CH₂)ₙCHR¹³COOR¹⁴, (CH₂)ₙCHR¹³CH₂OR¹⁴, (CH₂)ₙOCN and (CH₂)ₙNCO, wherein
- R¹¹, R¹²: are independently selected from a group consisting of H, A, (CH₂)ₘAr³ and (CH₂)ₘHet, or in NR¹¹R¹²,
- R¹¹ and R¹²: form, together with the N-atom they are bound to, a 5-, 6- or 7- membered heterocyclus which optionally contains 1 or 2 additional hetero atoms, selected from N, O and S,
- R¹³, R¹⁴: are independently selected from a group consisting of H, Hal, A, (CH₂)ₘAr⁴ and (CH₂)ₘHet,
- A: is selected from the group consisting of alkyl, alkenyl, cycloalkyl, alkylenecycloalkyl, alkoxy, alkoxyalkyl and saturated heterocyclyl, preferably from the group consisting of alkyl, alkenyl, cycloalkyl, alkylenecycloalkyl, alkoxy and alkoxyalkyl,
- Ar³, Ar⁴: are independently from one another aromatic hydrocarbon residues comprising 5 to 12 and preferably 5 to 10 carbon atoms which are optionally substituted by one or more substituents, selected from a group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOC^{R1}5,
- Het: is a saturated, unsaturated or aromatic heterocyclic residue which is optionally substituted by one ore more substituents, selected from a group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOCR¹⁵,
- Rⁱ⁵, R¹⁶: are independently selected from a group consisting of H, A, and (CH₂)ₘAr⁶, wherein
- Ar⁶: is a 5- or 6-membered aromatic hydrocarbon which is optionally substituted by one or more substituents selected from a group consisting of methyl, ethyl, propyl, 2-propyl, tert.-butyl, Hal, CN, OH, NH₂ and CF₃,
- k, n and m: are independently of one another 0, 1, 2, 3, 4, or 5,
- X: represents a bond or is (CR¹¹R¹²)ₕ, or (CHR¹¹)ₕ-Q-(CHR¹²)ᵢ, wherein
- Q: is selected from a group consisting of O, S, N-R¹⁵, (CHal₂)ⱼ, (O-CHR¹⁸)ⱼ, (CHR¹⁸-O)ⱼ, CR¹⁸=CR¹⁹, (O-CHR¹⁸CHR¹⁹)ⱼ, (CHR¹⁸CHR¹⁹-O)ⱼ, C=O, C=S, C=NR¹⁵, CH(OR¹⁵), C(OR¹⁵)(OR²⁰), C(=O)O, OC(=O), OC(=O)O, C(=O)N(R¹⁵), N(R15)C(=O) OC(=O)N(R¹⁵), N(R¹⁵)C(=O)O, CH=N-O, CH=N-NR¹⁵, OC(O)NR15, NR¹⁵C(O)O, S=O, SO₂, SO₂NR¹⁵ and NR¹⁵SO₂, wherein
- h, i: are independently from each other 0, 1, 2, 3, 4, 5, or 6, and
- j: is 1, 2, 3, 4, 5, or 6,
- Y: is selected from O, S, NR²¹, C(R²²)-NO₂, C(R²²)-CN and C(CN)₂, wherein
- R²¹: is independently selected from the meanings given for R¹³, R¹⁴ and
- R²²: is independently selected from the meanings given for R¹¹, R¹²,
- p, r: are independently from one another 0, 1, 2, 3, 4 or 5,
- q: is 0, 1, 2, 3 or 4, preferably 0, 1 or 2,
- u: is 0, 1, 2 or 3, preferably 0, 1 or 2,
and
- Hal: is independently selected from a group consisting of F, Cl, Br and I;
and the pharmaceutically acceptable derivatives, solvates, salts and stereoisomers thereof, including mixtures thereof in all ratios, and more preferred the salts and/or solvates thereof, and especially preferred the physiologically acceptable salts and/or solvates thereof.

Even more preferred are compounds of formula II
wherein
- Ar¹, Ar²: are selected independently from one another from aromatic hydrocarbons containing 6 to 10 and especially 6 carbon atoms and ethylenical unsaturated or aromatic heterocyclic residues containing 3 to 8 and especially 4 to 6 carbon atoms and one or two heteroatoms, independently selected from N, O and S and especially selected from N and O,
- R⁸, R⁹ and R¹⁰: are independently selected from a group consisting of H, A, cycloalkyl comprising 3 to 7 carbon atoms, Hal, CH₂Hal, CH(Hal)₂, C(Hal)₃, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)nNR¹¹(CH₂)ₖOR¹², (CH₂)ₙCOR¹³, (CH₂)ₙCOOR¹³, (CH₂)ₙCONR¹¹R¹², (CH2)nNR¹¹COR¹³, (CH₂)ₙNR¹¹CONR¹¹R¹², (CH₂)ₙNR¹¹SO₂A, (CH₂)ₙSO₂NR¹¹R¹², (CH₂)ₙS(O)ᵤR, (CH₂)ₙOC(O)R¹³, (CH₂)ₙCOR¹³, (CH2)ₙSR¹¹, (CH₂)ₙNHOA, (CH₂)ₙNR¹¹COOR¹³, (CH₂)ₙN(R₁₁)CH₂CH₂OR¹³, (CH₂)ₙN(R¹¹)CH₂CH₂OCF₃, (CH₂)ₙN(R¹¹)C(R¹³)HCOOR⁸, (CH₂)ₙN(R¹¹), C(R¹³)HCOR⁸, (CH₂)ₙN(COOR¹³)COOR¹⁴, (CH₂)ₙN(CONH₂)COOR¹³, (CH₂)ₙN(CONH₂)CONH₂, (CH₂)ₙN(CH₂COOR¹³)COOR¹⁴, (CH₂)nN(CH₂CONH₂)COOR¹³ (CH₂)ₙN(CH₂CONH₂)CONH₂, (CH₂)ₙCHR¹³COR¹⁴, (CH₂)ₙCHR¹³COOR¹⁴ and (CH₂)ₙCHR¹³CH₂OR¹⁴
- R⁶ R⁷: are independently selected from a the meanings given for R⁸, R⁹ and R¹⁰, more preferred independently selected from the group consiting of H, A, Hal, CH₂Hal, CH(Hal)₂, C(Hal)₃, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙNR¹¹COR¹³, (CH₂)ₙNR¹¹CONR¹¹R¹² (CH₂)ₙNR¹¹SO₂A, (CH₂)ₙSO₂NR¹¹R¹², (CH₂)ₙS(O)ᵤR¹³, (CH₂)ₙCOR¹³, (CH₂)ₙSR¹¹, (CH₂)ₙNHOA and (CH₂)ₙNR¹¹COOR¹³,
or
R⁶ and R⁷ together form a carbocyclic residue comprising 3 to 7 carbon atoms or a heterocyclic residue comprising 1, 2 or 3 hetero atoms, selected from the group consisting of O, N and S, and 2 to 6 carbon atoms, said carbocyclic or heterocyclic residue being unsubstituted or comprising 1, 2 or 3 substituents, selected from the meanings given for R⁸, R⁹ and R¹⁰, more preferred selected from the group consisting of H, A, Hal, CH₂Hal, CH(Hal)₂, C(Hal)₃, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙNR¹¹COR¹³, (CH₂)ₙNR¹¹CONR¹¹R¹², (CH₂)ₙNR¹¹SO₂A, (CH₂)ₙSO₂NR¹¹R¹²_{,} (CH₂)ₙS(O)ᵤR¹³, (CH₂)ₙCOR¹³, (CH₂)ₙSR¹¹, (CH₂)ₙNHOA and (CH₂)ₙNR¹¹COOR¹³,
- X: represents a bond or is (CR¹¹R¹²)_{h,} or (CHR¹¹)ₕ-Q-(CHR¹²)_{¡}, wherein
- Q: is selected from a group consisting of O, S, N-R¹⁵, (CHal₂)ⱼ. (O-CHR¹⁸)ⱼ, (CHR18-O)ⱼ, CR¹⁸=CR¹⁹, (O-CHR¹⁸CHR¹⁹)ⱼ, (CHR¹⁸CHR¹⁹-O)ⱼ, C=O, C=NR¹⁵, CH(OR¹⁵), C(OR¹⁵)(OR²⁰), C(=O)N(R¹⁵), N(R¹⁵)C(=O), CH=N-NR¹⁵, S=O, SO₂, SO₂NR¹⁵ and NR¹⁵SO₂, wherein
- h, i and k: are independently from each other 0, 1, 2, 3, 4, 5 or 6, preferably 0, 1, 2 or 3 and
- j: is 1, 2, 3, 4, 5 or 6, preferably 1, 2, 3 or 4,
- p: is 1, 2, 3 or 4, preferably 1, 2 or 3, and
- r: is 0, 1, 2, or 3, preferably 0, 1 or 2;
and the pharmaceutically acceptable derivatives, solvates, salts and stereoisomers thereof, including mixtures thereof in all ratios, and more preferred the salts and/or solvates thereof, and especially preferred the physiologically acceptable salts and/or solvates thereof.

Subject of the present invention are especially compounds of formula I and II, in which one or more substituents or groups, preferably the major part of the substituents or groups has a meaning which is indicated as preferred, more preferred, even more preferred or especially preferred.

In compounds of formula II, E, G, M, Q and U constitute, together with the carbon atom that E and U are bound to, a bivalent 6-membered aromatic or nitrogen containing heteroaromatic ring. Preferably, one or more of E, G, M, Q and U, more preferably two or more of E, G, M, Q and U and especially three or more of E, G, M, Q and U are carbon atoms. Especially preferred, none or one of E, G, M, Q and U is a nitrogen atom. Especially preferred, E, G, M, Q and U constitute, together with the carbon atom that E and U are bound to, a 6-membered aromatic or nitrogen containing heteroaromatic ring, selected from the group consisting of phenylen, pyridinylen and pyrimydylen, wherein X is preferably bonded to a carbon atom. The substituents R⁹ are preferably, bound to a carbon atom.

Especially preferred as compounds of formula II are compounds of formula II' 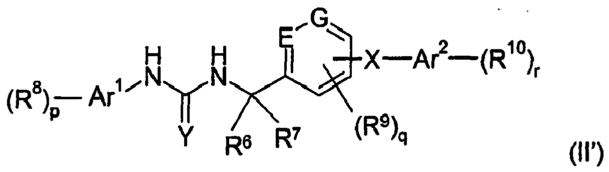 wherein E and G are as defined above, preferably E and G are both nitrogen atoms; more preferably one of E and G is a nitrogen atom or both E and G are carbon atoms. If E and/or G are carbon atoms, they can be unsubstituted or substituted by R⁹, i. e E and/or G are either CH or CR⁹.

In compounds of formula II, the term alkyl preferably refers to an unbranched or branched alkyl residue, preferably an unbranched alkyl residue comprising 1, 2, 3, 4, 5, 6, 7, 8, 9 or 10, preferably 1, 2, 3, 4, 5 or 6, more preferred 1, 2, 3 or 4 and especially 1 or 2 carbon atoms, or a branched alkyl residue comprising 3, 4, 5, 6, 7, 8, 9 or 10, preferably 3, 4, 5 or 6 more preferred 3 or 4 carbon atoms. The alkyl residues can be optionally substituted, especially by one or more halogen atoms, for example up to perhaloalkyl, by one or more hydroxy groups or by one or more amino groups, all of which can optionally be substituted by alkyl. If an alkyl residue is substituted by halogen, it usually comprises 1, 2, 3, 4 or 5 halogen atoms, depending on the number of carbon atoms of the alkyl residue. For example, a methyl group can comprise, 1, 2 or 3 halogen atoms, an ethyl group (an alkyl residue comprising 2 carbon atoms) can comprise 1, 2, 3, 4 or 5 halogen atoms. If an alkyl residue is substituted by hydroxy groups, it usually comprises one or two, preferably one hydroxy groups. If the hydroxy group is substituted by alkyl, the alkyl substituent comprises preferably 1 to 4 carbon atoms and is preferably unsubstituted or substituted by halogen and more preferred unsubstituted. If an alkyl residue is substituted by amino groups, it usually comprises one or two, preferably one amino groups. If the amino group is substituted by alkyl, the alkyl substituent comprises preferably 1 to 4 carbon atoms and is preferably unsubstituted or substituted by halogen and more preferred unsubstituted. According to compounds of formula II, alkyl is preferably selected from the group consisting of methyl, ethyl, trifluoro methyl, pentafluoro ethyl, isopropyl, tert.-butyl, 2-amino ethyl, N-methyl-2-amino ethyl, N,N-dimethyl-2-amino ethyl, N-ethyl-2-amino ethyl, N,N-diethyl-2-amino ethyl, 2-hydroxy ethyl, 2-methoxy ethyl and 2-ethoxy ethyl, further preferred of the group consisting of 2-butyl, n-pentyl, neo-nentyl, isopentyl, hexyl and n-decyl, more preferred of methyl, ethyl, trifluoro methyl, isoproply and tert.-butyl.

In compounds of formula II, alkenyl is preferably selected from the group consisting of allyl, 2- or 3-butenyl, isobutenyl, sec-butenyl, furthermore preferably 4-pentenyl, isopentenyl and 5-hexenyl.

In compounds of formula II, alkylene is preferably unbranched and is more preferably methylene or ethylene, furthermore preferably propylene or butylene.

In compounds of formula II, alkylenecycloalkyl preferably has 5 to 10 carbon atoms and is preferably methylenecyclopropyl, methylenencyclobutyl, furthermore preferably methylenecyclopentyl, methylenecyclohexyl or methylenecycloheptyl, furthermore alternatively ethylenecyclopropyl, ethylenecyclobutyl, ethylenecyclopentyl, ethylenecyclohexyl or ethylenencycloheptyl, propylenecyclopentyl, propylenecyclohexyl, butylenecyclopentyl or butylenecyclohexyl.

In compounds of formula II, the term "alkoxy" preferably comprises groups of formula O-alkyl, where alkyl is an alkyl group as defined above. More preferred, alkoxy is selected from group consisting of methoxy, ethoxy, n-propoxy, isopropoxy, 2-butoxy, tert.-butoxy and halogenated, especially perhalogenated, derivatives thereof. Preferred perhalogenated derivatives are selected from the group consisting of O-CCl₃, O-CF₃, O-C₂Cl₅, O-C₂F₅, O-C(CCl₃)₃ and O-C(CF₃)₃.

In compounds of formula II, the term "alkoxyalkyl" preferably comprises branched and unbranched residues, more preferred unbranched residues, of formula CᵤH₂ᵤ₊₁-O-(CH₂)ᵥ, wherein u and v are independently from each other 1 to 6. Especially preferred is u = 1 and v 1 to 4.

In compounds of formula II the term "alkoxyalkyl" includes alkoxyalkyl groups as defined above, wherein one or more of the hydrogen atoms are substituted by halogen, for example up to perhalo alkoxyalkyl.

In compounds of formula II, cycloalkyl preferably has 3 - 7 carbon atoms and is preferably cyclopropyl or cyclobutyl, furthermore preferably cyclopentyl or cyclohexyl, furthermore also cycloheptyl, particularly preferably cyclopentyl. The term "cycloalkyl", as used herein preferably also includes saturated heterocyclic groups, wherein one or two carbon atoms are substituted by hetero atoms, selected from the group consisting of O, NH, NA and S, wherein A is as defined as above/below.

In compounds of formula II, Ar³ to Ar⁶ are preferably selected independently from one another from phenyl, naphthyl and biphenyl which is optionally substituted by one or more substituents, selected from the group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOCR¹⁵_{.}

In compounds of formula II, het is preferably an optionally substituted aromatic heterocyclic residue and even more preferred and optionally substituted saturated heterocyclic residue, wherein the substituents are preferably selected from A, CN and hal. Even more preferred, het is selected from the group consisting of 1-piperidyl, 1-piperazyl, 1-(4-methyl)-piperazyl, 4-methylpiperazin-1-yl amine, 4-morpholinyl, 1-pyrrolidinyl, 2-pyrrolidinyl, 3-pyrrolidinyl, 1-pyrazolidinyl 1-(2-methyl)-pyrazolidinyl, 1-imidazolidinyl or 1-(3-methyl)-imidazolidinyl, thiophen-2-yl, thiophen-3-yl, 2-pyridyl, 3-pyridyl, 4-pyridyl, 2-oxazolyl, 4-oxazolyl, 5-oxazolyl, 2-thiazolyl, 4-thiazolyl, 5-thiazolyl, chinolinyl, isochinolinyl, 2-pyridazyl, 4-pyridazyl, 2-pyrimidyl, 4-pyrimidyl, 5-pyrimidyl, 2-pyrazinyl and 3-pyrazinyl. Especially the thiophenyl and the pyridyl residues can optionally be substituted by one or more cyano groups.

In compounds of formula II, saturated heterocyclyl is preferably a substituted or unsubstituted saturated heterocyclic residue, more preferred an unsubstituted saturated heterocyclic residue, preferably selected from the saturated groups given above in the definition of het.

In compounds of formula II, aromatic hydrocarbons containing 6 to 14 carbon atoms and ethylenical unsaturated or aromatic heterocyclic residues containing 3 to 10 carbon atoms and one or two heteroatoms, independently selected from N, O and S, are preferably selected from the definitions given herein for aryl, heteroaryl and/or het. Heteroaryl is more preferably furanyl, thiophenyl, pyrrolyl, imidazolyl, pyrazolyl, triazolyl, tetrazolyl, thiazolyl, oxazolyl, isoxazolyl, oxadiazolyl, oxo-pyridyl, thiadiazolyl, isothiazolyl, pyridyl, pyridazyl, pyrazinyl, pyrimidyl, quinolinyl, isoquinolinyl, benzofuranyl, benzothiophenyl, indolyl, indazolyl and even more preferably pyridinyl, pyrimidyl, chinolinyl, isochinolinyl, thiophenyl, thiadiazolyl, benzothiadiazolyl, oxazolyl, isoxazolyl, pyrazolyl and/or imidazolyl. Aryl more preferably refers to an optionally substituted benzene ring or to an optionally substituted benzene ring system fused to one or more optionally substituted benzene rings to form, for example, anthracene, phenanthrene, or napthalene ring systems. Even more preferably, aryl is selected from the group consisting of phenyl, 2-naphthyl, 1-naphthyl, biphenyl.

In compounds of formula II, Ar¹ is preferably selected from the group consisting of phenyl, pyridinyl, pyrimidyl, chinolinyl, isochinolinyl, thiophenyl, thiadiazolyl, benzothiadiazolyl, oxazolyl, isoxazolyl, pyrazolyl and imidazolyl, and especially from phenyl, pyridinyl, chinolinyl, isochinolinyl, thiophenyl, benzothiadiazolyl, oxazolyl, isoxazolyl and oxazolyl.

In compounds of formula II, Ar² is preferably selected from the group consisting of phenyl, pyridinyl, pyrimidyl, chinolinyl, isochinolinyl, thiophenyl, thiadiazolyl, benzothiadiazolyl, oxazolyl, isoxazolyl, pyrazolyl and imidazolyl, even more preferably from phenyl, pyridinyl and pyrimidyl and especially preferred from phenyl and pyridinyl.

Preferably, the sum of h and I exceeds 0.

A preferred aspect of the instant invention relates to compounds of formula II, wherein n is 0 or 1 and especially 0.

Another preferred aspect of the instant invention relates to compounds of formula II, wherein n is 0 in the residues R⁸, R⁹ and/or R¹⁰ and especially in R¹⁰.

Another preferred aspect of the instant invention relates to compounds of formula II, wherein n is 0 in the residues R⁶ and/or R⁷.

Another preferred aspect of the instant invention relates to compounds of formula II, wherein X represents a bridging group, selected from (CR¹¹R¹²)ₕ or (CHR¹¹)ₕ-Q-(CHR¹²)_{i.}

The invention relates in particular to compounds of the formula II in which at least one of said radicals has one of the preferred meanings given above.

Some more preferred groups of compounds may be expressed by the following sub-formulae II.1) to II.20), which correspond to the formula II and in which radicals not denoted in greater detail are as defined in the formula II, but in which
II.1)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl;
II.2)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl, and
   - p: is 1, 2 or 3;
II.3)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3, and
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)nO(CH2)kNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³;
II.4)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹² (CH₂)ₙO(CH₂)ₖOR¹¹, (CH₂)ₙNR¹¹(CH₂)ₖOR¹²_{,} (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³;
II.5)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹_{,} (CH₂)ₙNR¹¹(CH₂)ₖOR¹²_{,} (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, wherein
   - n: is 0 or 1;
II.6)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)_{kO}R¹¹, (CH₂)ₙNR¹¹(CH₂)ₖOR¹², (CH₂)ₙCOR¹³, (CH2)ₙCOOR¹³, (CH₂)ₙCONR¹¹R¹², (CH₂)ₙSO₂NR¹¹R¹² and (CH₂)ₙS(O)ᵤR¹³, wherein
   - n: is 0 or 1, and
   - u: is 0;
II.7)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH2)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, wherein
   - n: is 0 or 1,
   - u: is 0, and
   - q: is 0 or 1, and
   - X: is selected from the group consisting of 0, S, NR¹¹, CHOR¹¹, CH₂, CH₂CH₂, OCH₂, CH₂O, OCH₂CH₂, CH₂CH₂O, preferably O, S and CH₂ and especially O and S;
II.8)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)2, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, wherein
   - n: is 0 or 1,
   - u: is 0, and
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl;
II.9)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR₁₁(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³ wherein
   - n: is 0 or 1,
   - u: is 0, and
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH2)ₙO(CH₂)ₖNR¹¹R¹², (CH₂)ₙNR¹¹(CH₂)ₖNR¹¹R¹², (CH2)ₙO(CH₂)ₖOR¹¹, (CH₂)ₙNR¹¹(CH₂)ₖOR¹¹_{,} (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)¹¹CONR¹¹R¹²;
II.10)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, wherein
   - n: is 0 or 1,
   - u: is 0, and
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)nNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹² (CH₂)ₙCOR¹³, (CH₂)ₙCOOR¹³, (CH₂)ₙCONR¹¹R¹² and especially (CH₂)ₙCONR¹¹R¹², wherein
   - n: is 0, 1 or 2, preferably 0 or 1;
II.11)
   - Ar¹: is phenyl, pyridinyl, pyrimidyl, chinolinyl, isochinolinyl, thiophenyl, thiadiazolyl, benzothiadiazolyl, oxazolyl, isoxazolyl, pyrazolyl or imidazolyl, preferably phenyl, p pyridinyl, chinolinyl, isochinolinyl, thiophenyl, benzothiadiazolyl, oxazolyl, isoxazolyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)kNR¹¹R¹², (CH₂)ₙNR¹¹(CH₂)ₖNR¹¹R¹², (CH₂)ₙO(CH₂)KOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, wherein
   - n: is 0 or 1,
   - u: is 0, and
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl n comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH2)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹², wherein is 0, 1 or 2, preferably 0 or 1 and
   - r: is 0, 1 or 2, preferably 0 or 1;
II.12)
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, wherein
   - n: is 0 or 1,
   - u: is 0, and
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹² (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³ (CH₂)ₙCOOR¹³, (CH₂)ₙCONR¹¹R¹² and especially (CH₂)ₙCONR¹¹R¹², wherein
   - n: is 0, 1 or 2, preferably 0 or 1 and
   - r: is 0, 1 or 2, preferably 0 or 1;
II.13)
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹² (CN₂)ₙNR¹¹(CH₂)ₖNR¹¹R¹², (CH₂)ₙO(CH₂)ₖOR¹¹, (CH₂)ₙNR¹¹(CH₂)ₖOR¹², n (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, wherein is 0 or 1,
   - u: is 0, and
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹² (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹², wherein
   - n: is 0, 1 or 2, preferably 0 or 1 and
   - r: is 0, 1 or 2, preferably 0 or 1;
II.14)
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)kOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³ wherein
   - u: is 0, and
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)nNR¹¹(CH₂)ₖNR¹¹R¹², (CH₂)ₙO(CH₂)ₖOR¹¹, (CH₂)ₙNR¹¹(CH₂)ₖOR¹² (CH₂)ₙCOR¹³, (CH₂)ₙCOOR¹³, n (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹², wherein is 0, 1 or 2, preferably 0 or 1 and
   - r: is 0, 1 or 2, preferably 0 or 1;
II.15)
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³ (CH₂)ₙCOOR¹³, (CH₂)ₙCONR¹¹R¹², (CH₂)ₙSO₂NR¹¹R¹² and (CH₂)ₙS(O)ᵤR¹³, wherein
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR₁₁(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹² (CH₂)ₙO(CH₂)ₖNR¹R¹² (CH₂)ₙCOR¹³ (CH₂)ₙCOOR¹³, (CH₂)ₙCONR¹¹R¹² and especially (CH₂)ₙCONR¹¹R¹², wherein
   - n: is 0, 1 or 2, preferably 0 or 1 and
   - r: is 0, 1 or 2, preferably 0 or 1;
II.16)
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹², wherein
   - n: is 0, 1 or 2, preferably 0 or 1 and
   - r: is 0, 1 or 2, preferably 0 or 1;
II.17)
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is phenyl, pyridinyl or pyrimidyl, and especially is phenyl or pyridinyl; and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹², wherein
   - n: is 0, 1 or 2, preferably 0 or 1 and
   - r: is 0, 1 or 2, preferably 0 or 1;
II.18)
   - Ar²: is phenyl, pyridinyl or pyrimidyl, and especially is phenyl or pyridinyl; and
   - R¹⁰: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)kOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - n: is 0, 1 or 2, preferably 0 or 1 and
   - r: is 0, 1 or 2, preferably 0 or 1;
II.19)
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - n: is 0, 1 or 2, preferably 0 or 1 and
   - r: is 0, 1 or 2, preferably 0 or 1;
II.20)
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹², and
   - r: is 0, 1 or 2, preferably 0 or 1.

One preferred embodiment of the instant invention relates to compounds of formula II and preferably one or more of sub formulae II.1) to 11.20), wherein p is 1, 2 or 3 and R⁸ is independently selected from the group consisting of methyl, ethyl, isopropyl, tert.-butyl, F, Cl, Br, CF₃, C(CF₃)₃, SO₂CF₃, methoxy, ethoxy, tert.-butoxy, perfluoro tert.-butoxy (OC(CF₃)₃), methyl sulfanyl (SCH₃), ethyl sulfanyl (SCH₂CH₃), acetyl (COCH₃), propionyl (COCH₂CH₃), butyryl (COCH₂CH₂CH₃). If p is 2 or 3, all substituents can be the same or different.

Another preferred embodiment of the instant invention relates to compounds of formula II and preferably one or more of sub formulae II.1) to II.20), wherein X is selected from the group consisting of S, N-R²¹, CH₂, CH₂CH₂, OCH₂ and CH₂O.

Another preferred embodiment of the instant invention relates to compounds of formula II and preferably one or more of sub formulae II.1) to II.20), wherein X is selected from the group consisting of S, CH₂.

Another even more preferred embodiment of the instant invention relates to compounds of formula II and preferably one or more of sub formulae II.1) to II.20), wherein X is O.

Another preferred embodiment of the instant invention relates to compounds of formula II and preferably one or more of sub formulae II.1) to II.20), wherein Y is selected from the group consisting of C(R²²)-NO₂, C(R²²)-CN and C(CN)₂.

Another more preferred embodiment of the instant invention relates to compounds of formula II and preferably one or more of sub formulae II.1) to II.20), wherein Y is selected from the group consisting of O, S and NR²¹.

Another even more preferred embodiment of the instant invention relates to compounds of formula II and preferably one or more of sub formulae II.1) to II.20), wherein Y is selected from the group consisting of O and S.

Another even more preferred embodiment of the instant invention relates to compounds of formula II and preferably one or more of sub formulae II.1) to II.20), wherein Y is O.

Another preferred embodiment of the instant invention relates to compounds of formula II and preferably one or more of sub formulae II.1) to II.20), wherein R⁶ and R⁷ both are hydrogen.

Another preferred embodiment of the instant invention relates to compounds of formula II and preferably one or more of sub formulae II.1) to II.20), wherein R⁸ or R⁷ is a residue other than hydrogen. In this embodiment, the residue other than hydrogen is preferably selected from the meanings given for R⁸, R⁹ and R¹⁰, more preferably from A, and especially preferred from substituted or preferably unsubstituted alkyl, substituted or preferably unsubstituted alkenyl, substituted or preferably unsubstituted cycloalkyl and substituted or preferably unsubstituted alkylenecycloalkyl, even more preferred substituted or unsubstituted alkyl with 1 to 6 carbon atoms, for example methyl, ethyl, n-propyl, isopropyl, n-butyl, 2-butyl, tert.-butyl, optionally substituted by one or more hydroxy groups, preferably one or two hydroxy groups and/or one or more halogen atoms, up to perhalo. Examples for preferred substituted alkyl groups are CH₂Hal, especially CH₂F, CH₂Cl and CH₂Br, CHal₃, especially CF₃, CCl₃ and CBr₃, and (CH₂)_{z}OH, wherein Z is 1 to 6, especially CH₂OH and CH₂CH₂OH.

Another preferred embodiment of the instant invention relates to compounds of formula II and preferably one or more of sub formulae II.1) to II.20), wherein R⁶ or R⁷ is a residue other than hydrogen. In this embodiment, the residue other than hydrogen is preferably selected from (CH₂)ₙCOOR¹³, (CH₂)ₙCONR¹¹R¹², (CH₂)ₙNR¹¹COR¹³, (CH₂)ₙNR¹¹CONR¹¹R¹², (CH₂)ₙO(CH₂)ₖNR¹¹R¹² and (CH₂)ₙNR¹¹(CH₂)ₖNR¹¹R¹², more preferred (CH₂)ₙCOOR¹³, even more preferred (CH₂)ₙCOOA and (CH₂)ₙCOOH and especially preferred (CH₂)ₙCOOA, wherein A is C₁-C₄- alkyl and (CH₂)ₙCOOH. In this embodiment, n is as defined above/below and especially is 0.

Another preferred embodiment of the instant invention relates to compounds of formula II and preferably one or more of sub formulae II.1) to II.20), wherein R⁶ is hydrogen and R⁷ is methyl, or R⁶ is methyl and R⁷ is hydrogen.

Another preferred embodiment of the instant invention relates to compounds of formula II and preferably one or more of sub formulae II.1) to II.20), wherein R⁶ and R⁷ are residues other than hydrogen. In this embodiment, R⁶ and R⁷ are preferably selected, independently from one another, from the meanings given for R⁸, R⁹ and R¹⁰, more preferably from the meanings given for A, and especially preferred from substituted or preferably unsubstituted alkyl, substituted or preferably unsubstituted alkenyl, substituted or preferably unsubstituted cycloalkyl and substituted or preferably unsubstituted alkylenecycloalkyl, even more preferred substituted or unsubstituted alkyl with 1 to 6 carbon atoms, for example methyl, ethyl, n-propyl, isopropyl, n-butyl, 2-butyl; tert.-butyl, optionally substituted by one or more hydroxy groups, preferably one or two hydroxy groups and/or one or more halogen atoms, up to perhalo. Examples for preferred substituted alkyl groups are CH₂Hal, especially CH₂F, CH₂Cl and CH₂Br, CHal₃, especially CF₃, CCl₃ and CBr₃, and (CH₂)_{z}OH, wherein Z is 1 to 6, especially CH₂OH and CH₂CH₂OH. In this embodiment, R⁶ and R⁷ even more preferred form, together with the carbon atom they are bound to (i. e. the carbon atom of the methylene moiety of the methylene urea moiety), a carbocyclic residue comprising 3 to 6 carbon atoms or a heterocyclic residue comprising one or two heteroatoms, selected from the group consisting of O, N and S, and 2 to 5 carbon atoms, wherein the carbocyclic residue respectively the heterocyclic residue can be substituted by one or more substituents, preferably one or two substituents, selected, independently from one another, from the meanings given for R⁸, R⁹ and R¹⁰. If R⁶ and R⁷ form a cyclic residue together with the carbon atom they are bound to, carbocyclic residues are preferred. Even more preferred are carbocyclic residues comprising 3, 4 or 5 carbon atoms, especially 3 carbon atoms which can be substituted once or twice as given above and preferably are unsubstituted. In this respect, one preferred embodiment of the instant invention relates to compounds, wherein R⁶ and R⁷ form, together with the carbon atom they are bound to, a cyclopropane moiety.

Another preferred embodiment of the instant invention relates to compounds of formula II and preferably one or more of sub formulae II.1) to II.20), wherein one of the residues R⁶ or R⁷ or both residues R⁶ and R⁷ are other than hydrogen and are preferably as defined in the preferred embodiments relating to R⁶ and R⁷ given above.

Another preferred embodiment of the instant invention relates to compounds of formula II and preferably one or more of sub formulae II.1) to II.20), wherein Ar² is pyridinyl.

Another preferred embodiment of the instant invention relates to compounds of formula II and preferably one or more of sub formulae II.1) to II.20), wherein r is either 0 or 1. If r is 1, R¹⁰ is preferably (CH₂)ₙCONR¹¹R¹² and especially (CH₂)ₙCONR¹¹R¹², wherein n in 0. In this embodiment, R¹¹ is preferably selected from the group consisting of H and A and more preferred from H and alkyl, and R¹² is preferably selected from the group consisting of H and A and more preferred from H and alkyl. Especially preferred as residue R¹⁰ are carbamoyl, more preferred alkyl carbamoyl or dialkyl carbamoyl, even more preferred methyl carbamoyl or dimethyl carbamoyl, ethyl carbamoyl or diethyl carbamoyl and especially preferred methyl carbamoyl (-CONHCH₃). This embodiment is especially preferred when Ar² is pyridinyl. When Ar² is pyridinyl, R¹⁰ is preferably bonded in a vicinal position to the nitrogen atom of the pyrindiyl residue, i.e. in 2- and/or 6-position of the pyridinyl residue.

Another preferred embodiment of the instant invention relates to compounds of formula II and preferably one or more of sub formulae II.1) to II.20), wherein Ar¹ comprises two or more substituents R⁸, wherein one or more, preferably one substituent R⁸ is selected from the group consisting of (CH₂)ₙNR¹¹R¹², (CH2)ₙO(CH₂)ₖNR¹¹R¹². (CH₂)ₙNR¹(CH₂)ₖOR¹², (CH₂)ₙNR¹¹(CH₂)ₖNR¹²R¹², (CH₂)ₙCOOR¹³ and (CH₂)ₙS(O)ᵤR¹³ wherein R¹¹, R¹² and R¹³ are defined as above and n is as defined above, preferably n is 0, 1 or 2 and especially is 0, k is 1 to 4 and preferably 1 or 2, and u is preferably 2. In this embodiment R¹¹, R¹² and R¹³ are more preferably selected independently from each other from the group consisting of H, methyl and ethyl. In this embodiment, one or two substituents R⁸ and preferably one substituent R⁸ is especially preferably selected from the group consisting of NH₂, N(CH₃)₂, N(C₂H₅)₂, NHCH₂CH₂NH₂, N(CH₃)CH₂CH₂NH₂, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, SCH₃, SC₂H₅, S0₂CH₃, COOCH₃ and COOH. Accordingly, in this embodiment Ar¹ especially preferably comprises at least one substituent R⁸ other than (CH₂)ₙNR¹¹R¹², (CH₂)ₙO(CH₂)ₖNR¹¹R¹², (CH₂)ₙNR¹¹(CH₂)ₖOR¹² (CH₂)ₙNR¹¹(CH₂)ₖNR¹²R¹², (CH₂)ₙCOOR¹³ and (CH₂)ₙS(O)ᵤR¹³ as defined in this paragraph and especially other than NH₂, N(CH₃)₂, N(C₂H₅)₂, NHCH₂CH₂NH₂, N(CH₃)CH₂CH₂NH₂, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, SCH₃, SC₂H₅, SO₂CH₃, COOCH₃ and COOH.

Another preferred embodiment of the instant invention relates to compounds of formula II and preferably one or more of sub formulae II.1) to II.20), wherein q is 0, i.e. the phenyl group bound to the methylene group of the methylene urea moiety is unsubstituted.

Another preferred embodiment of the instant invention relates to compounds of formula I and preferably one or more of sub formulae II.1) to II.20), wherein q is 1, i.e. the phenyl group bound to the methylene group of the methylene urea moiety is substituted by one substituent, preferably a substituent as defined above and more preferably a substituent selected from alkyl and hal, and especially selected from CH₃, CH₂CH₃ and hal.

Another preferred embodiment of the instant invention relates to compounds of formula II and preferably one or more of formulae II.1) to II.20), wherein (R⁸)ₚ-Ar¹ is selected from the group consisting of 3-acetyl-phenyl, 4-acetyl-phenyl, 2-bromo-phenyl, 3-bromo-phenyl, 4-bromo-phenyl, 4-bromo-2-chloro-phenyl, 4-bromo-3-methyl-phenyl, 4-bromo-3-trifluoromethyl-phenyl, 2-chloro-phenyl, 2-chloro-4-trifluoromethyl-phenyl, 2-chloro-5-trifluoromethyl-phenyl, 3-chloro-phenyl, 3-chloro-4-methyl-phenyl, 3-chloro-4-methoxy-phenyl, 3-chloro-4-methoxy-phenyl, 4-chloro-phenyl, 4-chloro-2-trifluoromethyl-phenyl, 4-chloro-3-trifluoromethyl-phenyl, 4-chloro-2-methyl-phenyl, 5-chloro-2-methyl-phenyl, 5-chloro-2-methoxy-phenyl, 2,3-dichloro-phenyl, 2,4-dichloro-phenyl, 2,5-dichloro-phenyl, 3,4-dichloro-phenyl, 3,5-dichloro-phenyl, 2,4,5-trichloro-phenyl, 4-fluorophenyl, 4-fluoro-3-trifluoromethyl-phenyl, 4-ethoxy-phenyl, 2-methoxy-phenyl, 2-methoxy-5-trifluoromethyl-phenyl, 4-methoxy-phenyl, 2,5-dimethoxy-phenyl, 2-trifluoromethyl-phenyl, 3-trifluoromethyl-phenyl, 3-trifluoromethoxy-phenyl, 4-trifluoromethyl-phenyl, 4-trifluoromethoxy-phenyl, 3,5-bis-trifluoromethyl-phenyl, 3-methoxy-phenyl, 3-methylsulfanyl-phenyl, 4-methylsulfanyl-phenyl, o-tolyl (2-methyl-phenyl), m-tolyl (3-methyl-phenyl), p-tolyl (4-methyl-phenyl), 2,3-dimethyl-phenyl, 2,3-dimethyl-phenyl, 2,5-dimethyl-phenyl, 3,4-dimethyl-phenyl, 3,5-dimethyl-phenyl, 2-ethyl-phenyl, 3-ethyl-phenyl, 4-ethyl-phenyl, 4-isopropyl-phenyl, 4-tert-butyl-phenyl and 5-tert-butyl-isoxazol-3-yi.

Another preferred embodiment of the instant invention relates to compounds of formula II and the subformulae related thereto and preferably one or more of formulae II.1) to II.20), wherein the residues (R⁸)ₚ-Ar¹ are selected from the group consisting of compounds of the following formulae:
a) 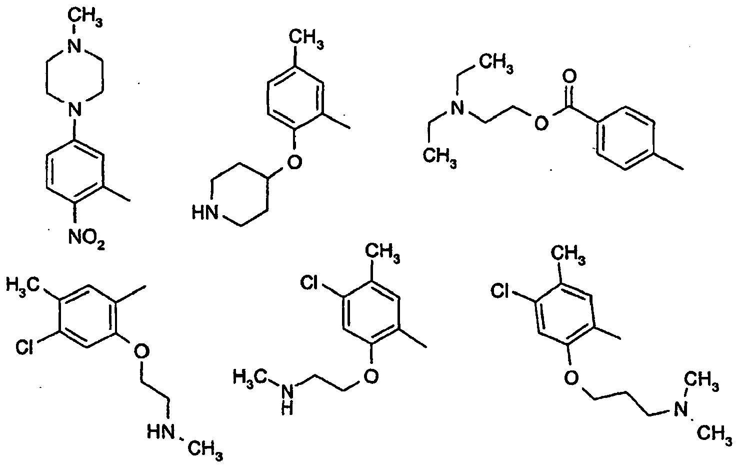 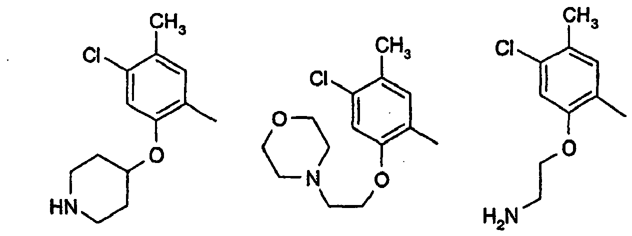 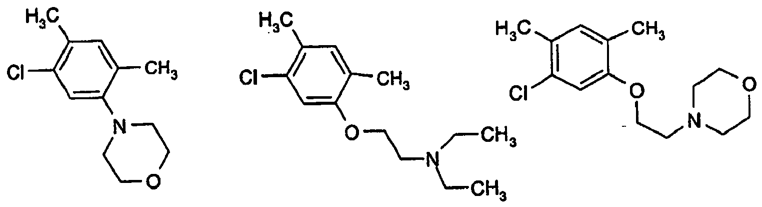 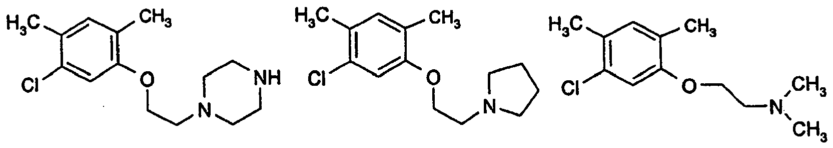
b) 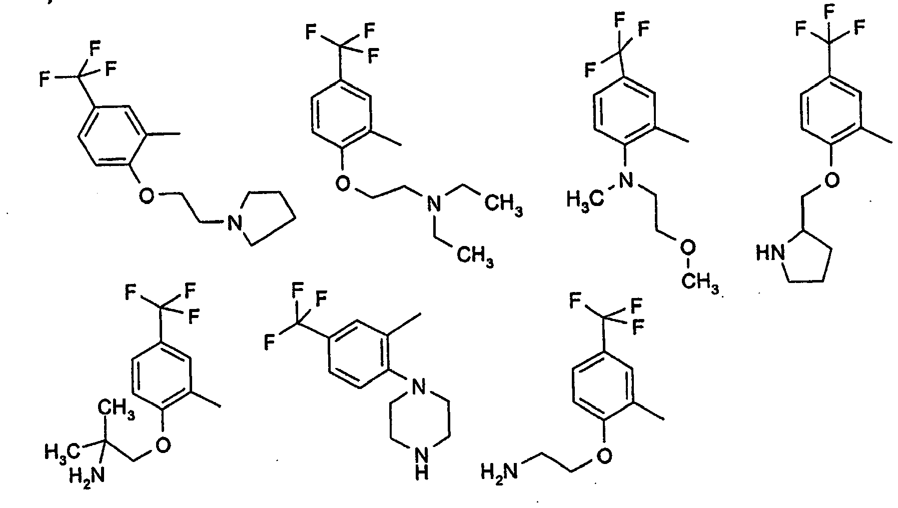 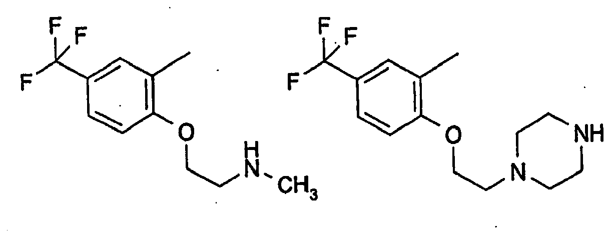 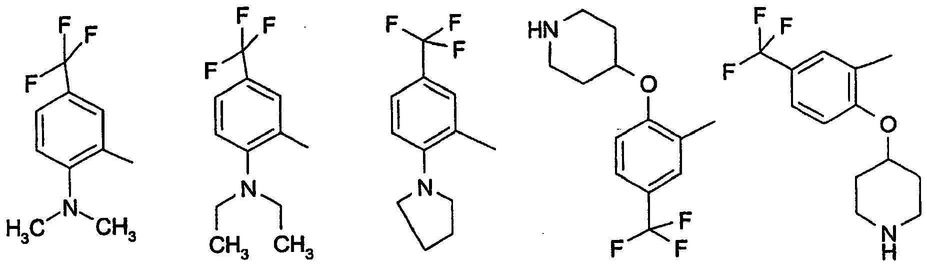 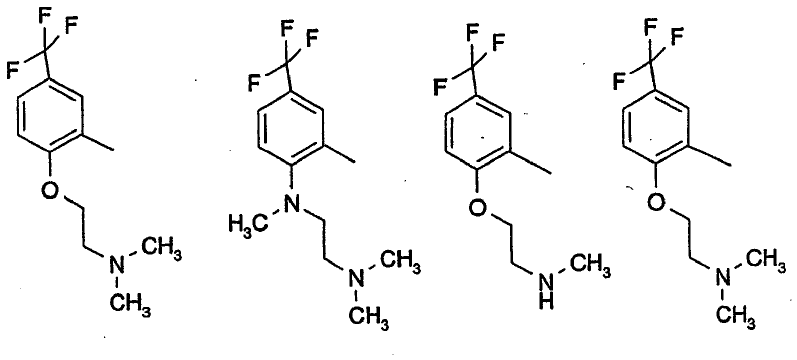
c) 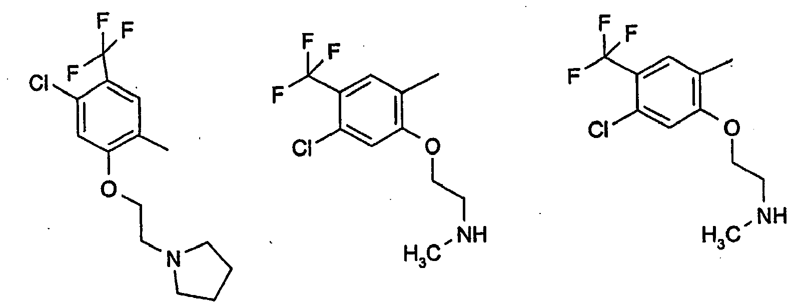 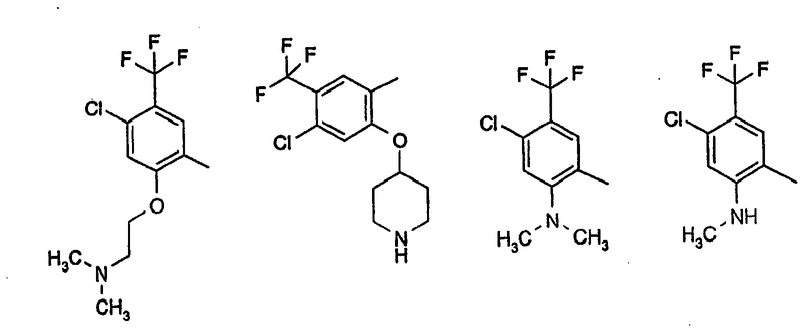 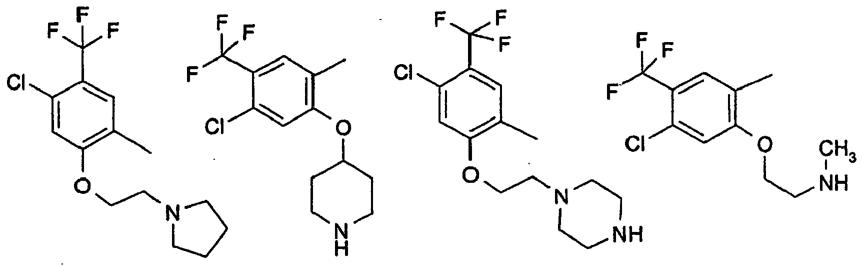 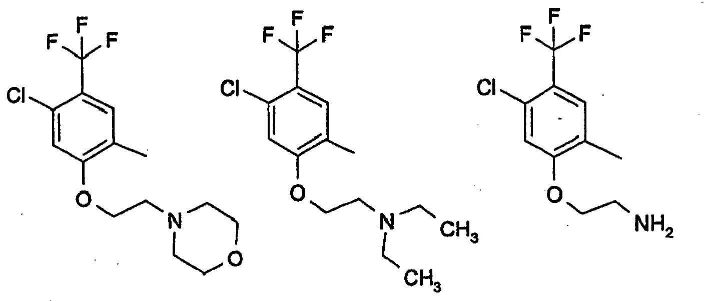
d) 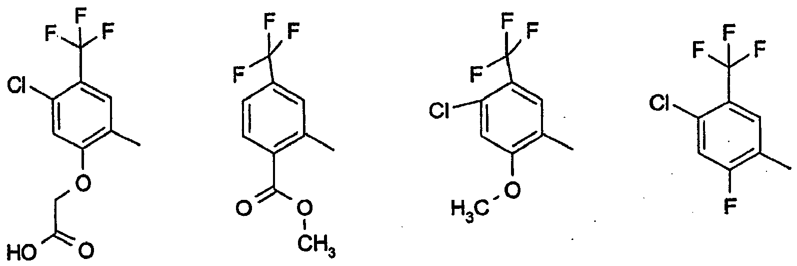 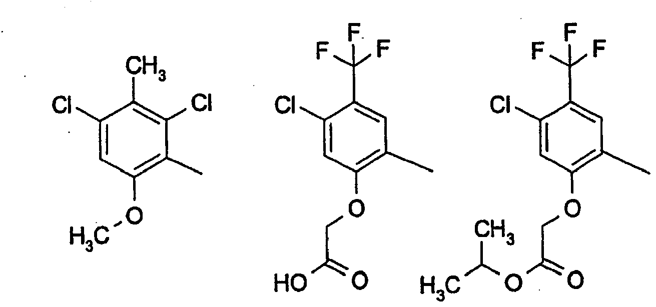
e) 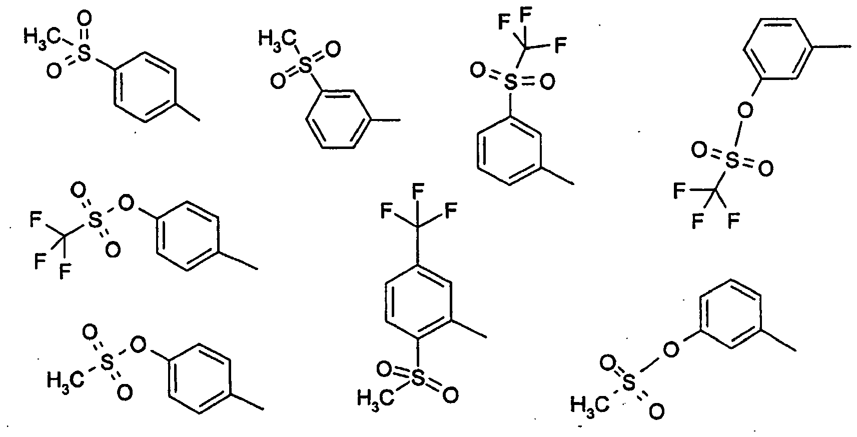 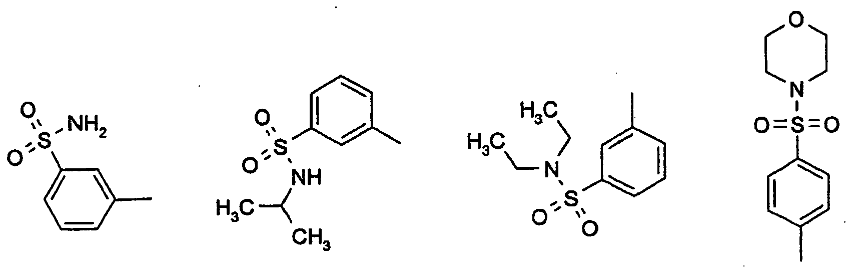 f) 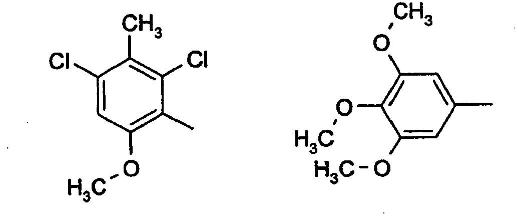 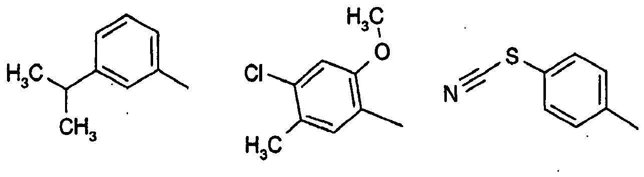 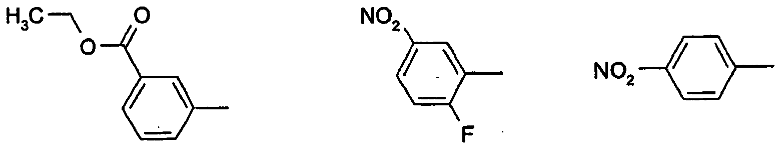 and/or 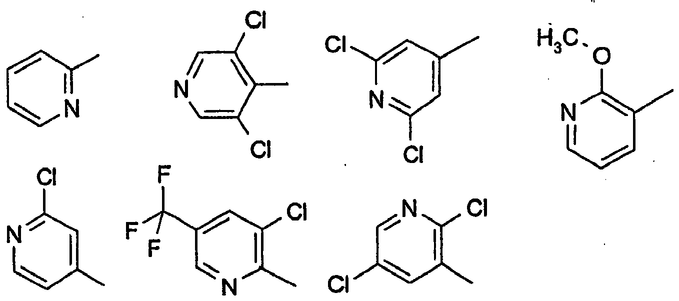 and/or 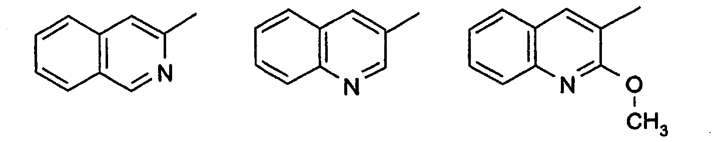 and/or 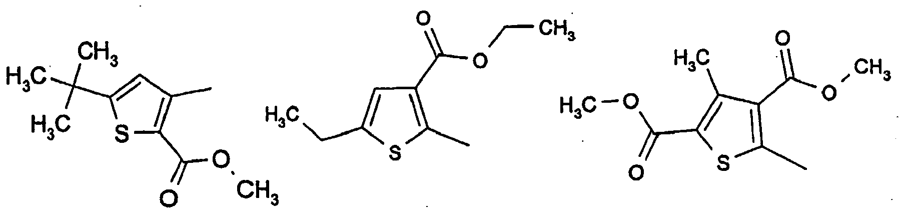 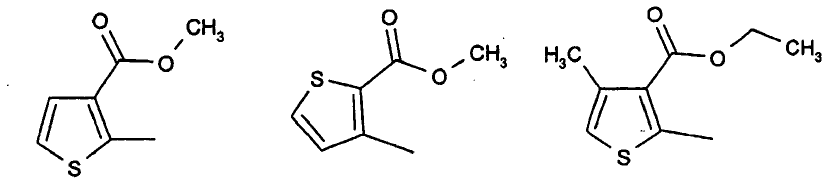 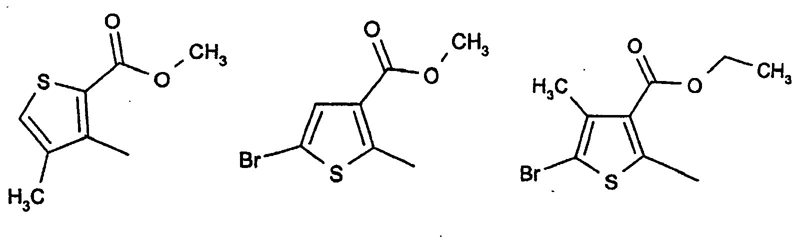 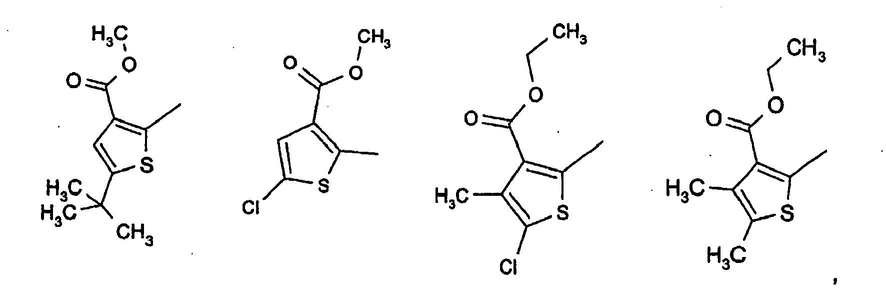 and/or residues of the structures given above that comprise one or two, preferably one additional substituent, independently selected from the meanings given for R³.

Another preferred embodiment of the instant invention relates to compounds of formula II and preferably one or more of sub formulae II.1) to II.20), wherein (R⁸)ₚ-Ar¹ is as defined above, but comprises one or more additional residues, preferably one additional residue. The additional residues are preferably selected from the meanings given for R⁸ and more preferably selected from the group consisting of (CH₂)ₙNR¹¹R¹², (CH₂)ₙO(CH₂)ₖNR¹¹R¹², (CH₂)ₙNR¹¹(CH₂)ₖOR¹², (CH₂)ₙNR¹¹(CH₂)ₖNR¹¹R¹², (CH₂)ₙCOOR¹³, (CH₂)ₙS(O)ᵤNR¹¹R¹² and (CH₂)ₙS(O)ᵤR¹³ wherein R¹¹, R¹² and R¹³ are defined as above and n is as defined above, preferably n is 0, 1 or 2 and especially is 0, k is 1 to 4 and preferably 1 or 2, and u is preferably 2. In this embodiment R¹¹, R¹² and R¹³ are more preferably selected independently from each other from the group consisting of H, methyl and ethyl. Even more preferred, the additional residue(s) is/are selected from the group consisting of NH₂, N(CH₃)₂, N(C₂H₅)₂, NHCH₂CH₂NH₂, N(CH₃)CH₂cH₂NH₂, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, SCH₃, SC₂H₅, SO₂CH₃, SO₂CF₃, OSO₂CH₃, OSO₂CF₃, SO₂NH₂, SO₂NHCH(CH₃)₂, SO₂N(CH₃)₂, SO₂N(CH₂CH₃)₂,4-Morpholino-sulfonyl, COOCH₃ and COOH.

Another preferred embodiment of the instant invention relates to compounds of formula II and the subformulae related thereto and preferably one or more of formulae II.1) to II.20), wherein the residues Ar²-(R¹⁰)ᵣ are selected from the group consisting of compounds of the following formulae: 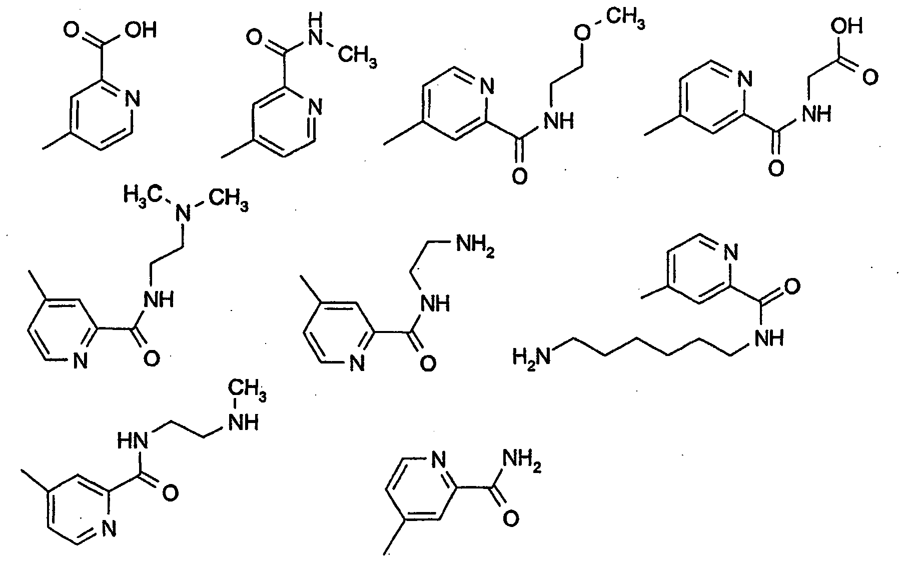 and/or residues of the structures given above that comprise one or two, preferably one additional substituent, independently selected from the meanings given for R¹⁰.

Another preferred embodiment of the instant invention relates to compounds of formula II and preferably one or more of sub formulae II.1) to II.20), wherein X is bonded in the para- (p-) or metha- (m-)position to the phenyl residue that is bonded directly to the methylene group of the methylene urea moiety.

Another preferred embodiment of the instant invention relates to compounds of formula II and preferably one or more of sub formulae II.1) to II.20), wherein Ar² is a pyridinyl residue and wherein said pyridinyl residue is bonded to X in the 3- or 4-position, preferably the 4-position, relative to the nitrogen atom of the pyridinyl residue.

Another preferred embodiment of the instant invention relates to compounds of formula II and preferably one or more of sub formulae II.1) to II.20), wherein Ar¹ comprises one or more substituents R⁸ and wherein one or two, preferably one substituent R⁸ is selected from the group consisting of NH₂, N(CH₃)₂, N(C₂H₅)₂, NHCH₂CH₂NH₂, N(CH₃)CH₂CH₂NH₂, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, SCH₃, SC₂H₅, SO₂CH₃, SO₂CF₃, OSO₂CH₃, OSO₂CF₃, SO₂NH₂, SO₂NHCH(CH₃)₂, SO₂N(CH₃)₂, SO₂N(CH₂CH₃)₂, 4-Morpholino-sulfonyl, COOCH₃ and COOH, more preferably NH₂, N(CH₃)₂, NHCH₃, N(C₂H₅)₂, HNCH₂CH₂NH₂, OCH₂CH₂NH₂, HOCH₂CH₂NH, OCH₂CH₂NHCH₃, N(CH₃)CH₂CH₂NH₂, HN(CH₃)CH₂CH2NH, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, OCH₂CH₂N(CH₂CH₃)₂, SCH₃, SC₂H₅, and/or compounds of the formulae 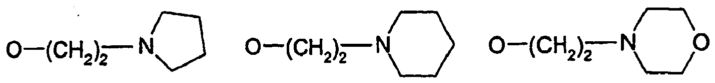 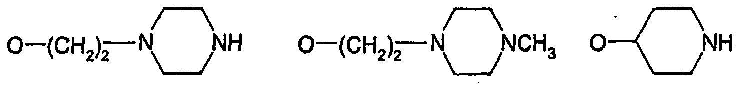 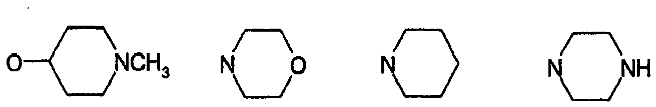 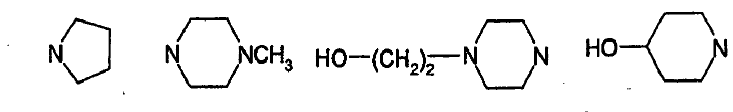 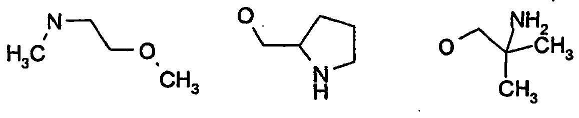 and/or Ar² comprises one or more substituents R¹⁰ and wherein one or two, preferably one substituent R¹⁰ is independently selected from the meanings given for R⁸ in this paragraph.

Another preferred embodiment of the instant invention relates to compounds of formula II and preferably one or more of sub formulae II.1) to II.20), wherein Ar¹ comprises one or more substituents R⁸ and wherein one or two, preferably one substituent R⁸ is selected from the group consisting of

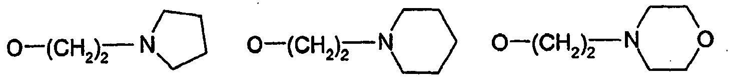

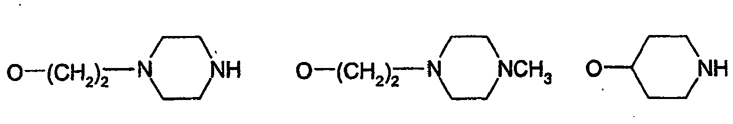

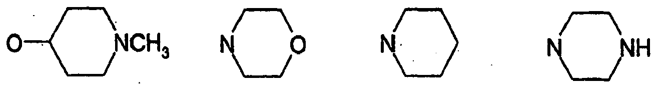

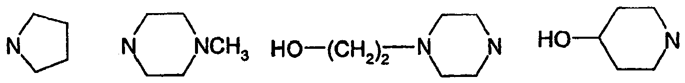

Another preferred embodiment of the instant invention relates to compounds of formula II and preferably one or more of sub formulae II.1) to II.20), wherein Ar¹ comprises one or more substituents R⁸ and wherein one or two, preferably one substituent R⁸ is selected from the group consisting of SO₂CH₃, SO₂CF₃, OSO₂CH₃, OSO₂CF₃, SO₂NH₂, SO₂NHCH(CH₃)₂, SO₂N(CH₃)₂, SO₂N(CH₂CH₃)₂ and 4-Morpholine-4-sulfonyl.

Another preferred embodiment of the instant invention relates to compounds of formula II and preferably one or more of sub formulae II.1) to II.20), wherein Ar² comprises one or more substituents R¹⁰ and wherein one or two, preferably one substituent R¹⁰ is selected from unsubstituted or substituted carbamoyl moieties. Substituted carbamoyl moieties are preferably selected from CONHR²³ or CONR²³R²⁴, preferably CONHR²³, wherein R²³ and R²⁴ are independently selected from the definitions given for R⁸, more preferably selected from alkyl, preferably methyl, ethyl, propyl and butyl, (CH₂)ₙNR¹¹R¹² and (CH₂)ₙOR¹², wherein R¹¹, R¹² and n are as defined above. In this embodiment, n is preferably not 0 and more preferred 1 to 3 and especially 1 or 2. Preferred examples for R²³ are selected from the group consisting of methyl, ethyl, CH₂CH₂NH₂, CH₂CH₂N(CH₃)₂, CH₂CH₂N(CH₂CH₃)₂, CH₂CH₂OH, CH₂CH₂OCH₃ and CH₂CH₂OCH₂CH₃.

Another preferred embodiment of the instant invention relates to compounds of formula II and preferably one or more of sub formulae II.1) to II.20), wherein Ar² comprises one or more substituents R¹⁰ and wherein one or two, preferably one substituent R¹⁰ is selected from substituted carbamoyl moieties. Substituted carbamoyl moieties are preferably selected from CONHR²³, wherein R²³ is preferably unsubstituted C₁-C₄-alkyl and especially methyl.

Another preferred embodiment of the instant invention relates to compounds of formula II and preferably one or more of sub formulae II.1) to II.20), wherein Ar² comprises one or more substituents R¹⁰ and wherein one or two, preferably one substituent R¹⁰ is selected from substituted carbamoyl moieties. Substituted carbamoyl moieties are preferably selected from CONHR²³, wherein R²³ is selected from (CH₂)ₙNR¹¹R¹² and (CH₂)ₙOR¹², wherein R¹¹, R¹² and n are as defined above. In this embodiment, n is preferably not 0 and more preferred 1 to 3 and especially 1 or 2. Preferred examples for R²³ are selected from the group consisting of CH₂CH₂NH₂, CH₂CH₂N(CH₃)₂, CH₂CH₂N(CH₂CH₃)₂, CH₂CH₂OH, CH₂CH₂OCH₃ and CH₂CH₂OCH₂CH₃.

Another preferred embodiment of the instant invention relates to compounds of formula II and preferably one or more of sub formulae II.1) to II.20), wherein -Ar²-(R¹⁰) is selected from the formulae 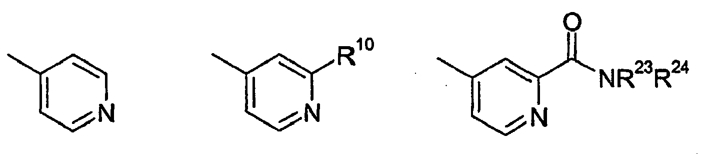 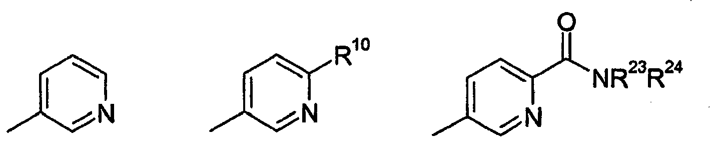 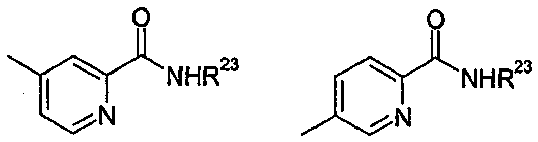 wherein R¹⁰, R²³ and R²⁴ are as defined above and below.

Another especially preferred embodiment of the instant invention relates to compounds of formula II and preferably one or more of sub formulae II.1) to II.20), wherein one or more features of the above and below mentioned embodiments are combined in one compound.

Subject of the present invention are therefore preferably compounds of formula II according to one or both of the formulae IIa and IIb, 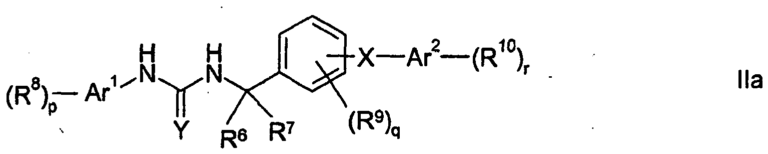 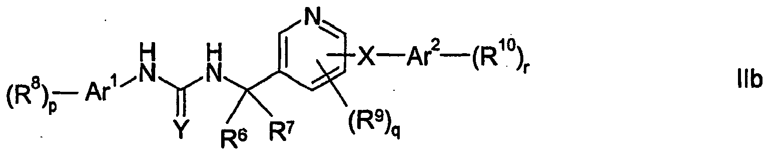 wherein Ar¹, R⁸, p, Y, R⁶, R⁷, X, R⁹, q, Ar², R¹⁰ and r are as defined above and below, and preferably as defined in sub formulae II.1) to II.20) and/or the embodiments related thereto.

Subject of the present invention are therefore especially preferred compounds of formula II according to one or both of the formulae IIc and IId, 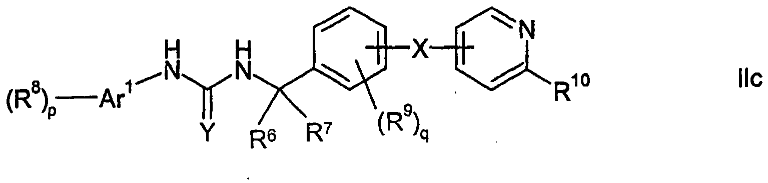 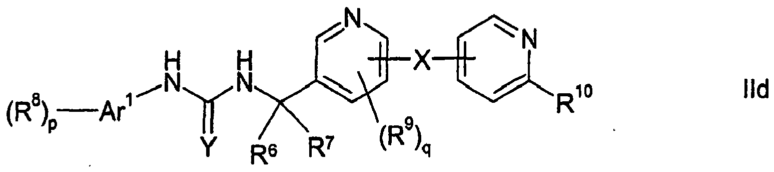 wherein Ar¹, R⁸, p, Y, R³, R⁷, X, R⁹ and q are as defined above and below, R¹⁰ is H or as defined above/below, and preferably as defined in sub formulae II.1) to II.20) and/or the embodiments related thereto;
and/or compounds of formula II according to one or more of the formulae IIe to IIx, 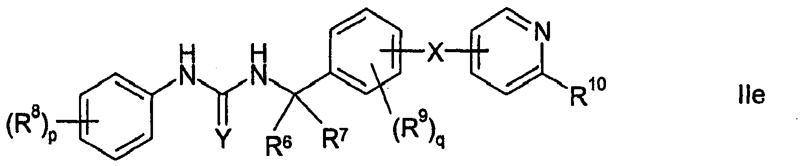 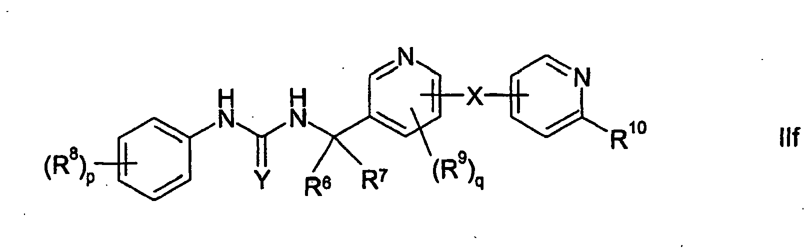 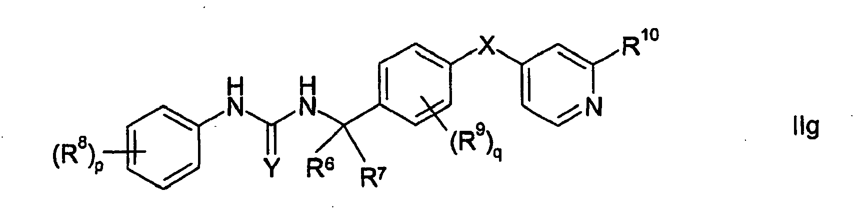 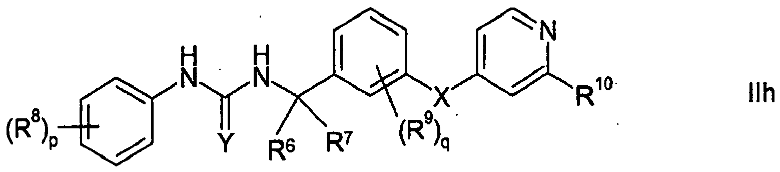 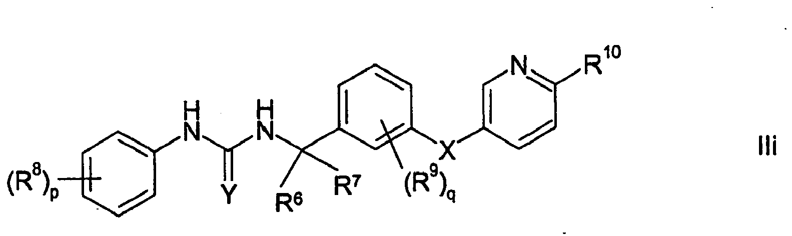 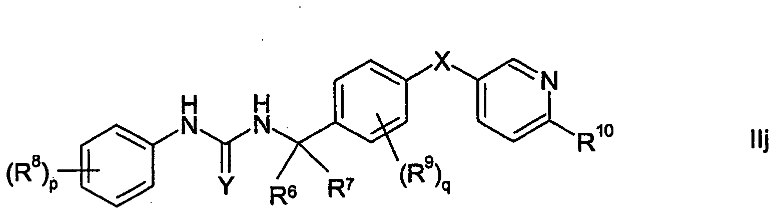 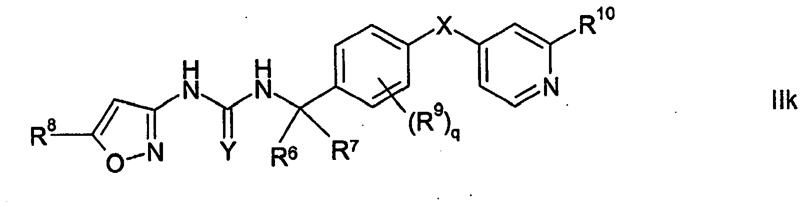 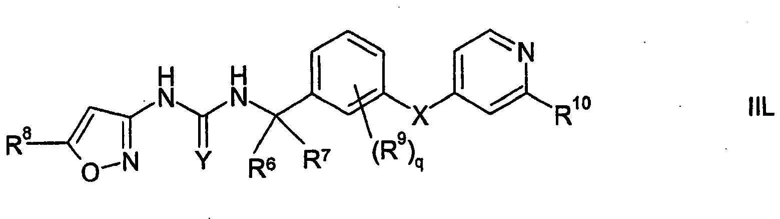 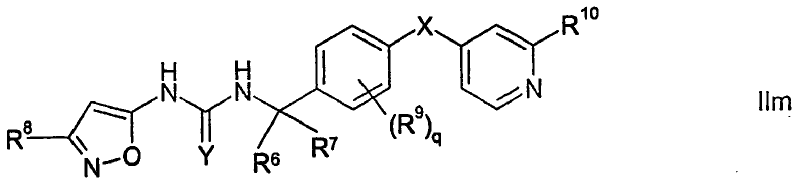 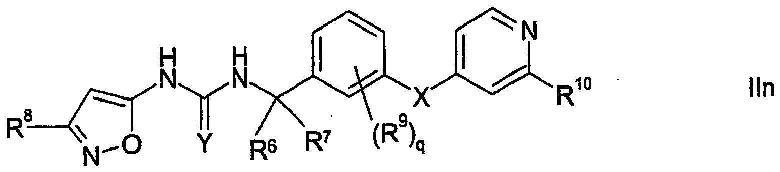 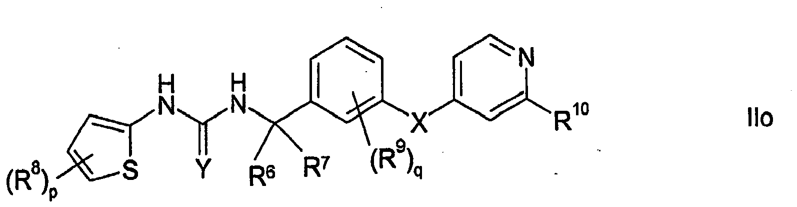 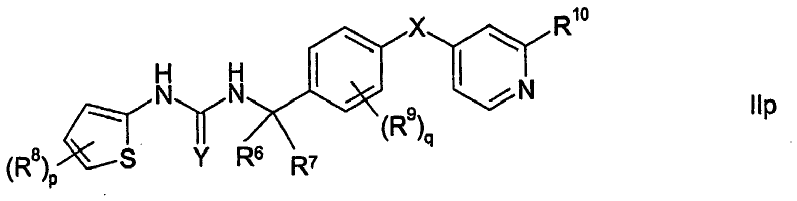 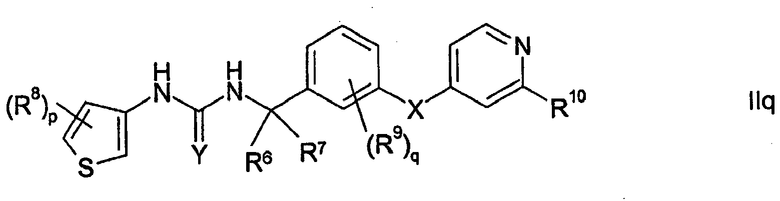 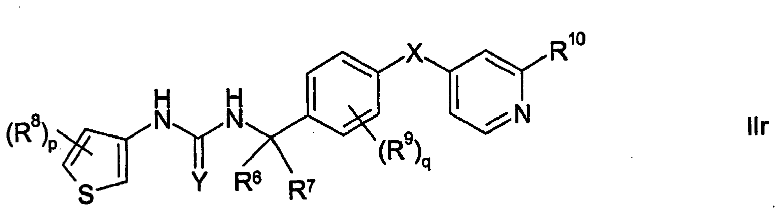 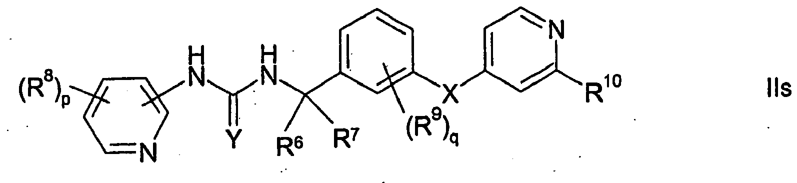 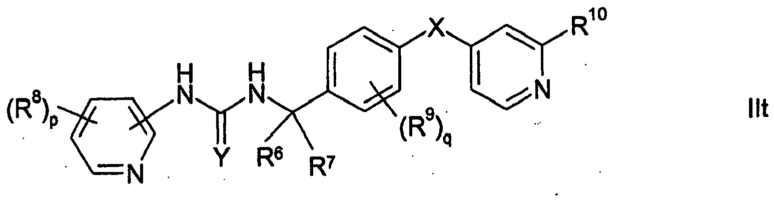 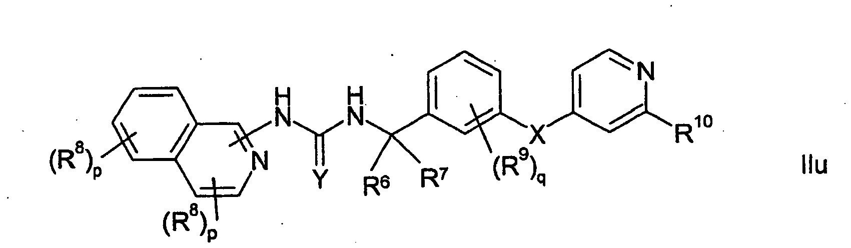 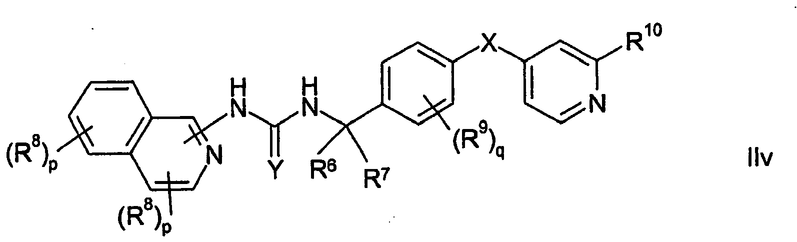 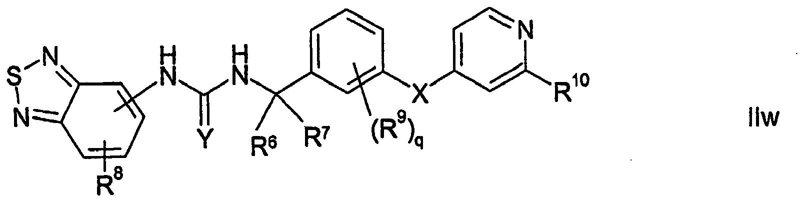 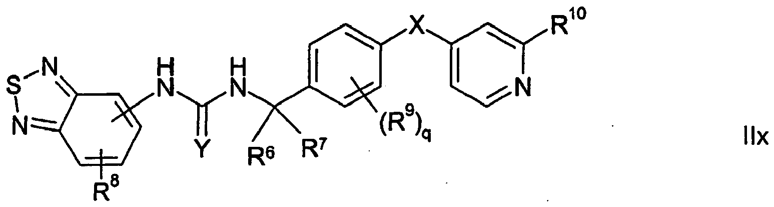 wherein R⁶, R⁷, R⁸, p, Y, X, R⁹ and q are as defined above and below, R¹⁰ is H or as defined above/below, and preferably as defined in sub formulae II.1) to II.20) and/or the embodiments related thereto.

One preferred aspect of the invention relates to compounds of formula II and especially to compounds of one or more of formulae IIa to IIx, wherein both R⁶ and R⁷ are hydrogen.

Another preferred aspect of the invention relates to compounds of formula II and especially to compounds of one or more of formulae IIa to IIx, wherein R⁶ and/or R⁷ are residues other than hydrogen.

Another preferred embodiment of the instant invention relates to compounds of formula II and preferably one or more of sub formulae II.1) to II.20) and IIa to IIx, wherein R¹⁰ is a substituted carbamoyl moiety CONHR²³ or CONR²³R²⁴, preferably CONHR²³, wherein R²³ and R²⁴ are independently selected from the definitions given for R⁸, more preferably selected from (CH₂)ₙNR¹¹R¹² and (CH₂)ₙOR¹², wherein R¹¹, R¹² and n are as defined above. In this embodiment, n is preferably not 0 and more preferred 1 to 3 and especially 1 or 2. Preferred examples for R²³ are selected from the group consisting of CH₂CH₂NH₂, CH₂CH₂N(CH₃)₂, CH₂CH₂N(CH₂CH₃)₂, CH₂CH₂OH, CH₂CH₂OCH₃ and CH₂CH₂OCH₂CH₃.

It is understood that when a residue, for example R⁸, R⁹, R¹⁰ or R¹⁴ or R²³, is comprised twice or more times in one or more of the formulae I, II and the sub formulae corresponding thereto, it is in each case independently from one another selected from the meanings given for the respective residue. For example, R¹¹ and R¹² are defined to be independently selected from a group consisting of H, A, (CH₂)ₘAr³ and (CH₂)ₘHet. Then (CH₂)ₙNR¹¹(CH₂)ₘNR¹²R¹² can be (CH₂)ₙNA(CH₂)ₘNA₂ (if R¹¹ = A, R¹² = A and R¹² = H) as well as (CH₂)ₙNA(CH₂)ₘNHA (if R¹¹ = A, R¹² = H and R¹² = A or (CH₂)ₙNA(CH₂)ₘNH(CH₂ₘHet (if R¹¹ = A, R¹² = H and R¹² = (CH₂)ₘHet). Accordingly, if a compound of formula II comprises one residue R⁸, R⁹ and R¹⁰, then for example R⁸, R⁹ and R¹⁰ can all be (CH₂)ₙCOOR¹³, wherein all residues R¹³ are the same (for example CH₂Hal, wherein Hal is Cl; then all residues R⁸, R⁹ and R¹⁰ are the same) or different (for example CH₂Hal, wherein in R⁸ Hal is Cl; in R⁹ Hal is F; and in R¹⁰ Hal is Br; then all residues R⁸, R⁹ and R¹⁰ are different); or for example R⁸ is (CH₂)ₙCOOR¹³, R⁹ is NO₂ and R¹⁰ is (CH₂)ₙSR¹¹, wherein R¹¹ and R¹³ can be the same (for example both can be H or both can be A which is methyl) of different (for example R¹¹ can be H and R¹³ can be A which is methyl).

If not stated otherwise, reference to compounds of formula I and formula II also includes the sub formulae related thereto, especially sub formulae II.1) to II.20) and IIa to IIx.

Subject of the instant invention are especially those compounds of formula I and/or formula II, in which at least one of the residues mentioned in said formulae has one of the preferred or especially preferred meanings given above and below.

The present invention further relates to compounds (1) to (224) of formula A-NH-CO-NH-CH₂-B, wherein A and B are as given in the table below:

| | A | B | Rt |
|---|---|---|---|
| (1) | 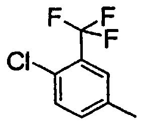 | 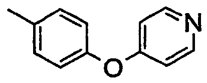 | 2.12 |
| (2) | 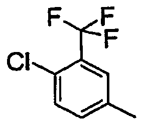 | 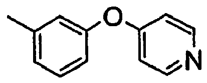 | 4.67^{a} |
| (3) | 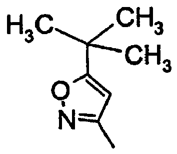 | 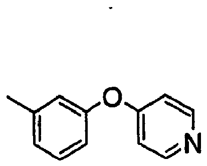 | 4.10^{a} |
| (4) | 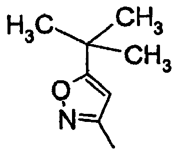 | 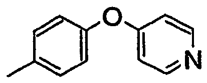 | 3.87 |
| (5) | 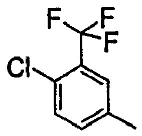 | 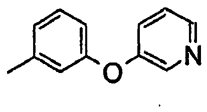 | 2.43 |
| (6) | 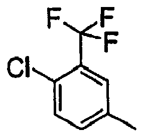 | 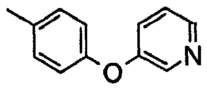 | 2.46 |
| (7) | 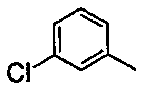 | 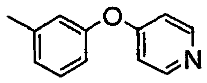 | 1.77 |
| (8) | 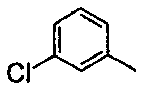 | 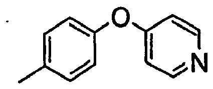 | 1.80 |
| (9) | 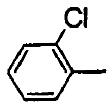 | 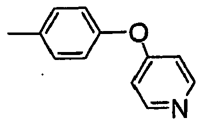 | 1.66 |
| (10) | 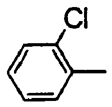 | 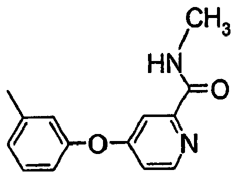 | 2.43 |
| (11) | 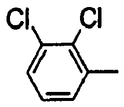 | 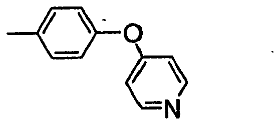 | 1.93 |
| (12) | 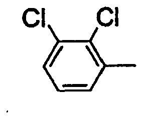 | 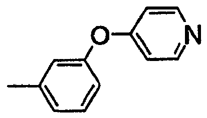 | 1.95 |
| (13) | 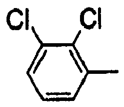 | 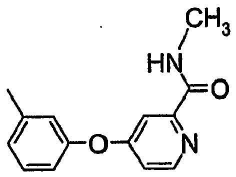 | 2.65 |
| (14) | 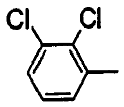 | 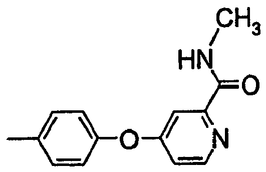 | 2.65 |
| (15) | 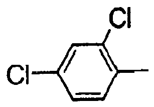 | 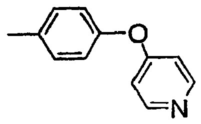 | 1.94 |
| (16) | 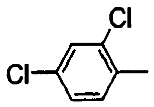 | 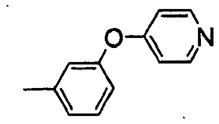 | 1.95 |
| (17) | 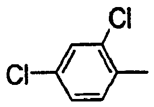 | 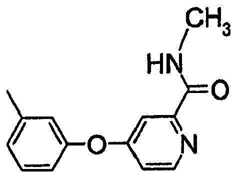 | 2.70 |
| (18) | 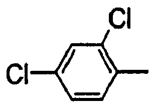 | 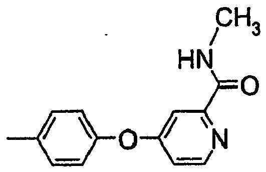 | 2.68 |
| (19) | 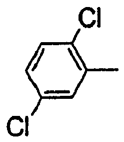 | 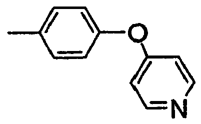 | 2.02 |
| (20) | 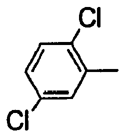 | 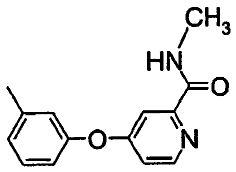 | 2.72 |
| (21) | 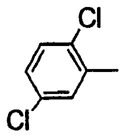 | 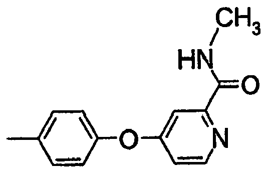 | 2.75 |
| (22) | 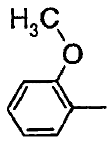 | 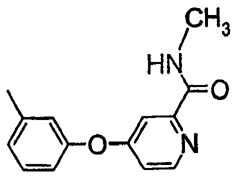 | 2.29 |
| (23) | 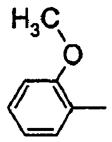 | 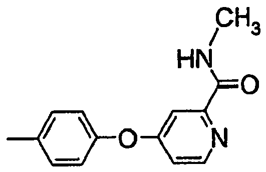 | 2.30 |
| (24) | 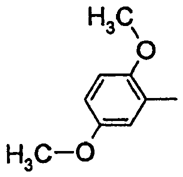 | 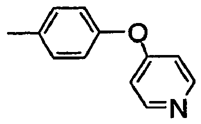 | 1.59 |
| (25) | 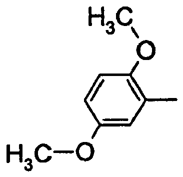 | 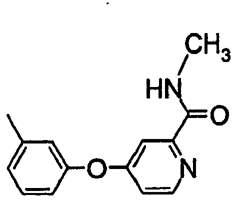 | 2.30 |
| (26) | 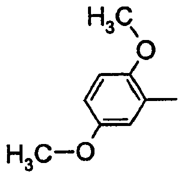 | 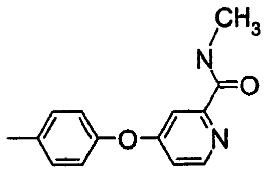 | 2.30 |
| (27) | 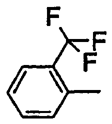 | 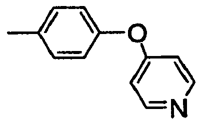 | 2.71 |
| (28) | 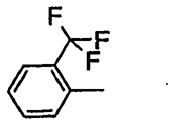 | 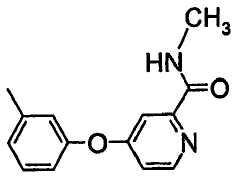 | 2.40 |
| (29) | 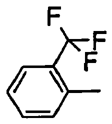 | 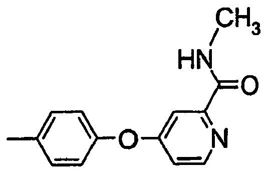 | 2.43 |
| (30) | 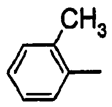 | 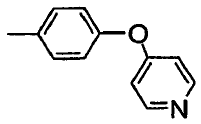 | 1.45 |
| (31) | 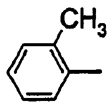 | 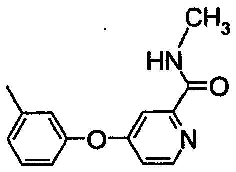 | 2.23 |
| (32) | 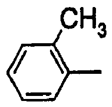 | 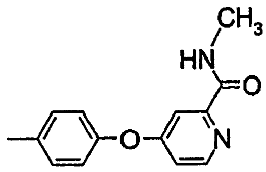 | 2.21 |
| (33) | 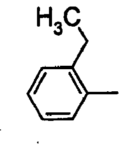 | 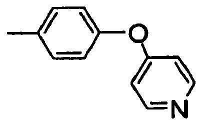 | 1.56 |
| (34) | 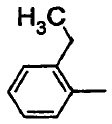 | 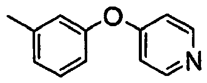 | 1.55 |
| (35) | 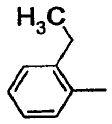 | 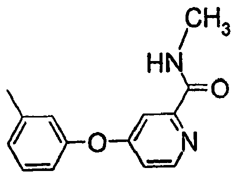 | 2.31 |
| (36) | 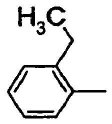 | 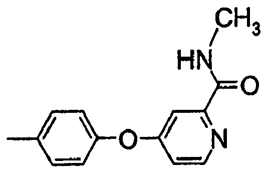 | 2.32 |
| (37) | 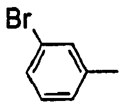 | 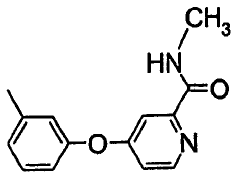 | 2.49 |
| (38) | 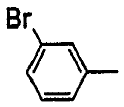 | 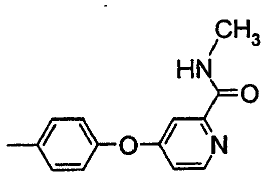 | 2.49 |
| (39) | 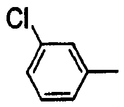 | 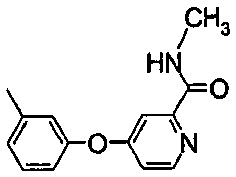 | 2.46 |
| (40) | 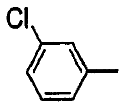 | 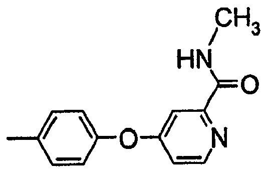 | 2.43 |
| (41) | 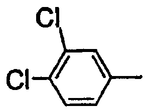 | 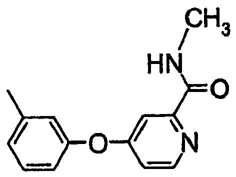 | 2.66 |
| (42) | 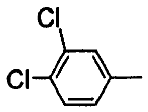 | 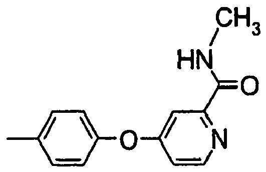 | 2.65 |
| (43) | 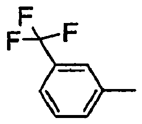 | 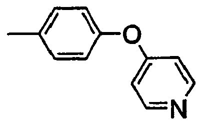 | 1.88 |
| (44) | 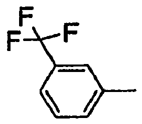 | 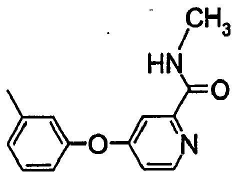 | 2.55 |
| (45) | 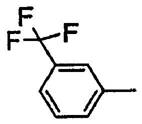 | 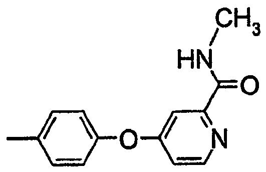 | 2.55 |
| (46) | 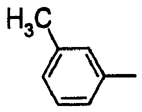 | 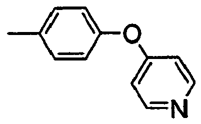 | 1.57 |
| (47) | 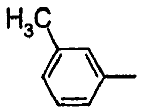 | 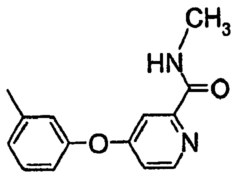 | 2.29 |
| (48) | 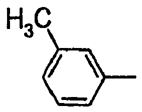 | 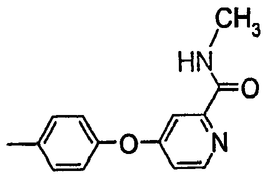 | 2.29 |
| (49) | 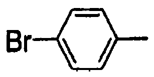 | 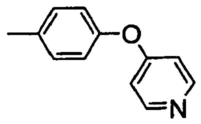 | 1.82 |
| (50) | 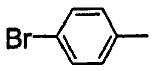 | 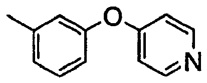 | 1.81 |
| (51) | 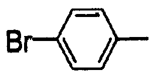 | 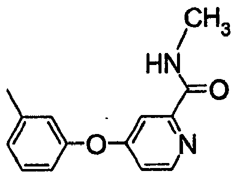 | 2.48 |
| (52) | 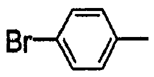 | 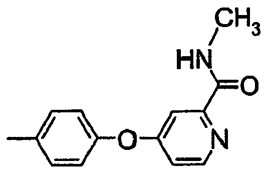 | 2.47 |
| (53) | 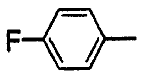 | 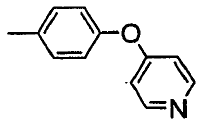 | 1.47 |
| (54) | 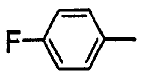 | 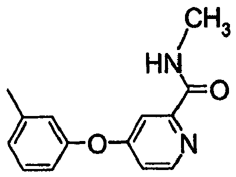 | 2.25 |
| (55) | 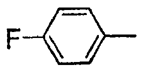 | 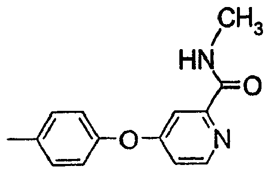 | 2.25 |
| (56) | 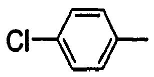 | 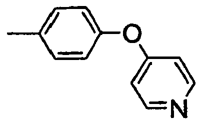 | 1.74 |
| (57) | 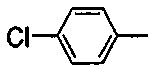 | 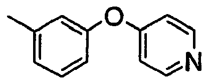 | 1.71 |
| (58) | 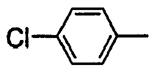 | 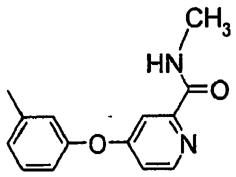 | 2.43 |
| (59) | 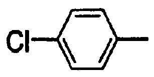 | 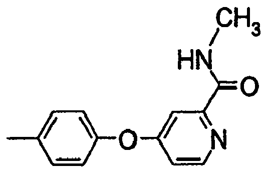 | 2.42 |
| (60) | 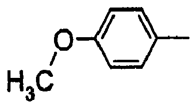 | 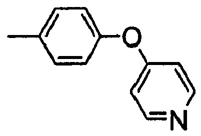 | 1.39 |
| (61) | 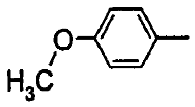 | 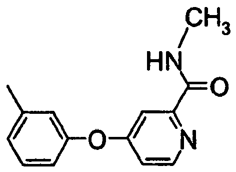 | 2.13 |
| (62) | 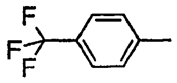 | 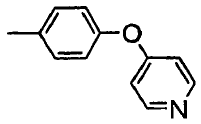 | 1.95 |
| (63) | 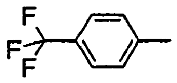 | 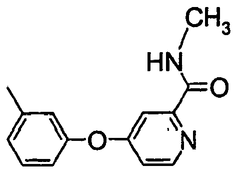 | 2.61 |
| (64) | 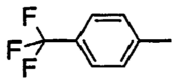 | 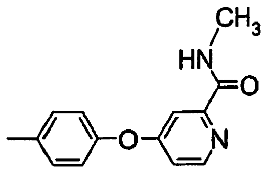 | 2.59 |
| (65) | 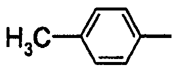 | 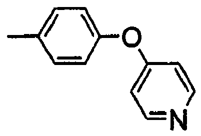 | 1.59 |
| (66) | 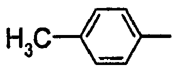 | 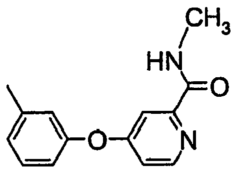 | 2.31 |
| (67) | 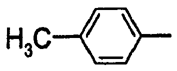 | 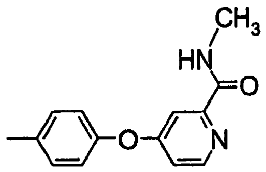 | 2.29 |
| (68) | 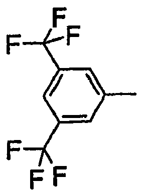 | 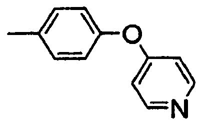 | 2.21 |
| (69) | 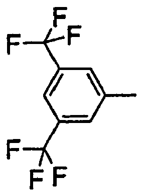 | 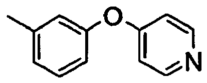 | 2.20 |
| (70) | 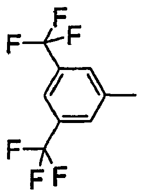 | 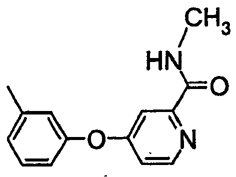 | 2.94 |
| (71) | 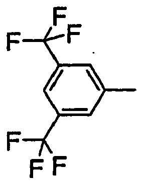 | 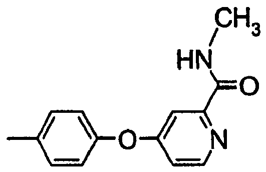 | 2.93 |
| (72) | 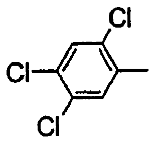 | 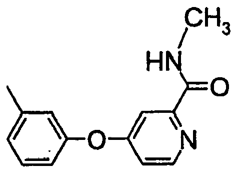 | 2.96 |
| (73) | 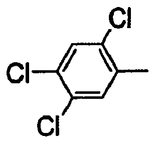 | 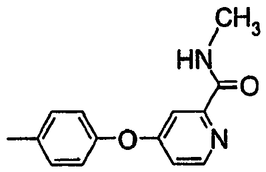 | 2.97 |
| (74) | 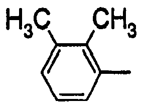 | 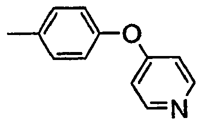 | 1.61 |
| (75) | 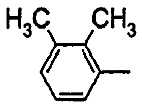 | 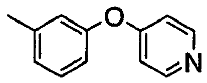 | 1.63 |
| (76) | 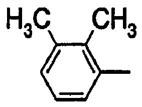 | 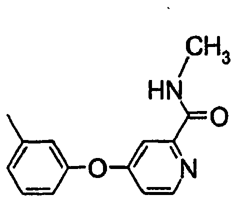 | 2.28 |
| (77) | 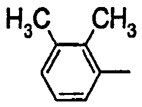 | 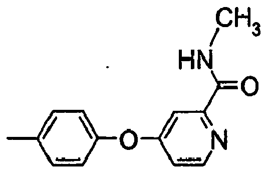 | 2.30 |
| (78) | 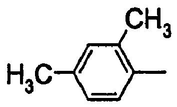 | 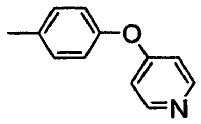 | 1.66 |
| (79) | 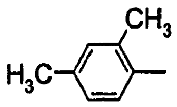 | 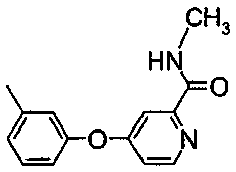 | 2.32 |
| (80) | 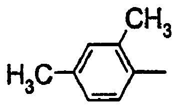 | 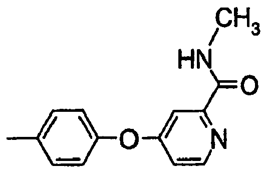 | 2.34 |
| (81) | 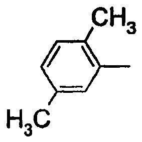 | 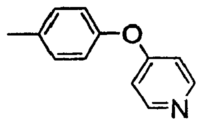 | 1.67 |
| (82) | 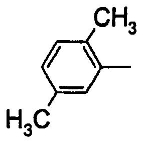 | 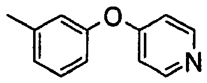 | 1.67 |
| (83) | 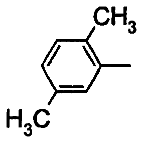 | 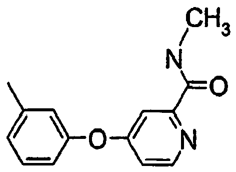 | 2.35 |
| (84) | 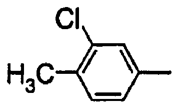 | 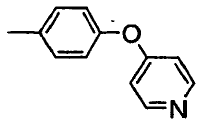 | 1.90 |
| (85) | 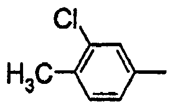 | 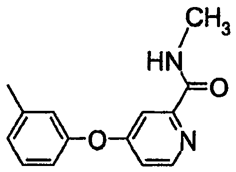 | 2.56 |
| (86) | 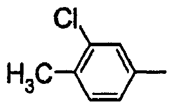 | 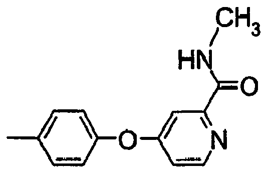 | 2.58 |
| (87) | 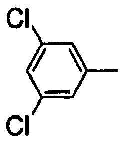 | 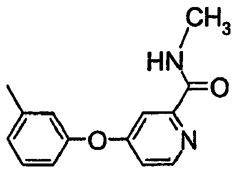 | 2.76 |
| (88) | 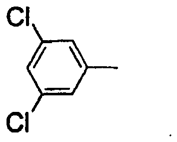 | 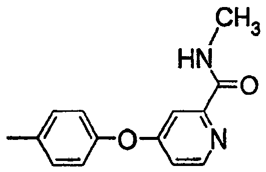 | 2.77 |
| (89) | 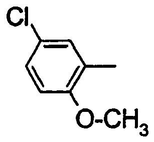 | 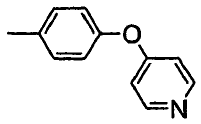 | 1.88 |
| (90) | 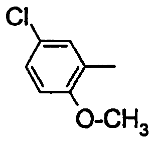 | 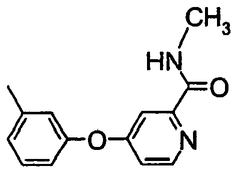 | 2.55 |
| (91) | 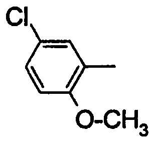 | 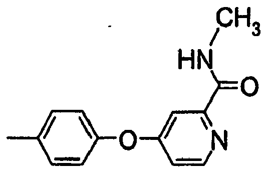 | 2.57 |
| (92) | 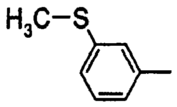 | 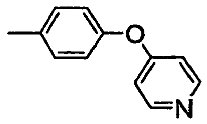 | 1.74 |
| (93) | 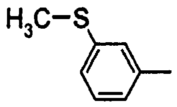 | 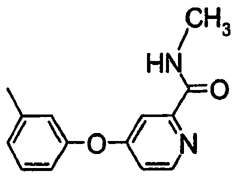 | 2.39 |
| (94) | 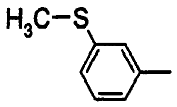 | 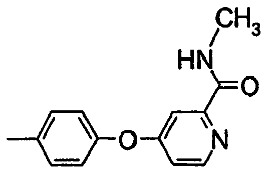 | 2.39 |
| (95) | 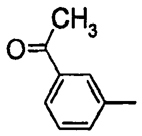 | 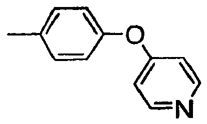 | 1.39 |
| (96) | 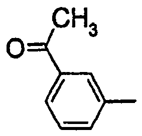 | 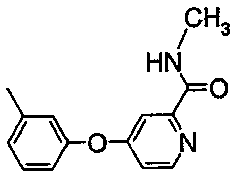 | 2.11 |
| (97) | 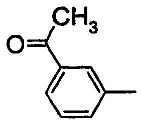 | 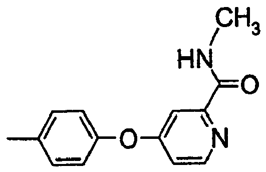 | 2.11 |
| (98) | 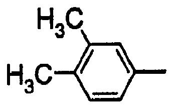 | 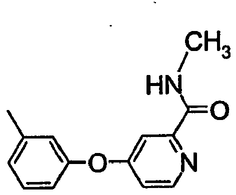 | 2.44 |
| (99) | 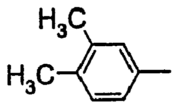 | 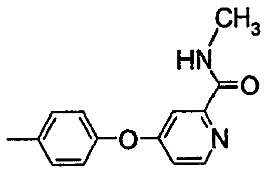 | 2.41 |
| (100) | 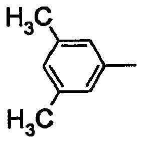 | 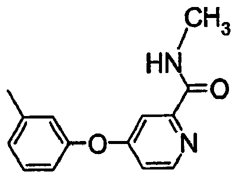 | 2.47 |
| (101) | 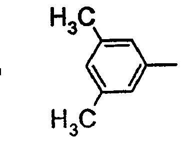 | 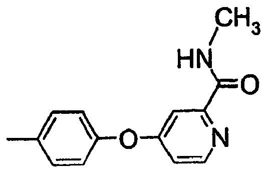 | 2.44 |
| (102) | 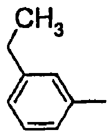 | 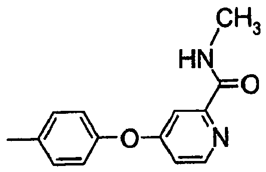 | 2.46 |
| (103) | 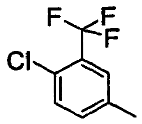 | 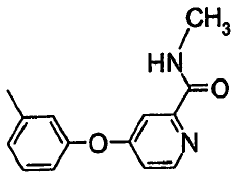 | 2.77 |
| (104) | 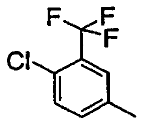 | 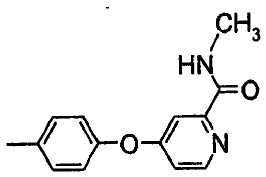 | 2.75 |
| (105) | 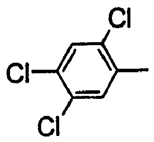 | 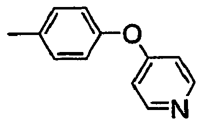 | 2.17 |
| (106) | 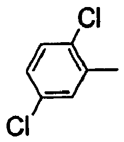 | 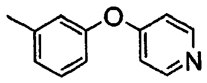 | 1.95 |
| (107) | 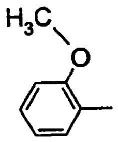 | 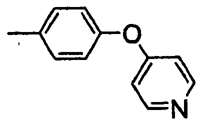 | 1.55 |
| (108) | 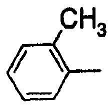 | 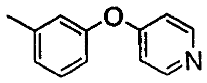 | 1.47 |
| (109) | 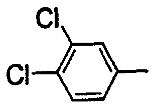 | 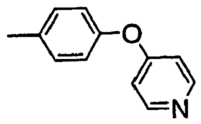 | 1.96 |
| (110) | 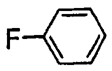 | 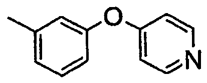 | 1.51 |
| (111) | 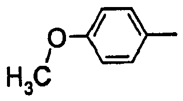 | 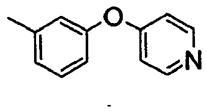 | 1.43 |
| (112) | 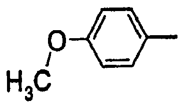 | 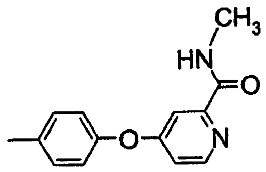 | 2.14 |
| (113) | 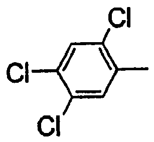 | 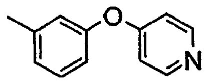 | 2.17 |
| (114) | 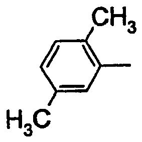 | 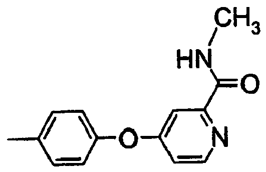 | 2.37 |
| (115) | 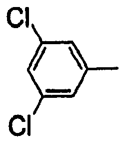 | 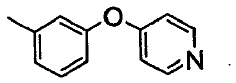 | 2.05 |
| (116) | 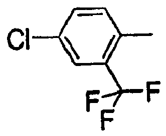 | 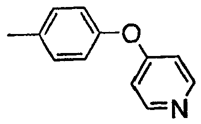 | 2.03 |
| (117) | 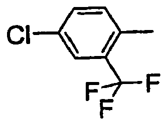 | 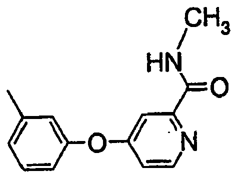 | 2.67 |
| (118) | 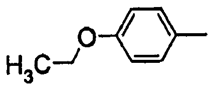 | 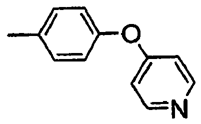 | 1.60 |
| (119) | 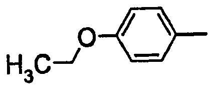 | 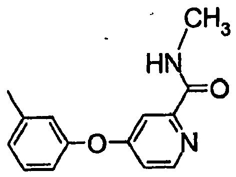 | 2.29 |
| (120) | 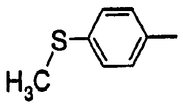 | 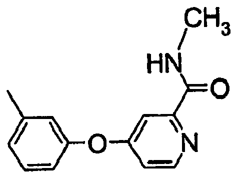 | 2.39 |
| (121) | 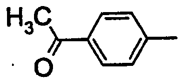 | 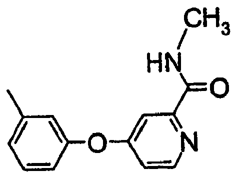 | 2.11 |
| (122) | 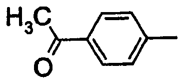 | 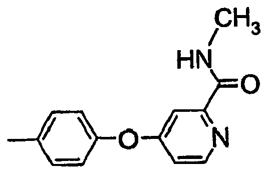 | 2.11 |
| (123) | 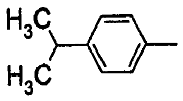 | 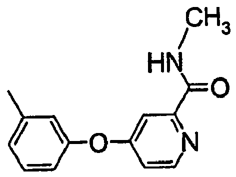 | 2.62 |
| (124) | 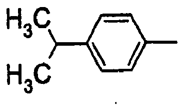 | 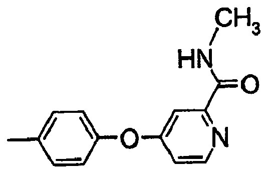 | 2.61 |
| (125) | 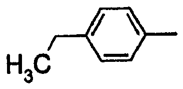 | 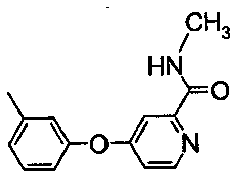 | 2.47 |
| (126) | 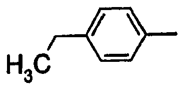 | 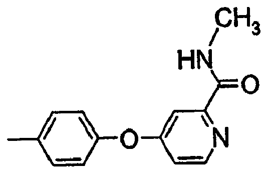 | 2.49 |
| (127) | 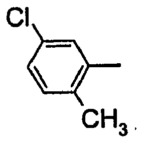 | 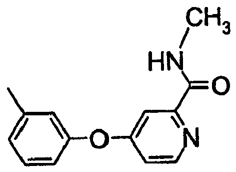 | 2.52 |
| (128) | 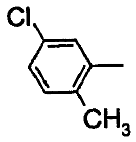 | 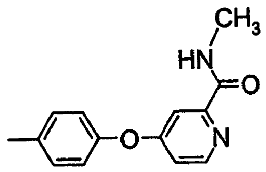 | 2.52 |
| (129) | 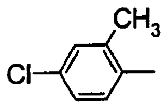 | 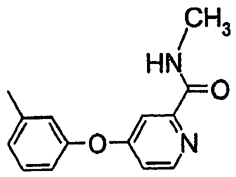 | 2.47 |
| (130) | 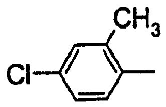 | 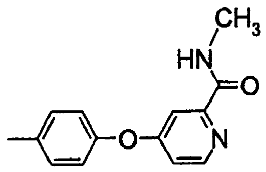 | 2.48 |
| (131) 2.62 | 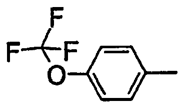 | 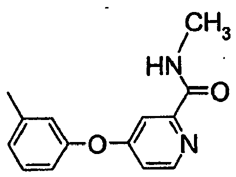 | 2.62 |
| (132) 2 | 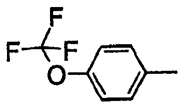 | 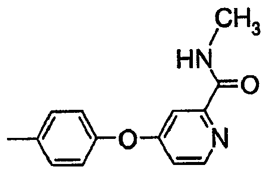 | 2.62 |
| (133) | 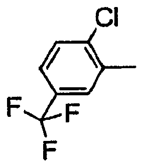 | 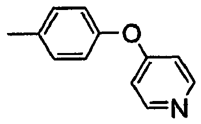 | 2.09 |
| (134) | 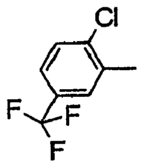 | 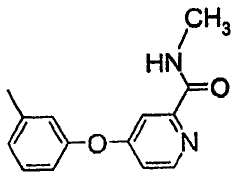 | 2.81 |
| (135) | 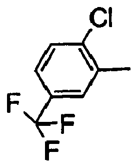 | 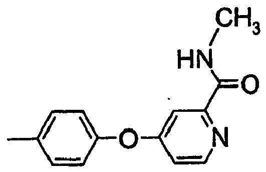 | 2.79 |
| (136) | 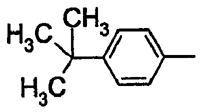 | 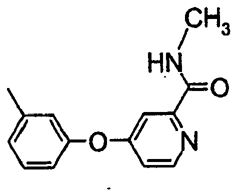 | 2.71 |
| (137) | 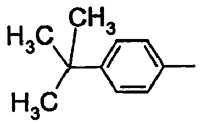 | 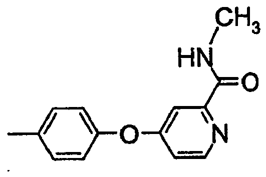 | 2.71 |
| (138) | 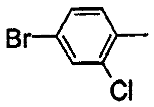 | 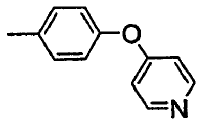 | 2.01 |
| (139) | 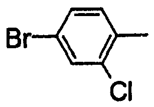 | 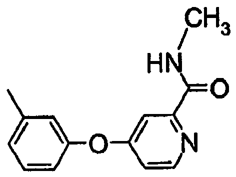 | 2.74 |
| (140) | 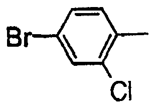 | 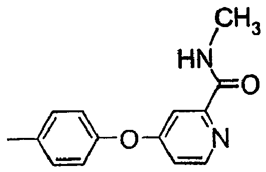 | 2.73 |
| (141) | 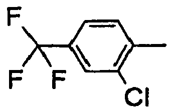 | 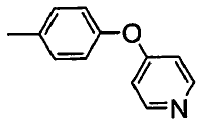 | 2.13 |
| (142) | 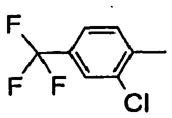 | 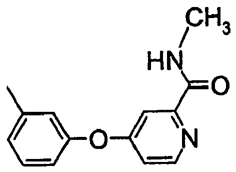 | 2.84 |
| (143) | 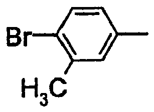 | 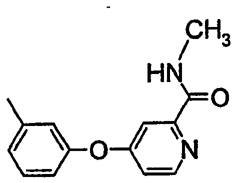 | 2.62 |
| (144) | 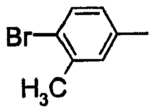 | 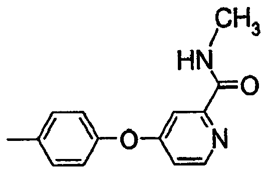 | 2.61 |
| (145) | 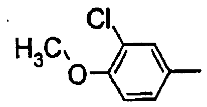 | 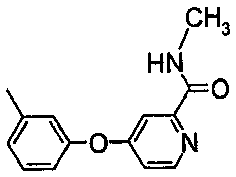 | 2.32 |
| (146) | 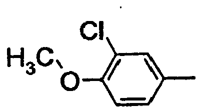 | 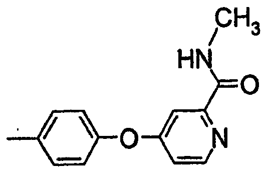 | 2.31 |
| (147) | 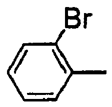 | 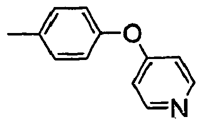 | 1.69 |
| (148) | 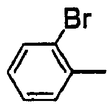 | 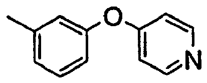 | 1.69 |
| (149) | 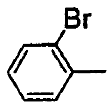 | 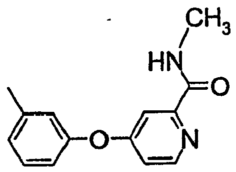 | 2.40 |
| (150) | 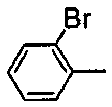 | 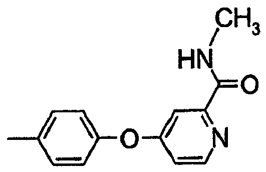 | 2.43 |
| (151) | 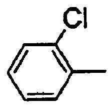 | 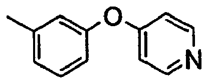 | 1.67 |
| (152) | 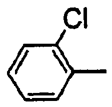 | 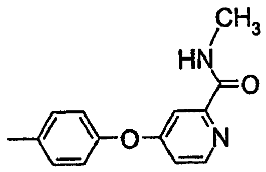 | 2.37 |
| (153) | 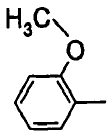 | 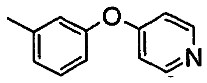 | 1.57 |
| (154) | 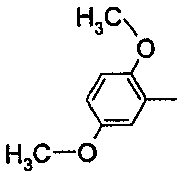 | 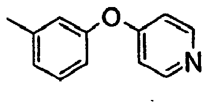 | 1.57 |
| (155) | 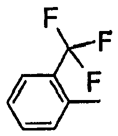 | 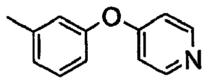 | 1.70 |
| (156) | 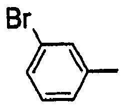 | 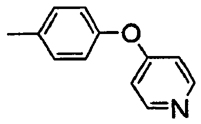 | 1.81 |
| (157) | 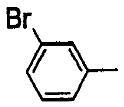 | 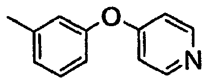 | 1.78 |
| (158) | 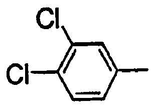 | 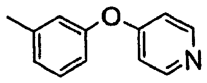 | 1.97 |
| (159) | 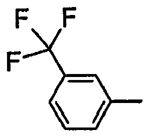 | 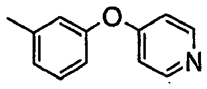 | 1.94 |
| (160) | 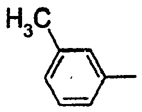 | 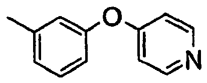 | 1.57 |
| (161) | 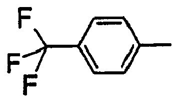 | 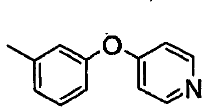 | 1.91 |
| (162) | 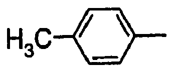 | 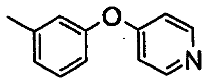 | 1.63 |
| (163) | 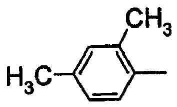 | 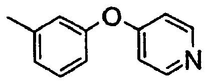 | 1.62 |
| (164) | 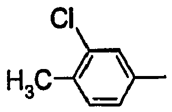 | 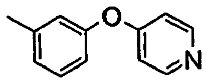 | 1.93 |
| (165) | 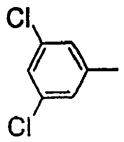 | 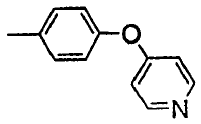 | 2.05 |
| (166) | 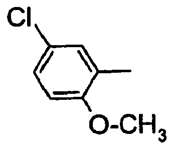 | 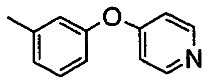 | 1.87 |
| (167) | 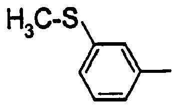 | 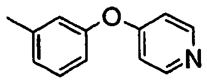 | 1.72 |
| (168) | 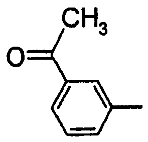 | 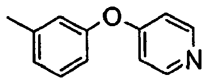 | 1.45 |
| (169) | 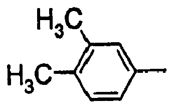 | 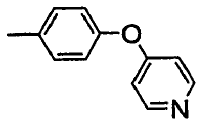 | 1.74 |
| (170) | 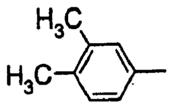 | 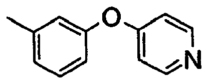 | 1.74 |
| (171) | 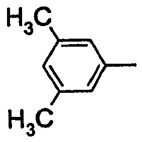 | 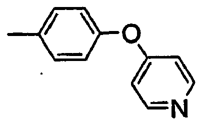 | 1.79 |
| (172) | 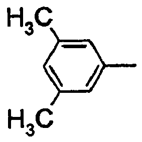 | 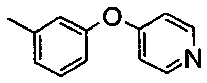 | 1.81 |
| (173) | 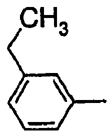 | 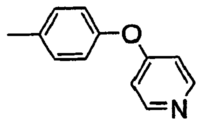 | 1.79 |
| (174) | 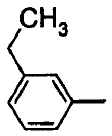 | 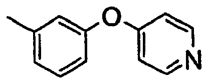 | 1.81 |
| (175) | 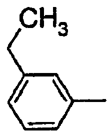 | 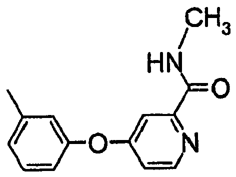 | 2.45 |
| (176) | 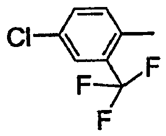 | 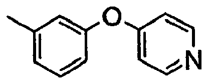 | 1.95 |
| (177) | 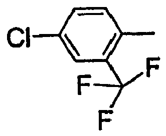 | 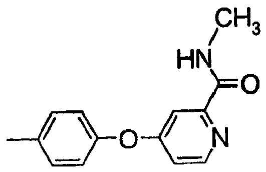 | 2.69 |
| (178) | 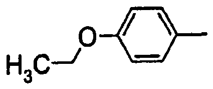 | 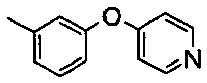 | 1.57 |
| (179) | 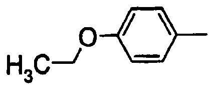 | 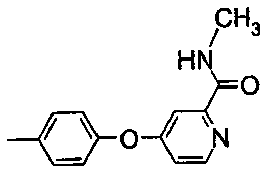 | 2.30 |
| (180) | 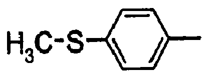 | 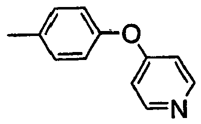 | 1.72 |
| (181) | 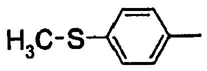 | 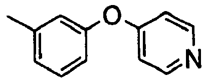 | 1.71 |
| (182) | 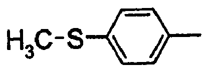 | 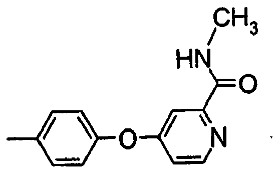 | 2.37 |
| (183) | 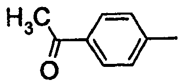 | 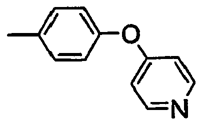 | 1.45 |
| (184) | 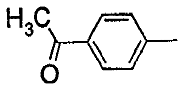 | 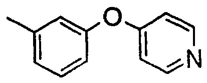 | 1.43 |
| (185) | 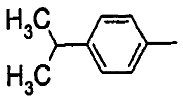 | 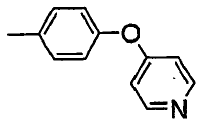 | 1.94 |
| (186) | 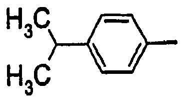 | 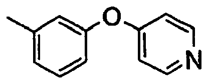 | 1.97 |
| (187) | 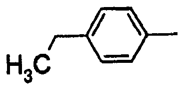 | 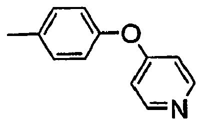 | 1.82 |
| (188) | 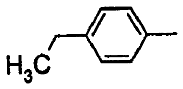 | 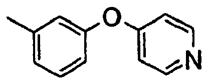 | 1.81 |
| (189) | 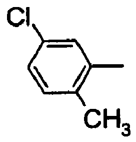 | 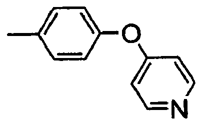 | 1.79 |
| (190) | 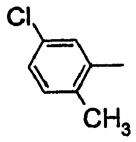 | 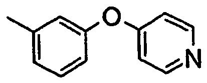 | 1.81 |
| (191) | 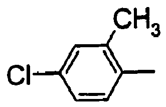 | 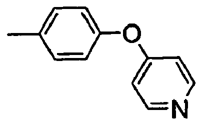 | 1.83 |
| (192) | 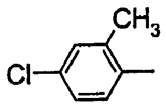 | 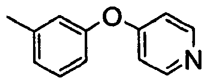 | 1.79 |
| (193) | 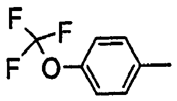 | 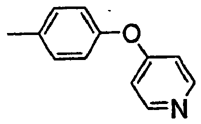 | 1.98 |
| (194) | 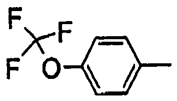 | 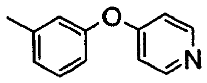 | 1.97 |
| (195) | 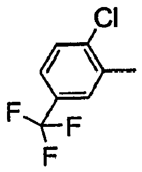 | 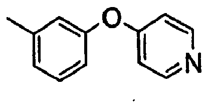 | 2.09 |
| (196) | 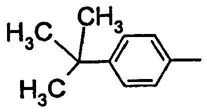 | 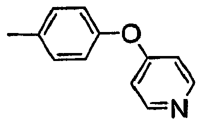 | 2.05 |
| (197) | 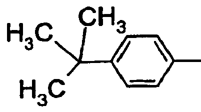 | 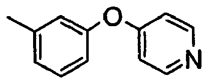 | 2.06 |
| (198) | 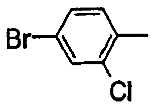 | 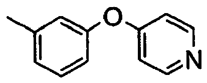 | 1.96 |
| (199) | 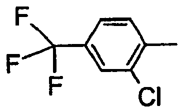 | 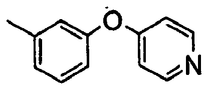 | 2.11 |
| (200) | 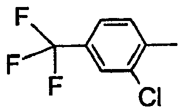 | 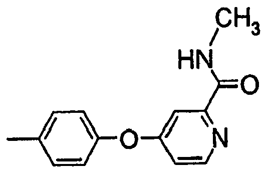 | 2.85 |
| (201) | 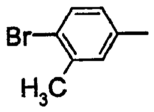 | 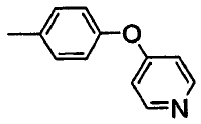 | 1.97 |
| (202) | 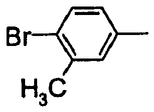 | 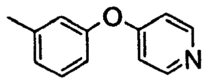 | 1.95 |
| (203) | 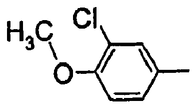 | 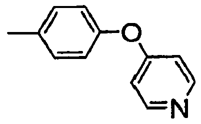 | 1.64 |
| (204) | 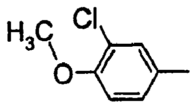 | 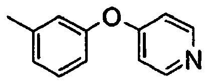 | 1.67 |
| (205) | 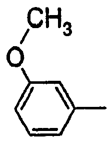 | 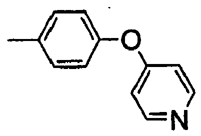 | 1.43 |
| (206) | 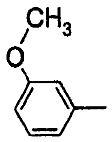 | 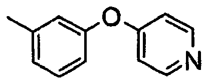 | 1.49 |
| (207) | 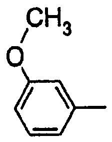 | 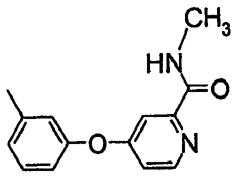 | 2.19 |
| (208) | 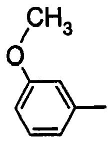 | 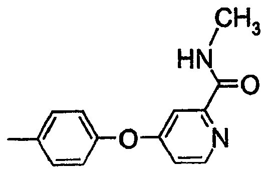 | 2.20 |
| (209) | 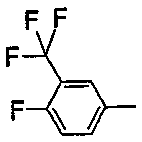 | 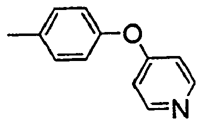 | 1.93 |
| (210) | 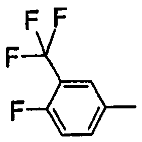 | 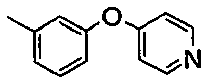 | 1.93 |
| (211) | 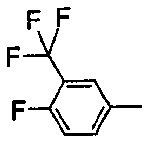 | 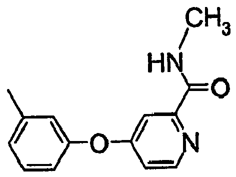 | 2.61 |
| (212) | 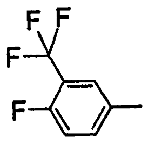 | 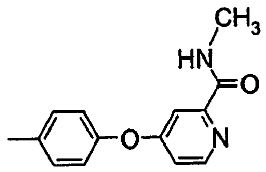 | 2.63 |
| (213) | 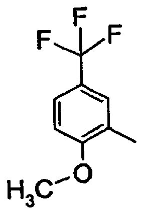 | 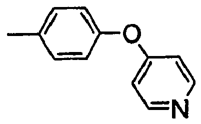 | 2.01 |
| (214) | 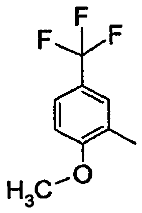 | 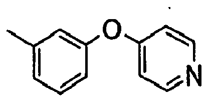 | 1.99 |
| (215) | 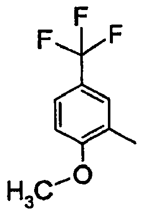 | 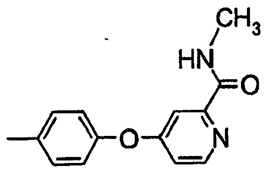 | 2.63 |
| (216) | 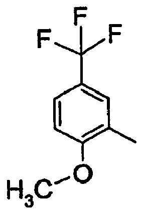 | 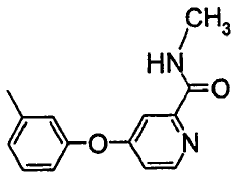 | 2.65 |
| (217) | 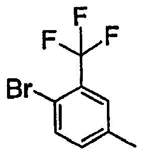 | 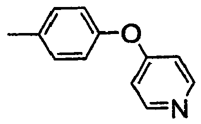 | 2.15 |
| (218) | 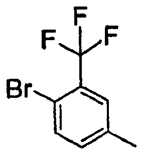 | 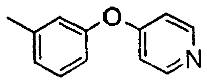 | 2.12 |
| (219) | 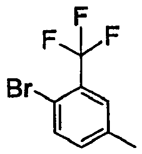 | 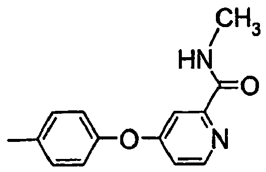 | 2.76 |
| (220) | 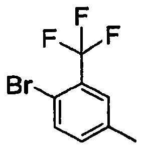 | 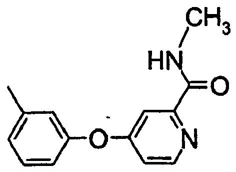 | 2.80 |
| (221) | 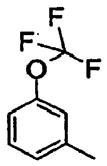 | 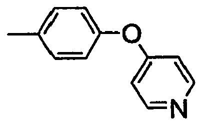 | 1.99 |
| (222) | 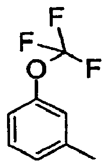 | 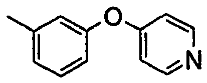 | 1.96 |
| (223) | 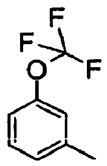 | 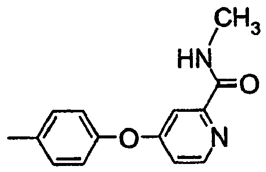 | 2.65 |
| (224) | 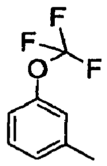 | 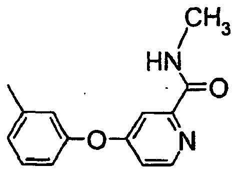 | 2.64 |

The present invention further relates to compounds (225) to (449) of formula A-NH-CO-NH-(CHMe)-B, wherein Me is a methyl group and A and B are as given in the table below:

| | A | B | Rt |
|---|---|---|---|
| (225) | 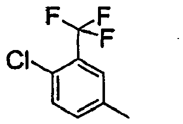 | 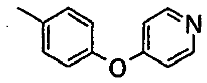 | |
| (226) | 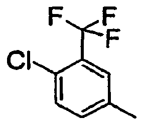 | 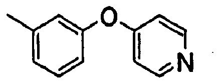 | |
| (227) | 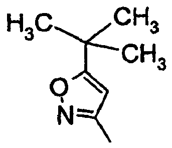 | 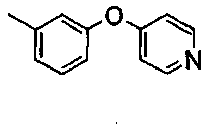 | |
| (228) | 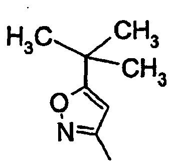 | 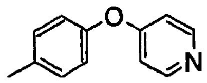 | |
| (229) | 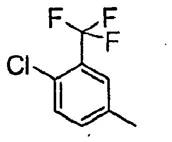 | 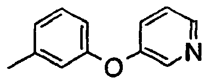 | |
| (230) | 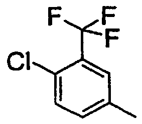 | 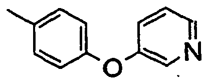 | |
| (231) | 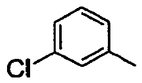 | 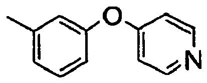 | |
| (232) | 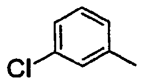 | 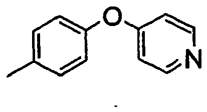 | |
| (233) | 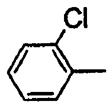 | 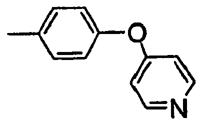 | |
| (234) | 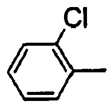 | 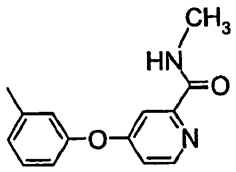 | |
| (235) | 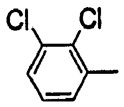 | 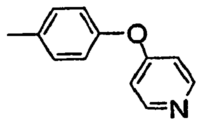 | |
| (236) | 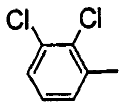 | 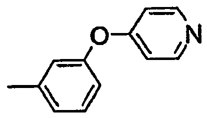 | |
| (237) | 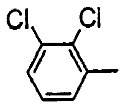 | 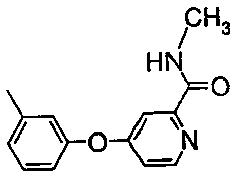 | |
| (238) | 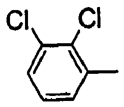 | 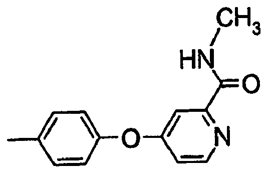 | |
| (239) | 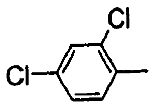 | 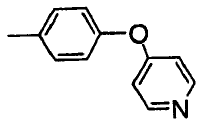 | |
| (240) | 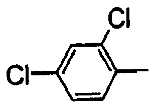 | 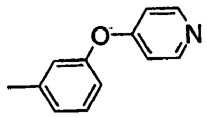 | |
| (241) | 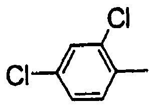 | 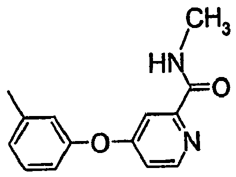 | |
| (242) | 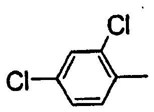 | 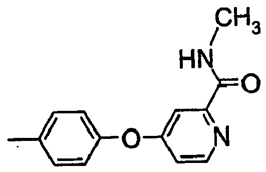 | |
| (243) | 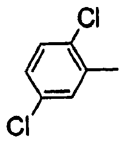 | 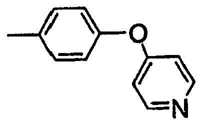 | |
| (244) | 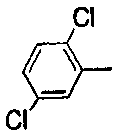 | 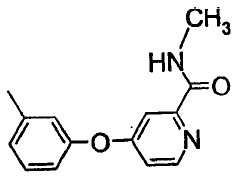 | |
| (245) | 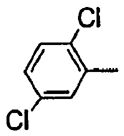 | 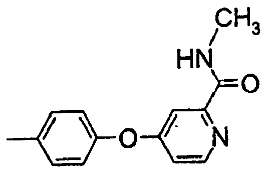 | |
| (246) | 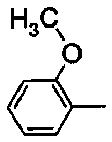 | 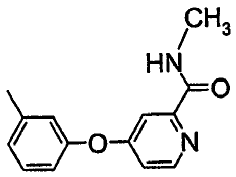 | |
| (247) | 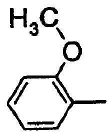 | 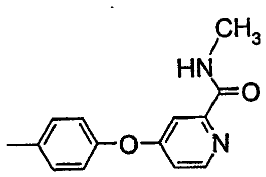 | |
| (248) | 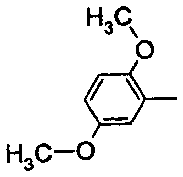 | 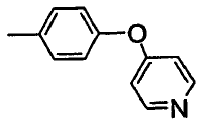 | |
| (249) | 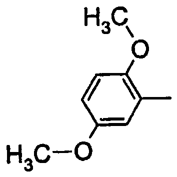 | 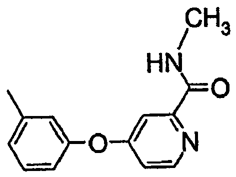 | |
| (250) | 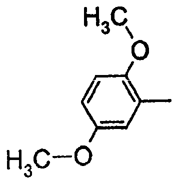 | 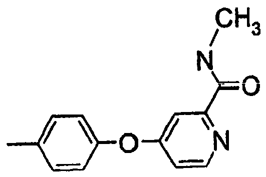 | |
| (251) | 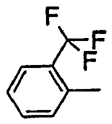 | 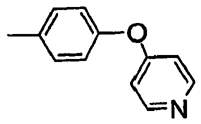 | |
| (252) | 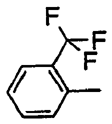 | 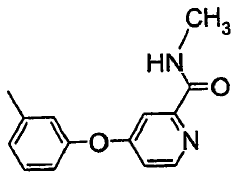 | |
| (253) | 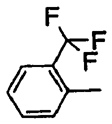 | 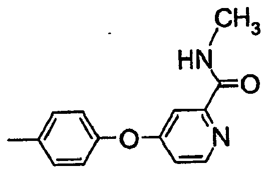 | |
| (254) | 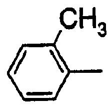 | 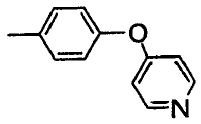 | |
| (255) | 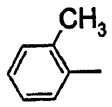 | 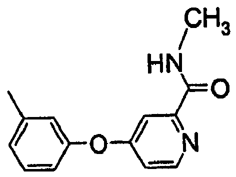 | |
| (256) | 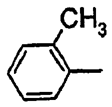 | 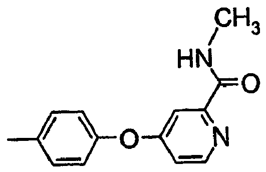 | |
| (257) | 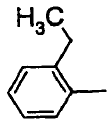 | 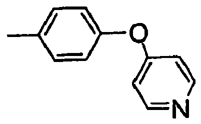 | |
| (258) | 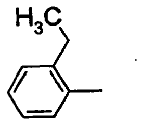 | 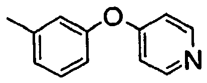 | |
| (259) | 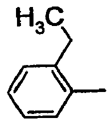 | 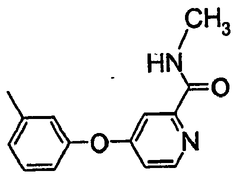 | |
| (260) | 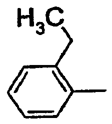 | 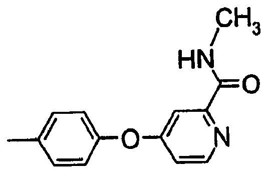 | |
| (261) | 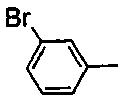 | 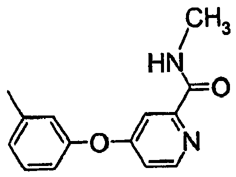 | |
| (262) | 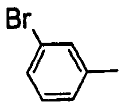 | 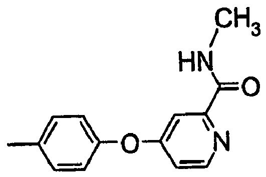 | |
| (263) | 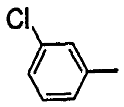 | 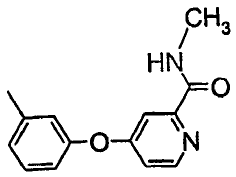 | |
| (264) | 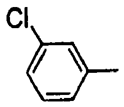 | 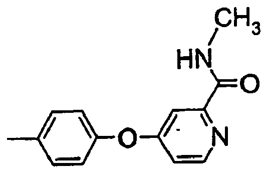 | |
| (265) | 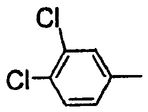 | 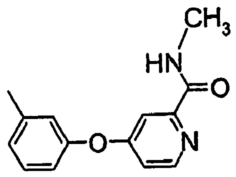 | |
| (266) | 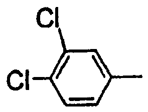 | 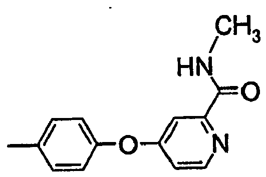 | 2.70 |
| (267) | 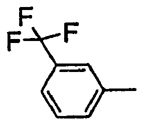 | 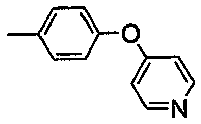 | |
| (268) | 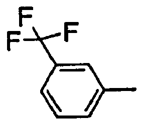 | 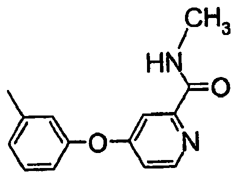 | |
| (269) | 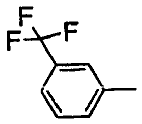 | 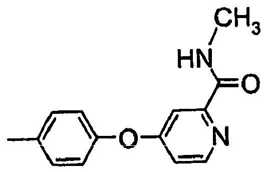 | 2.64 |
| (270) | 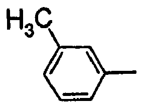 | 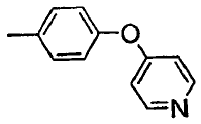 | |
| (271) | 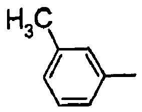 | 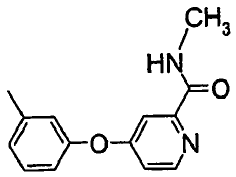 | |
| (272) | 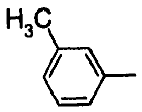 | 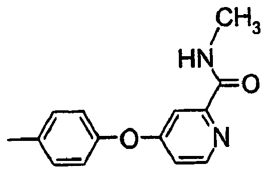 | |
| (273) | 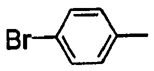 | 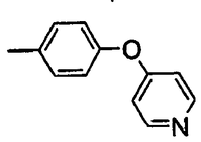 | |
| (274) | 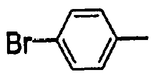 | 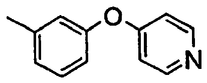 | |
| (275) | 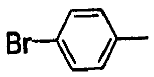 | 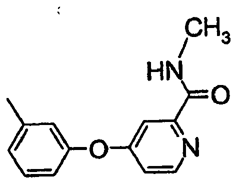 | |
| (276) | 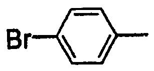 | 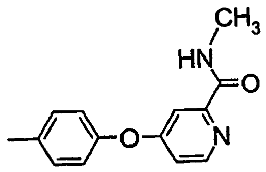 | |
| (277) | 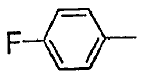 | 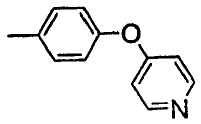 | |
| (278) | 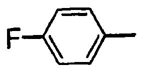 | 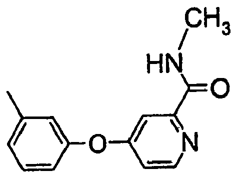 | |
| (279) | 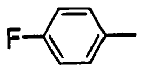 | 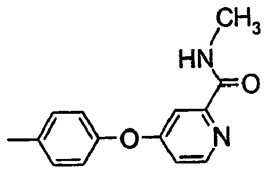 | |
| (280) | 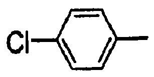 | 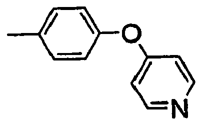 | |
| (281) | 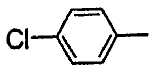 | 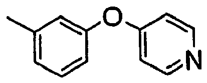 | |
| (282) | 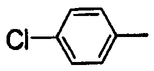 | 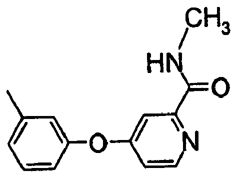 | |
| (283) | 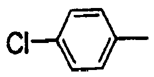 | 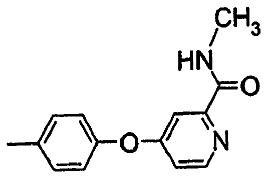 | |
| (284) | 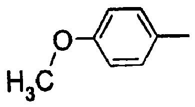 | 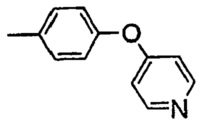 | |
| (285) | 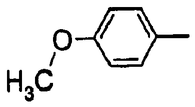 | 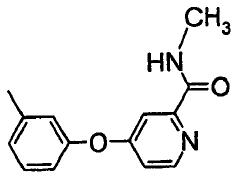 | |
| (286) | 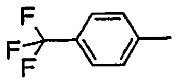 | 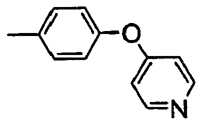 | |
| (287) | 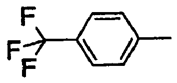 | 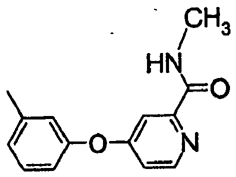 | |
| (288) | 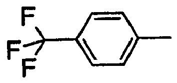 | 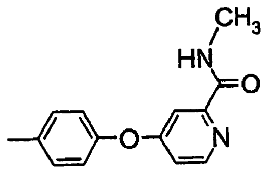 | 2.65 |
| (289) | 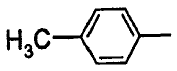 | 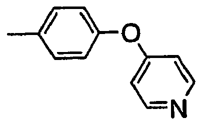 | |
| (290) | 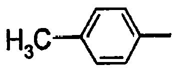 | 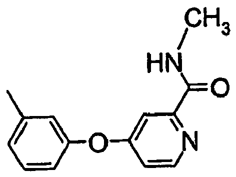 | |
| (291) | 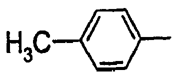 | 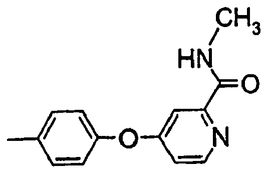 | |
| (292) | 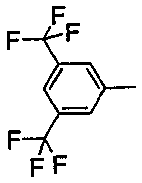 | 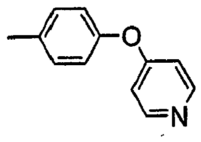 | |
| (293) | 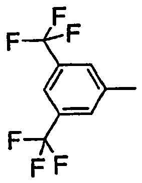 | 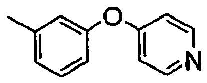 | |
| (294) | 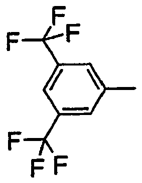 | 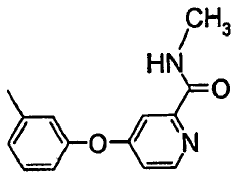 | |
| (295) | 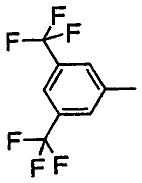 | 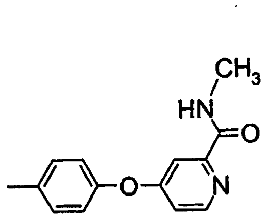 | |
| (296) | 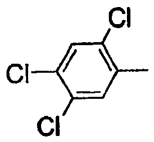 | 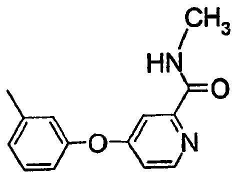 | |
| (297) | 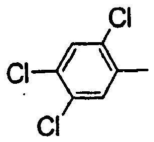 | 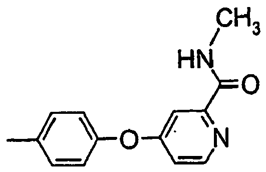 | 2.99 |
| (298) | 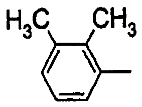 | 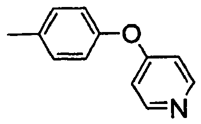 | |
| (299) | 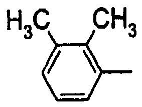 | 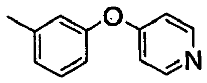 | |
| (300) | 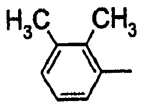 | 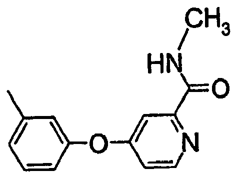 | |
| (301) | 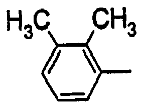 | 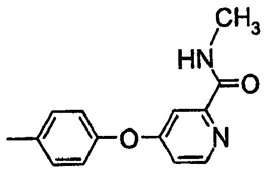 | |
| (302) | 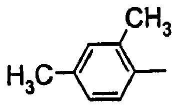 | 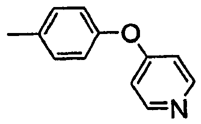 | |
| (303) | 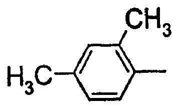 | 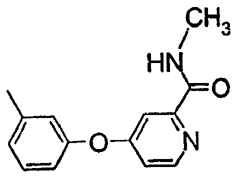 | |
| (304) | 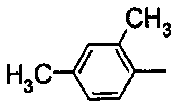 | 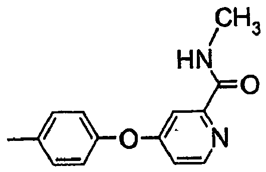 | |
| (305) | 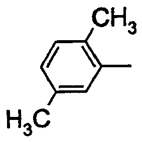 | 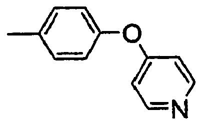 | |
| (306) | 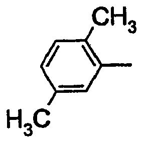 | 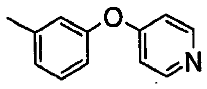 | |
| (307) | 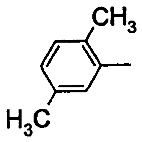 | 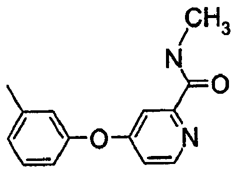 | |
| (308) | 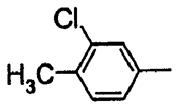 | 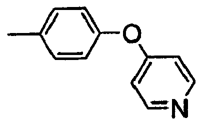 | |
| (309) | 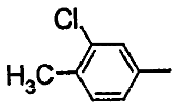 | 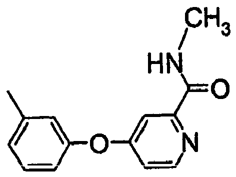 | |
| (310) | 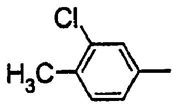 | 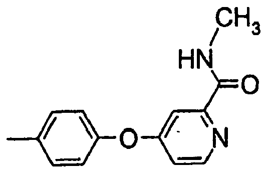 | 2.61 |
| (311) | 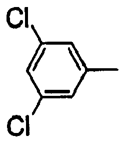 | 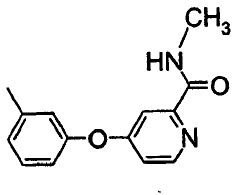 | |
| (312) | 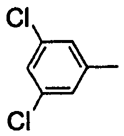 | 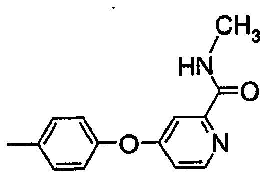 | |
| (313) | 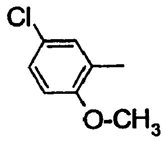 | 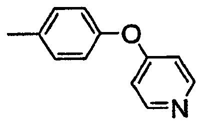 | |
| (314) | 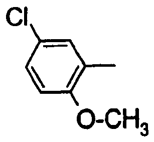 | 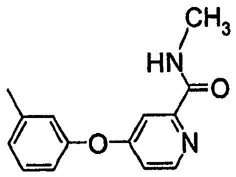 | |
| (315) | 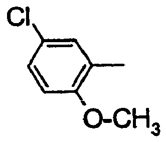 | 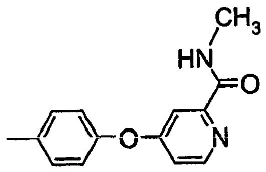 | 2.61 |
| (316) | 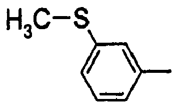 | 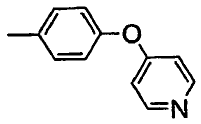 | |
| (317) | 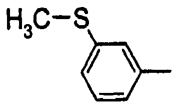 | 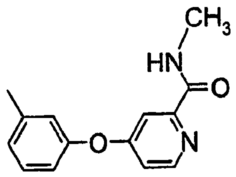 | |
| (318) | 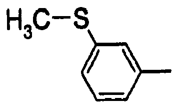 | 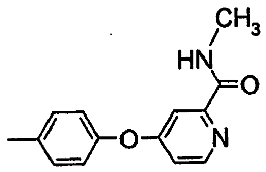 | |
| (319) | 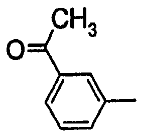 | 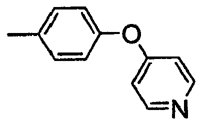 | |
| (320) | 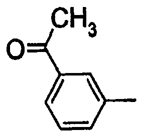 | 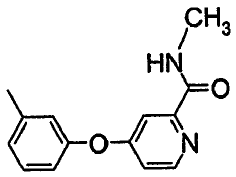 | |
| (321) | 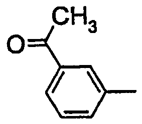 | 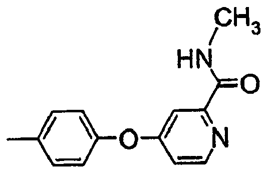 | |
| (322) | 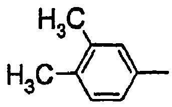 | 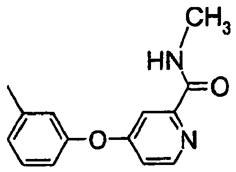 | |
| (323) | 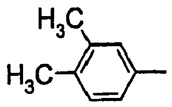 | 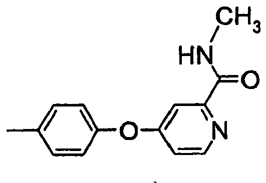 | |
| (324) | 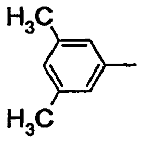 | 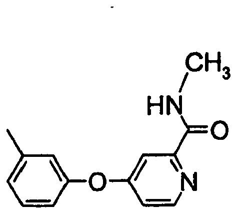 | |
| (325) | 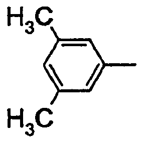 | 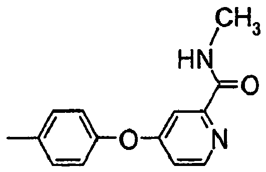 | |
| (326) | 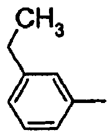 | 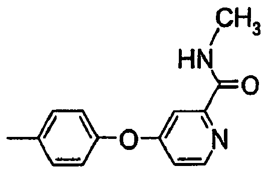 | |
| (327) | 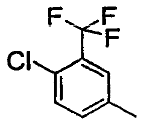 | 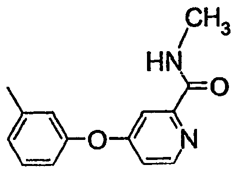 | |
| (328) | 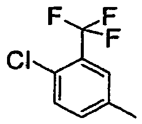 | 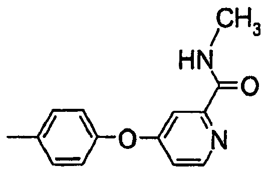 | 2.83 |
| (329) | 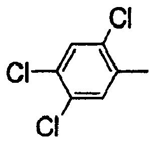 | 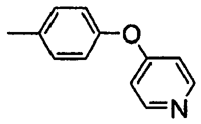 | |
| (330) | 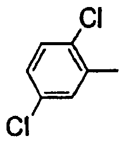 | 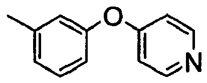 | |
| (331) | 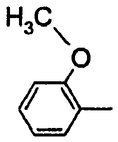 | 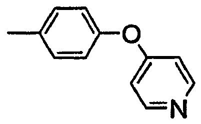 | |
| (332) | 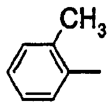 | 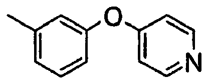 | |
| (333) | 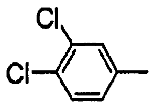 | 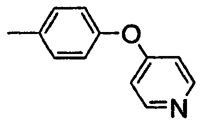 | |
| (334) | 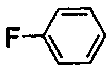 | 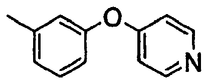 | |
| (335) | 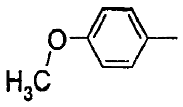 | 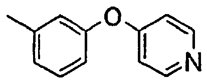 | |
| (336) | 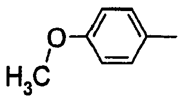 | 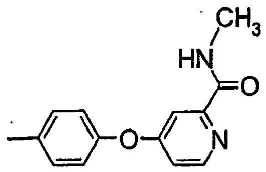 | |
| (337) | 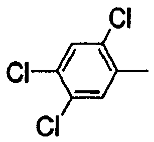 | 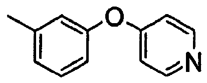 | |
| (338) | 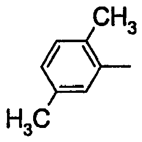 | 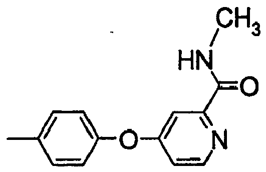 | |
| (339) | 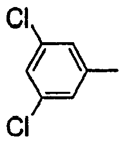 | 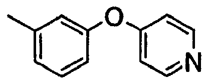 | |
| (340) | 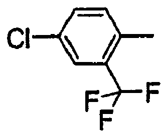 | 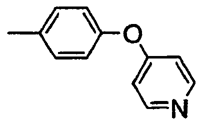 | |
| (341) | 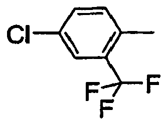 | 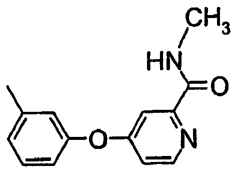 | |
| (342) | 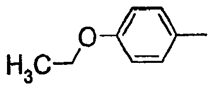 | 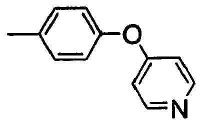 | |
| (343) | 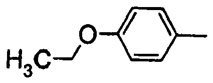 | 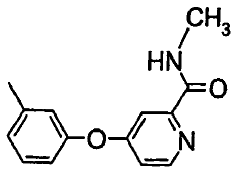 | |
| (344) | 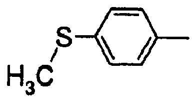 | 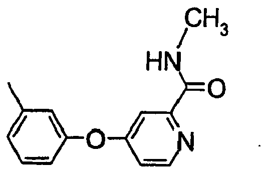 | |
| (345) | 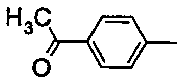 | 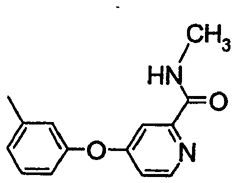 | |
| (346) | 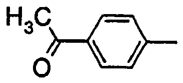 | 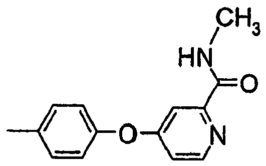 | |
| (347) | 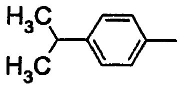 | 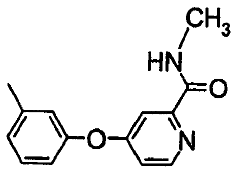 | |
| (348) | 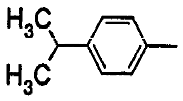 | 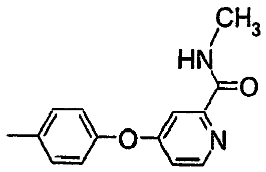 | |
| (349) | 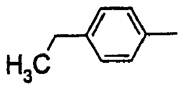 | 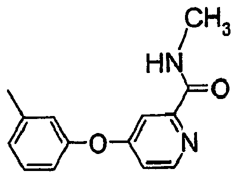 | |
| (350) | 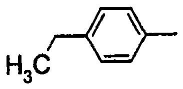 | 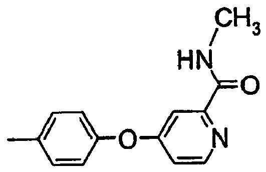 | |
| (351) | 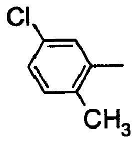 | 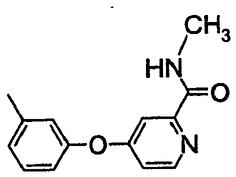 | |
| (352) | 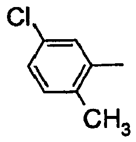 | 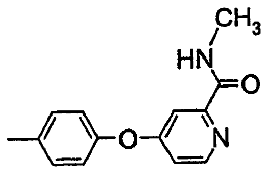 | |
| (353) | 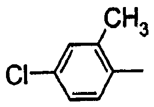 | 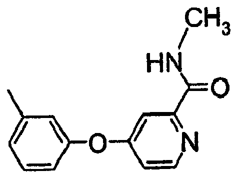 | |
| (354) | 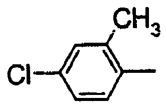 | 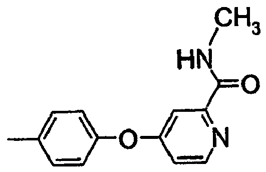 | |
| (355) 2.62 | 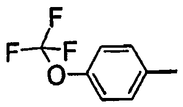 | 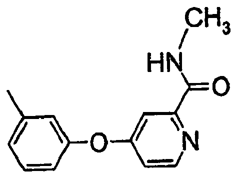 | |
| (356) 2 | 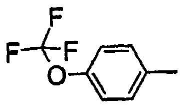 | 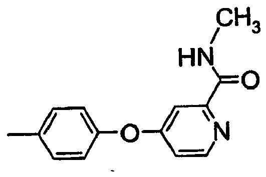 | |
| (357) | 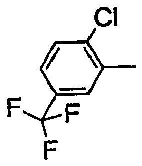 | 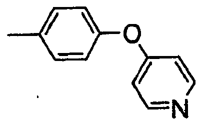 | |
| (358) | 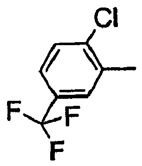 | 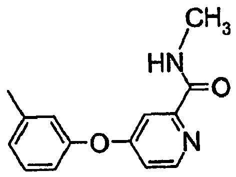 | |
| (359) | 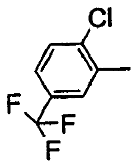 | 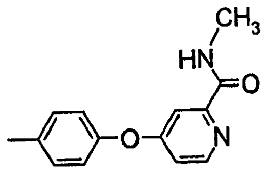 | 2.86 |
| (360) | 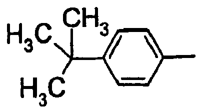 | 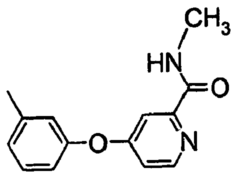 | |
| (361) | 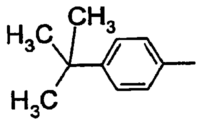 | 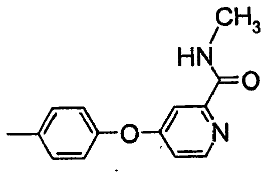 | |
| (362) | 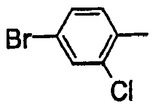 | 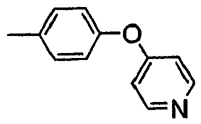 | |
| (363) | 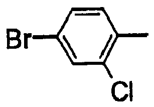 | 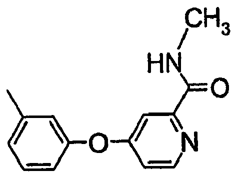 | |
| (364) | 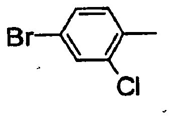 | 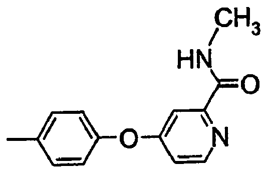 | |
| (365) | 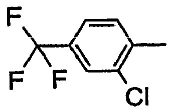 | 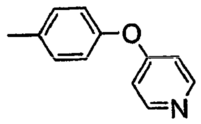 | |
| (366) | 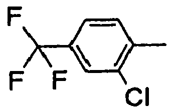 | 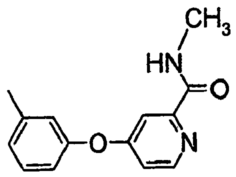 | |
| (367) | 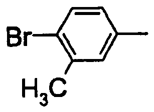 | 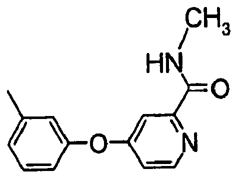 | |
| (368) | 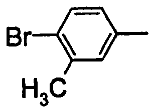 | 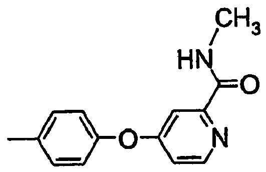 | |
| (369) | 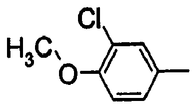 | 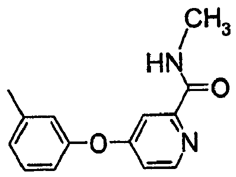 | |
| (370) | 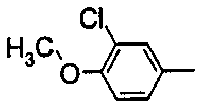 | 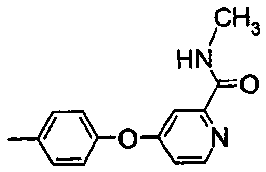 | |
| (371) | 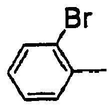 | 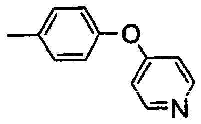 | |
| (372) | 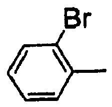 | 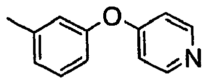 | |
| (373) | 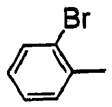 | 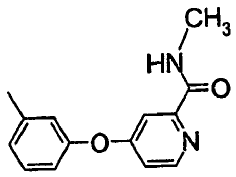 | |
| (374) | 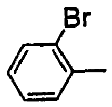 | 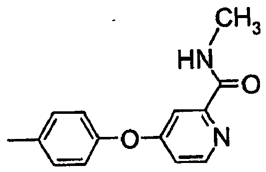 | |
| (375) | 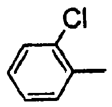 | 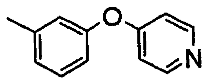 | |
| (376) | 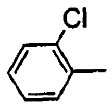 | 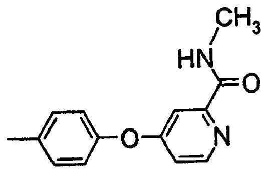 | |
| (377) | 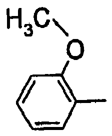 | 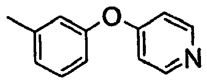 | |
| (378) | 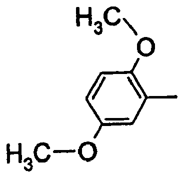 | 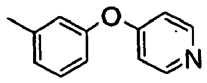 | |
| (379) | 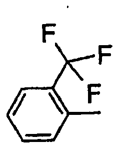 | 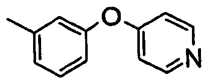 | |
| (380) | 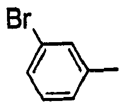 | 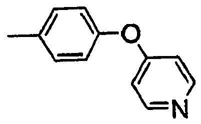 | |
| (381) | 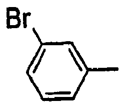 | 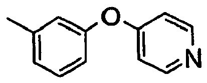 | |
| (382) | 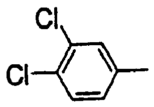 | 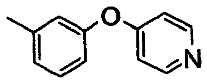 | |
| (383) | 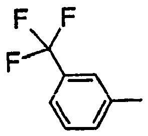 | 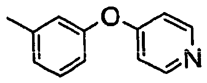 | |
| (384) | 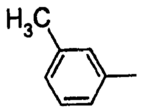 | 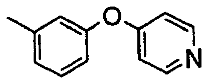 | |
| (385) | 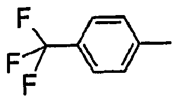 | 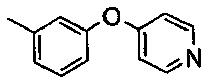 | |
| (386) | 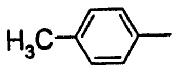 | 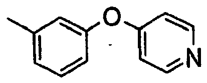 | |
| (387) | 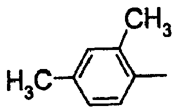 | 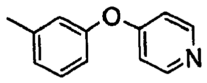 | |
| (388) | 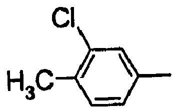 | 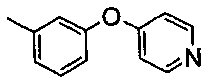 | |
| (389) | 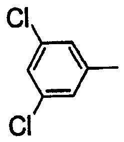 | 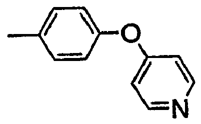 | |
| (390) | 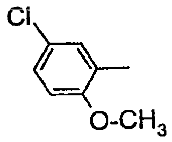 | 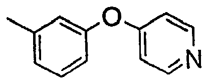 | |
| (391) | 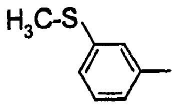 | 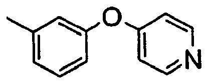 | |
| (392) | 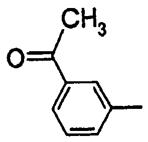 | 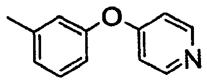 | |
| (393) | 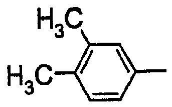 | 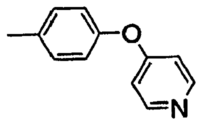 | |
| (394) | 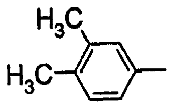 | 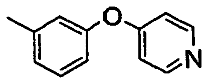 | |
| (395) | 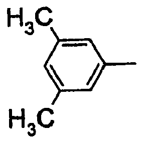 | 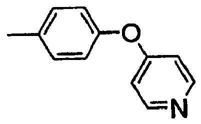 | |
| (396) | 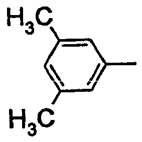 | 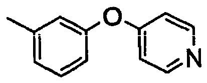 | |
| (397) | 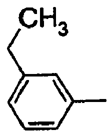 | 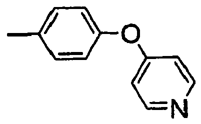 | |
| (398) | 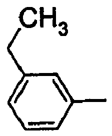 | 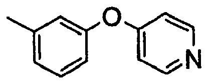 | |
| (399) | 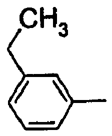 | 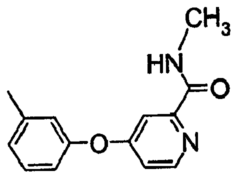 | |
| (400) | 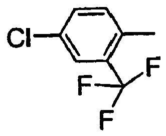 | 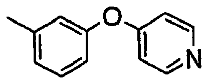 | |
| (401) | 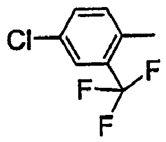 | 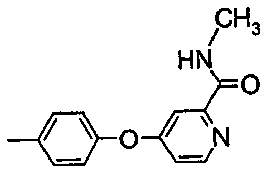 | |
| (402) | 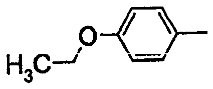 | 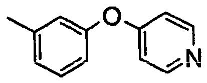 | |
| (403) | 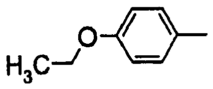 | 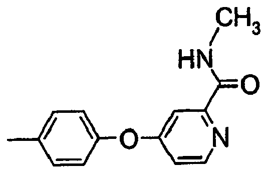 | |
| (404) | 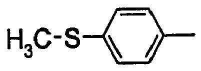 | 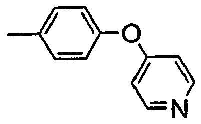 | |
| (405) | 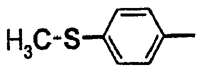 | 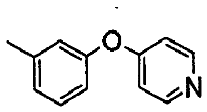 | |
| (406) | 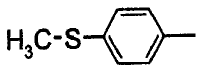 | 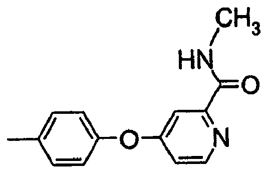 | |
| (407) | 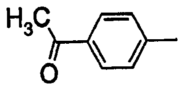 | 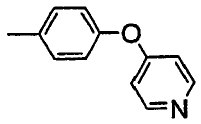 | |
| (408) | 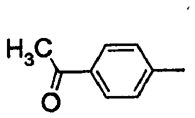 | 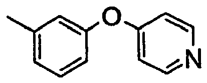 | |
| (409) | 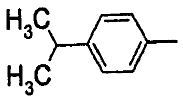 | 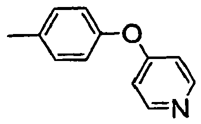 | |
| (410) | 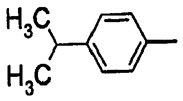 | 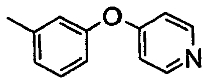 | |
| (411) | 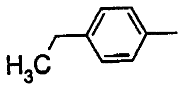 | 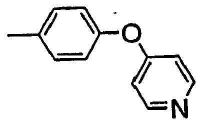 | |
| (412) | 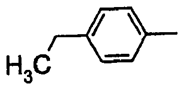 | 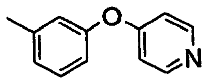 | |
| (413) | 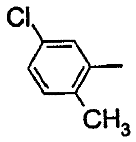 | 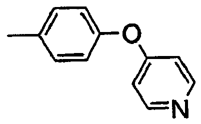 | |
| (414) | 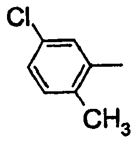 | 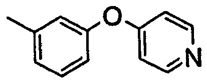 | |
| (415) | 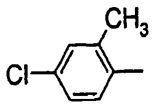 | 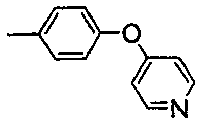 | |
| (416) | 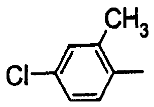 | 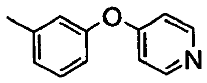 | |
| (417) | 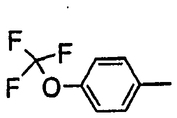 | 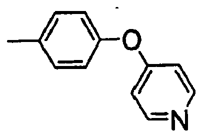 | |
| (418) | 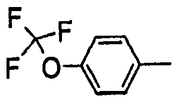 | 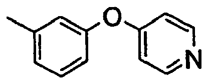 | |
| (419) | 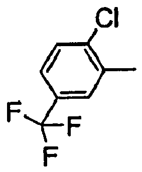 | 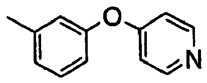 | |
| (420) | 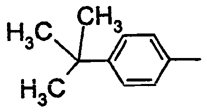 | 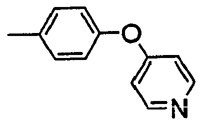 | |
| (421) | 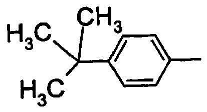 | 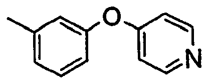 | |
| (422) | 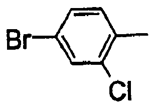 | 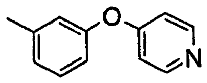 | |
| (423) | 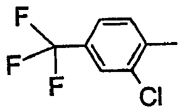 | 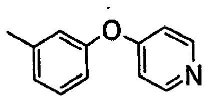 | |
| (424) | 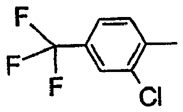 | 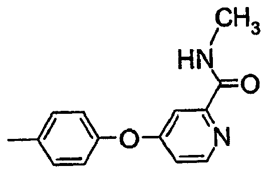 | |
| (425) | 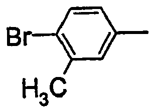 | 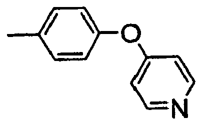 | |
| (426) | 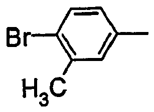 | 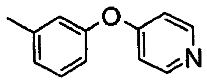 | |
| (427) | 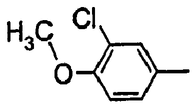 | 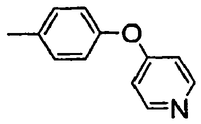 | |
| (428) | 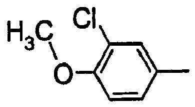 | 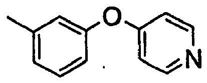 | |
| (429) | 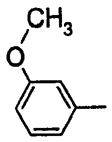 | 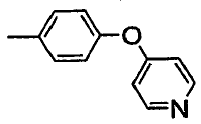 | |
| (430) | 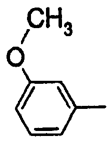 | 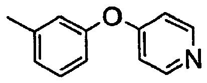 | |
| (431) | 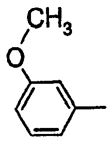 | 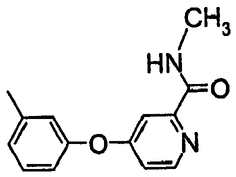 | |
| (432) | 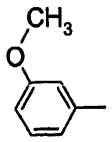 | 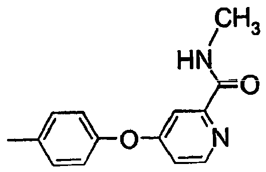 | |
| (433) | 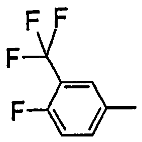 | 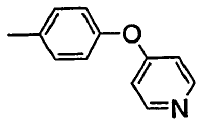 | |
| (434) | 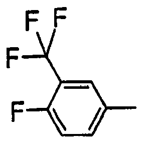 | 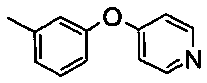 | |
| (435) | 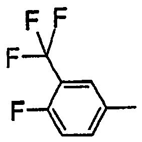 | 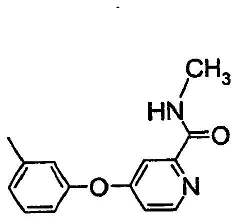 | |
| (436) | 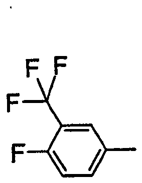 | 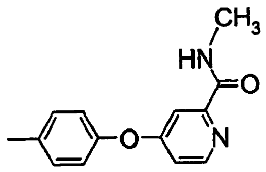 | 2.65 |
| (437) | 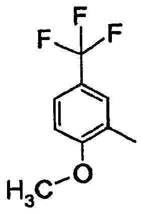 | 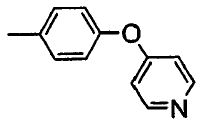 | |
| (438) | 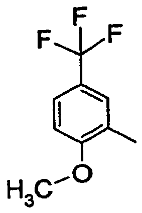 | 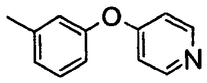 | |
| (439) | 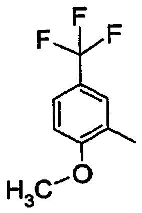 | 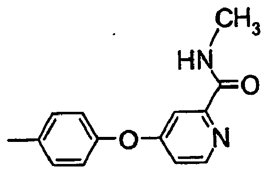 | 2.73 |
| (440) | 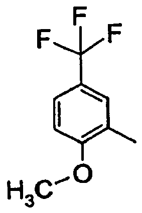 | 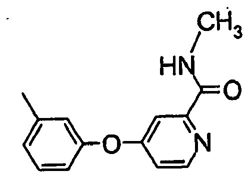 | |
| (441) | 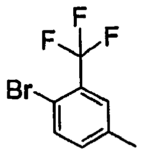 | 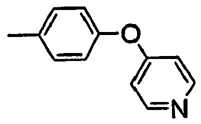 | |
| (442) | 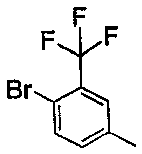 | 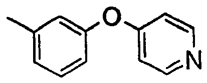 | |
| (443) | 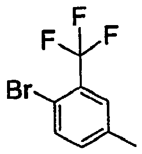 | 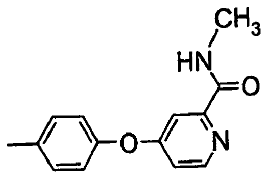 | 2.81 |
| (444) | 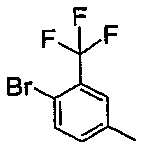 | 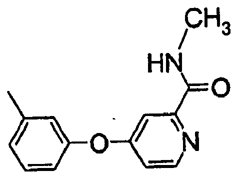 | |
| (445) | 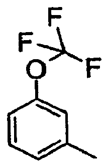 | 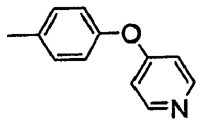 | |
| (446) | 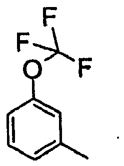 | 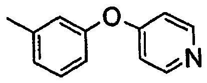 | |
| (447) | 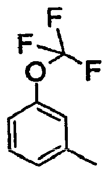 | 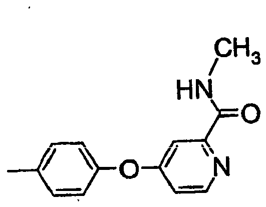 | 2.69 |
| (448) | 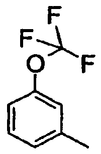 | 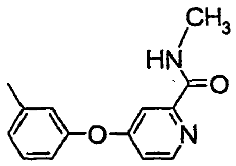 | |

The present invention further relates to compounds (449) to (672) of formula A-NH-CO-CR⁶R⁷-NH-B, wherein R⁶ and R⁷ form, together with the carbon atom of the methylene moiety they are bound to, a cyclopropane moiety, and wherein A and B are as given in the table below:

| | A | B | Rt |
|---|---|---|---|
| (449) | 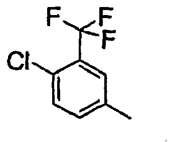 | 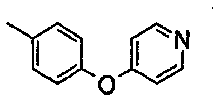 | 2.13 |
| (450) | 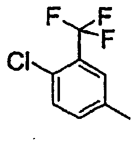 | 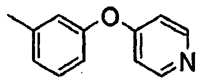 | 2.11 |
| (451) | 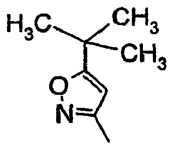 | 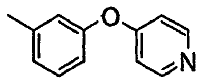 | |
| (452) | 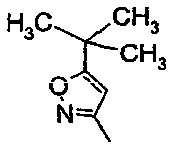 | 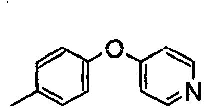 | |
| (453) | 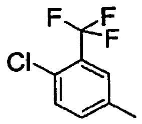 | 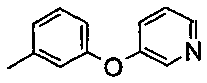 | |
| (454) | 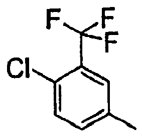 | 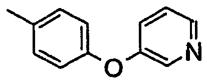 | |
| (455) | 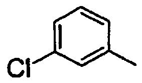 | 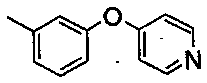 | |
| (456) | 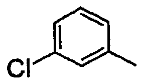 | 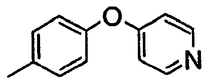 | |
| (457) | 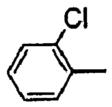 | 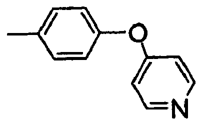 | |
| (458) | 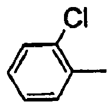 | 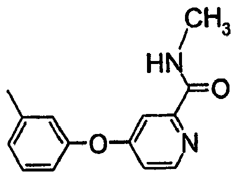 | |
| (459) | 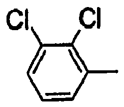 | 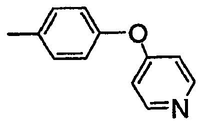 | |
| (460) | 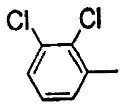 | 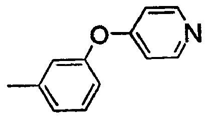 | |
| (461) | 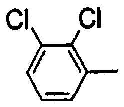 | 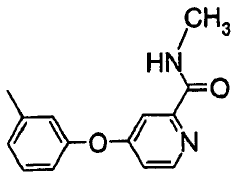 | |
| (462) | 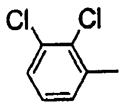 | 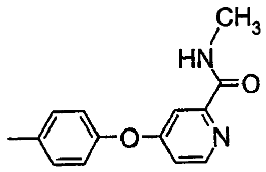 | |
| (463) | 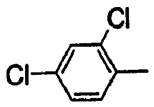 | 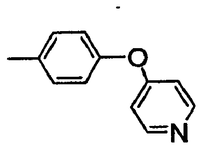 | |
| (464) | 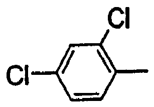 | 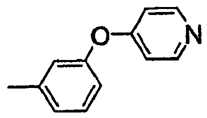 | |
| (465) | 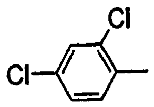 | 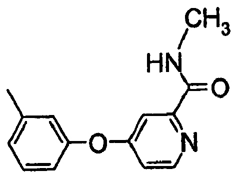 | |
| (466) | 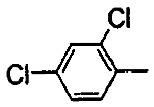 | 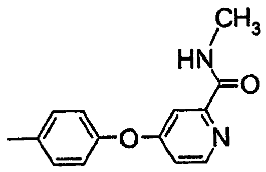 | |
| (467) | 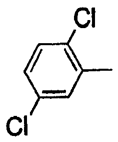 | 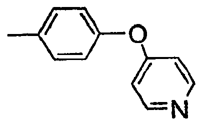 | |
| (468) | 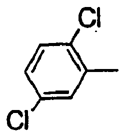 | 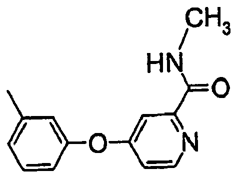 | |
| (469) | 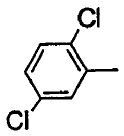 | 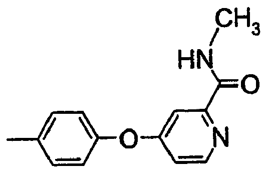 | |
| (470) | 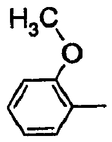 | 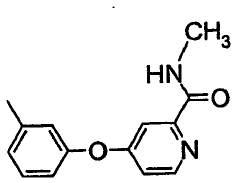 | |
| (471) | 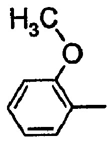 | 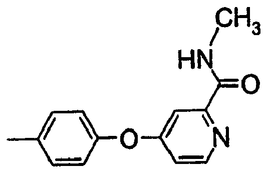 | |
| (472) | 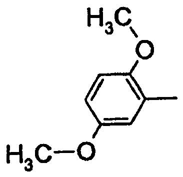 | 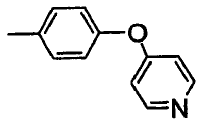 | |
| (473) | 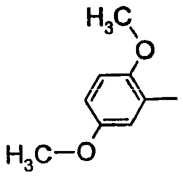 | 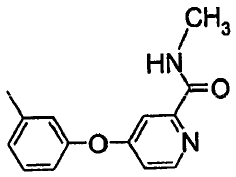 | |
| (474) | 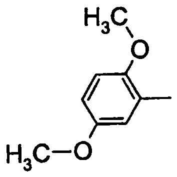 | 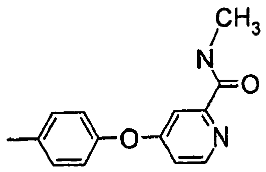 | |
| (475) | 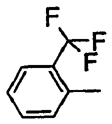 | 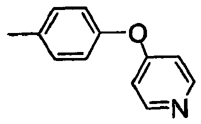 | |
| (476) | 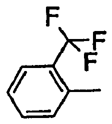 | 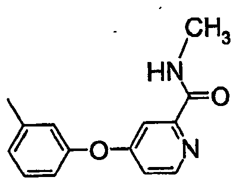 | |
| (477) | 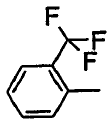 | 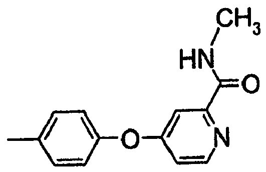 | |
| (478) | 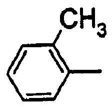 | 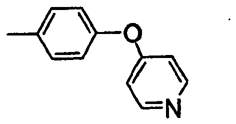 | |
| (479) | 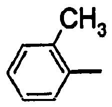 | 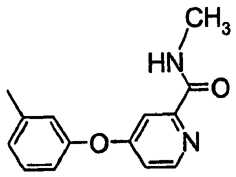 | |
| (480) | 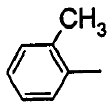 | 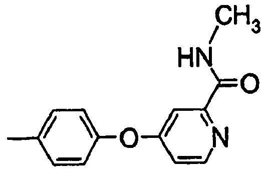 | |
| (481) | 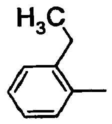 | 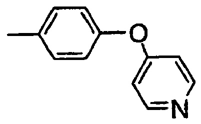 | |
| (482) | 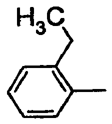 | 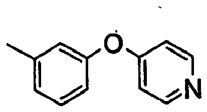 | |
| (483) | 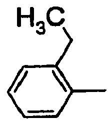 | 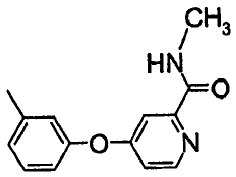 | |
| (484) | 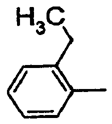 | 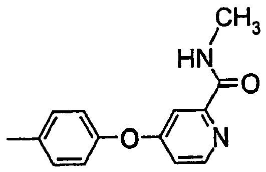 | |
| (485) | 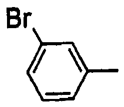 | 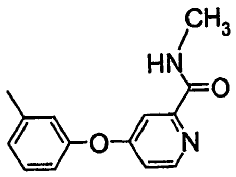 | |
| (486) | 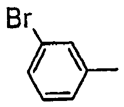 | 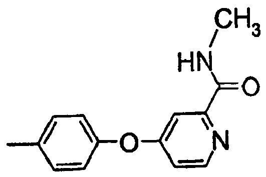 | |
| (487) | 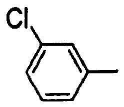 | 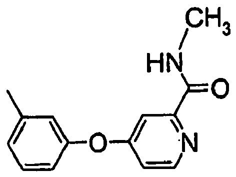 | |
| (488) | 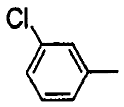 | 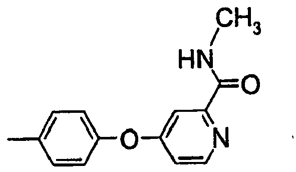 | |
| (489) | 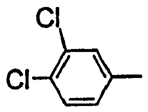 | 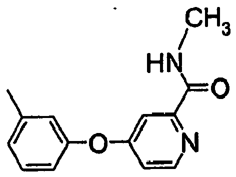 | |
| (490) | 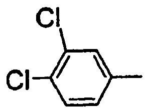 | 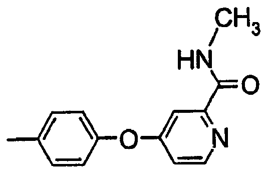 | |
| (491) | 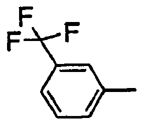 | 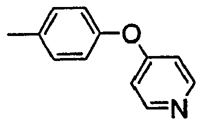 | |
| (492) | 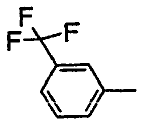 | 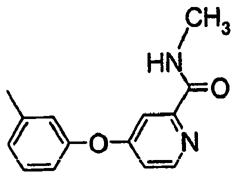 | |
| (493) | 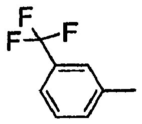 | 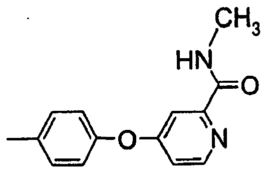 | |
| (494) | 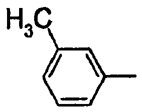 | 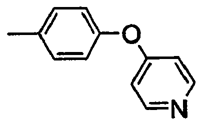 | |
| (495) | 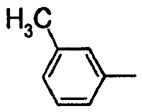 | 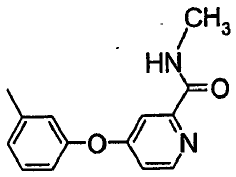 | |
| (496) | 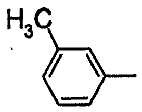 | 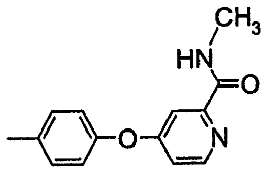 | |
| (497) | 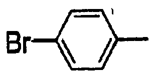 | 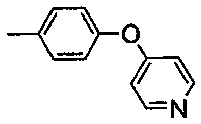 | |
| (498) | 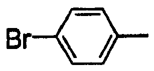 | 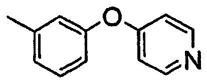 | |
| (499) | 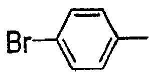 | 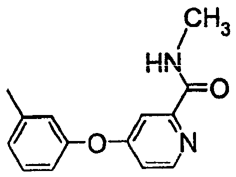 | |
| (500) | 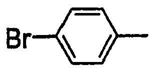 | 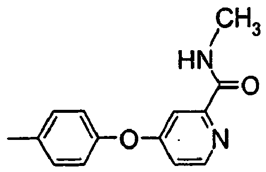 | |
| (501) | 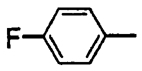 | 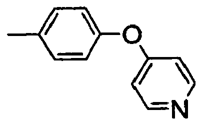 | |
| (502) | 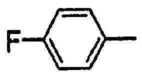 | 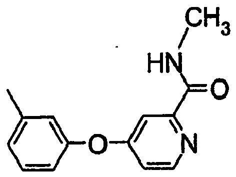 | |
| (503) | 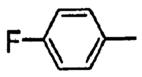 | 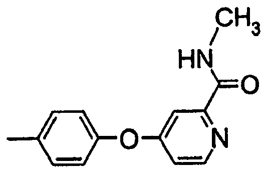 | |
| (504) | 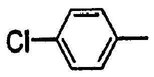 | 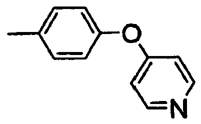 | |
| (505) | 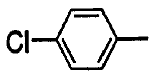 | 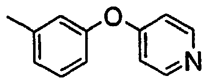 | |
| (506) | 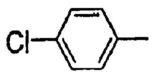 | 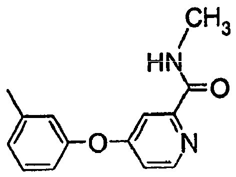 | |
| (507) | 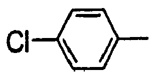 | 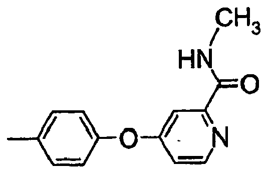 | |
| (508) | 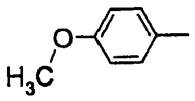 | 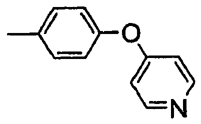 | |
| (509) | 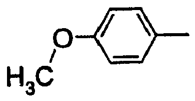 | 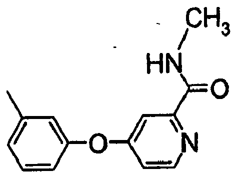 | |
| (510) | 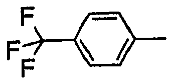 | 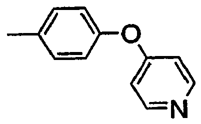 | |
| (511) | 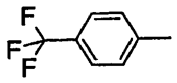 | 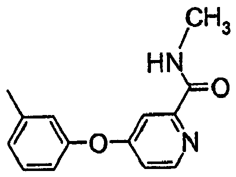 | |
| (512) | 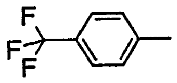 | 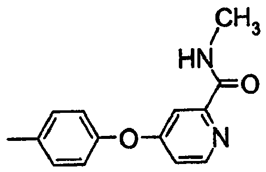 | |
| (513) | 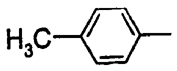 | 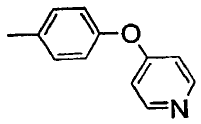 | |
| (514) | 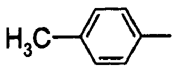 | 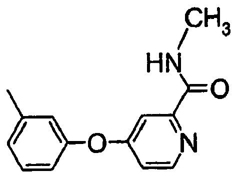 | |
| (515) | 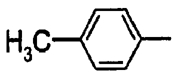 | 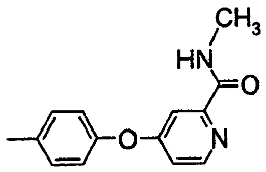 | |
| (516) | 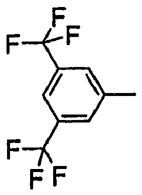 | 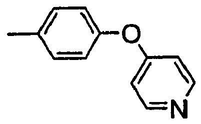 | |
| (517) | 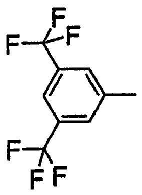 | 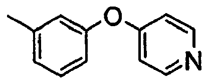 | |
| (518) | 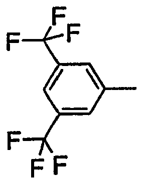 | 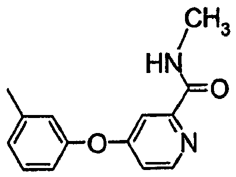 | |
| (519) | 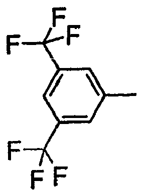 | 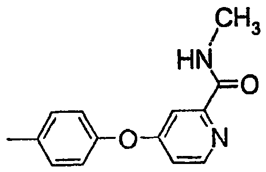 | |
| (520) | 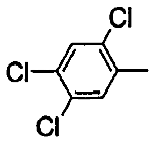 | 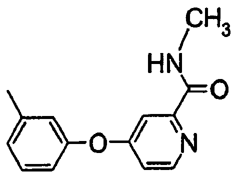 | |
| (521) | 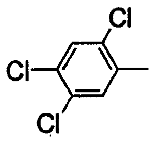 | 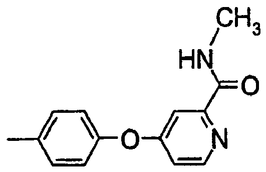 | |
| (522) | 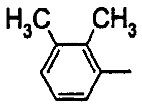 | 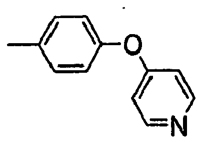 | |
| (523) | 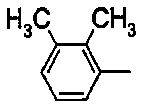 | 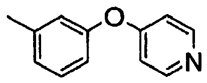 | |
| (524) | 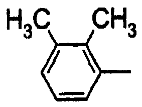 | 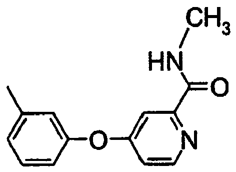 | |
| (525) | 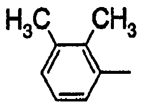 | 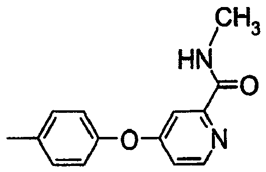 | |
| (526) | 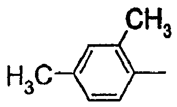 | 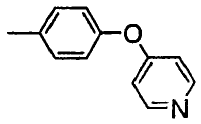 | |
| (527) | 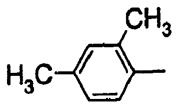 | 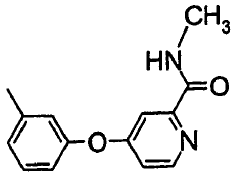 | |
| (528) | 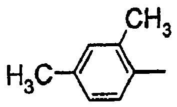 | 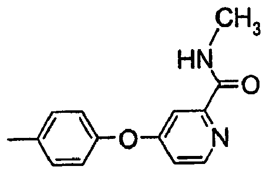 | |
| (529) | 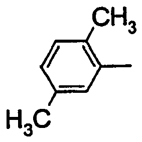 | 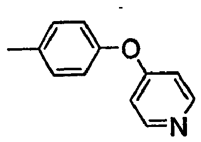 | |
| (530) | 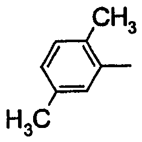 | 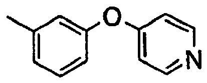 | |
| (531) | 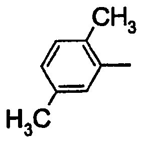 | 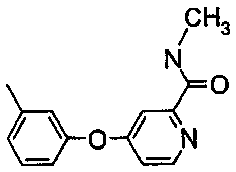 | |
| (532) | 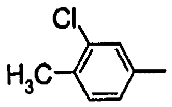 | 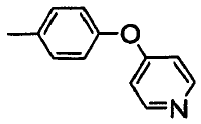 | |
| (533) | 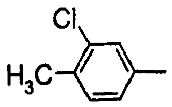 | 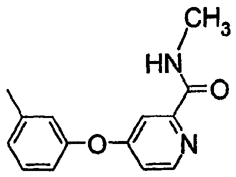 | |
| (534) | 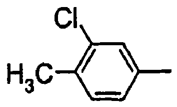 | 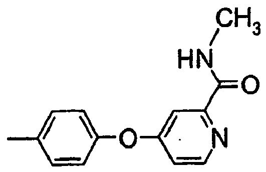 | |
| (535) | 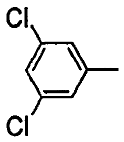 | 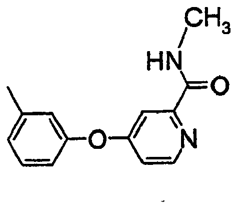 | |
| (536) | 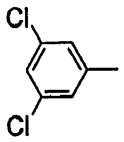 | 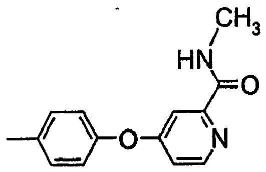 | |
| (537) | 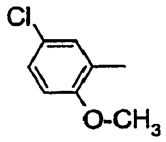 | 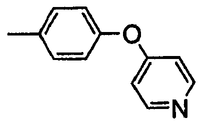 | |
| (538) | 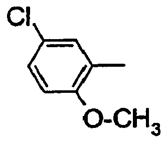 | 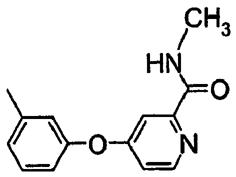 | |
| (539) | 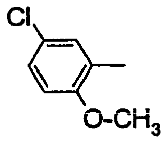 | 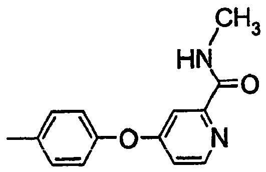 | |
| (540) | 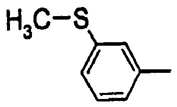 | 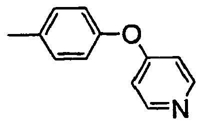 | |
| (541) | 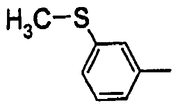 | 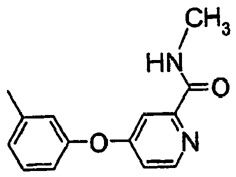 | |
| (542) | 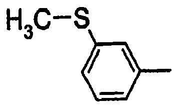 | 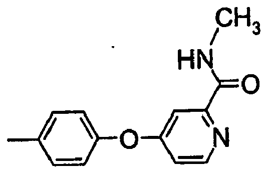 | |
| (543) | 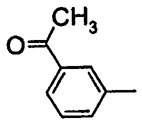 | 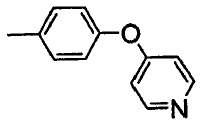 | |
| (544) | 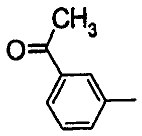 | 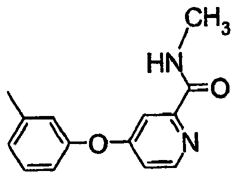 | |
| (545) | 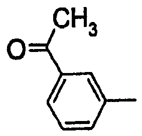 | 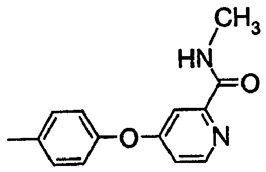 | |
| (546) | 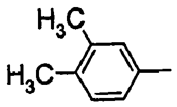 | 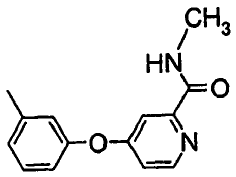 | |
| (547) | 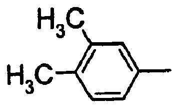 | 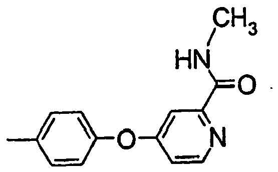 | |
| (548) | 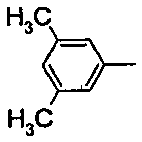 | 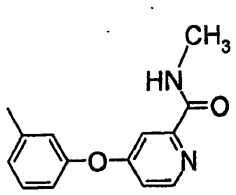 | |
| (549) | 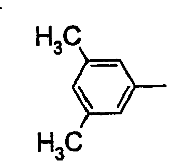 | 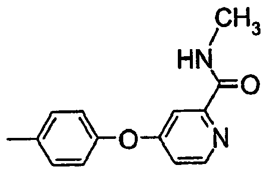 | |
| (550) | 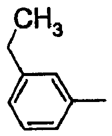 | 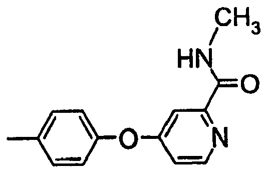 | |
| (551) | 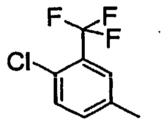 | 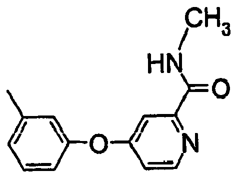 | |
| (552) | 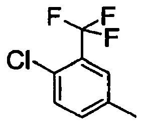 | 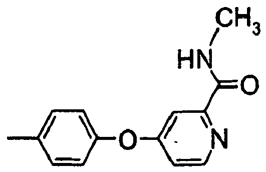 | |
| (553) | 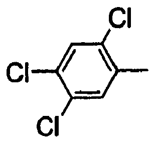 | 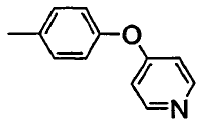 | |
| (554) | 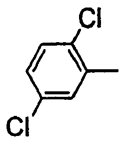 | 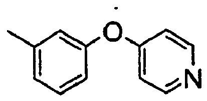 | |
| (555) | 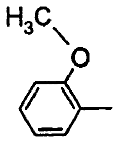 | 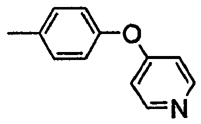 | |
| (556) | 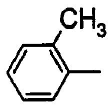 | 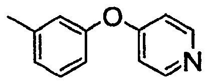 | |
| (557) | 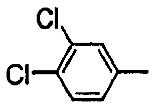 | 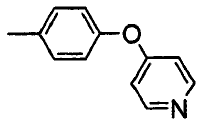 | |
| (558) | 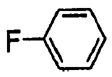 | 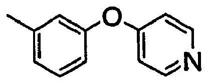 | |
| (559) | 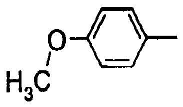 | 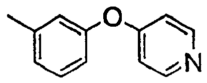 | |
| (560) | 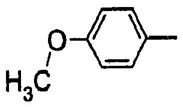 | 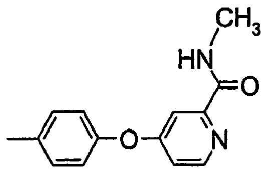 | |
| (561) | 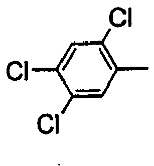 | 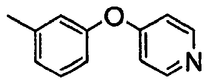 | |
| (562) | 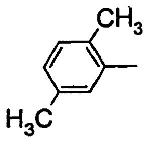 | 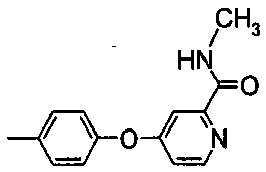 | |
| (563) | 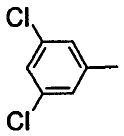 | 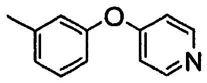 | |
| (564) | 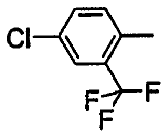 | 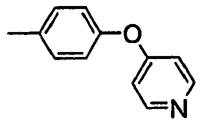 | |
| (565) | 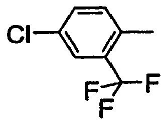 | 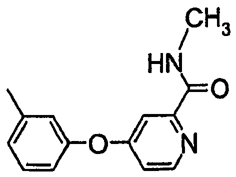 | |
| (566) | 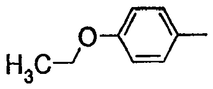 | 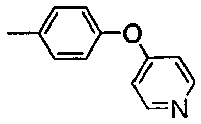 | |
| (567) | 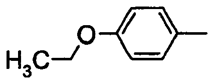 | 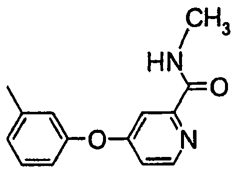 | |
| (568) | 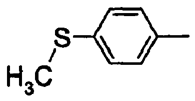 | 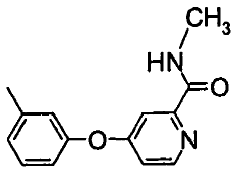 | |
| (569) | 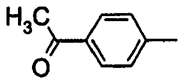 | 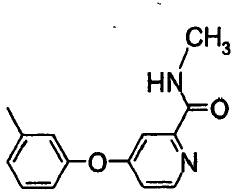 | |
| (570) | 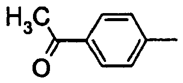 | 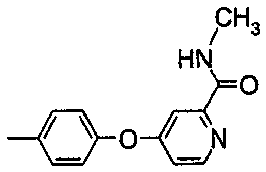 | |
| (571) | 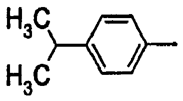 | 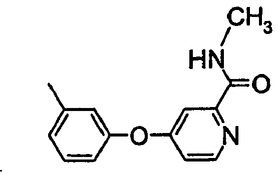 | |
| (572) | 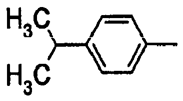 | 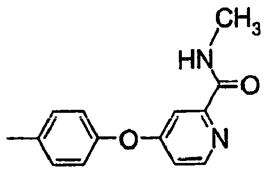 | |
| (573) | 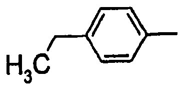 | 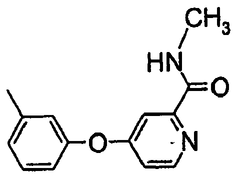 | |
| (574) | 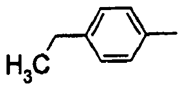 | 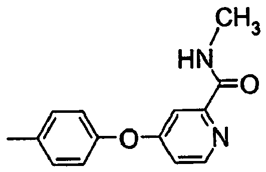 | |
| (575) | 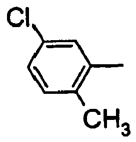 | 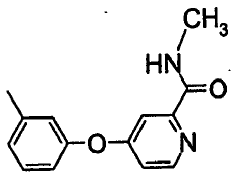 | |
| (576) | 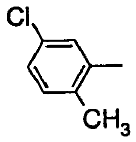 | 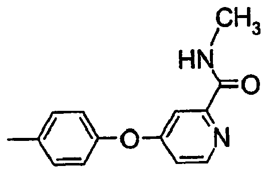 | |
| (577) | 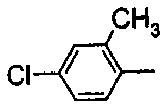 | 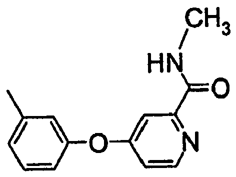 | |
| (578) | 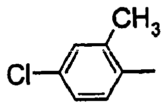 | 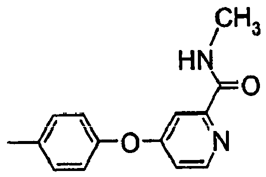 | |
| (579) | 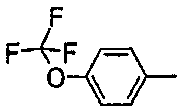 | 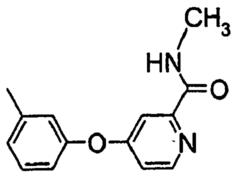 | |
| (580) | 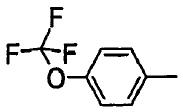 | 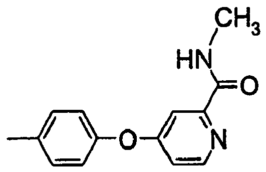 | |
| (581) | 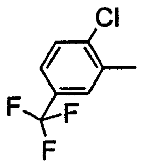 | 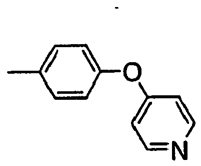 | |
| (582) | 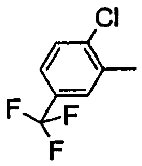 | 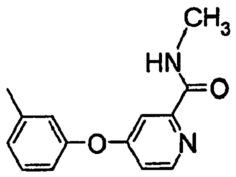 | |
| (583) | 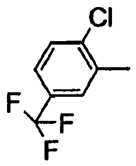 | 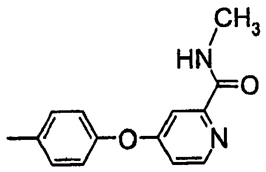 | |
| (584) | 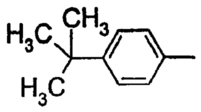 | 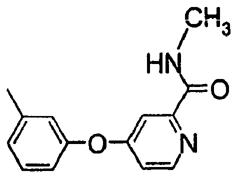 | |
| (585) | 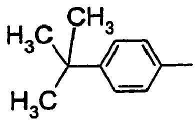 | 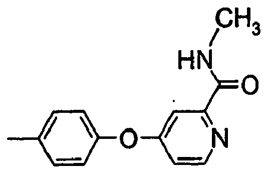 | |
| (586) | 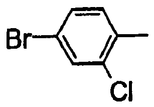 | 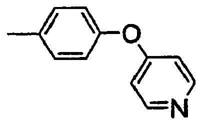 | |
| (587) | 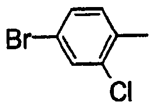 | 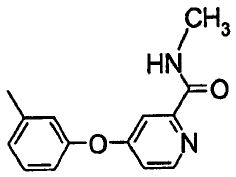 | |
| (588) | 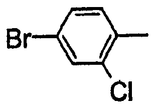 | 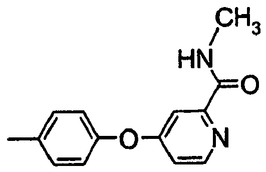 | |
| (589) | 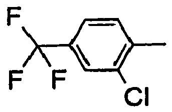 | 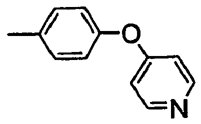 | |
| (590) | 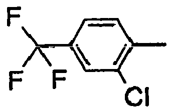 | 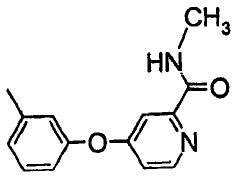 | |
| (591) | 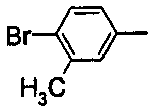 | 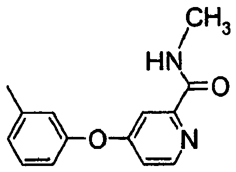 | |
| (592) | 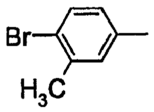 | 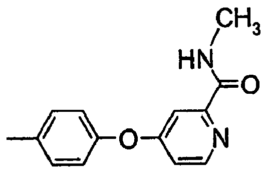 | |
| (593) | 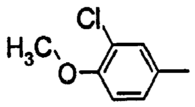 | 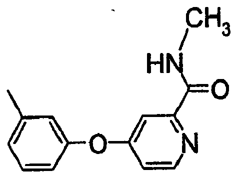 | |
| (594) | 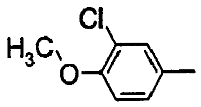 | 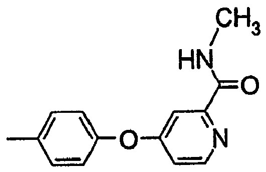 | |
| (595) | 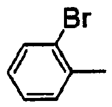 | 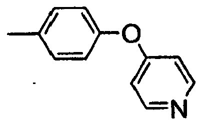 | |
| (596) | 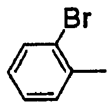 | 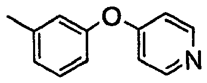 | |
| (597) | 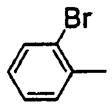 | 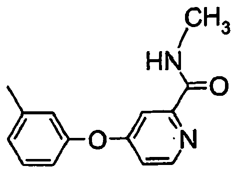 | |
| (598) | 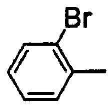 | 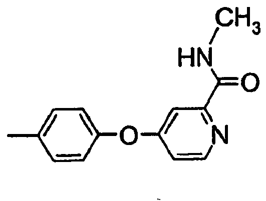 | |
| (599) | 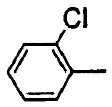 | 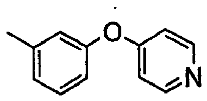 | |
| (600) | 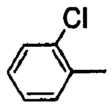 | 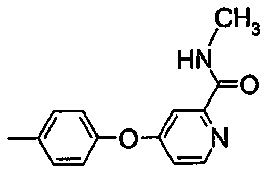 | |
| (601) | 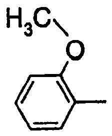 | 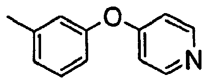 | |
| (602) | 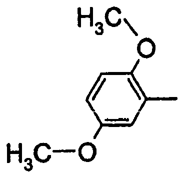 | 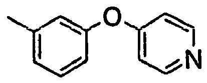 | |
| (603) | 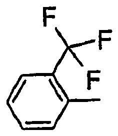 | 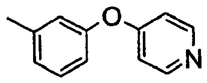 | |
| (604) | 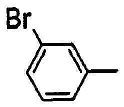 | 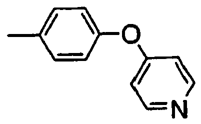 | |
| (605) | 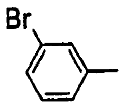 | 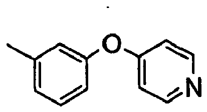 | |
| (606) | 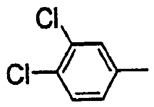 | 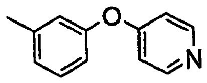 | |
| (607) | 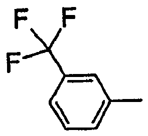 | 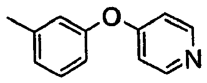 | |
| (608) | 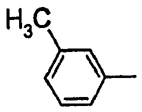 | 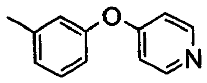 | |
| (609) | 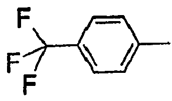 | 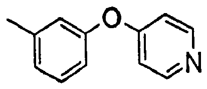 | |
| (610) | 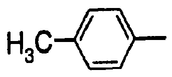 | 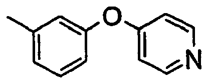 | |
| (611) | 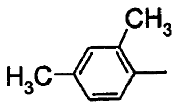 | 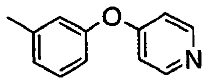 | |
| (612) | 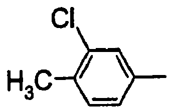 | 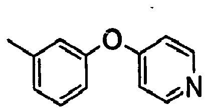 | |
| (613) | 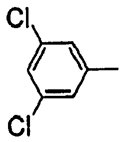 | 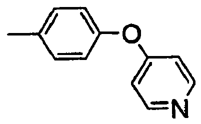 | |
| (614) | 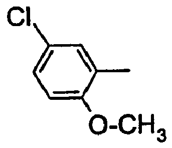 | 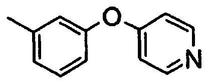 | |
| (615) | 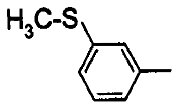 | 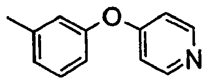 | |
| (616) | 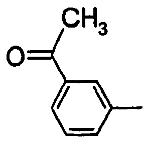 | 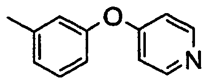 | |
| (617) | 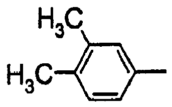 | 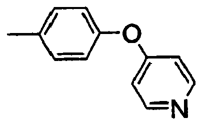 | |
| (618) | 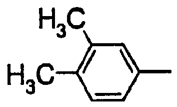 | 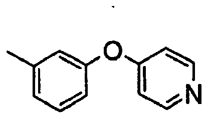 | |
| (619) | 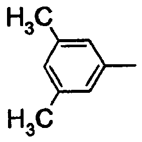 | 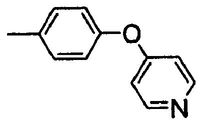 | |
| (620) | 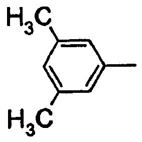 | 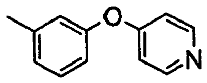 | |
| (621) | 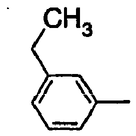 | 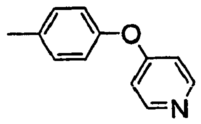 | |
| (622) | 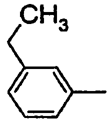 | 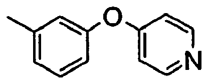 | |
| (623) | 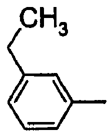 | 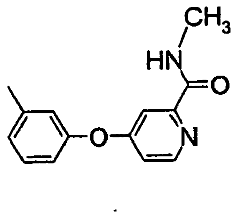 | |
| (624) | 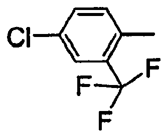 | 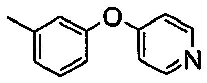 | |
| (625) | 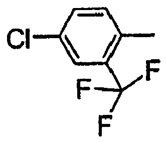 | 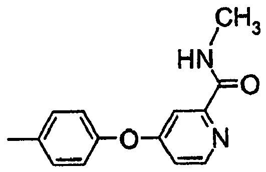 | |
| (626) | 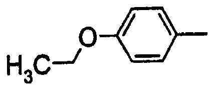 | 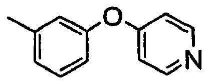 | |
| (627) | 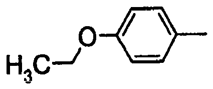 | 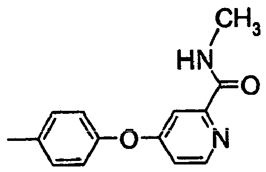 | |
| (628) | 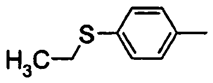 | 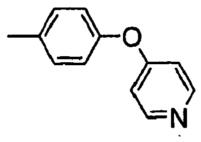 | |
| (629) | 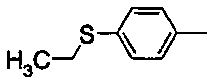 | 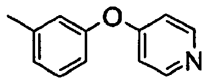 | |
| (630) | 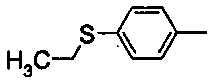 | 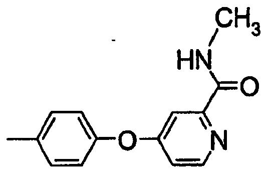 | |
| (631) | 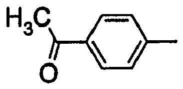 | 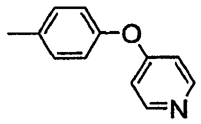 | |
| (632) | 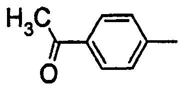 | 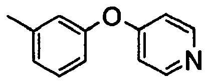 | |
| (633) | 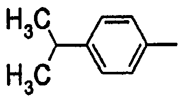 | 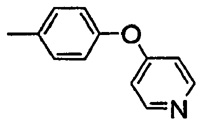 | |
| (634) | 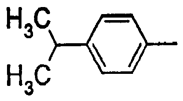 | 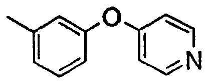 | |
| (635) | 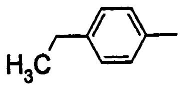 | 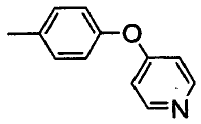 | |
| (636) | 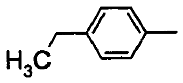 | 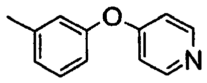 | |
| (637) | 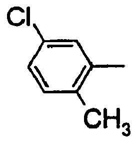 | 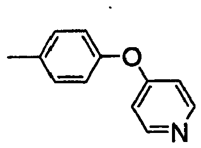 | |
| (638) | 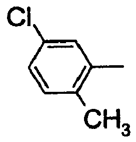 | 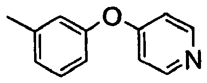 | |
| (639) | 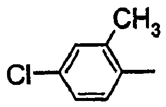 | 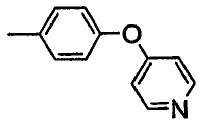 | |
| (640) | 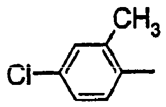 | 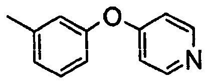 | |
| (641) | 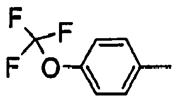 | 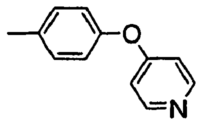 | |
| (642) | 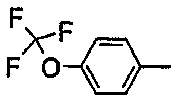 | 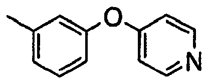 | |
| (643) | 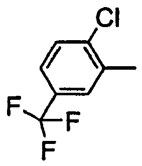 | 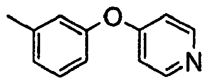 | |
| (644) | 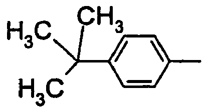 | 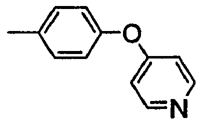 | |
| (645) | 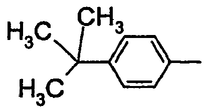 | 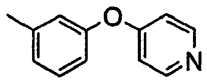 | |
| (646) | 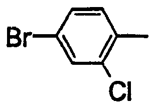 | 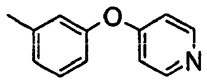 | |
| (647) | 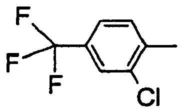 | 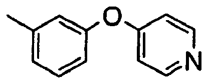 | |
| (648) | 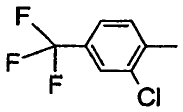 | 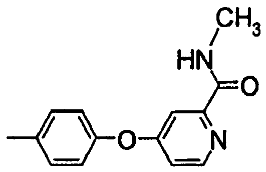 | |
| (649) | 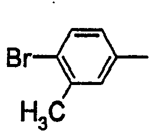 | 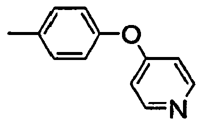 | |
| (650) | 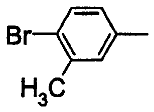 | 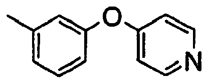 | |
| (651) | 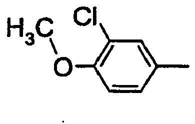 | 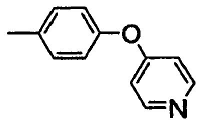 | |
| (652) | 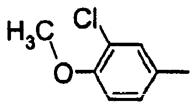 | 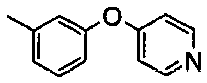 | |
| (653) | 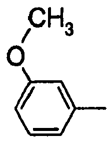 | 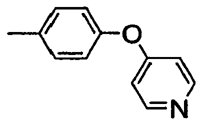 | |
| (654) | 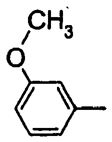 | 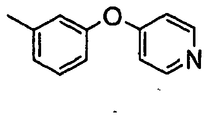 | |
| (655) | 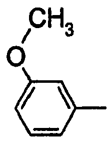 | 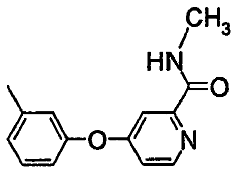 | |
| (656) | 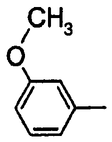 | 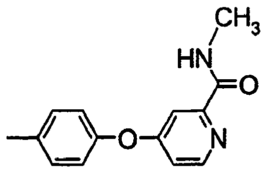 | |
| (657) | 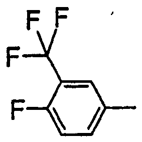 | 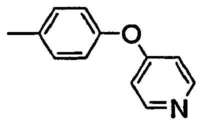 | |
| (658) | 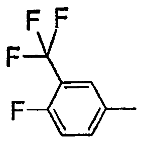 | 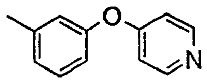 | |
| (659) | 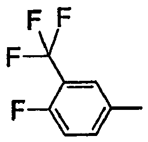 | 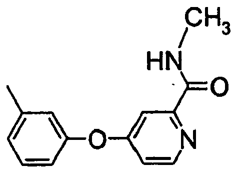 | |
| (660) | 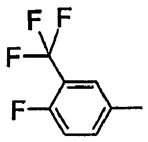 | 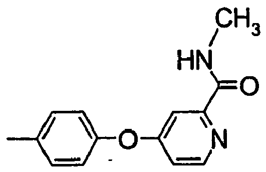 | |
| (661) | 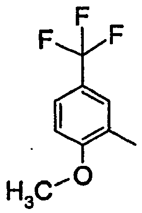 | 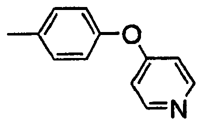 | |
| (662) | 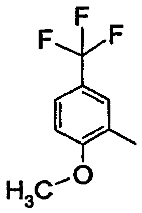 | 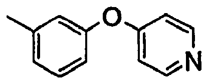 | |
| (663) | 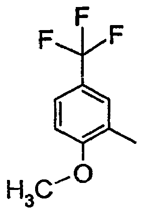 | 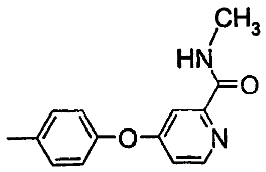 | |
| (664) | 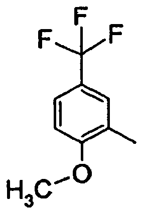 | 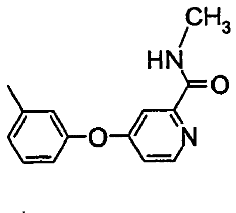 | |
| (665) | 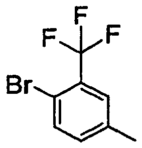 | 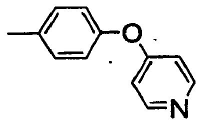 | |
| (666) | 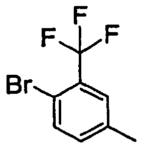 | 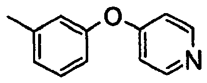 | |
| (667) | 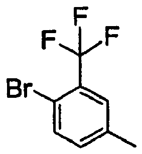 | 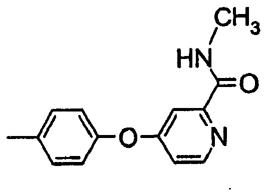 | |
| (668) | 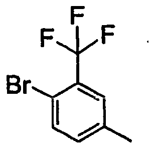 | 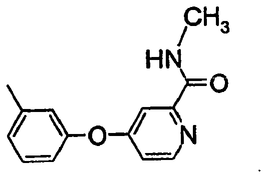 | |
| (669) | 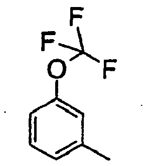 | 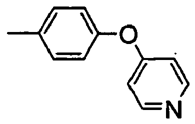 | |
| (670) | 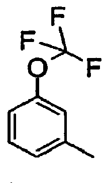 | 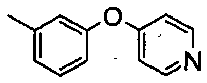 | |
| (671) | 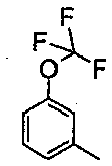 | 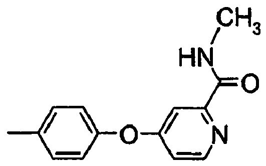 | |
| (672) | 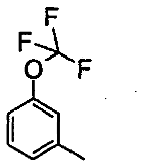 | 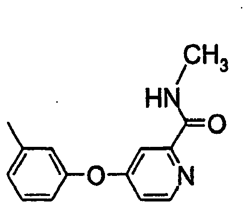 | |

The present invention further relates to compounds (673) to (758) as given in the table below:

| Compound | | Rt |
|---|---|---|
| (673) | 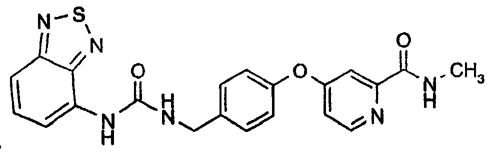 | 2.32 |
| (674) | 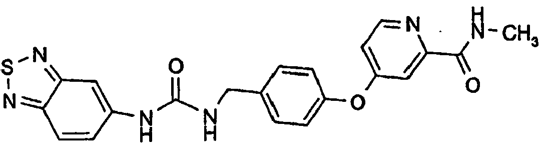 | 2.20 |
| (675) | 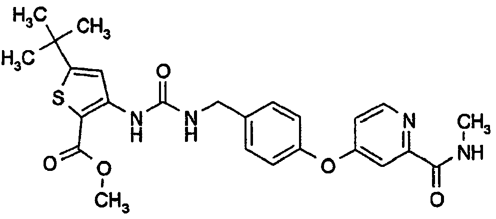 | 2.99 |
| (676) | 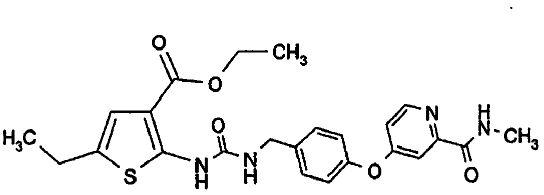 | 2.93 |
| (677) | 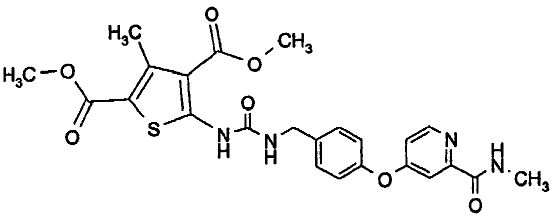 | 2.68 |
| (678) | 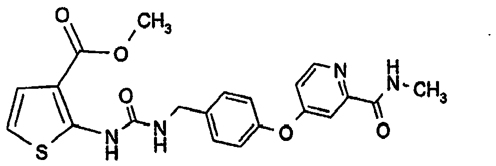 | 2.44 |
| (679) | 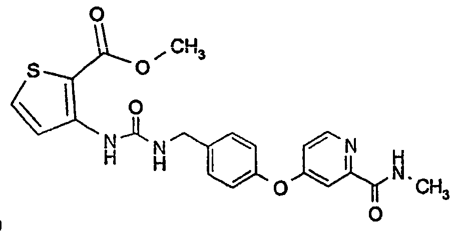 | 2.40 |
| (680) | 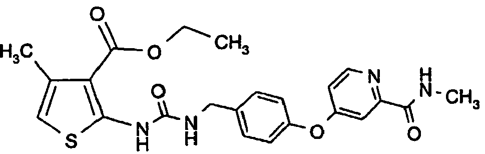 | 2.77 |
| (681) | 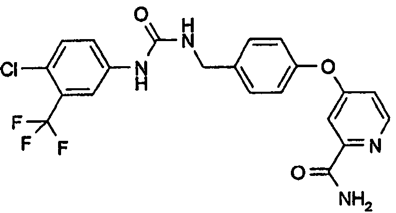 | 2.55 |
| (682) | 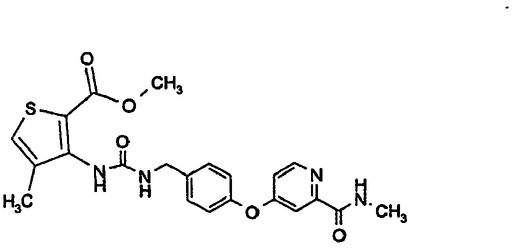 | 2.25 |
| (683) | 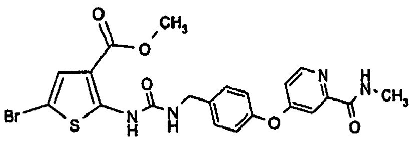 | 2.86 |
| (684) | 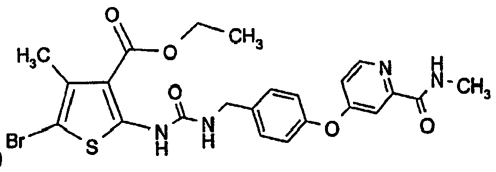 | 3.20 |
| (685) | 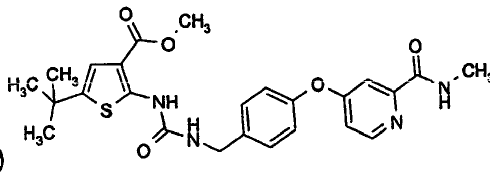 | 3.01 |
| (686) | 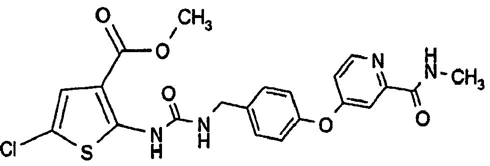 | 2.87 |
| (687) | 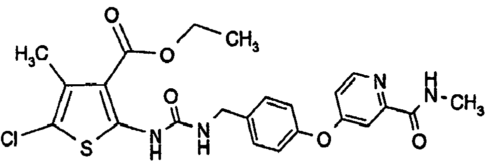 | 3.19 |
| (688) | 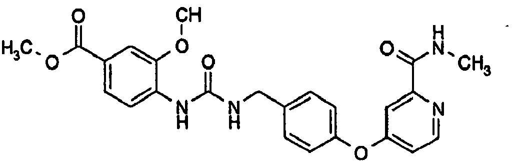 | 4.52^{a} |
| (689) | 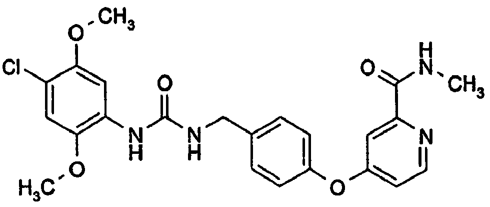 | 4.78^{a} |
| (690) | 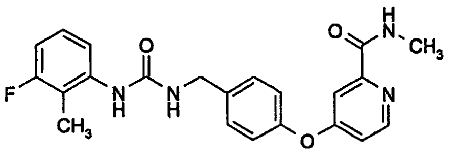 | 4.41^{a} |
| (691) | 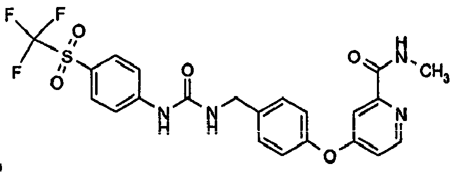 | 4.97^{a} |
| (692) | 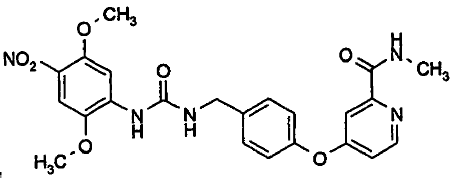 | 4_{.}59^{a} |
| (693) | 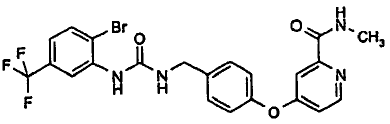 | 5_{.}28^{a} |
| (694) | 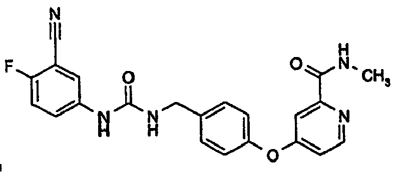 | 4.42^{a} |
| (695) | 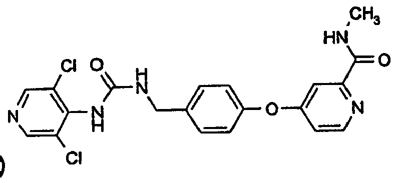 | 3.06^{b} |
| (696) | 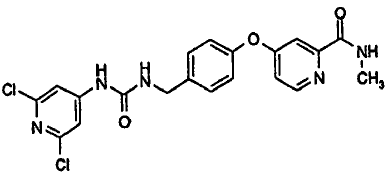 | 3.02^{b} |
| (697) | 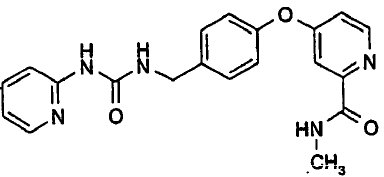 | 1.30^{b} |
| (698) | 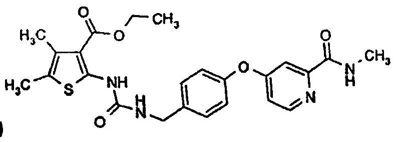 | 2.91 |
| (699) | 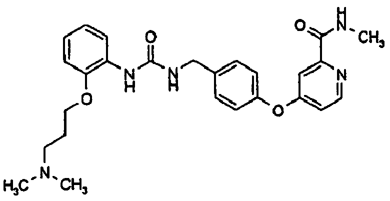 | 3.49^{a} |
| (700) | 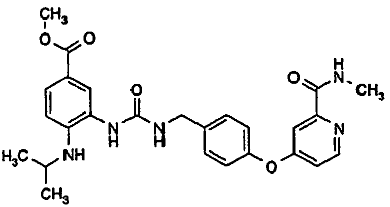 | 3.94^{a} |
| (701) | 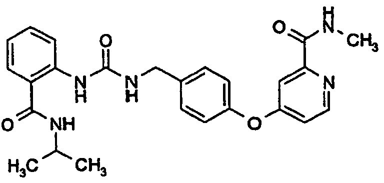 | 4.31^{a} |
| (702) | 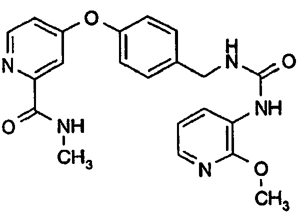 | 2.44^{b} |
| (703) | 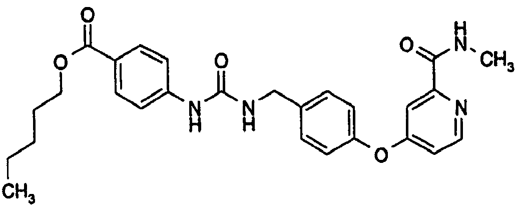 | 5.39^{a} |
| (704) | 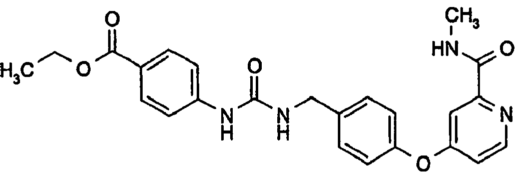 | 4.59^{a} |
| (705) | 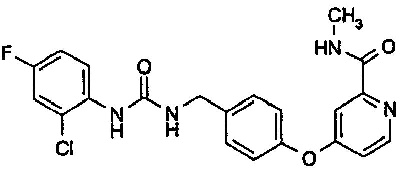 | 4.61^{a} |
| (706) | 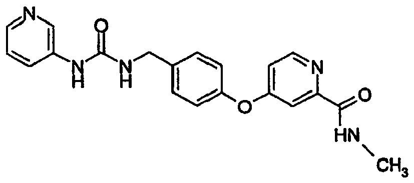 | 4.71^{c} |
| (707) | 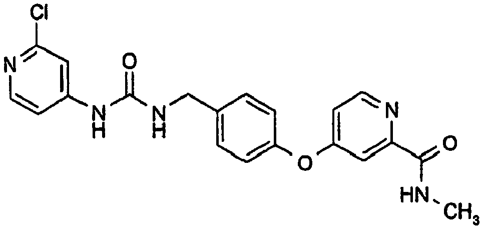 | 2.31^{b} |
| (708) | 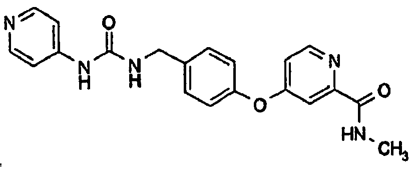 | 4.85^{c} |
| (709) | 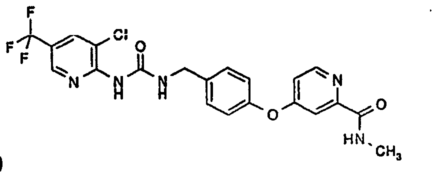 | 2.33^{b} |
| (710) | 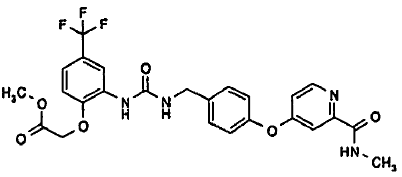 | 2.66 |
| (711) | 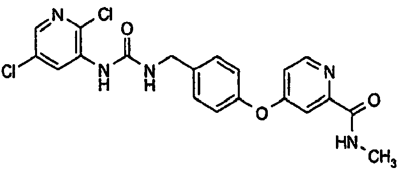 | 3.03^{b} |
| (712) | 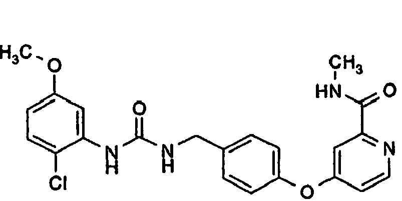 | 4.68^{a} |
| (713) | 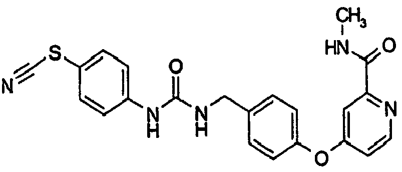 | 4.47^{a} |
| (714) | 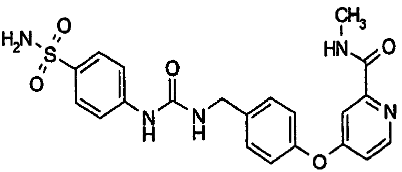 | 3.29^{a} |
| (715) | 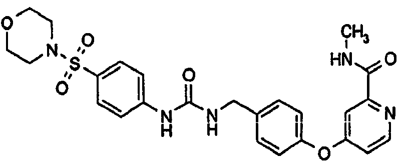 | 4.03^{a} |
| (716) | 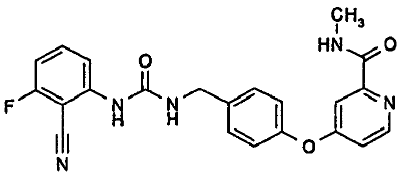 | 4.35^{a} |
| (717) | 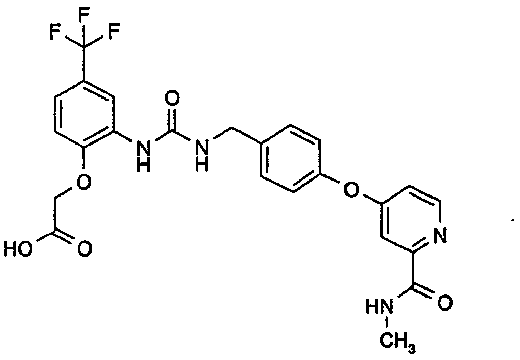 | 2.41 |
| (718) | 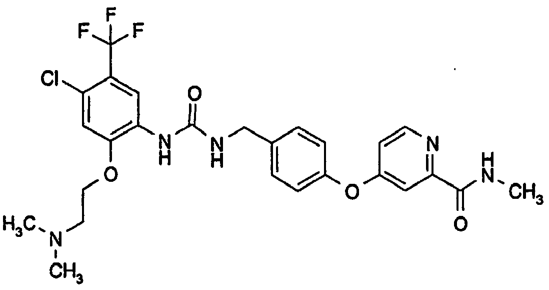 | 2.13 |
| (719) | 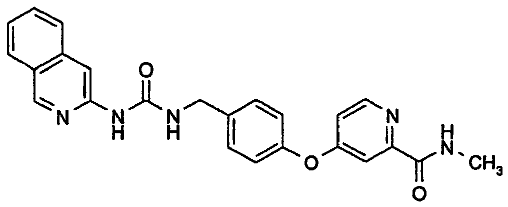 | 2.51^{b} |
| (720) | 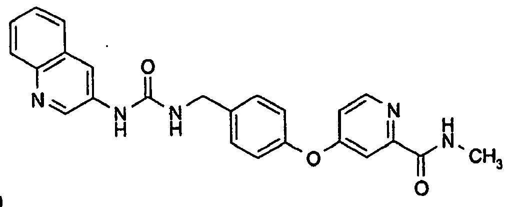 | 2.09^{b} |
| (721) | 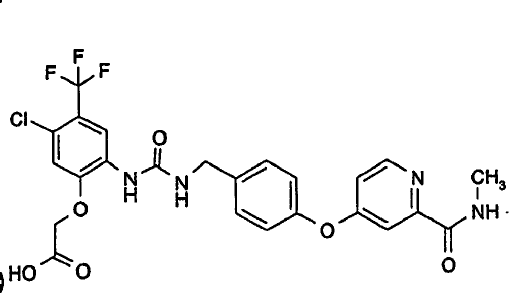 | 2.55 |
| (722) | 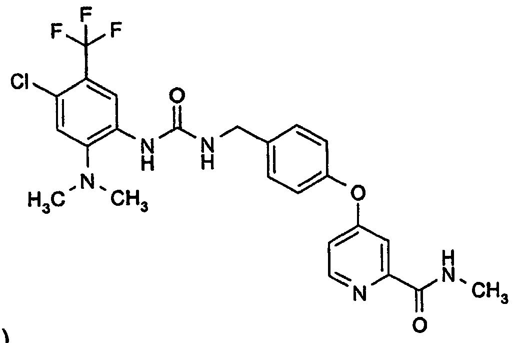 | 2.95 |
| (723) | 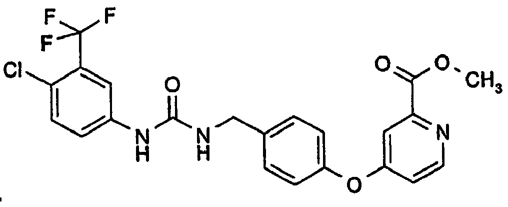 | 2.68 |
| (724) | 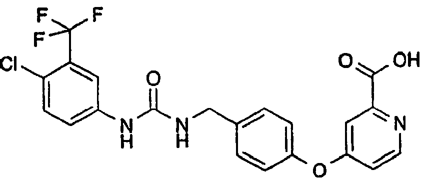 | 2.19 |
| (725) | 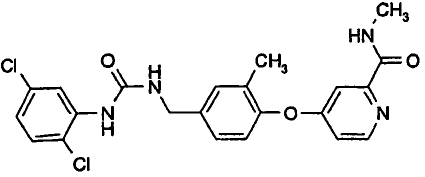 | 3.42^{b} |
| (726) | 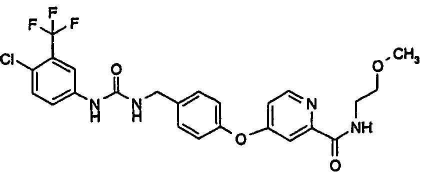 | 2.83 |
| (727) | 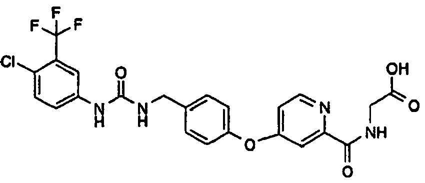 | 2.61 |
| (728) | 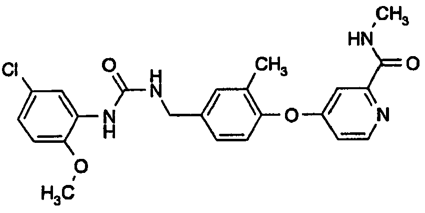 | 3.19^{b} |
| (729) | 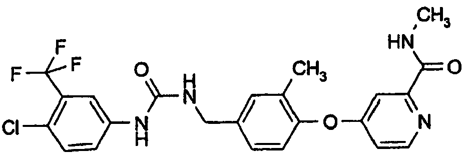 | 3.38^{b} |
| (730) | 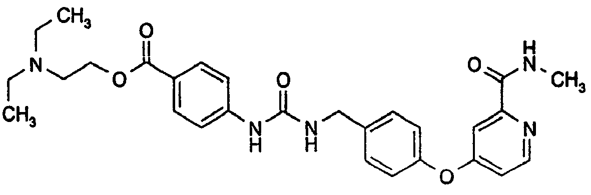 | 3.59^{a} |
| (731) | 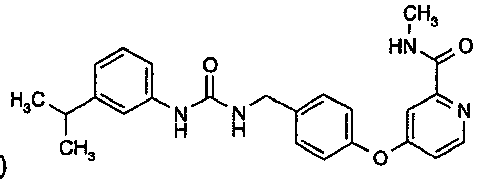 | 4.91^{a} |
| (732) | 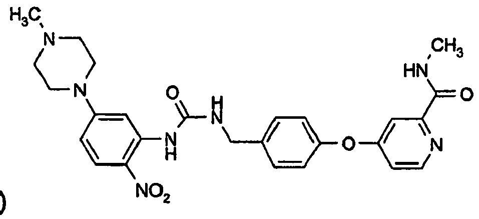 | 3.59^{a} |
| (733) | 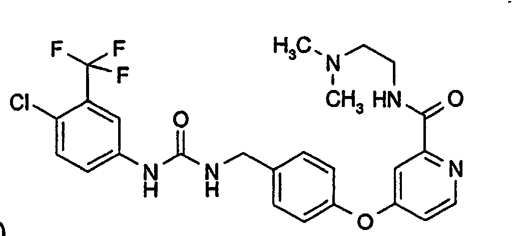 | 2.17 |
| (734) | 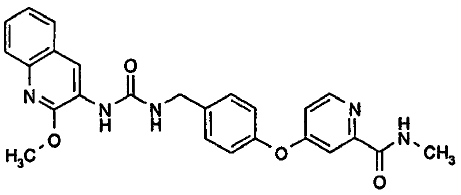 | 3.11^{b} |
| (735) | 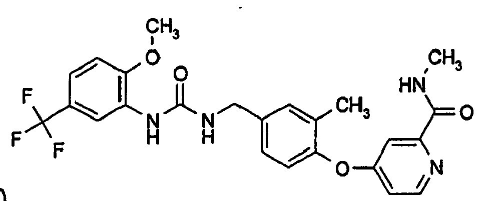 | 3.29^{b} |
| (736) | 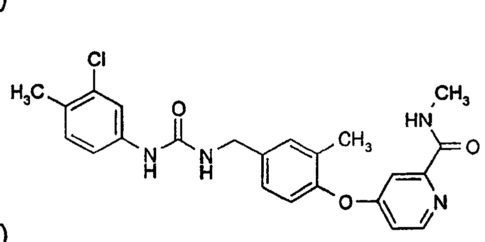 | 3.21^{b} |
| (737) | 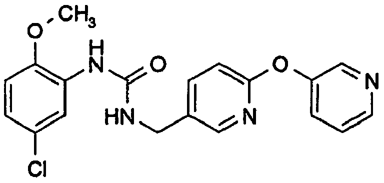 | 2.51^{b} |
| (738) | 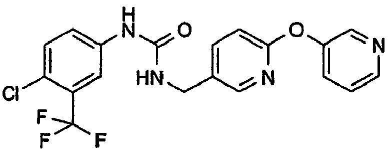 | 2.79^{b} |
| (739) | 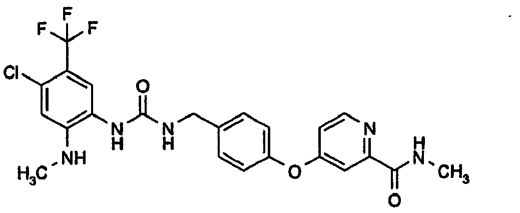 | 2.60 |
| (740) | 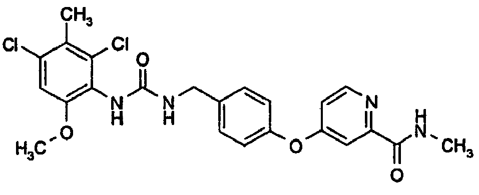 | 2.47 |
| (741) | 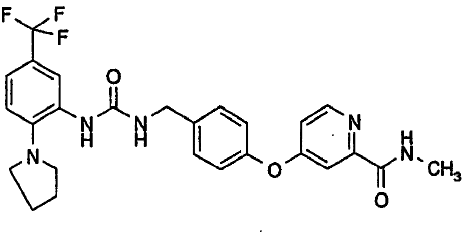 | 2.69 |
| (742) | 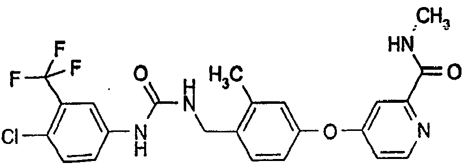 | 3.41^{b} |
| (743) | 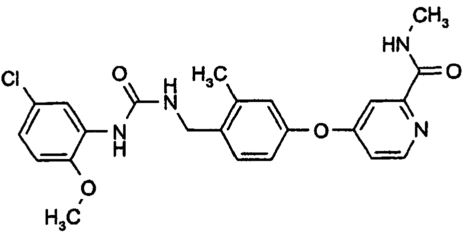 | 3.23^{b} |
| (744) | 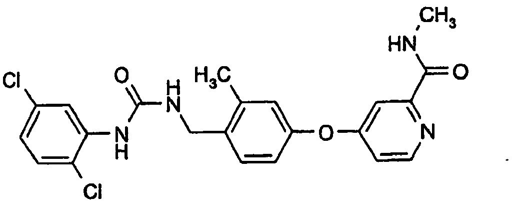 | 3.39^{b} |
| (745) | 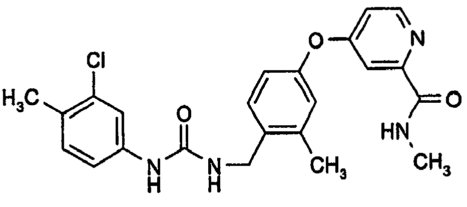 | 3.23^{b} |
| (746) | 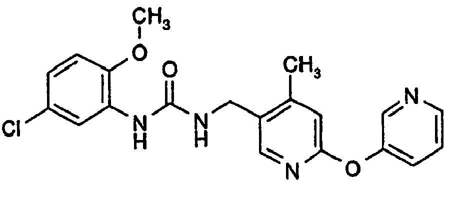 | 2.64^{b} |
| (747) | 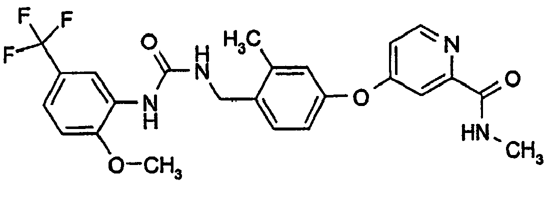 | 3.31^{b} |
| (748) | 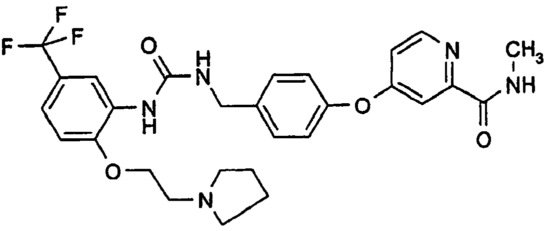 | 2.05 |
| (749) | 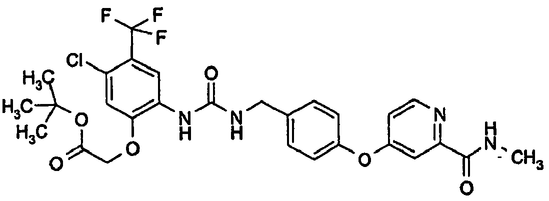 | 3.13 |
| (750) | 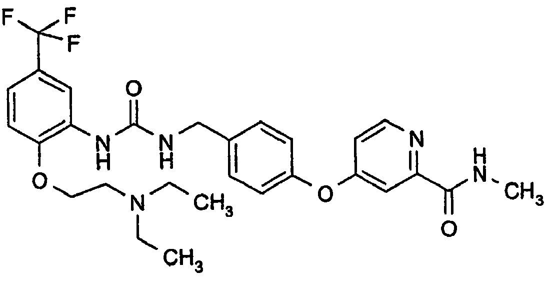 | 2.06 |
| (751) | 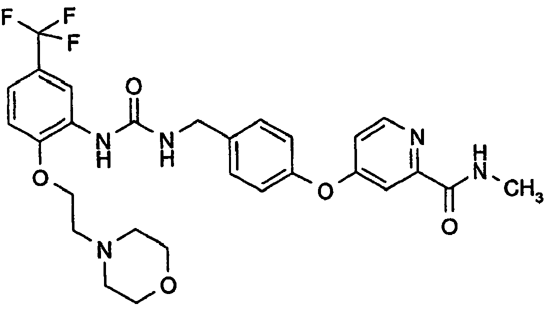 | 2.03 |
| (752) | 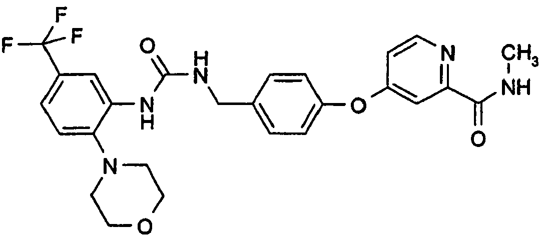 | 2.65 |
| (753) | 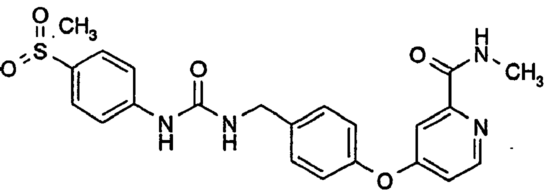 | 3.71^{a} |
| (754) | 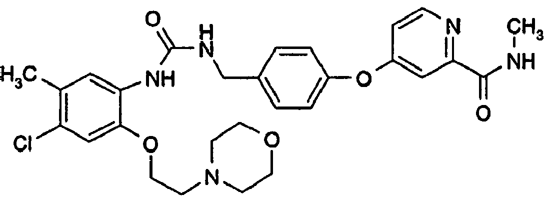 | 1.95 |
| (755) | 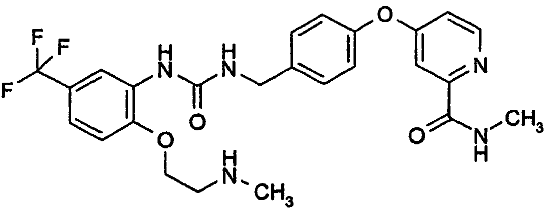 | 1.99 |
| (756) | 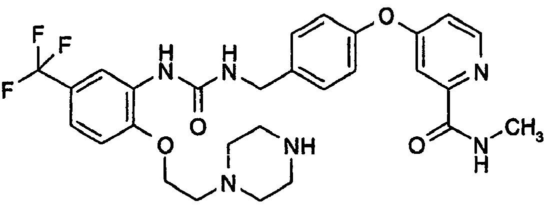 | 1.93 |
| (757) | 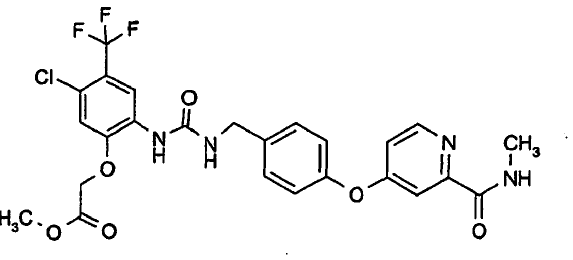 | 2.83 |
| (758) | 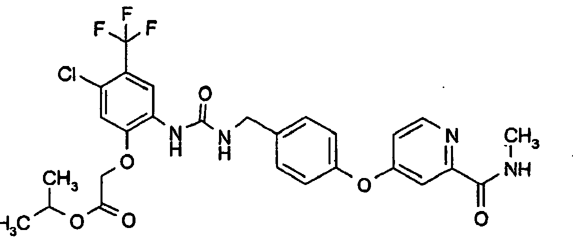 | 3.04 |

The present invention further relates to compounds (759) to (825) as given in the table below:

| Compound | | Rt |
|---|---|---|
| (759) | 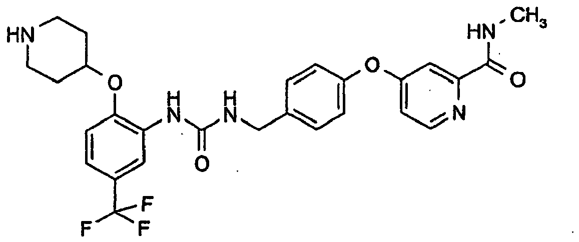 | 2.03 |
| (760) | 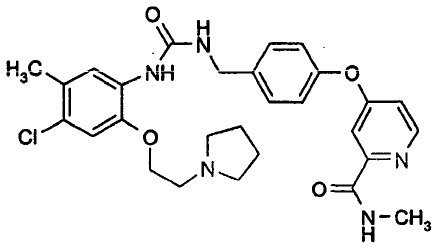 | 1.99 |
| (761) | 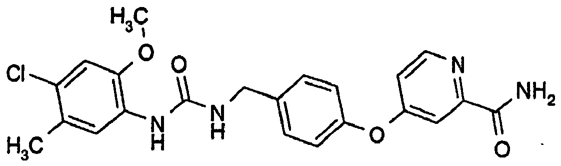 | 2.55 |
| (762) | 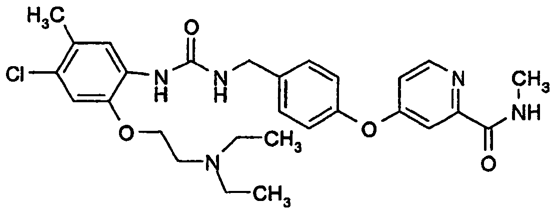 | 2.00 |
| (763) | 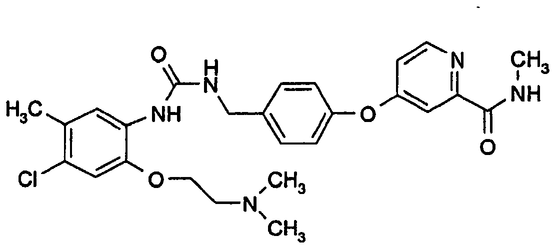 | 1.95 |
| (764) | 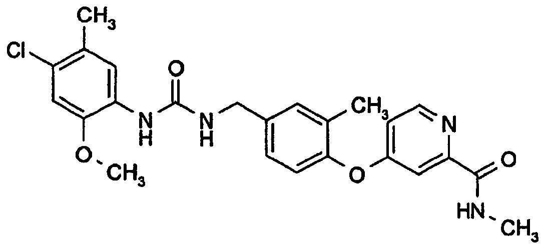 | 3.39^{b} |
| (765) | 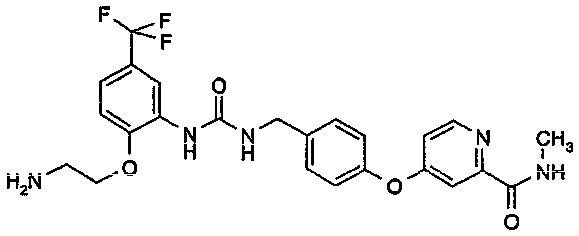 | 1.99 |
| (766) | 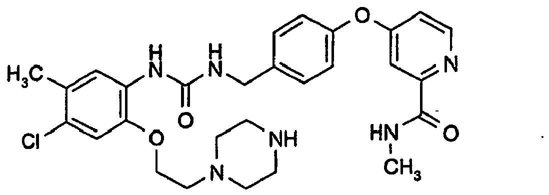 | 1.88 |
| (767) | 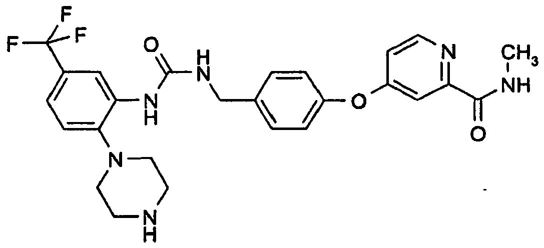 | 2.02 |
| (768) | 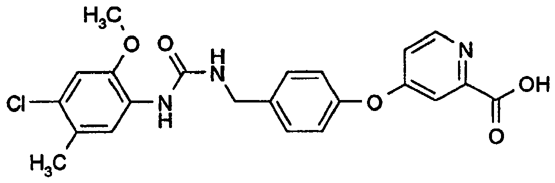 | 2.11 |
| (769) | 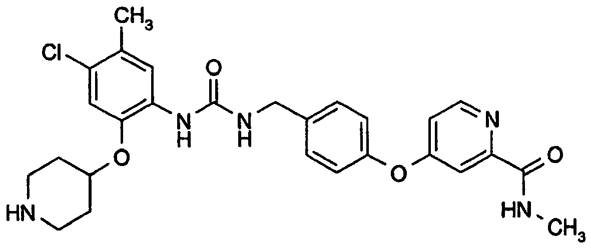 | 2.01 |
| (770) | 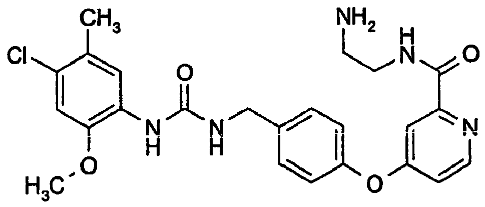 | 2.07 |
| (771) | 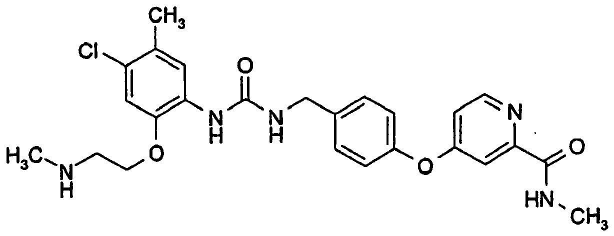 | 2.01 |
| (772) | 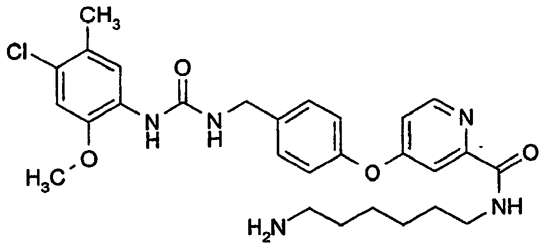 | 2.23 |
| (773) | 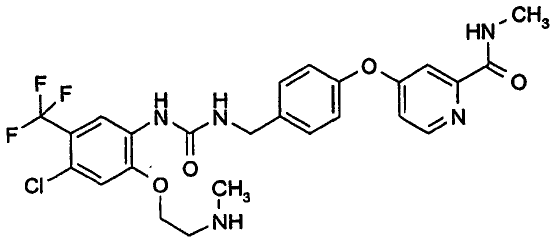 | 2.12 |
| (774) | 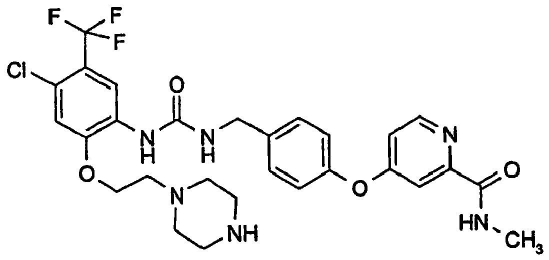 | 2.11 |
| (775) | 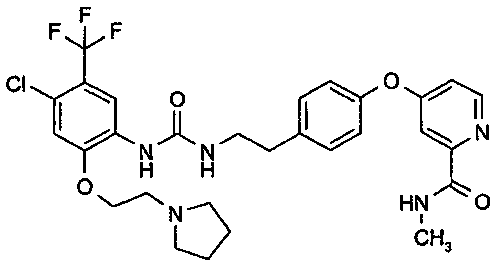 | 2.2 |
| (776) | 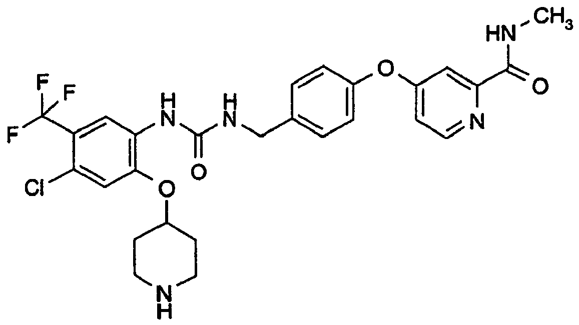 | 2.19 |
| (777) | 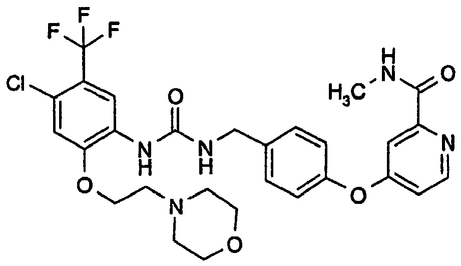 | 2.18 |
| (778) | 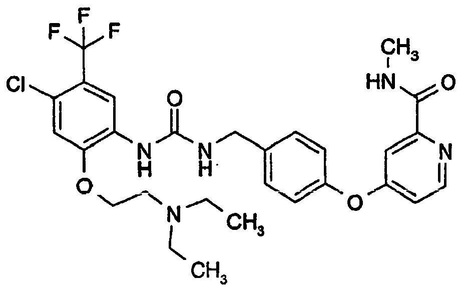 | 2.23 |
| (779) | 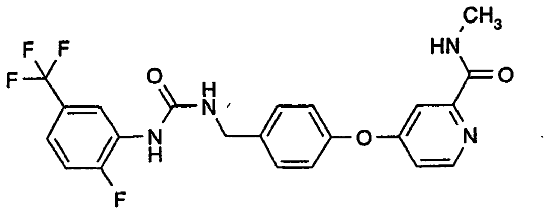 | 2.66 |
| (780) | 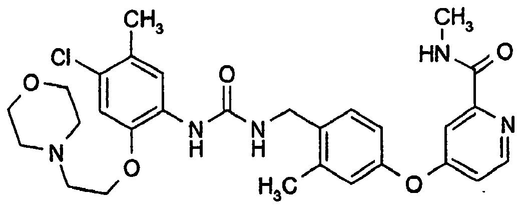 | 2.09 |
| (781) | 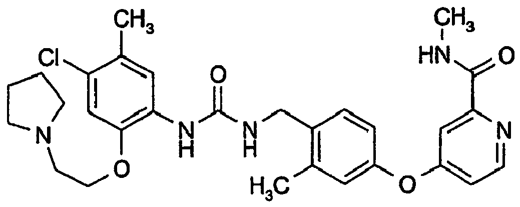 | 2.13 |
| (782) | 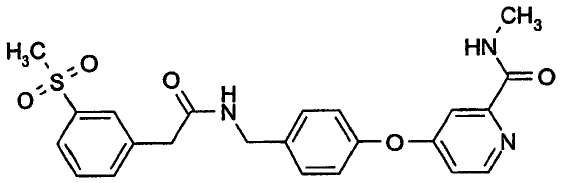 | |
| (783) | 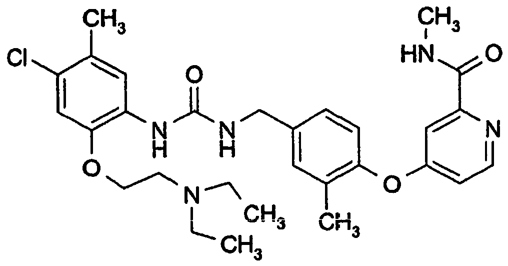 | 3.01^{a} |
| (784) | 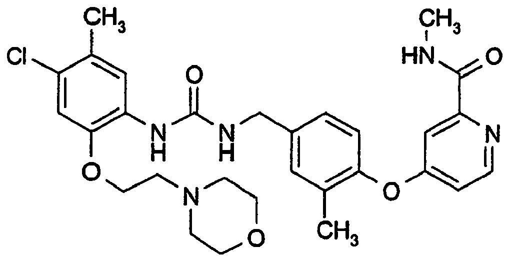 | 2.95^{a} |
| (785) | 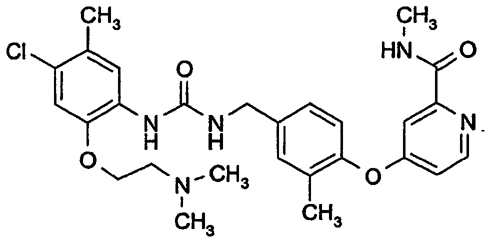 | 2.07^{a} |
| (786) | 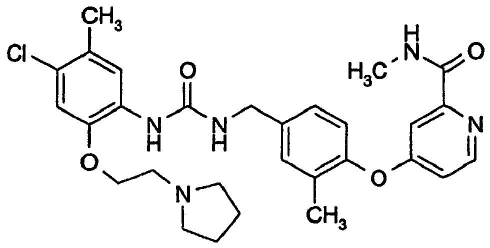 | 3.01^{a} a |
| (787) | 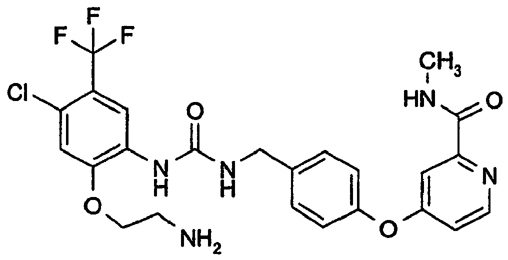 | 2.11 |
| (788) | 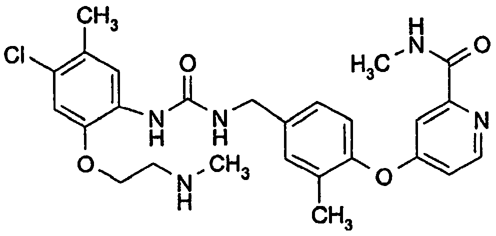 | 2.03 |
| (789) | 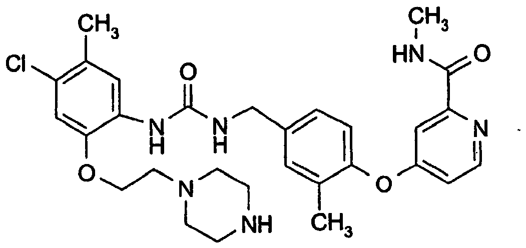 | 1.93 |
| (790) | 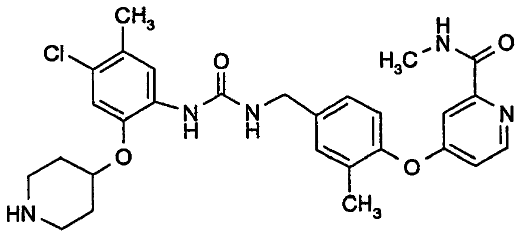 | 2.08 |
| (791) | 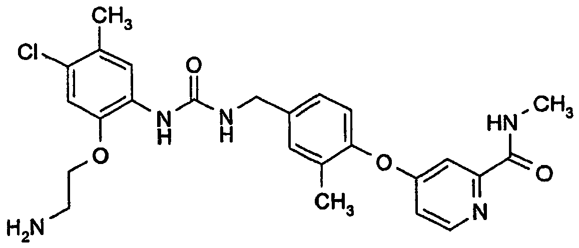 | 2.05 |
| (792) | 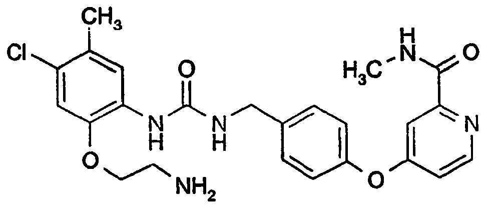 | 1.97 |
| (792b) | 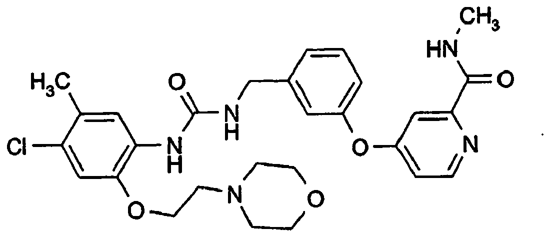 | 2.89b |
| (793) | 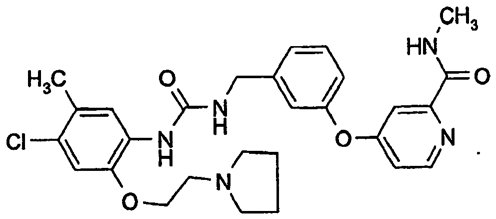 | 2.93 |
| (794) | 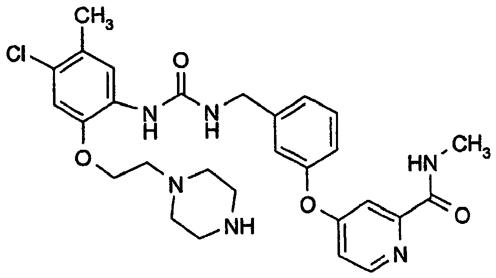 | 1.93 |
| (795) | 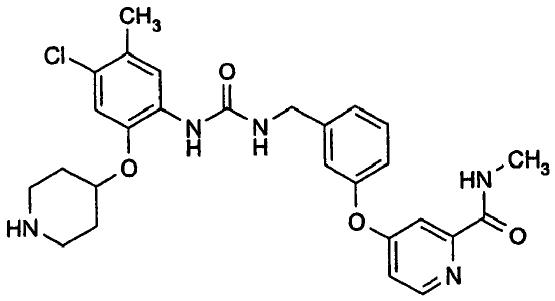 | 2.03 |
| (796) | 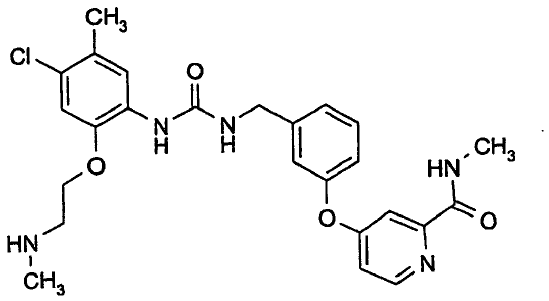 | 2 |
| (797) | 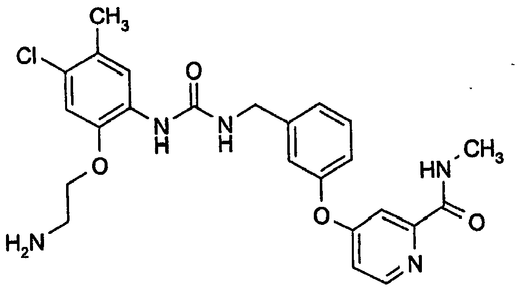 | 1.97 |
| (798) | 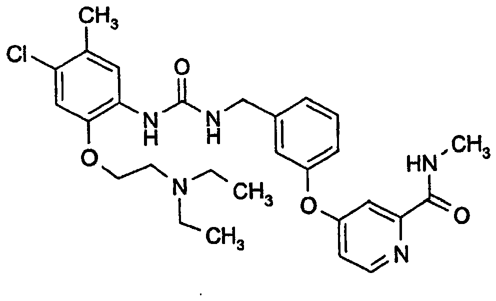 | 2.91 |
| (799) | 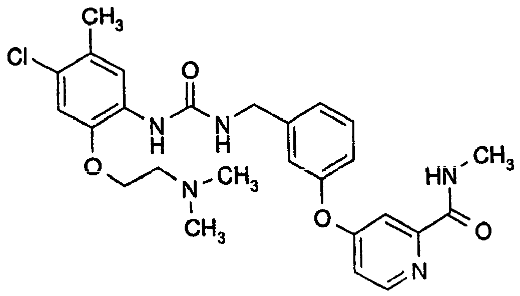 | 2.91 |
| (800) | 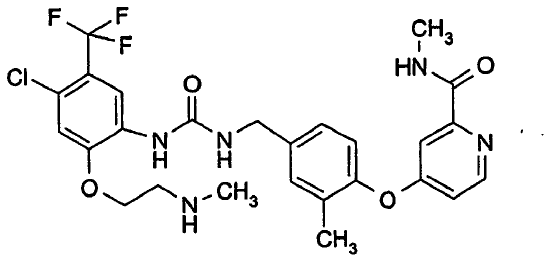 | 2.23 |
| (801) | 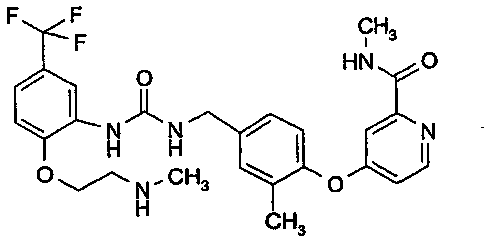 | 2.09 |
| (802) | 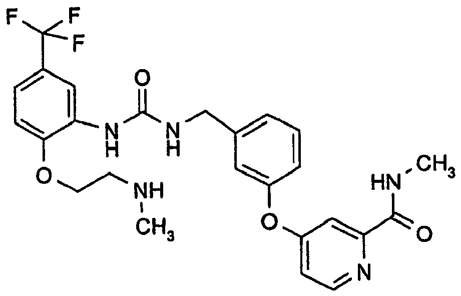 | 2.01 |
| (803) | 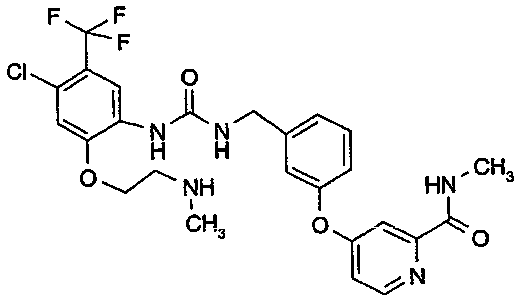 | 2.13 |
| (804) | 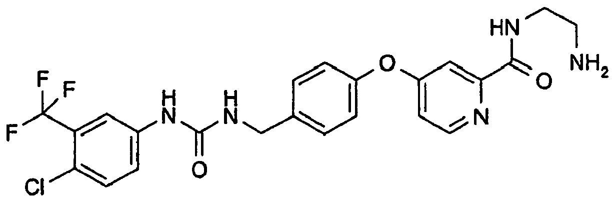 | 2.19 |
| (805) | 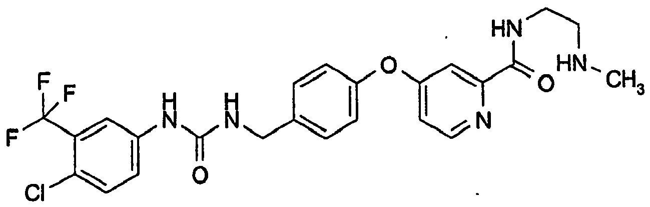 | 2.2 |
| (806) | 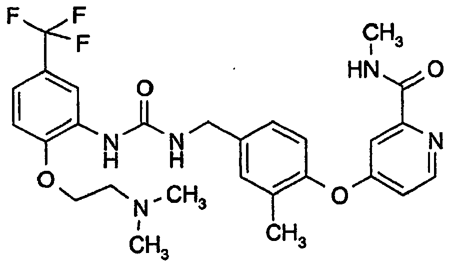 | 2.13 |
| (807) | 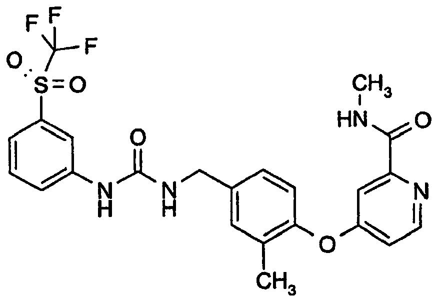 | 2.73 |
| (808) | 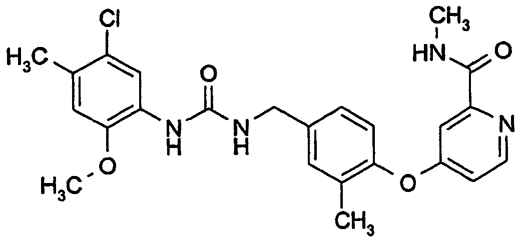 | 2.79 |
| (809) | 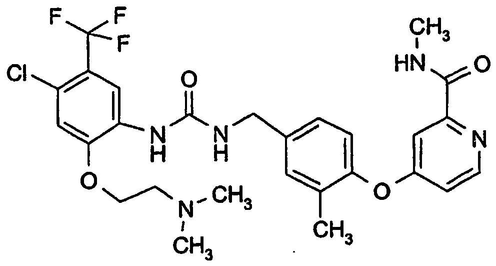 | 2.21 |
| (810) | 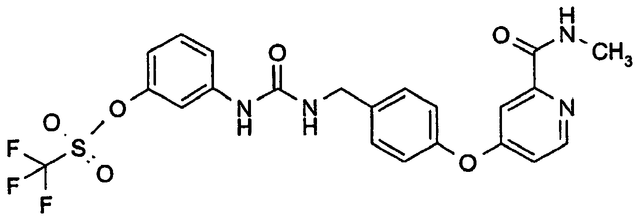 | 5.11^{c} |
| (811) | 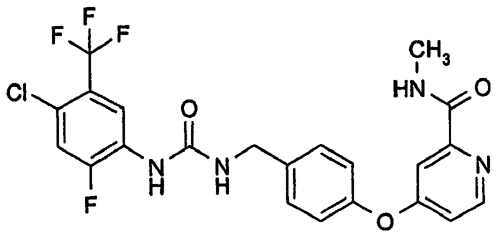 | 2.81 |
| (812) | 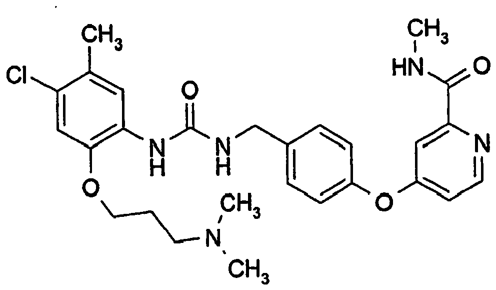 | 2.03^{b} |
| (813) | 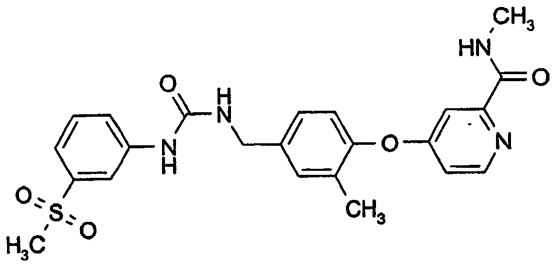 | 1.91^{b} |
| (814) | 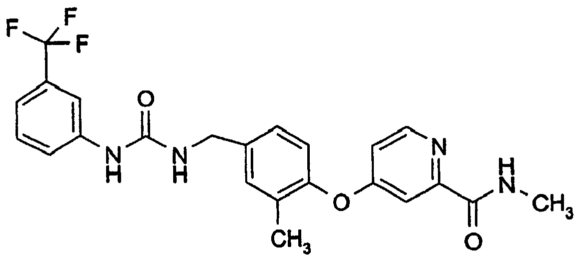 | 2.67 |
| (815) | 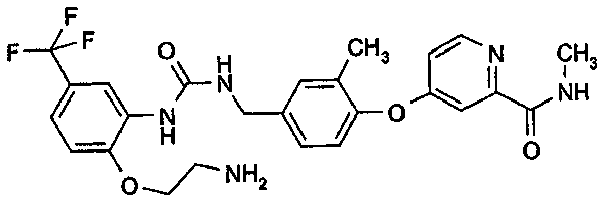 | 2.1 |
| (816) | 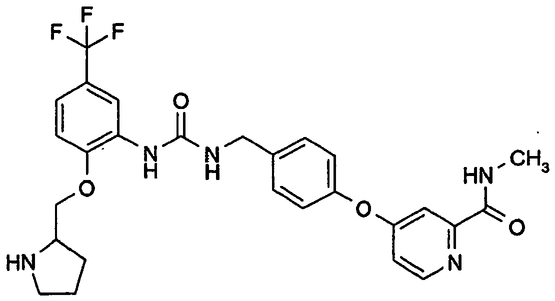 | 2.11 |
| (817) | 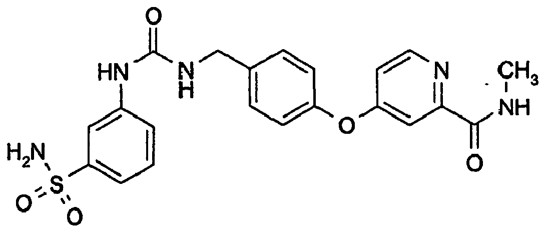 | 1.65^{b} |
| (818) | 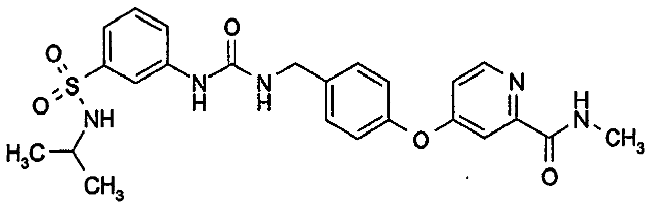 | 2.05^{b} |
| (819) | 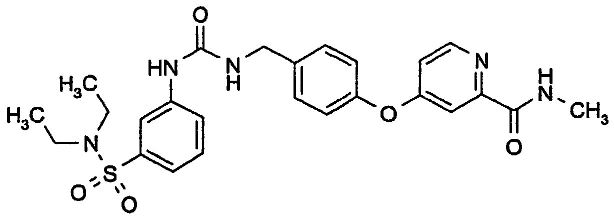 | 2.23^{b} |
| (820) | 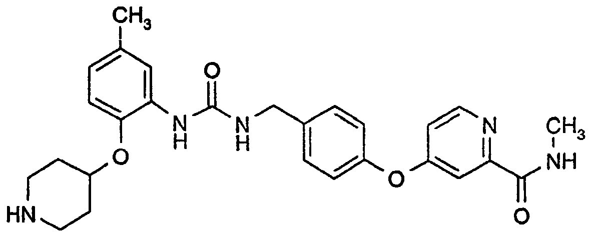 | 1.85^{b} |
| (821) | 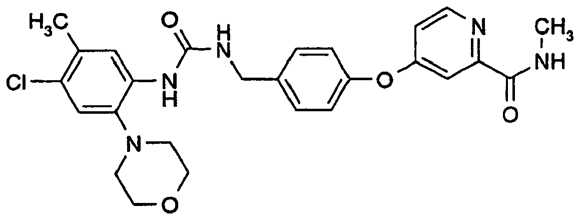 | 2.41^{b} |
| (822) | 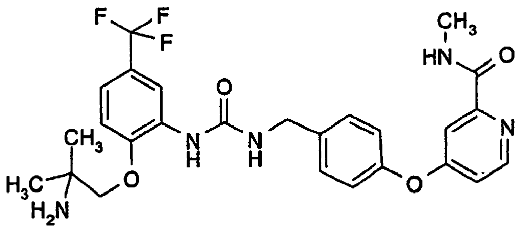 | 2.11 |
| (823) | 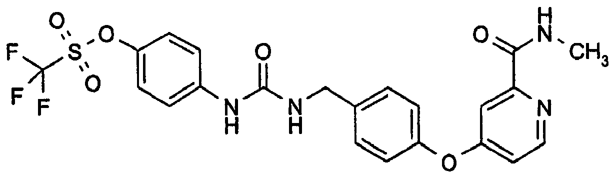 | 5.05^{c} |
| (824) | 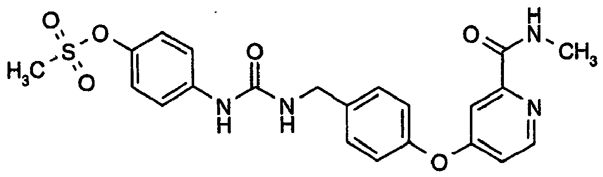 | 4.07^{c} |
| (825) | 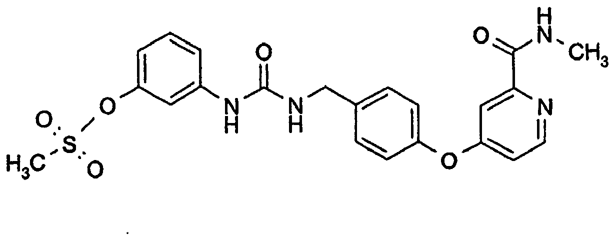 | 4.18^{c} |

The present invention further relates to compounds (826) to (874) as given in the table below:

| Compound | | Rt |
|---|---|---|
| (826) | 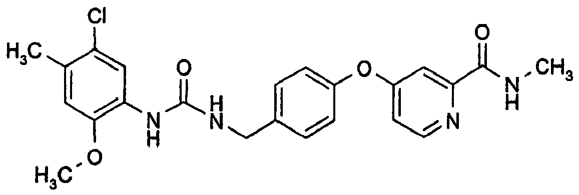 | |
| (827) | 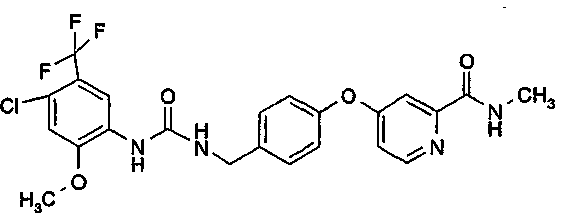 | |
| (828) | 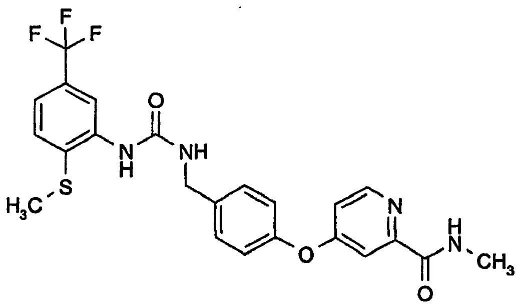 | |
| (829) | 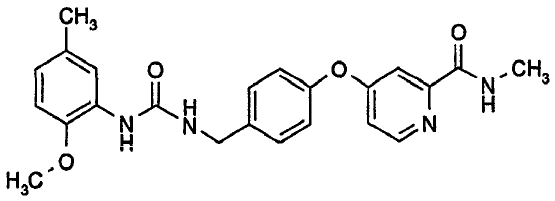 | |
| (830) | 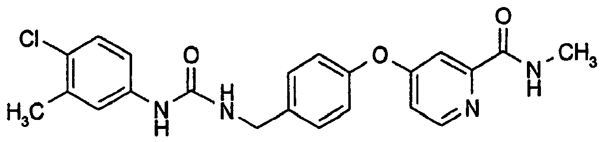 | |
| (831) | 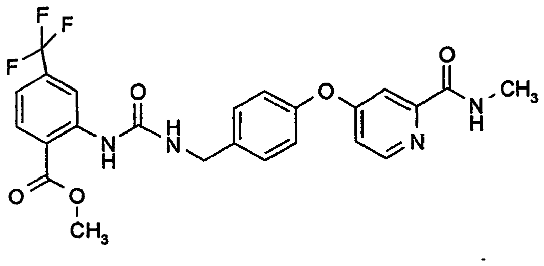 | |
| (832) | 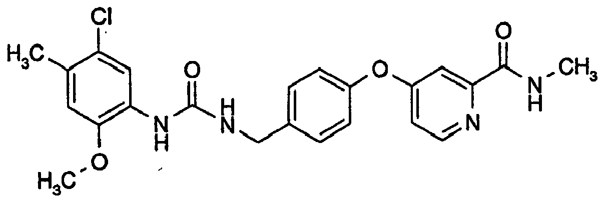 | 2.67 |
| (833) | 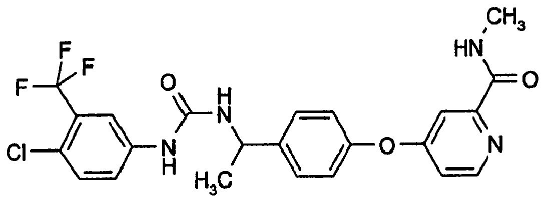 | |
| (834) | 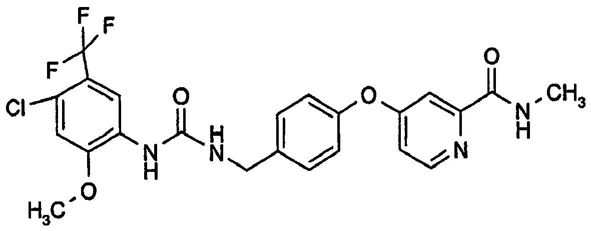 | 2.81 |
| (835) | 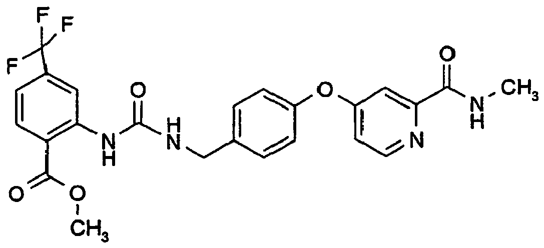 | 2.83 |
| (836) | 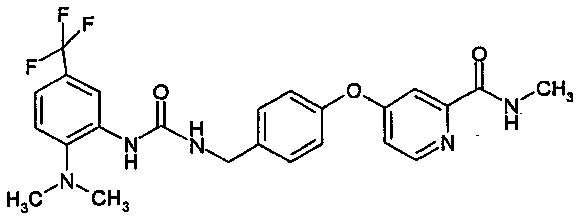 | 2,69 |
| (837) | 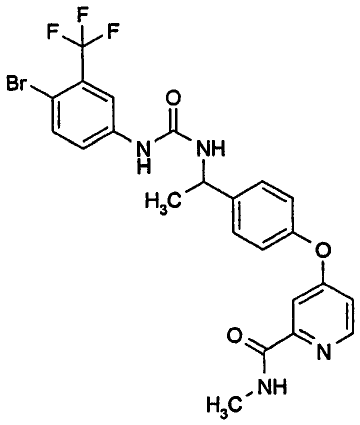 | |
| (838) | 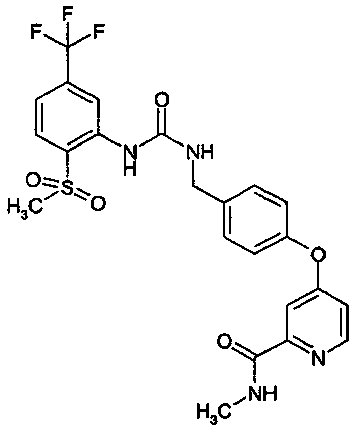 | 2.61 |
| (839) | 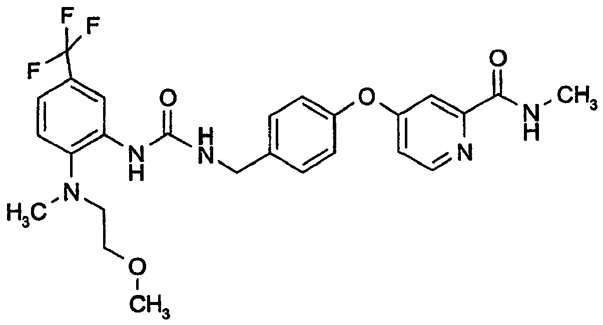 | 2.84 |
| (840) | 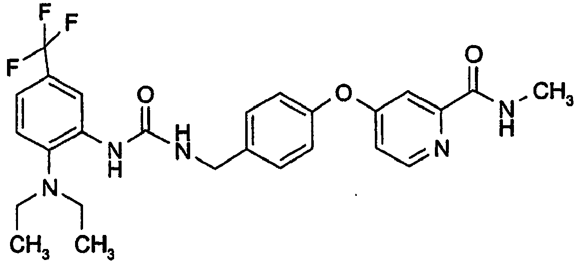 | 2.88 |
| (841) | 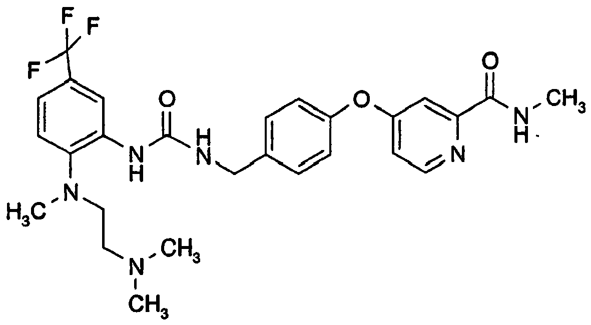 | 2.09 |
| (842) | 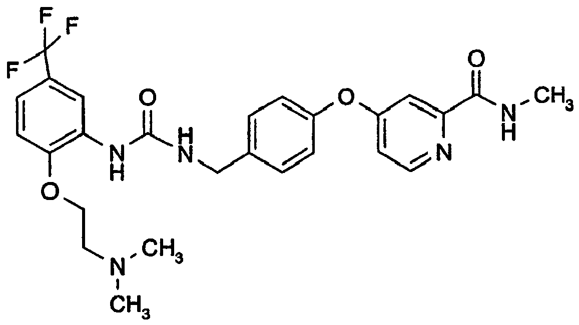 | 2.02 |
| (843) | 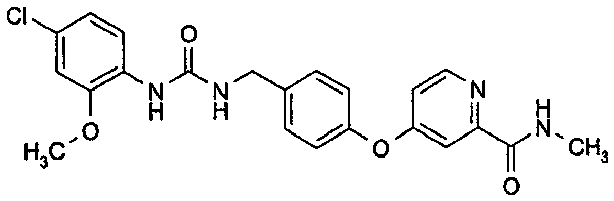 | 2.54 |
| (844) | 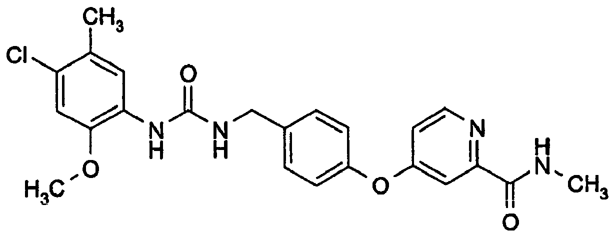 | 2.64 |
| (845) | 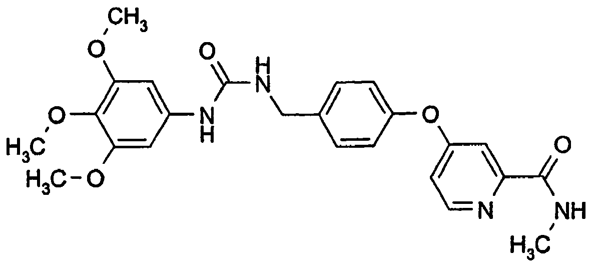 | 3.85^{a} |
| (846) | 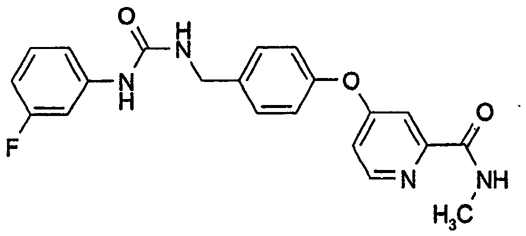 | 4.43^{a} |
| (847) | 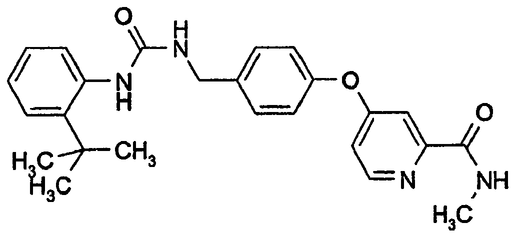 | 4.69^{a} |
| (848) | 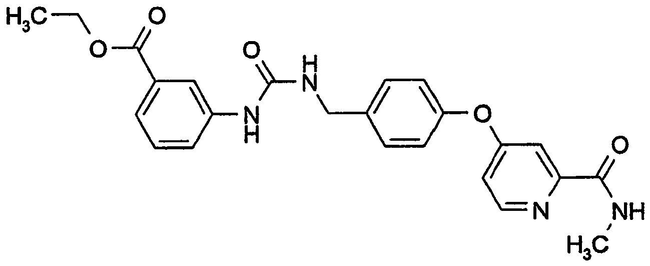 | 4.56^{a} |
| (849) | 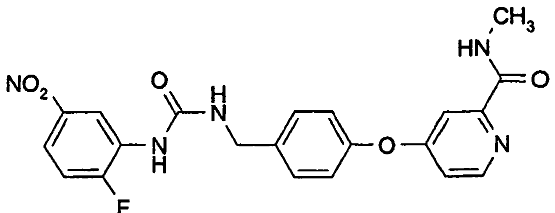 | 4.60^{a} |
| (850) | 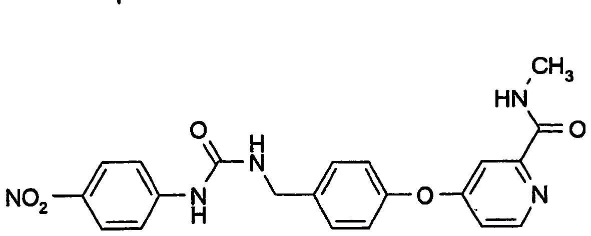 | 4.53 |
| (851) | 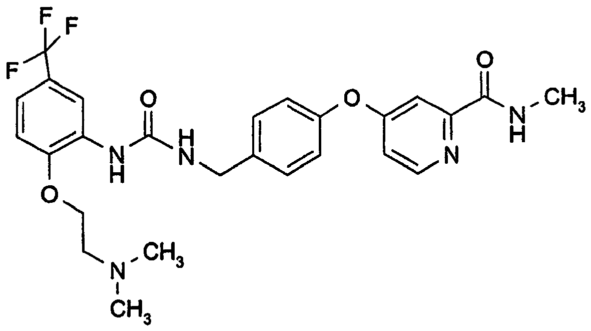 | |
| (852) | 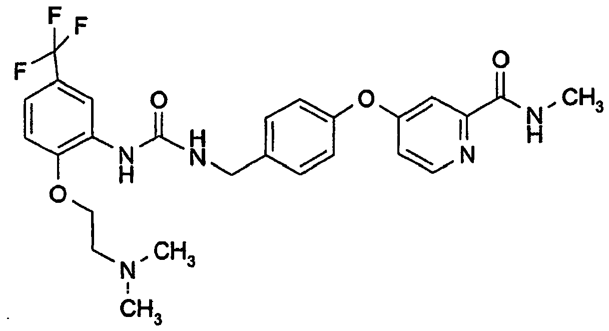 | |
| (853) | 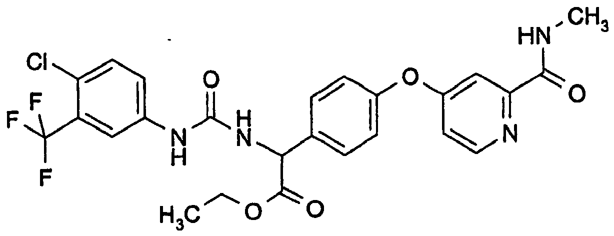 | |
| (854) | 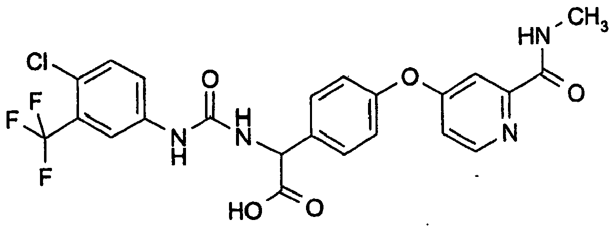 | |
| (855) | 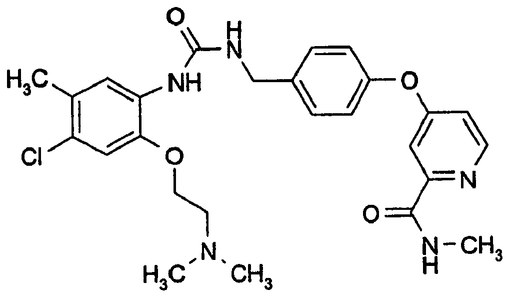 | |
| (856) | 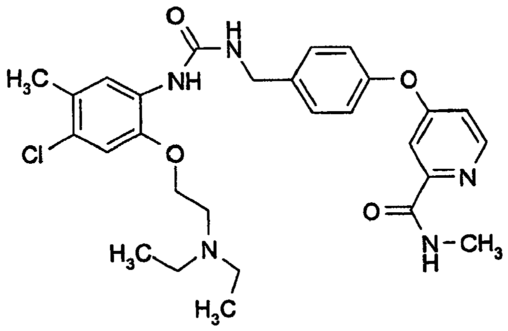 | |
| (857) | 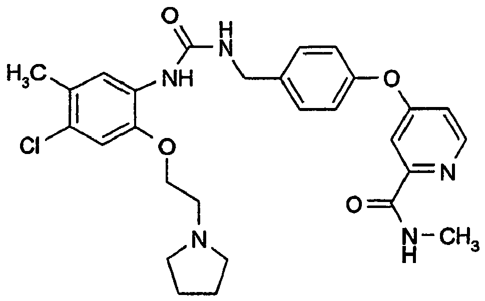 | |
| (858) | 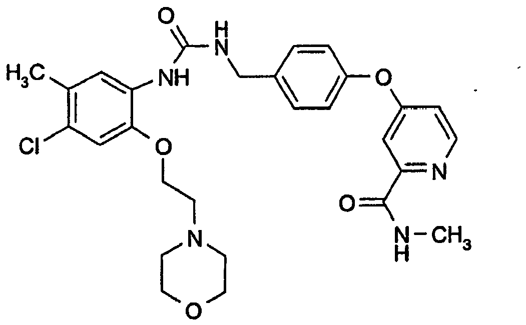 | |
| (859) | 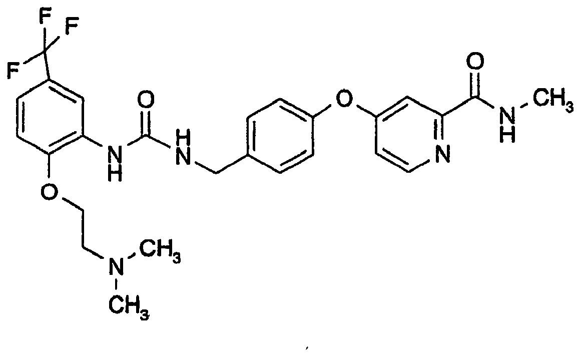 | |
| (860) | 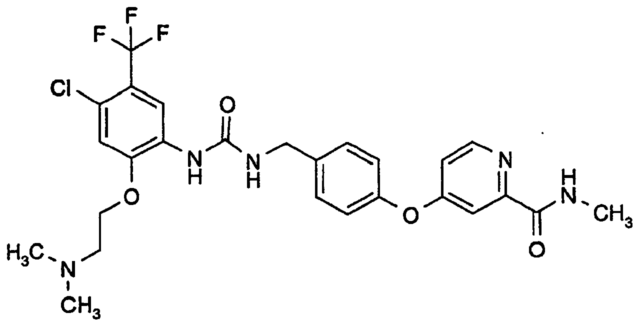 | |
| (861) | 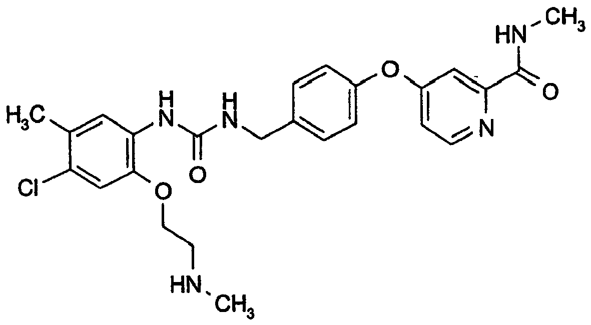 | |
| (862) | 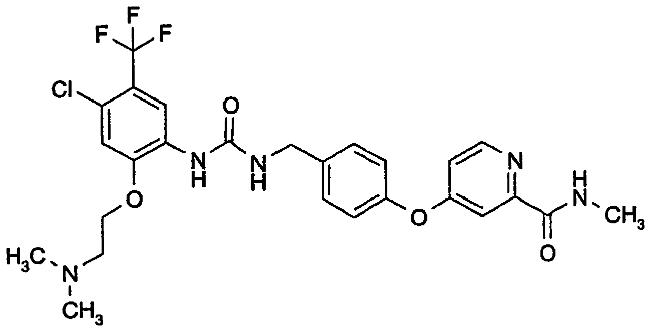 | |
| (863) | 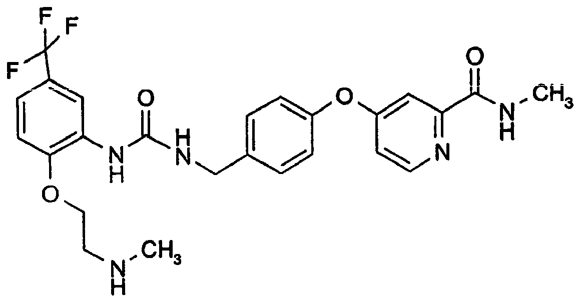 | |
| (864) | 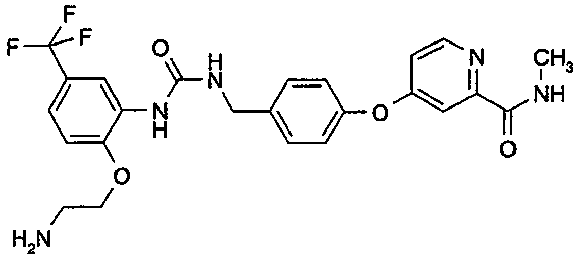 | |
| (865) | 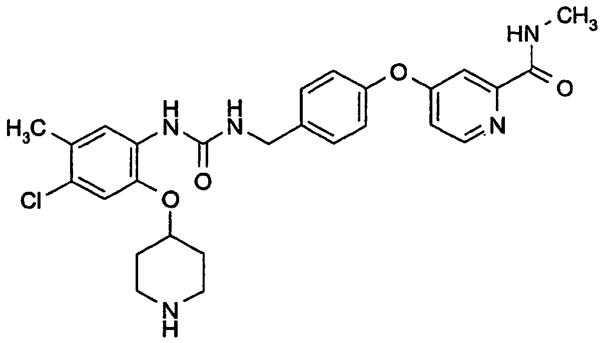 | |
| (866) | 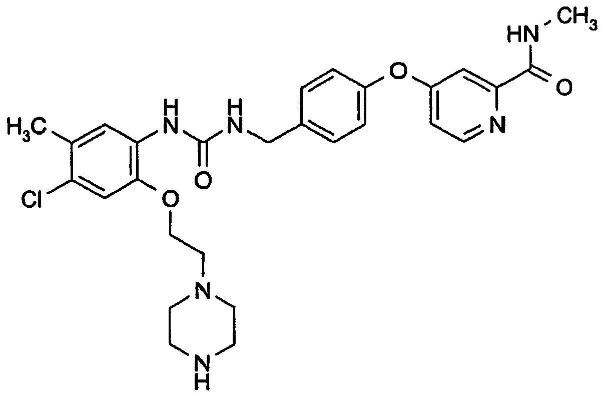 | |
| (867) | 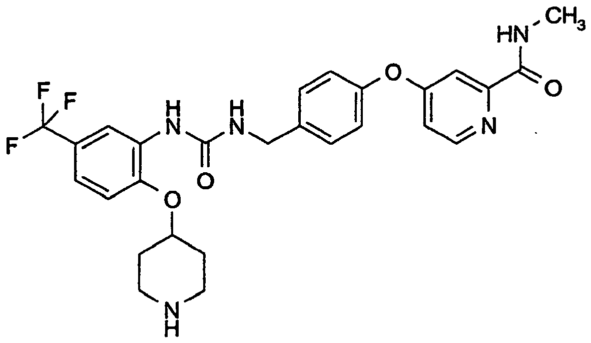 | |
| (868) | 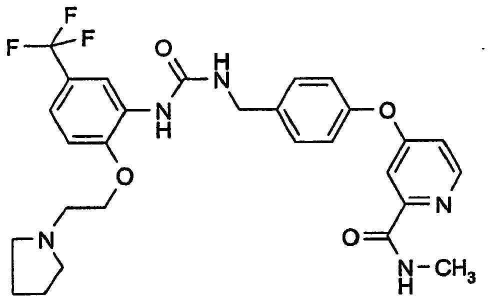 | |
| (869) | 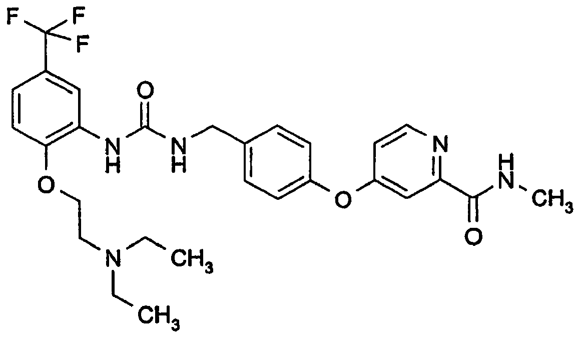 | |
| (870) | 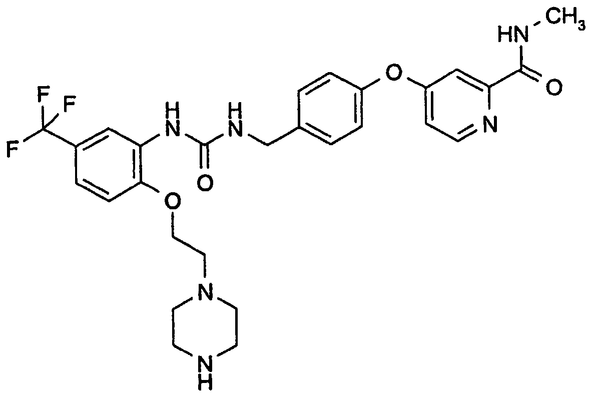 | |
| (871) | 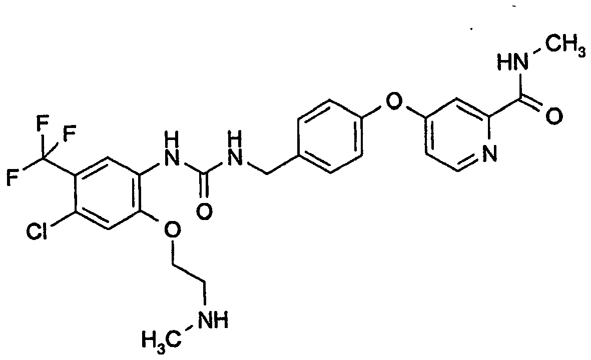 | |
| (872) | 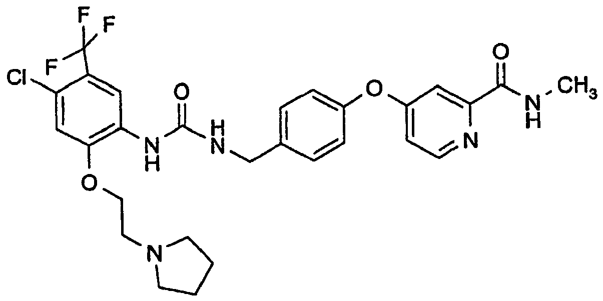 | |
| (873) | 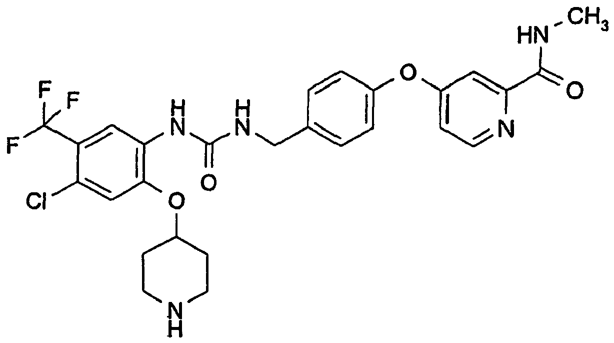 | |
| (874) | 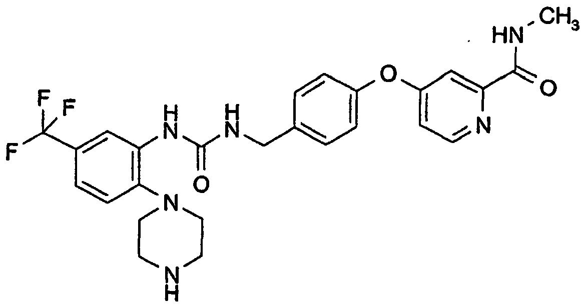 | |

The nomenclature as used herein for defining compounds, especially the compounds according to the invention, is in general based on the rules of the IUPAC-organisation for chemical compounds and especially organic compounds.

In a special embodiment, one or more of the methylene urea derivatives according to sub formulae IIa to IIx and/or compounds (1) to (224), compounds (225) to (448), compounds (449) to (672), compounds (673) to (758), compounds (759) to (825) and/or compounds (826) to (874) additionally comprise one or two substituents selected from the group consisting of O(CH₂)ₙNR¹¹R¹², NR¹¹(CH₂)ₙNR¹¹R¹², O(CH₂)ₙOR¹² and NR¹¹(CH₂)ₙOR¹²,
wherein
- R¹¹, R¹²: are independently selected from a group consisting of H, A, (CH₂)ₘAr³ and (CH₂)ₘHet, or in NR¹¹R¹²,
- R¹¹ and R¹²: form, together with the N-Atom they are bound to, a 5-, 6- or 7-membered heterocyclus which optionally contains 1 or 2 additional hetero atoms, selected from N, O an S, and
- n i: is 1, 2, 3, 4, 5 or 6, preferably 2, 3 or 4.

In this special embodiment, the substituents are preferably selected from the group consisting of HNCH₂CH₂NH₂, OCH₂CH₂NH₂, NHCH₂CH₂OH, OCH₂CH₂NHCH₃, N(CH₃)CH₂CH₂NH₂, HN(CH₃)CH₂CH₂NH, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, OCH₂CH₂N(CH₂CH₃)₂ and compounds of the formulae 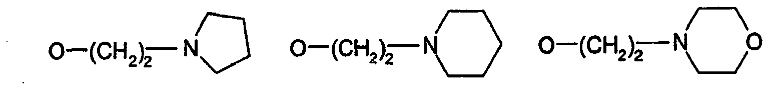 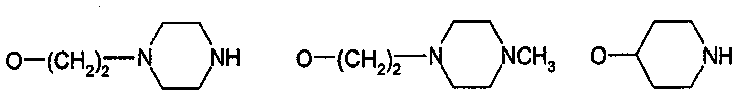 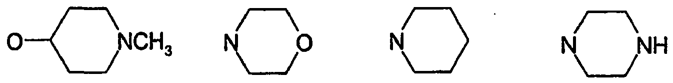 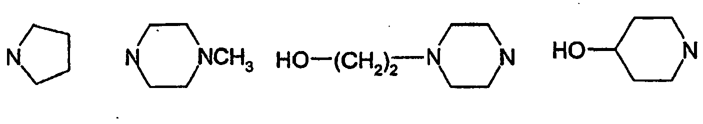 and/or compounds of formulae

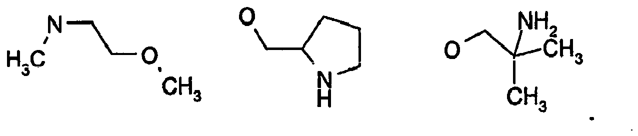

In a further special embodiment, one or more of the methylene urea derivatives according to sub formulae IIa to IIx and/or compounds (1) to (224), compounds (225) to (448), compounds (449) to (672), compounds (673) to (758), compounds (759) to (825) and/or compounds (826) to (874) additionally comprise one or two substituents selected from the group consisting of (CH₂)ₙS(O)ᵤNR¹¹R¹² and (CH₂)ₙS(O)ᵤR¹³ wherein R¹¹, R¹² and R¹³ are defined as above and n is as defined above, preferably n is 0, 1 or 2 and especially is 0, and u is preferably 2 or 3. In this embodiment, the residues are preferably selected from SO₂CH₃, SO₂CF₃, OSO₂CH₃, OSO₂CF₃, SO₂NH₂, SO₂NHCH(CH₃)₂, SO₂N(CH₃)₂, SO₂N(CH₂CH₃)₂ and 4-Morpholino-sulfonyl.

In this special embodiments, the additional substituents are preferably bound to one of the aromatic residues directly bound to the methylene urea moiety and/or the pyridinyl residue. More preferably, one or two additional substituents are bound to the residue Ar¹ according to formula II. Even more preferably, in one or more of the formulae IIa to IId, one or two additional substituents are bound to the phenyl moiety directly bound to the nitrogen atom of the methylene urea moiety, i. e. the phenyl moiety at the left hand side of the respective formulae. Especially preferred are compounds (1) to (224), compounds (225) to (448) and/or compounds (449) to (672), wherein one or two additional substituents are bound to the moiety A.

Another aspect of the invention relates to a method for producing compounds of formula II, characterised in that
a) A compound of formula III 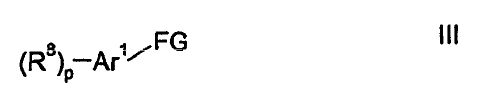 wherein
   - FG: is a functional group, selected from
   -N=C=Y and -NH-(C=Y)-LG,
   wherein Y is as defined above and below, LG is a leaving group, preferably a leaving group selected from OR²⁵ and CHal₃, wherein R²⁵ is selected from the group consisting of unsubstituted or substituted aromatic residues, unsubstituted or substituted heteroaromatic residues and (O)₂S-R²⁶, wherein R²⁶ is selected from unsubstituted or substituted aromatic residues and unsubstituted or substituted alkyl residues residues, and wherein R⁸, p and Ar¹ are as defined above and below,
   is reacted
b) with a compound of formula IV, 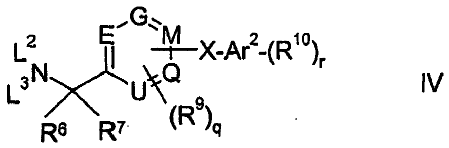 wherein
   - L², L³: are independently from one another H or a metal ion, and R⁶, R⁷, E, G, M, Q, U, R⁹, q, X, Ar², R¹⁰ and r are as defined above and below,
   and optionally
c) isolating and/or treating the compound of formula II obtained by said reaction withan acid, to obtain the salt thereof.

The compounds of the formula I and preferably the compounds of the formula II and also the starting materials for their preparation are, in addition, prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and suitable for the said reactions. Use can also be made here of variants which are known per se, but are not mentioned here in greater detail.

If desired, the starting materials can also be formed in situ by not isolating them from the reaction mixture, but instead immediately converting them further into the compounds of the formula I or II, respectively. On the other hand, it is possible to carry out the reaction stepwise.

The compounds of the formula I and especially the compounds of formula II can preferaby be obtained by reacting compounds of the formula III with compounds of the formula IV.
In compounds of formula III, the group FG is a suitable functional group that this preferably selected from -N=C=Y and -NH-(C=Y)-LG. In the functional groups -N=C=Y and/or -NH-(C=Y)-LG, Y is preferably selected from the group consisting of O, S, NR²¹, C(R²²)-NO₂, C(R²²)-CN and C(CN)₂, and more preferably selected from O, S and NR²¹, even more preferably selected from O and S and especially is O, wherein R²¹ and R²² are as defined above/below.

In the compounds of formula III, wherein FG is -NH-(C=Y)-LG, LG is a suitable leaving group. Suitable leaving groups are known in the art, for example from Houben-Weyl, Methods of Organic chemistry. Preferably, the leaving group is selected from CHal₃, wherein Hal is as defined above/below and preferably is chlorine or bromine and especially is chlorine, and OR²⁵, wherein R²⁵ is selected from the group consisting of unsubstituted or substituted aromatic residues, unsubstituted or substituted heteroaromatic residues and (O)₂S-R²⁶, wherein R²⁶ is selected from unsubstituted or substituted aromatic residues and unsubstituted or substituted alkyl residues residues, and wherein R⁸, p and Ar¹ are as defined above and belowl.

In compounds of formula III in which FG is -NH-(C=Y)-LG and LG is CHal₃, Hal is preferably selected independently from one another from the group consisting of chlorine, bromine and iodine, even more preferably chlorine and bromine and especially preferred chlorine. Preferably, CHal₃ is selected from the group consisting of CCl₃ and CBr₃ and especially preferred CHal₃ is CCl₃.

In compounds of formula III in which FG is -NH-(C=Y)-LG and LG is OR²⁵, R²⁵ is preferably selected from unsubstituted or substituted phenyl moieties, preferably substituted phenyl moieties which comprises one or more nitro groups (-NO₂) and/or one or more sulfonic acid groups (-SO₃H) or salts thereof as substituents, and (O)₂S-R²⁶, wherein R²⁶ is selected from unsubstituted or substituted phenyl moieties, preferably alkyl substituted phenyl moieties, and unsubstituted or substituted alkyl residues residues, preferably unsubstituted or substituted C₁-C₄-alkyl moieties and especially unsubstituted or substituted methyl moieties. Substituted alkyl moieties preferably comprise one or more halogen substituents up to perhalo. Preferred as halogen substituents are fluorine and chlorine and especially preferred is chlorine. Especially preferred as substituted alkyl moiety is -CF₃. Examples of preferred leaving groups OR²⁵ are the para-Tosyl- (i. e. p-Me-C₆H₄-SO₃-) group, the para-Nitro-phenolate-group (i.e the p-O₂N-C₆H₄-O-) group and the triflate- (i. e. the F₃C-SO₃-) group.

If compounds of formula II are desired wherein Y is other than O, it can be advantageous however to to carry out the reaction of a compound of formula III, wherein Y is O, and a compound of formula IV according to the invention to obtain a compound of formula II, wherein Y is O, and to modify or convert the corresponding C=O group (i. e. the C=Y group, wherein Y is O) in the compound of formula II into a C=NR²¹, C=C(R²²)-NO₂, C=C(R²²)-CN or C=C(CN)₂ group according to methods known in the art, for example from Houben-Weyl, Methods of Organic Chemistry.

In the compounds of formula IV, L² and/or L³ is preferably H or a moiety which activates the amino group it is bonded to, for example a metal ion. Suitable metal ions are preferably selected from the group consisting of alkaline metal ions, alkaline-earth metal ions and aluminium ions. Especially preferred metal ions are alkaline metal ions, of which Li, Na K are especially preferred. In case of multi-valent metal ions, the metal ions and the compounds of formula IV form a complex containing one or more compounds of formula IV and one or more metal ions wherein the ratio between compounds of formula IV and metal ions is depending on the valency of the metal ion(s) according to the rules of stoichiometry and/or electroneutrality. Preferably, L² or L³ and more preferred L² and L³ are hydrogen.

In detail, the reaction of the compounds of the formula III with the compounds of the formula IV is carried out in the presence or absence of a preferaby inert solvent at temperatures between about -20 °C and about 200 °C, preferably between 0 °C and 150 °C and especially between room temperature (25°) and 120°. In many cases, it is advantageous to combine one compound of formula III with one compound of formula IV at the lower end of the given temperature range, preferably between -20 °C and 75 °C, more preferred between 0 °C and 60 °C and especially between 10 °C and 40 °C, for example at about room temperature, and heat the mixture up to a temperature at the upper end of the given temperature range, preferably between 65 °C and 180 °C, more preferred between 75 °C and 150 °C and especially between 80 °C and 120 °C, for example at about 80 °C, at about 90 °C or at about 100 °C. Proceeding in that manner can be advantageous especially in the case that RG is selected from -NH-(C=Y)-LG. If RG is selected from -N=C=Y and preferably is -N=C=O or -N=C=S and especially is -N=C=O, the reaction can be regularly carried out without prolonged heating to higher temperatures. For example it can be carried out at a temperature between 0 °C and 60 °C and preferably at about room temperature.

The reaction between the compounds of formula III, wherein FG is -NH-(C=Y)-LG and especially wherein LG is CHal₃, and compounds of formula IV is preferably carried out in the presence of an acid binding means, for example one or more bases. Suitable acid binding means are known in the art. Preferred as acid binding means are inorganic bases and especially organic bases. Examples for inorganic bases are alkaline or alkaline-earth hydroxides, alkaline or alkaline-earth carbonates and alkaline or alkaline-earth bicarbonates or other salts of a weak acid and alkaline or alkaline-earth metals, preferably of potassium, sodium, calcium or cesium. Examples for organic bases are triethyl amine, diisopropyl ethyl amine (DIPEA), diaza bicyclo undecen (DBU), dimethyl aniline, pyridine or chinoline. If an organic base is used, it is advantageous in general to use a base with a boiling point that is higher than the highest reaction temperature employed during the reaction. Especially preferred as organic bases are DBU and DIPEA. DBU is especially preferred in the case that LG is CHal₃. DIPEA is especially preferred in the case that LG is OR²⁵.

Reaction times are generally in the range between some minutes and several days, depending on the reactivity of the respective compounds and the respective reaction conditions. Suitable reaction times are readily determinable by methods known in the art, for example reaction monitoring. Based on the reaction temperatures given above, suitable reaction times generally lie in the range 10 min and 36 hrs, preferably 30 min and 24 hrs and especially between 45 min and 16 hrs, for example about 1 h, about 2 hrs, about 4 hrs, about 6 or about 16 hrs.

Preferably, the reaction of the compounds of the formula III with the compounds of the formula IV is carried out in the presence of a suitable solvent, that is preferably inert under the respective reaction conditions. Examples of suitable solvents are hydrocarbons, such as hexane, petroleum ether, benzene, toluene or xylene; chlorinated hydrocarbons, such as trichlorethylene, 1,2-dichloroethane, tetrachloromethane, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, dimethylformamide (DMF) or N-methyl pyrrolidinone (NMP); nitriles, such as acetonitrile; sulfoxides, such as dimethyl sulfoxide (DMSO); nitro compounds, such as nitromethane or nitrobenzene; esters, such as ethyl acetate, or mixtures of the said solvents. Polar solvents are in general preferred. Examples for suitable polar solvents are chlorinated hydrocarbons, alcohols, glycol ethers, nitriles, amides and sulfoxides or mixtures thereof. More preferred are chlorinated hydrocarbons, especially dichloromethane, and sulfoxides, especially DMSO.

Preferably, the reaction between a compound of formula III, wherein FG is -N=C=Y and preferably is -N=C=O or-N=C=S and especially is -N=C=O, and a compound of formula IV, especially a compound of formula IV, wherein L² and L³ is H, is carried out in an inert solvent at the lower end of the given temperature range, for example in a chlorinated hydrocarbon, for example dichloromethane, in a temperature range between 0 °C and 60 °C, preferably at about room temperature. Reaction times generally lie in the range of 2 hours to 24 hrs, for example at about 16 hrs. Preferably, no acid binding means is present.

Preferably, the reaction between a compound of formula III, wherein FG is -NH-(C=Y)-LG and especially wherein LG is CHal₃, and compounds of formula IV, especially a compound of formula IV, wherein L² and L³ is H, is carried out in an inert solvent, preferably a solvent boiling at higher temperatures, for example a sulfoxide and especially DMSO, in a temperature range between 60 °C and 120 °C, for example at about 80 °C. Reaction times generally lie in the range of 1 hrs to 10 hrs, for example between 2 and 6 hrs. Preferably, the reaction is carried out in the presence of an acid binding means, preferably one of the afore mentioned acid binding means, more preferably an organic base and especially in the presence of DBU.

Preferably, the reaction between a compound of formula III, wherein FG is -NH-(C=Y)-LG and especially wherein LG is OR²⁵, and compounds of formula IV, especially a compound of formula IV, wherein L² and L³ is H, is carried out in an inert solvent at the lower end of the given temperature range, for example in a chlorinated hydrocarbon, for example dichloromethane, in a temperature range between 0 °C and 60 °C, preferably at about room temperature. Reaction times generally lie in the range of 2 hours to 24 hrs. Preferably, the reaction is carried out in the presence of an acid binding means, preferably one of the afore mentioned acid binding means , more preferably an organic base and especially in the presence of DIPEA.

In general, the compounds of formula III and/or formula IV are new. In any case, they can be prepared according to methods known in the art.

The compounds of formula III can be obtained according to methods known in the art. In an advantageous manner, they can be readily obtained by one or more of the reaction routes given below:

Compounds of formula III, wherein FG is -N=C=Y and Y is O or S can be readily obtained from suitable substituted derivatives of (R⁸)ₚ-Ar¹ according to known procedures for producing isocyanates and thioisocyanates. When FG is -N=C=O, the compounds of formula III can be readily obtained via Curtius-, Hoffmann or Lossen rearrangement starting from (R⁸)ₚ-Ar¹-COOH or the respective acid halides, as described in the art. If desired, compounds of formula III, wherein Y is O can be readily derivatized to compounds of formula III, wherein Y is S, according to procedures known in the art.

Compounds of formula III, wherein FG is -NH-(C=Y)-LG and especially wherein LG is CHal₃ can be readily obtained from the reaction of suitable amino substituted derivatives of (R⁸)p-Ar¹ of formula V 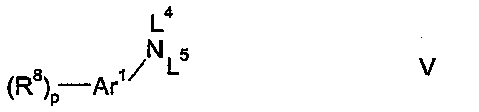 wherein R⁸, p, and Ar¹ are as defined above/below and L⁴ and L⁵ are selected independently from each other from the meanings given for L² and L³ and more preferred are hydrogen, with a compound of formula VI 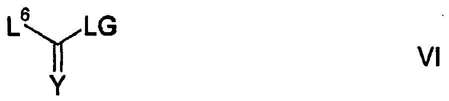 wherein Y is as defined above/below and L⁶ is preferably selected from Cl, Br, I, OH, reactive derivatized OH-moieties, especially reactive esterified OH-moieties, for example alkylsulfonyloxy-moieties comprising 1 to 6 carbon atoms (preferably methylsulfonyloxy) or and arylsulfonyloxy-moiety comprising 6 to 10 carbon atoms (preferably phenyl- oder p-tolylsulfonyloxy), and diazonium moieties, and more preferred selected from Cl, Br or I, and even more preferred is Cl.

Compounds of formula III, wherein FG is -NH-(C=Y)-LG and especially wherein LG is CHal₃ can be readily obtained from the reaction of suitable amino substituted derivatives of (R⁸)ₚ-Ar¹ of formula V 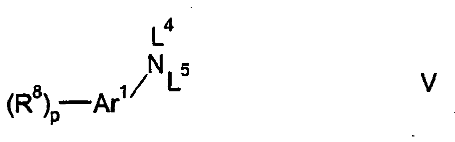 wherein R⁸, p, and Ar¹ are as defined above/below and L⁴ and L⁵ are selected independently from each other from the meanings given for L² and L³ and more preferred are hydrogen, with a compound of formula Via 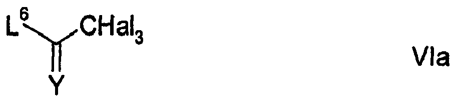 wherein Y and L⁶ are as defined above/below.

The reaction between compounds of formula V and compounds of formula VI can be carried out in the presence of a suitable solvent, that is preferably inert at the chosen reaction conditions. Suitable solvents are known in the art. Exemples of suitable solvents include hydrocarbons, such as hexane, petroleum ether, benzene, toluene or xylene; chlorinated hydrocarbons, such as trichloroethylene, 1,2-dichloroethane, tetrachloromethane, chloroform or dichloromethane; ethers, such as diethyl ether, diisopropyl ether, tetrahydrofuran (THF) or dioxane; nitriles, such as acetonitrile; esters, such as ethyl acetate, or mixtures of said solvents. Non-protic solvents are in general preferred.

The reaction between compounds of formula V and compounds of formula VI can be carried out in the presence of a suitable acid binding means, especially organic or anorganic bases. Examples for inorganic bases are alkaline or alkaline-earth hydroxides, alkaline or alkaline-earth carbonates and alkaline or alkaline-earth bicarbonates or other salts of a weak acid and alkaline or alkaline-earth metals, preferably of potassium, sodium, calcium or cesium. Examples for organic bases are triethyl amine, diisopropyl ethal amine (DIPEA), diaza bicyclo undecan (DBU), dimethyl aniline, pyridine or chinoline. If an organic base is used, it is advantageous in general to use a base with a boiling point that is higher than the highest reaction temperature employed during the reaction.

The reaction between compounds of formula V and compounds of formula VI can be carried out in the presence of a suitable solvent, that is preferably inert at the chosen reaction conditions. Suitable solvents are known in the art. Exemples of suitable solvents include hydrocarbons, such as hexane, petroleum ether, benzene, toluene or xylene; chlorinated hydrocarbons, such as trichloroethylene, 1,2-dichloroethane, tetrachloromethane, chloroform or dichloromethane; ethers, such as diethyl ether, diisopropyl ether, tetrahydrofuran (THF) or dioxane; nitriles, such as acetonitrile; esters, such as ethyl acetate, or mixtures of said solvents. Non-protic solvents are in general preferred.

If the reaction between a compound of formula V and a compound of formula VI is carried out in presence of an organic base that is liquid at the chosen reaction conditions, it can be advantagous not to add an additional solvent.

Compounds of formula III, wherein FG is -NH-(C=Y)-LG and preferably wherein LG is OR²⁵ and especially wherein R²⁵ is an unsubstituted or substituted phenyl moiety, can be readily obtained from the reaction of suitable amino substituted derivatives of (R⁸)ₚ-Ar¹ of formula V 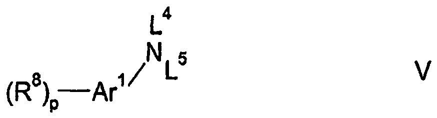 wherein R⁸, p, and Ar¹ are as defined above/below and L⁴ and L⁵ are selected independently from each other from the meanings given for L² and L³ and more preferred are hydrogen, with a compound of formula Vlb 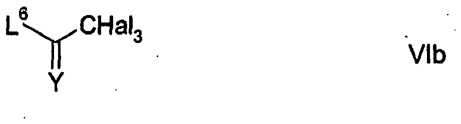 wherein Y and L⁶ are as defined above/below.

Suitable reaction conditions for carrying out reaction of compounds of formula V with compounds of formula VI, VIa and VIb, respectively, are known in the art. In detail, the reaction of the compounds of the formula V with the compounds of the formula VI is carried out in the presence or absence, preferably in the presence of an inert solvent, preferably one of the afore mentioned inert solvents, more preferably ethers and chlorinated hydrocarbons, and especially in dichloromethane, preferably in a temperature range between 0 °C and 60 °C and more preferably at about room temperature.

The reaction between compounds of formula V and compounds of formula VI is preferably carried out in the presence of an acid binding means, for example one or more bases. Suitable acid binding means are known in the art. Preferred as acid binding means are organic bases, more preferably one of the afore mentioned organic bases and especially pyridine.

In general, the reaction times for the reaction between compounds of formula V and compounds of formula VI lie in the range between 6 hrs and 36 hrs, preferably 12 hrs to 24hrs, for example at about 16 hrs.

Some of the starting materials of the formula V and/or the formula VI are known and preferably commercially available. If they are not known, they can be prepared by methods known per se.

The compounds of formula IV can be obtained according to methods known in the art.

If the compound of formula IV is a compound according to formula IVa, 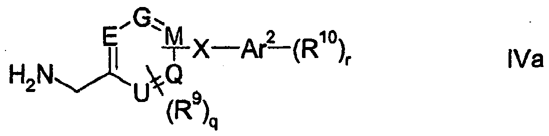 it can be readily obtained in an advantageous manner (reaction route A) by reacting a compound of formula VIIa, 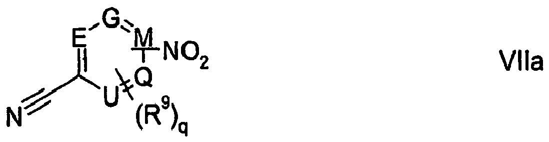 wherein E, G, M, Q, U, R⁹ and q are as defined above/below,
with a compound of formula VIII,

L⁸-X-Ar²-(R¹⁰)ᵣ VIII

wherein L⁸ is H or a metal ion, preferably a metal ion selected from the group consisting of alkaline metal ions, alkaline-earth metal ions and aluminum ions, especially preferred alkaline metal ions, of which Li, Na and K are especially preferred, and even more preferred is H; and Ar², R¹⁰, r and X are as defined above/below, and preferably wherein X is (CHR¹¹)ₕ-Q-(CHR¹²)ᵢ, wherein R¹¹, h and i and R¹² are defined above/below, and more preferred wherein h and/or i is 0 and Q is selected from a group consisting of O, S, N-R¹⁵, (O-CHR¹⁸O)ⱼ, (O-CHR¹⁸CHR¹⁹)ⱼ, O-N=CH, NR¹⁵-N=CH, NR¹⁵SO₂, wherein R¹⁵, R¹⁸, R¹⁹ and j are as defined above/below, and even more preferred wherein h and i is 0 and Q is selected from a group consisting of O, S, N-R¹⁵;
optionally isolating the reaction product,
and transferring the obtained reaction product of formula IX 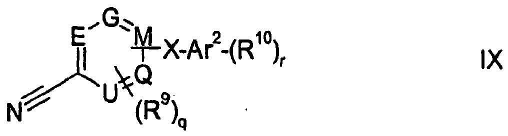 into a compound of formula IVa, preferably by reducing and more preferred by hydrogenating the CN-moiety of the compound of formula IX into a H₂NCH₂-moiety. Methods and reaction conditions for hydrogenating said CN-moiety into a H₂NCH₂-moiety are known in the art. In general, it is advantageous to carry out the hydrogenation reaction in the presence of a hydrogen delivering means, for example hydrogen gas, in the presence of a suitable catalyst, preferably a Nickel catalyst, for example Raney-Nickel. In general, such hydrogenation reactions are carried out in a suitable solvent. Suitable solvents for hydrogenation reactions are known in the art. Suitable solvents, for example, are alcohols, especially methanol and ethanol and ethers, especially THF, and mixtures thereof. Preferably, the hydrogenation reaction is carried out in a methanol/ammonia mixture, preferably in the presence of Raney nickel. In general, the hydrogenation reactions are carried out at about normal pressure or elevated pressure, for example between normal pressure and 10 bar pressure, preferably at about 5 par pressure (about 500 kPa). The hydrogenation reaction is usually carried out in the temperature range between -20 °C and 150 °C, preferably +20 °C and 100 °C, for example at about 45 °C.

Ar² is preferably pyridinyl. Accordingly, the compound of formula VIII is preferably selected from the group consisting of formulae VIIIa and VIIIb, 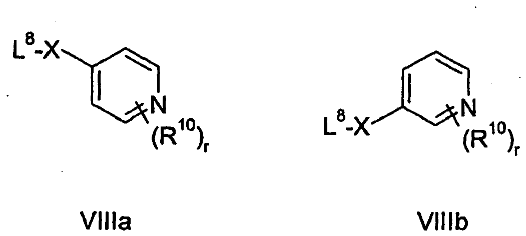 wherein L⁸, X, R¹⁰ and r are as defined above, and especially preferred from the group consisting of formulae VIIIc and VIIId, 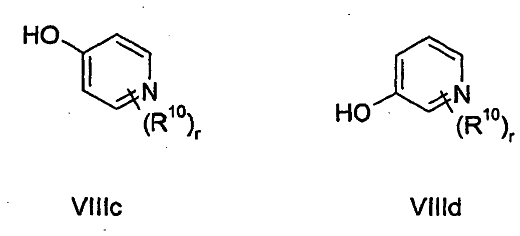 wherein R¹⁰ and r are as defined above, or the alkaline metal salts and especially the sodium or potasium salts thereof.

Accordingly, in formulae IVa, VIII, VIIIa, Vlllb and IX, the bridging group X is preferably O, S, OCH₂ and OCH₂CH₂ and especially is O.

In the formulae VIII, VIIIa and VIIb, L⁸ is preferably H or selected from the group consisting of Na, K and Cs and especially preferred is H.

In general, this reaction is advantageous to produce compounds of formula IVaa, 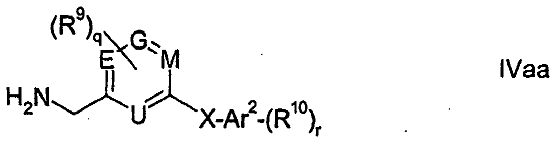 wherein E, G, M, U, R⁹, q, X, Ar², R¹⁰ and r are as defined above/below.

To obtain compounds of formula IVaa, it is reasonable to employ a compound of formula VII that is selected from the compounds of formula VIIa, 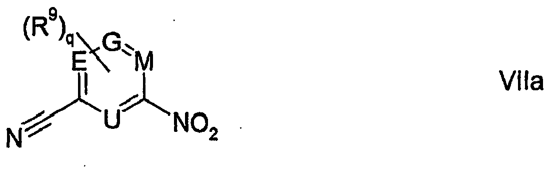 and proceed the reaction as described above/below.

Accordingly, by starting from a compound of formula VIIa and a compound of formula VIIIa, the reaction preferably leads to compounds of formula IVaaa, 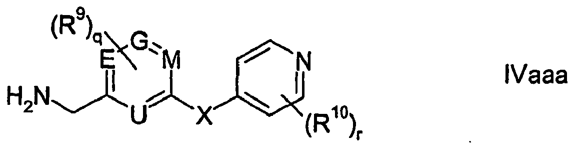 wherein E, G, M, U, R⁹, q, X, R¹⁰ and r are as defined above/below.

Accordingly, by starting from a compound of formula VIIa and a compound of formula Vlllb, the reaction preferably leads to compounds of formula IVaab, 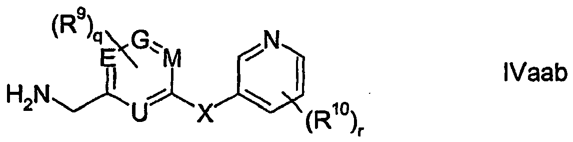 wherein E, G, M, U, R⁹, q, X, R¹⁰ and r are as defined above/below.

Accordingly, by starting from a compound of formula Vlla and a compound of formula VIIIc, the reaction preferably leads to compounds of formula IVaac, 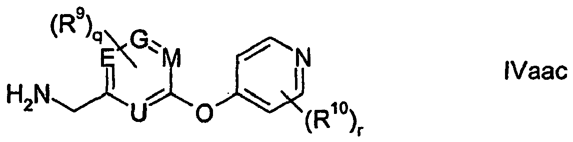 wherein E, G, M, U, R⁹, q, R¹⁰ and r are as defined above/below.

Accordingly, by starting from a compound of formula VIIa and a compound of formula VIIId, the reaction preferably leads to compounds of formula 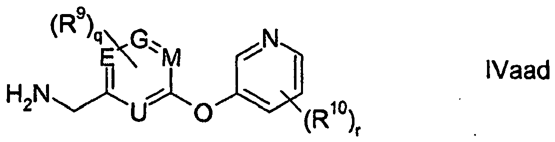 wherein E, G, M, U, R⁹, q, R¹⁰ and r are as defined above/below.

Some of the starting materials of the formula VII and/or the formula VIII are known and preferably commercially available. If they are not known, they can be prepared by methods known per se.

The reaction between the compound of formula VII and VIII is preferably carried out in the temperature range between 0 °C and 250 °C, more preferred room temperature and 200 °C, for example at about 120 °C, at about 150 °C or at about 180°. Reaction times depend on the respective reactants and the respective reaction temperature, but generally lie in the range between 30 min and 36 hrs, preferably 3 hrs and 24 hrs, more preferably 8 hrs and 20 hrs for example about 10 hrs, about 16 hrs or about 18 hrs.

The reaction can be carried out in the absence of solvent or preferably in the presence of a solvent, preferable a solvent that is inert under the respective reaction conditions. Suitable inert solvents for carrying out the reaction are known in the art. Examples for suitable solvents are high boiling aliphatic hydrocarbons, high boiling aromatic carbons, for example toluene, xylenes, high boiling chlorinated hydrocarbons, such as trichloroethylene, tetrachloroethanes, pentachloroethanes and hexachloroethanes; high boiling ethers, such as ethylene glycol and propylene glycols; glycol ethers, such as ethylene glycol monomethyl or monoethyl ether or ethylene glycol dimethyl ether (diglyme); amides, such as acetamide, dimethylacetamide, dimethylformamide (DMF) or N-methyl pyrrolidinone (NMP); sulfoxides, such as dimethyl sulfoxide (DMSO); or mixtures of the said solvents. Preferred are amides, especially dimethylformamide (DMF) or N-methyl pyrrolidinone (NMP).

Preferably, the reaction is carried out in the presence of a base. Suitable bases are known in the art. Preferred bases are organic bases and especially inorganic bases. Examples for inorganic bases are alkaline or alkaline-earth hydroxides, alkaline or alkaline-earth carbonates and alkaline or alkaline-earth bicarbonates or other salts of a weak acid and alkaline or alkaline-earth metals, preferably of potassium, sodium, calcium or cesium. Preferred inorganic bases are K₂CO₃, Na₂CO₃, MgCO₃, CaCO₃, NaOH and KOH, especially preferred is K₂CO₃. Examples for organic bases are triethyl amine, diisopropyl ethyl amine (DIPEA), dimethyl aniline, pyridine or chinoline. If an organic base is used, it is advantageous in general to use a base with a boiling point that is higher than the highest reaction temperature employed during the reaction.

Alternatively, if the compound of formula IV is a compound according to formula IVb, 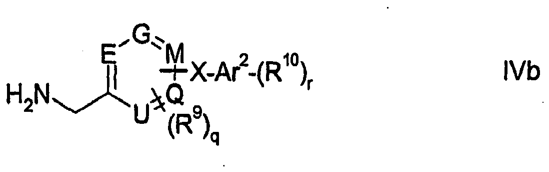 it can be readily obtained in an advantageous manner (reaction route B) by reacting a compound of formula Vllb, 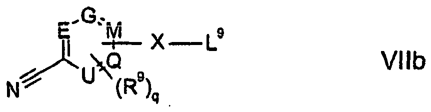 wherein E, G, M, Q, U, R⁹ and q are as defined above/below and wherein L⁹ is selected independently from H or a moiety which activates the group (and preferably a hetero atom such as N, S and especially O which is part of the group) it is bonded to, for example a metal ion. Suitable metal ions are preferably selected from the group consisting of alkaline metal ions, alkaline-earth metal ions and aluminium ions. More preferred, L⁹ is selected from H, Na and K, and is even more preferred H, especially if X is selected from the group consisting of wherein X is (CHR¹¹)ₕ-Q-(CHR¹²)ᵢ, wherein R¹¹, h and i and R¹² are defined above/below, and more preferred wherein h and/or i is 0 and Q is selected from a group consisting of O, S, N-R¹⁵, (CHR¹⁸-O)ⱼ, (CHR¹⁸CHR¹⁹-O)ⱼ, CH=N-O, CH=N-NR¹⁵, SO₂NR¹⁵, wherein R¹⁵, R¹⁸, R¹⁹ and j are as defined above/below, and even more preferred wherein h and i is 0 and Q is selected from a group consisting of O, S, N-R¹⁵;
with a compound of formula VIIIb,

L¹⁰-Ar²-(R10)ᵣ VIIIb

wherein L¹⁰ is preferably Cl, Br, I or diazonium moiety, more preferred Cl, Br or I and even more preferred Br and Cl;
optionally isolating the reaction product,
and transferring the obtained reaction product of formula IXb 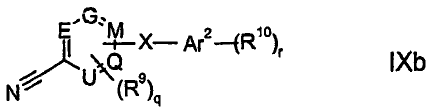 into a compound of formula IVa, preferably by reducing and more preferred by hydrogenating the CN-moiety of the compound of formula IXa into a H₂NCH₂-moiety, preferably as described above for the compound IX.

Ar² is preferably pyridinyl. Accordingly, the compound of formula VIIIb is preferably selected from the group consisting of formulae Vllle and VIIIf, 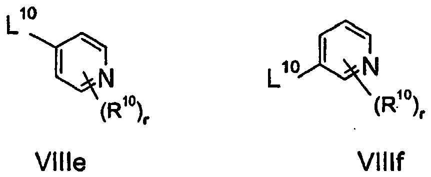 wherein L¹⁰, R¹⁰ and r are as defined above, and especially preferred from the group consisting of formulae Vlllg and VIIIh, 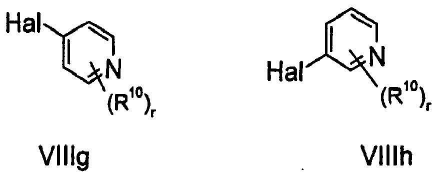 wherein Hal, R¹⁰ and r are as defined above, and wherein Hal is preferably Cl in compounds of formula Vlllg and preferably Br in compounds of formula VIIIh.

Accordingly, in formulae IVb, VIIIb, Vllle, VIIIf and IXb, the bridging group X is preferably O, S, OCH₂ and OCH₂CH₂ and especially is O.

In general, this alternative reaction is advantageous to produce compounds of formula IVbb, 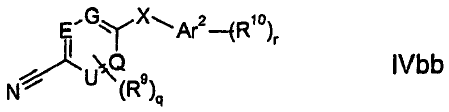 wherein E, G, Q, U, R⁹, q, X, Ar², R¹⁰ and r are as defined above/below.

To obtain compounds of formula IVbb, it is reasonable to employ a compound of formula Vllb that is selected from the compounds of formula Vllbb, 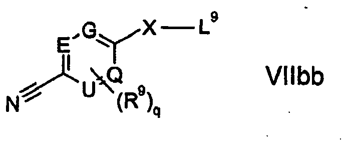 wherein E, G, Q, U, X and L⁹ are as defined above/below, more preferred wherein X-L⁹ is selected from the group consisting of SH, OH and HN-R¹⁷ and especially wherein X-L⁹ is OH, and proceed the alternative reaction as described above/below.

Accordingly, by starting from a compound a formula VIIbb and a compound of formula VIIe, the reaction preferably leads to compounds of formula IVbbe, 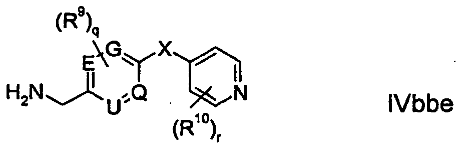 wherein E, G, Q, U, R⁹, q, X, R¹⁰ and r are as defined above/below.

Accordingly, by starting from a compound of formula Vllbb and a compound of formula VIIIf, the reaction preferably leads to compounds of formula IVbbf, 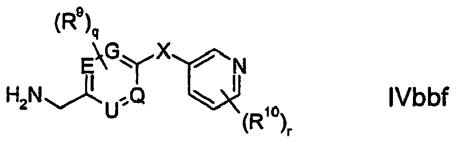 wherein E, G, Q, U, R⁹, q, X, R¹⁰ and r are as defined above/below.

Accordingly, by starting from a compound of formula Vllbb and a compound of formula Vlllg, the reaction preferably leads to compounds of formula IVbbg, 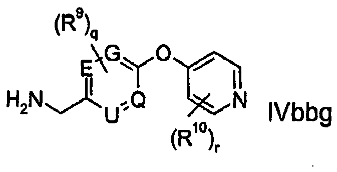 wherein E, G, Q, U, R⁹, q, R¹⁰ and r are as defined above/below.

Accordingly, by starting from a compound of formula Vllb and a compound of formula VIIIh, the reaction preferably leads to compounds of formula IVbbh, 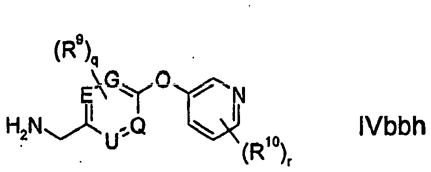 wherein E, G, Q, U, R⁹, q, R¹⁰ and r are as defined above/below.

Some of the starting materials of the formula VIIb and/or the formula Vlllb are known and preferably commercially available. If they are not known, they can be prepared by methods known per se.

The reaction between the compound of formula VIIb and Vlllb is preferably carried out in the temperature range between 0 °C and 250 °C, more preferred 50 °C and 220 °C, for example at about 90 °C, at about 120 °C, at about 160 °C, at about 180 °C or at about 200°. Reaction times depend on the respective reactants and the respective reaction temperature, but generally lie in the range between 10 min and 36 hrs, preferably between 60 min and 24 hrs, more preferably 3 h and 20 hrs for example about 6 hrs, about 12 hrs, about 15 hrs or about 18 hrs.

The reaction can be carried out in the absence or the presence of a solvent, preferable a solvent that is inert under the respective reaction conditions. Suitable inert solvents for carrying out the reaction are known in the art. Examples for suitable solvents are high aliphatic hydrocarbons, aromatic carbons, for example toluene and xylenes, high boiling chlorinated hydrocarbons, such as dichlormethane, trichloromethane trichloroethylene, tetrachloroethanes, pentachloroethanes and hexachloroethanes; ethers, such as diethylether, tert.-butyl methyl ether, ethylene glycol and propylene glycols; glycol ethers, such as ethylene glycol monomethyl or monoethyl ether or ethylene glycol dimethyl ether (diglyme); nitriles, such as acetonitrile, amides such as acetamide, diemthyacetamide, dimethylformamide (DMF) or N-methyl pyrrolidinone (NMP); sulfoxides, such as dimethyl sulfoxide (DMSO); or mixtures of the said solvents.

In many cases, it is advantageous to carry out the reaction in the presence of a catalyst. Suitable catalysts are known in the art. Preferred catalysts are compounds comprising catalytically active metals and especially compounds comprising catalytically active copper. A preferred compound comprising catalytically active copper is copper iodide and especially is Cul. Carrying out the reaction in the presence of a catalyst as described above is preferred if a compound of formula VIII is used, wherein L¹⁰ or Hal is bromine, and is especially preferred if a compound of formula VIIIf or Vlllh is used, wherein L¹⁰ or Hal is bromine.

In many cases, it is advantageous to carry out the reaction in the presence of an acid binding means, preferably an organic base as described above and more preferred an inorganic base. Preferred inorganic bases are K₂CO₃, Na₂CO₃, MgCO₃, CaCO₃, NaOH and KOH, especially preferred is K₂CO₃. Carrying out the reaction in the presence of and acid binding means as described above is preferred if a compound of formula VIII is used, wherein L¹⁰ or Hal is bromine, and is especially preferred if a compound of formula VIII for VIIIh is used, wherein L¹⁰ or Hal is bromine.

Preferably, the reaction is carried out by heating up a reaction mixture comprising one compound of formula VIIb and one compound of formula VIIIb to a suitable reaction temperature, which preferably lies at the upper end of the given temperature ranges and more preferred is in the range between 150 °C and 200 °C, for example at about 180 °C, preferably in the presence of the suitable catalyst and especially in the presence of copper. Reaction times at this temperature are preferably as given above and especially in the range between 1 h and 5 hrs, for example about 3 hrs. Preferably, the reaction mixture is then allowed to cool down to a temperature in the lower range of the given temperature, more preferred to a temperature in the range between 50 °C and 150 °C, for example to about 90°. Preferably, a suitable solvent, especially tert.-butyl methyl ether, is then added and the reaction mixture is preferably kept at about the same temperature for some more time, preferably for 30 min to 2 hrs and more preferred for about one hour.

If the compound IV is a compound according to formula IVc, 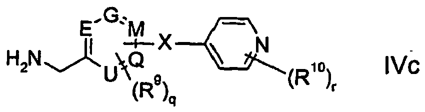 it can be readily obtained in an advantageous manner (reaction route C) by reacting a compound of formula XI 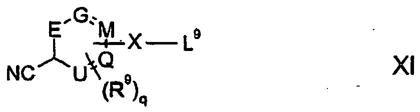 wherein L⁹ is H or a metal ion, preferably a metal ion selected from the group consisting of alkaline metal ions, alkaline-earth metal ions and aluminium ions, especially preferred alkaline metal ions, of which Li, Na, and K are especially preferred, and even more preferred H; and E, G, M, Q, U, R⁹, q and X are as defined above/below, and especially wherein X is selected from the group consisting of wherein X is (CHR¹¹)ₕ-Q-(CHR¹²)ₗ, wherein R¹¹, h and i and R¹² are defined above/below, and more preferred wherein h and/or i is 0 and Q is selected from a group consisting of O, S, N-R¹⁵, (CHR¹⁸-O)ⱼ, (CHR¹⁸CHR¹⁹-O)ⱼ, CH=N-O, CH=N-NR¹⁵, SO₂NR¹⁵, wherein R¹⁵, R¹⁸, R¹⁹ and j are as defined above/below, and even more preferred wherein h and i is 0 and Q is selected from a group consisting of O, S, N-R¹⁵;
with a compound of formula XII, 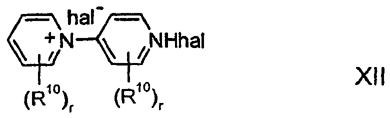 wherein hal is independently select selected from the group consisting of Cl, Br and I, the residue R¹⁰ are the same or different and have the meanings given above/below and preferably have both the same meaning, and the indices r are the same or different and have the meanings given above/below and preferably are the same,

optionally isolating the reaction product, and transferring the obtained reaction product of formula XIII 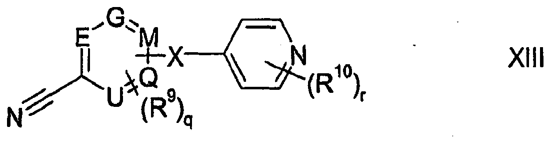 into a compound of formula IVc, preferably by reducing or hydrogenating the CN-moiety of the compound of formula IX into a H₂NCH₂-moiety, for example as described above for the compound of formula IX.

In the compounds IVc, XII and XIII, r is preferably in each case identical and even more preferred in each case 0.

In formulae IVc, XI and XIII, the bridging group X is preferably O, S, OCH₂ and OCH₂CH₂ and especially is O.

In the formula XI, L⁹ is preferably H or selected from the group consisting of Na, K and Cs and especially preferred is H.

The reaction between the compound of formula XI and XII is preferably carried out in the temperature range between 0 °C and 250 °C, more preferred room temperature and 200 °C, for example at about 120 °C, at about 150 °C or at about 180°. Reaction times depend on the respective reactants and the respective reaction temperature, but generally lie in the range between 30 min and 24 hrs, preferably one hour and 12 hrs, for example about 2 hrs, about 3 hrs or about 6 hrs. The reaction can be carried out in the absence of solvent or in the presence of a solvent, preferable a solvent that is inert under the respective reaction conditions. Suitable inert solvents for carrying out the reaction are known in the art.

In the methods according to the invention for producing compounds, E, G, M, Q, and U are as defined above/below, for example as defined above/below for the compounds according to the invention. More preferably, two or more of E, G, M, Q, and U are carbon atoms. In one embodiment of the method according to the invention for producing compounds, E, G, M, Q, and U all are carbon atoms.

Some of the starting materials of the formula XI and/or the formula XII are known and preferably commercially available. If they are not known, they can be prepared by methods known per se.

Independently of the chosen reaction route, it is in many cases possible or even feasible to introduce residues R⁸, R⁹ and/or R¹⁰ into one or more of the compounds described above, or, if the compound already comprises one or more residues R⁸, R⁹ and/or R¹⁰, to introduce additional residues R⁸, R⁹ and/or R¹⁰ into said compound. The introduction of additional residues can be readily performed by methods known in the art and especially by aromatic substitution, for example nucleophilic aromatic substitution or electrophilic aromatic substitution. For example, in compounds comprising Ar¹, wherein Ar¹ comprises one or more halogen and preferably fluorine substituents, one or more of the halogen/fluorine substituents can be easily substituted by hydroxy, thio and/or amino substituted hydrocarbons, preferably selected from the group consisting of HO(CH₂)ₙNR¹¹R¹², HO(CH₂)ₙO(CH₂)ₖNR¹¹R¹², HO(CH₂)ₙNR¹¹(CH₂)ₖOR¹², HO(CH₂)ₙNR¹¹(CH₂)ₖNR¹¹R¹², HO(CH₂)ₙCOOR¹³ , HO(CH₂)ₙS(O)ᵤR¹³ HNR¹¹(CH₂)ₙNR¹¹R¹², HNR¹¹(CH₂)ₙO(CH₂)ₖNR¹¹R¹², HNR¹¹(CH₂)ₙNR¹¹(CH₂)ₖOR¹², HNR¹¹(CH₂)ₙNR¹¹(CH₂)ₖNR¹¹R¹², HNR¹¹(CH₂)ₙCOOR¹² and HNR¹¹(CH₂)ₙS(o)ᵤR¹³ wherein R¹¹, R¹² and R¹³ are defined as above and n is as defined above, preferably n is 0, 1 or 2 and especially is 0, k is 1 to 4 and preferably 1 or 2, and u is preferably 2. In this embodiment R¹¹, R¹² and R¹³ are more preferably selected independently from each other from the group consisting of H, methyl and ethyl. Even more preferred, the hydroxy, thio and/or amino substituted hydrocarbons are selected from the group consisting of NH₃, HN(CH₃)₂, NH₂CH₃, HN(C₂H₅)₂, H₂NCH₂CH₂NH₂, HOCH₂CH₂NH₂, HOCH₂CH₂NHCH₃, HN(CH₃)CH₂CH₂NH₂, HN(CH₃)CH₂CH₂N(CH₃)₂, HN(CH₃)CH₂CH₂N(CH₃)₂, HN(CH₃)CH₂CH₂OCH₃, HOCH₂CH₂N(CH₃)₂, HOCH₂CH₂N(CH₂CH₃)₂, HSCH₃, HSC₂H₅, and compounds of the formulae 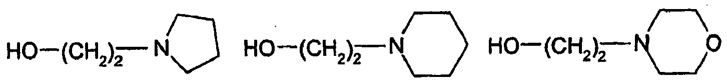 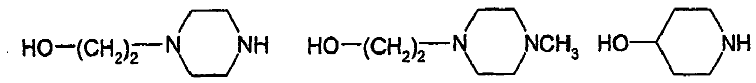 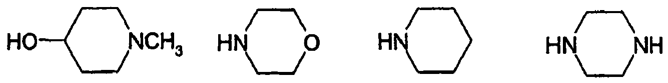 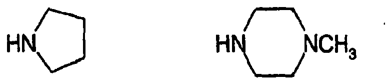 or salts and especially metal salts thereof.

On the other hand, it is in many cases possible or even feasible to modify or derivatize one or more of the residue is R⁸, R⁹ and R¹⁰ into residues R⁸, R⁹ and/or R¹⁰ other than the ones originally present. For example, CH₃-groups can be oxidised into aldehyde groups or carbonic acid groups, thio atom containing groups, for example S-alkyl or S-aryl groups, can be oxidised into SO₂-alkyl or SO₂-aryl groups, respectively, carbonic acid groups can be derivatized to carbonic acid ester groups or carbon amide groups and carbonic acid ester groups or carbon amide groups can be hydrolysed into the corresponding carbonic acid groups. Methods for performing such modifications or derivatizations are known in the art, for example from Houben-Weyl, Methods of Organic Chemistry.

Every reaction step described herein can optionally be followed by one or more working up procedures and/or isolating procedures. Suitable such procedures are known in the art, for example from standard works, such as Houben-Weyl, Methoden der organischen Chemie [Methods of Organic Chemistry], Georg-Thieme-Verlag, Stuttgart). Examples for such procedures include, but are not limited to evaporating a solvent, distilling, crystallization, fractionised crystallization, extraction procedures, washing procedures, digesting procedures, filtration procedures, chromatography, chromatography by HPLC and drying procedures, especially drying procedures in vacuo and/or elevated temperature.

A base of the formula I or the formula II can be converted into the associated acid-addition salt using an acid, for example by reaction of equivalent amounts of the base and the acid in a preferably inert solvent, such as ethanol, followed by evaporation. Suitable acids for this reaction are, in particular, those which give physiologically acceptable salts. Thus, it is possible to use inorganic acids, for example sulfuric acid, sulfurous acid, dithionic acid, nitric acid, hydrohalic acids, such as hydrochloric acid or hydrobromic acid, phosphoric acids, such as, for example, orthophosphoric acid, sulfamic acid, furthermore organic acids, in particular aliphatic, alicyclic, araliphatic, aromatic or heterocyclic monobasic or polybasic carboxylic, sulfonic or sulfuric acids, for example formic acid, acetic acid, propionic acid, hexanoic acid, octanoic acid, decanoic acid, hexadecanoic acid, octadecanoic acid, pivalic acid, diethylacetic acid, malonic acid, succinic acid pimelic acid, fumaric acid, maleic acid, lactic acid, tartaric acid, malic acid, citric acid, gluconic acid, ascorbic acid, nicotinic acid, isonicotinic acid, methane- or ethanesulfonic acid, ethanedisulfonic acid, 2-hydroxyethanesulfonic acid, benzenesulfonic acid, trimethoxybenzoic acid, adamantanecarboxylic acid, p-toluenesulfonic acid, glycolic acid, embonic acid, chlorophenoxyacetic acid, aspartic acid, glutamic acid, proline, glyoxylic acid, palmitic acid, parachlorophenoxyisobutyric acid, cyclohexanecarboxylic acid, glucose 1-phosphate, naphthalenemono- and -disulfonic acids or laurylsulfuric acid. Salts with physiologically unacceptable acids, for example picrates, can be used to isolate and/or purify the compounds of the formula I. On the other hand, compounds of the formula I can be converted into the corresponding metal salts, in particular alkali metal salts or alkaline earth metal salts, or into the corresponding ammonium salts, using bases (for example sodium hydroxide, potassium hydroxide, sodium carbonate or potassium carbonate). Suitable salts are furthermore substituted ammonium salts, for example the dimethyl-, diethyl- and diisopropyl-ammonium salts, monoethanol-, diethanol- and diisopropanolammonium salts, cyclohexyl- and dicyclohexylammonium salts, dibenzylethylenedi-ammonium salts, furthermore, for example, salts with arginine or lysine.

On the other hand, if desired, the free bases of the formula I or the formula II can be liberated from their salts using bases (for example sodium hydroxide, potassium hydroxide, sodium carbonate or potassium carbonate).

The invention relates to compounds of the formula I and of the formula II and physiologically acceptable salts and solvates thereof as medicaments.

The invention also relates to the compounds for the formula I and of the formula II and physiologically acceptable salts and solvates thereof as kinase inhibitors.

The invention furthermore relates to the use of the compounds of the formula I and/or physiologically acceptable salts and/or solvates thereof for the preparation of pharmaceutical compositions and/or pharmaceutical preparations, in particular by non- chemical methods. The invention furthermore relates to the use of the compounds of the formula II and/or physiologically acceptable salts and/or solvates thereof for the preparation of pharmaceutical compositions and/or pharmaceutical preparations, in particular by non-chemical methods. In this cases, one or more compounds according to the invention can be converted into a suitable dosage form together with at least one solid, liquid and/or semi-liquid excipient or adjuvant and, if desired, in combination with one or more further active ingredients.

The invention further relates to the use of one or more of the compounds according to the invention, selected from the group consisting of compounds of the formula I as free bases, solvates of compounds of the formula I, salts of compounds of formula I, of compounds of the formula II as free bases, solvates of compounds of the formula II and salts of compounds of formula II, for the production of pharmaceutical compositions and/or pharmaceutical preparations, in particular by a non-chemical route. In general, non-chemical routes for the production of pharmaceutical compositions and/or pharmaceutical preparations comprise processing steps on suitable mechanical means known in the art that transfer one or more compounds according to the invention into a dosage form suitable for administration to a patient in need of such a treatment. Usually, the transfer of one or more compounds according to the invention into such a dosage form comprises the addition of one or more compounds, selected from the group consisting of carriers, excipients, auxiliaries and pharmaceutical active ingredients other than the compounds according to the invention. Suitable processing steps include, but are not limited to combining, milling, mixing, granulating, dissolving, dispersing, homogenizing, casting and/or compressing the respective active and non-active ingridients. In this respect, active ingredients are preferably at least one compound according to this invention and one or more additional compounds other than the compounds according to the invention, which show valuable pharmaceutical properties, preferably those pharmaceutical active agents other than the compounds according to invention which are disclosed herein.

The process for preparing pharmaceutical compositions and/or pharmaceutical preparations preferably comprises one or more processing steps, selected from the group consisting of combining, milling, mixing, granulating, dissolving, dispersing, homogenizing and compressing. The one or more processing steps are preferably performed on one or more of the ingredients which are to form the pharmaceutical composition and/or pharmaceutical preparation preferably according to invention. Even more preferred, said processing steps are performed on two or more of the ingredients which are to form the pharmaceutical composition and/or pharmaceutical preparation, said ingredients comprising one or more compounds according to the invention and, additionally, one or more compounds, preferably selected from the group consisting of active ingredients other than the compounds according to the invention, excipients, auxiliaries, adjuvants and carriers. Mechanical means for performing said processing steps are known in the art, for example from Ullmann's Encyclopedia of Industrial Chemistry, 5th Edition.

Preferably, one or more compounds according to the invention are converted into a suitable dosage form together with at least one compound selected from the group consisting of excipients, auxiliaries, adjuvants and carriers, especially solid, liquid and/or semi-liquid excipients, auxiliaries, adjuvants and carriers, and, if desired, in combination with one or more further active ingredients.

Suitable dosage forms include, but are not limited to tablets, capsules, semi-solids, suppositories, aerosols, which can be produced according to methods known in the art, for example as described below:
- tablets: mixing of active ingredient/s and auxiliaries, compression of said mixture into tablets (direct compression), optionally granulation of part of mixture before compression
- capsules: mixing of active ingredient/s and auxiliaries to obtain a flowable powder, optionally granulating powder, filling powders/granulate into opened capsules, capping of capsules
- semi-solids (ointments, gels, creams): dissolving/dispersing active ingredient/s in an aqueous or fatty carrier; subsequent mixing of aqueous/fatty phase with complementary fatty resp. aqueous phase, homogenisation (creams only)
- suppositories (rectal and vaginal): dissolving/dispersing active ingredient/s in carrier material liquified by heat (rectal: carrier material normally a wax; vaginal: carrier normally a heated solution of a gelling agent), casting said mixture into suppository forms, annealing and withdrawal suppositories from the forms
- aerosols:: dispersing/dissolving active agent/s in a propellant, bottling said mixture into an atomizer

The invention thus relates to pharmaceutical compositions and/or pharmaceutical preparations comprising at least one compound of the formula I and/or one of its physiologically acceptable salts and/or solvates and especially to pharmaceutical compositions and/or pharmaceutical preparations comprising at least one compound of the formula II and/or one of its physiologically acceptable salts and/or solvates.

Preferably, the pharmaceutical compositions and/or pharmaceutical preparations according to the invention contain a therapeutic effective amount of one or more compounds according to the invention. Said therapeutic effective amount of one or more of the compounds according to the invention is known to the skilled artisan or can be easily determined by standard methods known in the art. For example, the compounds according to the invention can be administered to a patient in an analogous manner to other compounds that are effective as raf-kinase inhibitors, especially in an analogous manner to the compounds described in WO 00/42012 (Bayer). Usually, suitable doses that are therapeutically effective lie in the range between 0.0005 mg and 1000 mg, preferably between 0.005 mg and 500 mg and especially between 0.5 and 100 mg per dose unit. The daily dose comprises preferably more than 0.001 mg, more preferred more than 0.01 milligram, even more preferred more than 0.1 mg and especially more than 1.0 mg, for example more than 2.0 mg, more than 5 mg, more than 10 mg, more than 20 mg, more than 50 mg or more than 100 mg, and preferably less than 1500 mg, more preferred less than 750 mg, even more preferred less than 500 mg, for example less than 400 mg, less than 250 mg, less than 150 mg, less than 100 mg, less than 50 mg or less than 10 mg.

The specific dose for the individual patient depends, however, on the multitude of factors, for example on the efficacy of the specific compounds employed, on the age, body weight, general state of health, the sex, the kind of diet, on the time and route of administration, on the excretion rate, the kind of administration and the dosage form to be administered, the pharmaceutical combination and severity of the particular disorder to which the therapy relates. The specific therapeutic effective dose for the individual patient can readily be determined by routine experimentation, for example by the doctor or physician which advises or attends the therapeutic treatment.

However, the specific dose for each patient depends on a wide variety of factors, for example on the efficacy of the specific compound employed, on the age, body weight, general state of health, sex, on the diet, on the time and method of administration, on the rate of excretion, medicament combination and severity of the particular illness to which the therapy applies. Parenteral administration is preferred. Oral administration is especially preferred.

These compositions and/or preparations can be used as medicaments in human or veterinary medicine. Suitable excipients are organic or inorganic substances which are suitable for enteral (for example oral), parenteral or topical administration and do not react with the novel compounds, for example water, vegetable oils, benzyl alcohols, alkylene glycols, polyethylene glycols, glycerol triacetate, gelatine, carbohydrates, such as lactose or starch, magnesium stearate, talc or vaseline. Examples for suitable dosage forms, which are especially suitable for oral administration are, in particular, tablets, pills, coated tablets, capsulees, powders, granules, syrups, juices or drops. Further examples for suitable dosage forms, which are especially suitable for rectal administration are suppositories, further examples for suitable dosage forms, which are especially suitable for parenteral administration are solutions, preferably oil-based or aqueous solutions, furthermore suspensions, emulsions or implants, and suitable for topical application are ointments, creams or powders. The novel compounds may also be lyophilised and the resultant lyophilisates used, for example, for the preparation of injection preparations. The compositions and/or preparations indicated may be sterilized and/or comprise assistants, such as lubricants, preservatives, stabilizers and/or wetting agents, emulsifiers, salts for modifying the osmotic pressure, buffer substances, dyes and flavors and/or one or more further active ingredients, for example one or more vitamins.

For administration as an inhalation spray, it is possible to use sprays in which the active ingredient is either dissolved or suspended in a propellant gas or propellant gas mixture (for example CO₂ or chlorofluorocarbons). The active ingredient is advantageously used here in micronized form, in which case one or more additional physiologically acceptable solvents may be present, for example ethanol. Inhalation solutions can be administered with the aid of conventional inhalers.

The compounds of the formula I and their physiologically acceptable salts and solvates and especially the compounds of formula II and their physiologically acceptable salts and solvates can be employed for combating one or more diseases, for example allergic diseases, psoriasis and other skin diseases, especially melanoma, autoimmune diseases, such as, for example, rheumatoid arthritis, multiple sclerosis, Crohn's disease, diabetes mellitus or ulcerative colitis.

In General, the substances according to the invention are preferably administered in doses corresponding to the compound rolipram of between 1 and 500 mg, in particular between 5 and 100 mg per dosage unit. The daily dose is preferably between about 0.02 and 10 mg/kg of body weight. However, the specific dose for each patient depends on a wide variety of factors, for example on the efficacy of the specific compound employed, on the age, body weight, general state of health, sex, on the diet, on the time and method of administration, on the excretion rate, medicament combination and severity of the particular illness to which the therapy applies. Oral administration is preferred.

The compounds of the formula I according to claim 1 and/or their physiologically acceptable salts are also used in pathological processes which are maintained or propagated by angiogenesis, in particular in tumors, restenoses, diabetic retinopathy, macular degenerative disease or rheumatois arthritis.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

For use in the subject methods, the subject compounds may be formulated with pharmaceutically active agents other than the compounds according to the invention, particularly other anti-metastatic, antitumor or anti-angiogenic agents. Angiostatic compounds of interest include angiostatin, enclostatin, carboxy terminal peptides of collagen alpha (XV), etc. Cytotoxic and cytostatic agents of interest include adriamycin, aleran, Ara-C, BICNU, busulfan, CNNU, cisplatinum, cytoxan, daunorubicin, DTIC, 5-FU, hydrea, ifosfamicle, methotrexate, mithramycin, mitomycin, mitoxantrone, nitrogen mustard, velban, vincristine, vinblastine, VP-16, carboplatinum, fludarabine, gemcitabine, idarubicin, irinotecan, leustatin, navelbine, taxol, taxotere, topotecan, etc.

The compounds of the invention have been shown to have antiproliferative effect in an in vivo xenograft tumor model. The subject compounds are administered to a subject having a hyperproliferative disorders, e.g., to inhibit tumor growth, to decrease inflammation associated with a lymphoproliferative disorder, to inhibit graft rejection, or neurological damage due to tissue repair, etc. The present compounds are useful for prophylactic or therapeutic purposes. As used herein, the term "treating" is used to refer to both prevention of disease, and treatment of pre-existing conditions. The prevention of proliferation is accomplished by administration of the subject compounds prior to development of overt disease, e.g., to prevent the regrowth of tumors, prevent metastatic growth, diminish restenosis associated with cardiovascular surgery, etc. Alternatively the compounds are used to treat ongoing disease, by stabilizing or improving the clinical symptoms of the patient.

The host, or patient, may be from any mammalian species, e.g., primate sp., particularly human; rodents, including mice, rats and hamsters; rabbits; equines, bovines, canines, felines; etc. Animal models are of interest for experimental investigations, providing a model for treatment of human disease.

The susceptibility of a particular cell to treatment with the subject compounds may be determined by in vitro testing. Typically a culture of the cell is combined with a subject compound at varying concentrations for a period of time sufficient to allow the active agents to induce cell death or inhibit migration, usually between about one hour and one week. For in vitro testing, cultured cells from a biopsy sample may be used. The viable cells left after treatment are then counted.

The dose will vary depending on the specific compound utilized, specific disorder, patient status, etc. Typically a therapeutic dose will be sufficient to substantially decrease the undesirable cell population in the targeted tissue, while maintaining patient viability. Treatment will generally be continued until there is a substantial reduction, e.g., at least about 50 %, decrease in the cell burden, and may be continued until there are essentially none of the undesirable cells detected in the body.

The compounds according to the invention are preferably administered to human or nonhuman animals, more preferred to mammalian animals and especially to humans.

The compounds also find use in the specific inhibition of a signaling pathway mediated by protein kinases. Protein kinases are involved in signaling pathways for such important cellular activities as responses to extracellular signals and cell cycle checkpoints. Inhibition of specific protein kinases provided a means of intervening in these signaling pathways, for example to block the effect of an extracellular signal, to release a cell from cell cycle checkpoint, etc. Defects in the activity of protein kinases are associated with a variety of pathological or clinical conditions, where there is a defect in the signaling mediated by protein kinases. Such conditions include those associated with defects in cell cycle regulation or in response to extracellular signals, e.g., immunological disorders, autoimmune and immunodeficiency diseases; hyperproliferative disorders, which may include psoriasis, arthritis, inflammation, endometriosis, scarring, cancer, etc. The compounds of the present invention are active in inhibiting purified kinase proteins preferably raf kinases, e.g., there is a decrease in the phosphorylation of a specific substrate in the presence of the compound. The compounds of the invention may also be useful as reagents for studying signal transduction or any of the clinical disorders listed throughout this application.

There are many disorders associated with a dysregulation of cellular proliferation. The conditions of interest include, but are not limited to, the following conditions. The subject compounds are useful in the treatment of a variety of conditions where there is proliferation and/or migration of smooth muscle cells, and/or inflammatory cells into the intimal layer of a vessel, resulting in restricted blood flow through that vessel, e.g., neointimal occlusive lesions. Occlusive vascular conditions of interest include atherosclerosis, graft coronary vascular disease after transplantation, vein graft stenosis, peri-anastomatic prothetic graft stenosis, restenosis after angioplasty or stent placement, and the like.

Diseases where there is hyperproliferation and tissue remodelling or repair or reproductive tissue, e.g., uterine, testicular and ovarian carcinomas, endometriosis, squamous and glandular epithelial carcinomas of the cervix, etc. are reduced in cell number by administration of the subject compounds. The growth and proliferation of neural cells is also of interest.

Tumor cells are characterized by uncontrolled growth, invasion to surrounding tissues, and metastatic spread to distant sites. Growth and expansion requires an ability not only to proliferate, but also to down-modulate cell death (apoptosis) and activate angiogenesis to product a tumor neovasculature.

Tumors of interest for treatment include carcinomas, e.g., colon, duodenal, prostate, breast, melanoma, ductal, hepatic, pancreatic, renal, endometrial, stomach, dysplastic oral mucosa, polyposis, invasive oral cancer, non-small cell lung carcinoma, transitional and squamous cell urinary carcinoma etc.; neurological malignancies; e.g. neuroplastoma, gliomas, etc.; hematological malignancies, e.g., childhood acute leukaemia, non-Hodgkin's lymphomas, chronic lymphocytic leukaemia, malignant cutaneous T-cells, mycosis fungoides, non-MF cutaneous T-cell-lymphoma, lymphomatoid papulosis, T-cell rich cutaneous lymphoid hyperplasia, bullous pemphigoid, discoid lupus erythematosus, lichen planus, etc.; and the like.

Tumors of neural tissue are of particular interest, e.g., gliomas, neuromas, etc. Some cancers of particular interest include breast cancers, which are primarily adenocarcinoma subtypes. Ductal carcinoma in situ is the most common type of noninvasive breast cancer. In DCIS, the malignant cells have not metastasized through the walls of the ducts into the fatty tissue of the breast. Infiltration (or invasive) ductal carcinoma (IDC) has metastasized through the wall of the duct and invaded the fatty tissue of the breast. Infiltrating (or invasive) lobular carcinoma (ILC) is similar to IDC, in that it has the potential to metastasize elsewhere in the body. About 10 % to 15 % of invasive breast cancers are invasive lobular carcinomas.

Also of interest is non-small cell lung carcinoma. Non-small cell lung cancer (NSCLC) is made up of three general subtypes of lung cancer. Epidermoid carcinoma (also called squamos cell carcinoma) usually starts in one of the larger bronchial tubes and grows relatively slowly. The size of these tumors can range from very small to quite large. Adenocarcinoma starts growing near the outside surface of the lung and may vary in both size and growth rate. Some slowly growing adenocarcinomas are described as alveolar cell cancer. Large cell carcinoma starts near the surface of the lung, grows rapidly, and the growth is usually fairly large when diagnosed. Other less common forms of lung cancer are carcinoid, cylindroma, mucoepidermoid, and malignant mesothelioma.

Melanoma is a malignant tumor of melanocytes. Although most melanomas arise in the skin, they also may arise from mucosal surfaces or at other sites to which neural crest cells migrate. Melanoma occurs predominantly in adults, and more than half of the cases arise in apparently normal areas of the skin. Prognosis is affected by clinical and histological factors and by anatomic location of the lesion. Thickness and/or level of invasion of the melanoma, mitotic index, tumor infiltrating lymphocytes, and ulceration or bleeding at the primary site affect the prognosis. Clinical staging is based on whether the tumor has spread to regional lymph nodes or distant sites. For disease clinically confined to the primary site, the greater the thickness and depth of local invasion of the melanoma, the higher the chance of lymph node metastases and the worse the prognosis. Melanoma can spread by local extension (through lymphatics) and/or by hematogenous routes to distant sites. Any organ may be involved by metastases, but lungs and liver are common sites.

Other hyperproliferative diseases of interest relate to epidermal hyperproliferation, tissue, remodeling and repair. For example, the chronic skin inflammation of psoriasis is associated with hyperplastic epidermal keratinocyctes as well as infiltrating mononuclear cells, including CD4+ memory T cells, neutrophils and macrophages.

The proliferation of immune cells is associated with a number of autoimmune and lymphoproliferative disorders. Diseases of interest include multiple sclerosis, rheumatoid arthritis and insulin dependent diabetes mellitus. Evidence suggests that abnormalities in apoptosis play a part in the pathogenesis of systemic lupus erythematosus (SLE). Other lymphoproliferative conditions the inherited disorder of lymphocyte apoptosis, which is an autoimmune lymphoproliferative syndrome, as well as a number of leukemia's and lymphomas. Symptoms of allergies to environmental and food agents, as well as inflammatory bowel disease, may also be alleviated by the compounds of the invention.

Surprisingly, it has been found that methylene urea derivatives according to invention are able to interact with signaling pathways, especially the signaling pathways described herein and preferably the raf-kinase signaling pathway. Methylene urea derivatives according to the invention preferably show advantageous biological activity which can easily be demonstrated according to methods known in the art, for example by enzyme based assays. Suitable assays are known in the art, for example from the literature cited herein and the references cited in the literature, or can be developed and/or performed in an analogous manner thereof. In such enzyme based assays, methylene urea derivatives according to invention show an effect, preferably a modulating and especially an inhibiting effect which is usually documented by IC₅₀ values in a suitable range, preferably in the micromolar range and more preferred in the nanomolar range.

In general, compounds according to the invention are to be regarded as suitable kinase-modulators and especially suitable kinase-inhibitors according to the invention if they show an effect or an activity to one or more kinases, preferably to one or more raf-kinases that preferably lies, determined as IC₅₀-value, in the range of 100 µmol or below, preferably 10 µmol or below, more preferably in the range of 3 µmol or below, even more preferably in the range of 1 µmol or below and most preferably in the nanomolar range. Especially preferred for use according to the invention are kinase-inhibitors as defined above/below, that show an activity, determined as IC₅₀-value, to one or more raf-kinases, preferably including A-raf, B-raf and c-raf1 or consisting of A-raf, B-raf and c-raf1 and more preferred including c-raf1 or consisting of c-raf1, in the range of 0.5 µmol or below and especially in the range of 0.1 µmol or below. In many cases an IC₅₀-value at the lower end of the given ranges is advantageous and in some cases it is highly desirable that the IC₅₀-value is as small as possible or the he IC₅₀-values are as small as possible, but in general IC₅₀-values that lie between the above given upper limits and a lower limit in the range of 0.0001 µmol, 0.001 µmol, 0.01 µmol or even above 0.1 µmol are sufficient to indicate the desired pharmaceutical activity. However, the activities measured can vary depending on the respective testing system or assay chosen.

Alternatively, the advantageous biological activity of the compounds according to the invention can easily be demonstrated in *in vitro* assays, such as *in vitro* proliferation assays or *in vitro* growth assays. Suitable *in vitro* assays are known in the art, for example from the literature cited herein and the references cited in the literature or can be performed as described below, or can be developed and/or performed in an analogous manner thereof.

As an example for an *in vitro* growth assay, human tumor cell lines, for example HCT116, DLD-1 or MiaPaCa, containing mutated K-ras genes can be used in standard proliferation assays, for example for anchorage dependent growth on plastic or anchorage independent growth in soft agar. Human tumor cell lines are commercially available, for example from ATCC (Rockville MD), and can be cultured according to methods known in the art, for example in RPMI with 10% heat inactivated fetal bovine serum and 200 mM glutamine. Cell culture media, fetal bovine serum and additives are commercially available, for example from Invitrogen/Gibco/BRL (Karlsruhe, Germany) and/or QRH Biosciences (Lenexa, KS). In a standard proliferation assay for anchorage dependent growth, 3X10³ cells can be seeded into 96-well tissue culture plates and allowed to attach, for example overnight at 37 °C in a 5% CO₂ incubator. Compounds can be titrated in media in dilution series and added to 96 well cell cultures. Cells are allowed to grow, for example for 1 to 5 days, typically with a feeding of fresh compound containing media at about half of the time of the growing period, for example on day 3, if the cells are allowed to grow 5 days. Proliferation can be monitored by methods known in the art, such as measuring metabolic activity, for example with standard XTT colorimetric assay (Boehringer Mannheim) measured by standard ELISA plate reader at OD 490/560, by measuring ³H-thymidine incorporation into DNA following an 8 h culture with 1µCu ³H-thymidine, harvesting the cells onto glass fiber mats using a cell harvester and measuring ³H-thymidine incorporation by liquid scintillation counting, or by staining techniques, such as crystal violet staining. Other suitable cellular assay systems are known in the art.

Alternatively, for anchorage independent cell growth, cells can be plated at 1 x 10³ to 3 x 10³ in 0.4% Seaplaque agarose in RPMI complete media, overlaying a bottom layer containing only 0.64% agar in RPMI complete media, for example in 24-well tissue culture plates. Complete media plus dilution series of compounds can be added to wells and incubated, for example at 37 °C in a 5% CO₂ incubator for a sufficient time, for example 10-14 days, preferably with repeated feedings of fresh media containing compound, typically at 3-4 day intervals. Colony formation and total cell mass can be monitored, average colony size and number of colonies can be quantitated according to methods known in the art, for example using image capture technology and image analysis software. Image capture technology and image analysis software, such as Image Pro Plus or media Cybernetics.

As discussed herein, these signaling pathways are relevant for various disorders. Accordingly, by interacting with one or more of said signaling pathways, methylene urea derivatives are useful in the prevention and/or the treatment of disorders that are dependent from said signaling pathways.

The compounds according to the invention are preferably kinase modulators and more preferably kinase inhibitors. According to the invention, kinases include, but are not limited to one or more Raf-kinases, one or more Tie-kinases, one or more VEGFR-kinases, one or more PDGFR-kinases, p38-kinase and/or SAPK2alpha.

Raf-kinases in this respect are respect preferably include or consist of A-Raf, B-Raf and c-Raf1.

Tie-kinases in this respect preferably include or consist of Tie-2 kinase.

VEGFR-kinases in this respect preferably include or consist of VEGFR-2 kinase.

Due to the kinase modulating or inhibting properties of the compounds according to the invention, the compounds according to the invention preferably interact with one or more signalling pathways which are preferably cell signalling pathways, preferably by downregulating or inhibiting said signaling pathways. Examples for such signalling pathways include, but are not limited to the raf-kinase pathway, the Tie-kinase pathway, the VEGFR-kinase pathway, the PDGFR-kinase pathway, the p38-kinase pathway, the SAPK2alpha pathway and/or the Ras-pathway.

Modulation of the raf-kinase pathway plays an important role in various cancerous and noncancerous disorders, preferably cancerous disorders, such as dermatological tumors, haematological tumors, sarcomas, squamous cell cancer, gastric cancer, head cancer, neck cancer, oesophageal cancer, lymphoma, ovary cancer, uterine cancer and/or prostate cancer. Modulation of the raf-kinase pathway plays a even more important role in various cancer types which show a constitutive activation of the raf-kinase dependent signalling pathway, such as melanoma, colorectal cancer, lung cancer, brain cancer, pancreatic cancer, breast cancer, gynaecological cancer, ovarian cancar, thyroid cancer, chronic leukaemia and acute leukaemia, bladder cancer, hepatic cancer and/or renal cancer. Modulation of the raf-kinase pathway plays also an important role in infection diseases, preferably the infection diseases as mentioned above/below and especially in Helicobacter pylori infections, such as Helicobacter pylori infection during peptic ulcer disease.

One or more of the signalling pathways mentioned above/below and especially the VEGFR-kinase pathway plays an important role in angiogenesis. Accordingly, due to the kinase modulating or inhibting properties of the compounds according to the invention, the compounds according to the invention are suitable for the prophylaxis and/or treatment of pathological processes or disorders caused, mediated and/or propagated by angiogenesis, for example by inducing anti-angiogenesis. Pathological processes or disorders caused, mediated and/or propagated by angiogenesis include, but are not limited to tumors, especially solid tumors, arthritis, especially heumatic or rheumatoid arthritis, diabetic retinopathy, psoriasis, restenosis; fibrotic disorders; mesangial cell proliferative disorders, diabetic nephropathy, malignant nephrosclerosis, thrombotic microangiopathy syndromes, organ transplant rejection, glomerulopathies, metabolic disorders, inflammation and neurodegenerative diseases, and especially solid tumors, rheumatic arthritis, diabetic retinopathy and psoriasis.

Modulation of the p38-signalling pathway plays an important role in various cancerous and although in various noncancerous disorders, such as fibrosis, atherosclerosis, restenosis, vascular disease, cardiovascular disease, inflammation, renal disease and/or angiogenesis, and especially noncancerous disorders such as rheumatoid arthritis, inflammation, autoimmune disease, chronic obstructive pulmonary disease, asthma and/or inflammatory bowel disease.

Modulation of the PDGF-signalling pathway plays an important role in various cancerous and although in various noncancerous disorders, such as rheumatoid arthritis, inflammation, autoimmune disease, chronic obstructive pulmonary disease, asthma and/or inflammatory bowel disease, and especially noncancerous disorders such as fibrosis, atherosclerosis, restenosis, vascular disease, cardiovascular disease, inflammation, renal disease and/or angiogenesis.

Subject of the present invention are therefore methylene urea derivatives according to the invention as promoters or inhibitors, preferably as inhibitors, of the signaling pathways described herein. Preferred subject of the invention are therefore methylene urea derivatives according to the invention as promoters or inhibitors, preferably as inhibitors of the raf-kinase pathway. More preferred subject of the invention are therefore methylene urea derivatives according to the invention as promoters or inhibitors, preferably as inhibitors of the raf-kinase. Even more preferred subject of the invention are methylene urea derivatives according to invention as promoters or inhibitors, preferably as inhibitors of one or more raf-kinases, selected from the group consisting of A-raf, B-raf and c-raf1. Especially preferred subject of the invention are methylene urea derivatives according to the invention as promoters or inhibitors, preferably as inhibitors of c-raf1.

Thus, subject of the present invention are methylene urea derivatives according to the invention as medicaments. Subject of the present invention are methylene urea derivatives according to the invention as medicament active ingredients. Further subject of the present invention is the use of one or more methylene urea derivatives according to the invention as a pharmaceutical. Further subject of the present invention is the use of one or more methylene urea derivatives according to the invention in the treatment and/or the prophylaxis of disorders, preferably the disorders described herein, more preferred disorders that are caused, mediated and/ or propagated by signalling pathways discussed herein, even more preferred disorders that are caused, mediated and/or propagated by raf-kinases and especially disorders that are caused, mediated and/or propagated by raf-kinases, selected from the group consisting of A-raf, B-raf and c-raf1. Usually, the disorders discussed herein are divided into two groups, hyperproliferative and non hyperproliferative disorders. In this context, psioarsis, arthritis, inflammation, endometriosis, scarring, begnin prostatic hyperplasia, immunological diseases, autoimmune diseases and immunodeficiency diseases are to be regarded as noncancerous disorders, of which arthritis, inflammation, immunological diseases, autoimmune diseases and immunodeficiency diseases are usually regarded as non hyperproliferative disorders. In this context, brain cancer, lung cancer, squamous cell cancer, bladder cancer, gastric cancer, pancreatic cancer, hepatic cancer, renal cancer, colorectal cancer, breast cancer, head cancer, neck cancer, oesophageal cancer, gynaecological cancer, thyroid cancer, lymphoma, chronic leukaemia and acute leukaemia are to be regarded as cancerous disorders, all of which are usually regarded as hyperproliferative disorders. Especially cancerous cell growth and especially cancerous cell growth mediated by raf-kinase is a disorder which is a target of the present invention. Subject of the present invention therefore are methylene urea derivatives according to the invention as medicaments and/or medicament active ingredients in the treatment and/or the prophylaxis of said disorders and the use of methylene urea derivatives according to the invention for the manufacture of a pharmaceutical for the treatment and/or the prophylaxis of said disorders as well as a method of treatment of said disorders, comprising administering one or more methylene urea derivatives according to the invention to a patient in need of such an administration. Subject of the present invention therefore are methylene urea derivatives according to the invention as medicaments and/or medicament active ingredients in the treatment and/or the prophylaxis said disorders and the use of methylene urea derivatives according to the invention for the manufacture of a pharmaceutical for the treatment and/or the prophylaxis of said disorders as well as a method of treatment of said disorders, comprising administering one or more methylene urea derivatives according to the invention to a patient in need of such an administration.

Accordingly, subject of the present invention are pharmaceutical compositions that contain one or more methylene urea derivatives according to the invention. Subject of the present invention are especially pharmaceutical compositions that contain one or more methylene urea derivatives according to the invention and one or more additional compounds (other than the compounds of the instant invention), preferably selected from the group consisting of physiologically acceptable excipients, auxiliaries, adjuvants, carriers and pharmaceutically active ingredients other than the compounds according to the invention.
Accordingly, subject of the present invention is a process for the manufacture of a pharmaceutical composition, wherein one or more methylene urea derivatives according to the invention and one or more compounds (other than the compounds of the instant invention), preferably selected from the group consisting of carriers, excipients, auxiliaries, adjuvants and pharmaceutically active ingredients other than the compounds according to the invention.

Accordingly, the use of the compounds according to the invention in the treatment of Hyperproliferative disorders is a subject of the instant invention.

Accordingly, the use of the compounds according to the invention for producing a medicament for the treatment of hyperproliferative disorders is a subject of the instant invention.

Above and below, all temperatures are given in °C. In the examples below, "conventional work-up" means that the organic phase is washed with saturated NaHCO₃ solution, if desired with water and saturated NaCl solution, the phases are separated, the organic phase is dried over sodium sulfate and evaporated, and the product is purified by chromatography on silica gel, by preparative HPLC and/or by crystallization.

The present invention relates to methylene urea derivatives of formula I, the use of the compounds of formula I as inhibitors of raf-kinase, the use of the compounds of formula I for the manufacture of a pharmaceutical composition and a method of treatment, comprising administering said pharmaceutical composition to a patient.

### Examples

### Experimental part

### Synthesis of the benzylamine moieties

### 4-(4-Pyridinyloxy)benzylamine

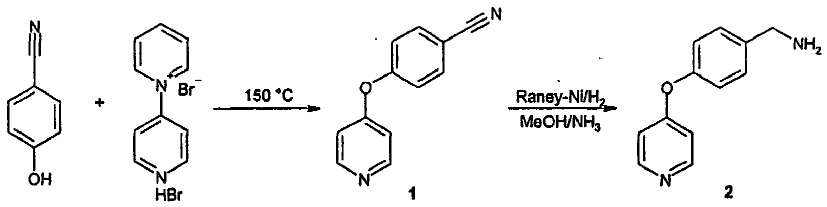

a) 5 g (42 mmol) of 4-hydroxybenzonitrile and 13.36 g (42 mmol) of bipyridine are mixed and heated to 150°C. After the reaction mixture has been stirred at 150°C for 3 hours, it is cooled, 500 ml of 10% Na₂CO₃ solution are added, and the mixture is stirred. The resultant precipitate is filtered off with suction, rinsed with 500 ml of water and dried under reduced pressure. Extraction of the aqueous phase with ethyl acetate followed by drying and evaporation gave further product.
   Yield: 3.86 g (47%) of **1**, pale-brown solid
b) Compound **1** is hydrogenated using Raney nickel in methanolic ammonia solution at 50°C and 5 bar. The reaction solution is filtered through kieselguhr and rinsed with MeOH, and the filtrate is subsequently evaporated.
   Yield: 3.49 g (78%) of **2**, brown oil

### 3-(4-Pyridinyloxy)benzylamine

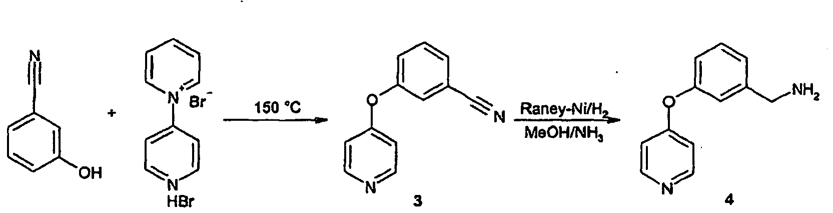

a) 5 g (42 mmol) of 3-hydroxybenzonitrile and 13.36 g (42 mmol) of bipyridine are mixed and heated to 150°C. After the reaction mixture has been stirred at 150°C for 3 hours, it is cooled, diluted with 600 ml of ethyl acetate and washed with 600 ml of 10% Na₂CO₃ solution. The organic phase is dried using Na₂SO₄, filtered and evaporated. The residue is purified by column chromatography (100 g of silica gel; eluent: ethyl acetate:petroleum ether = 1:1).
   Yield: 2.46 g (35%) of **3**, pale-yellow crystals
b) Compound **3** is hydrogenated using Raney nickel in methanolic ammonia solution at 50°C and 5 bar. The reaction solution is filtered through kieselguhr and rinsed with MeOH, and the filtrate is subsequently evaporated.
   Yield: 2.33 g (96%) of **4,** brown oil

### 4-(3-Pyridinyloxy)benzylamine

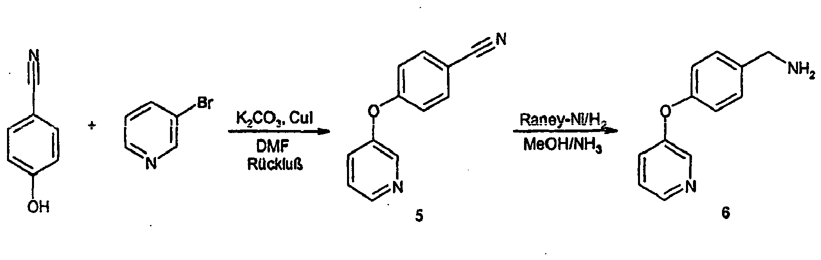

a) 3 g (25 mmol) of 4-hydroxybenzonitrile, 2.44 g (25 mmol) of 3-bromopyridine and 5.71 g (30 mmol) of copper iodide are dissolved in DMF, 6.91 g (50 mmol) of K₂CO₃ are added, and the mixture is refluxed for 18 hours. The reaction mixture is cooled, 200 ml of dichloromethane are added, and the mixture is stirred for 15 minutes and filtered. The filtrate is washed with water and extracted with 10% HCl solution. The HCl phase is neutralized using NH₄OH solution and extracted with dichloromethane. The combined organic phases are washed with NaOH solution (2M), dried using Na₂SO₄, filtered and evaporated. The residue is purified by column chromatography (25 g of silica gel, eluent: ethyl acetate:petroleum ether = 1:2).
   Yield: 375 mg (8%) of **5,** yellow crystals
b) Compound **5** is hydrogenated using Raney nickel in methanolic ammonia solution at 50°C and 4.8 bar. The reaction solution is filtered through kieselguhr and rinsed with MeOH, and the filtrate is subsequently evaporated.
   Yield: 440 mg (97%) of **6**, brown oil

### 3-(3-Pyridinyloxy)benzylamine

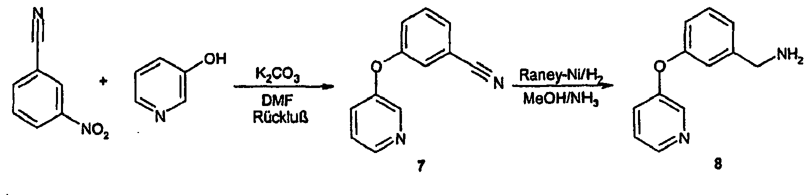

a) 1 g (11 mmol) of 3-hydroxypyridine and 3.26 g (22 mmol) of 3-nitrobenzonitrile are dissolved in DMF, 3.34 g (24 mmol) of potassium carbonate are added, and the mixture is refluxed overnight. The reaction mixture is evaporated, and the residue is taken up in 150 ml of dichloromethane, stirred for 30 minutes and filtered. The filtrate is washed with water and extracted with 10% HCl solution. The HCl phase is neutralized using NH₄OH and extracted with dichloromethane. The combined organic phases are dried using Na₂SO₄, filtered and evaporated. The residue is purified by column chromatography (33 g of silica gel, eluent: ethyl acetate:petroleum ether = 1:2).
   Yield: 956 mg (44%) of **7**, yellow oil
b) Compound **7** is hydrogenated using Raney nickel in methanolic ammonia solution at 50°C and 4.8 bar. The reaction solution is filtered through kieselguhr and rinsed with MeOH, and the filtrate is subsequently evaporated.
   Yield: 945 mg (97%) of **8,** brown oil

### Synthesis of the benzylureas

### Variant A

### 1-(4-Chloro-3-trifluoromethylphenyl)-3-[4-(4-pyridinyloxy)benzyl]urea

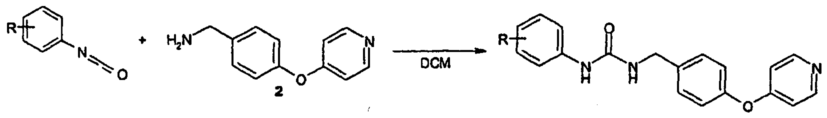

150 mg (0.75 mmol) of **2** are dissolved in dichloromethane together with 166 mg (0.75 mmol) of 4-chloro-3-trifluoromethylphenyl isocyanate, and the mixture is stirred at room temperature for 2 hours. The reaction mixture is evaporated, and the residue is purified by column chromatography (11 g of silica gel, eluent: ethyl acetate:petroleum ether = 2:1).
Yield: 119 mg (37%), colourless oil **1-(4-Chloro-3-trifluoromethylphenyl)-3-[3-(4-pyridinyloxy)benzyl]urea**

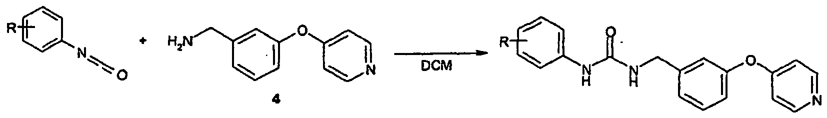

100 mg (0.5 mmol) of **4** are dissolved in dichloromethane together with 133 mg (0.6 mmol) of 4-chloro-3-trifluoromethylphenyl isocyanate and 0.1 ml (0.6 mmol) of N-ethyldiisopropylamine, and the mixture is stirred at room temperature for 2 hours. The resultant precipitate is filtered off with suction, washed with dichloromethane and dried under reduced pressure.
Yield: 201 mg (96%), colourless solid

### 1-(4-Chloro-3-trifluoromethylphenyl)-3-[4-(3-pyridinyloxy)benzyl]urea

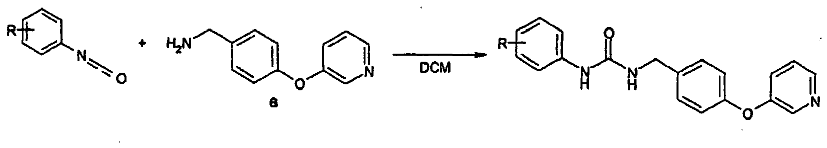

100 mg (0.5 mmol) of **6** are dissolved in dichloromethane together with 111 mg (0.5 mmol) of 4-chloro-3-trifluoromethylphenyl isocyanate. After the reaction mixture has been stirred at room temperature for 4 hours, it is refluxed for 1 hour and subsequently stirred again at room temperature overnight. The reaction mixture is diluted with dichloromethane, extracted with saturated Na₂HCO₃ solution, dried using Na₂SO₄, filtered and evaporated. The residue is purified by preparative HPLC.
Yield: 40 mg (19%), yellow oil

### 1-(4-Chloro-3-trifluoromethylphenyl)-3-[3-(3-pyridinyloxy)benzyl]urea.

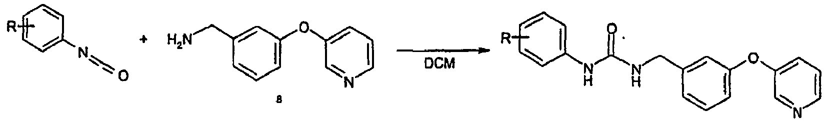

100 mg (0.5 mmol) of **8** are dissolved in dichloromethane together with 111 mg (0.5 mmol) of 4-chloro-3-trifluoromethylphenyl isocyanate. After the reaction mixture has been stirred at room temperature for 4 hours, it is refluxed for 1 hour and subsequently stirred again at room temperature overnight. The reaction mixture is diluted with dichloromethane, extracted with saturated Na₂HCO₃ solution, dried using Na₂SO₄, filtered and evaporated. The residue is purified by column chromatography (3 g of silica gel, eluent: ethyl acetate:petroleum ether = 1:3).
Yield: 34 mg (16%), colourless solid

### Variant B

### 5-t-Butyl-3-isoxazolyl)carbamic acid, 4-nitrophenyl ester

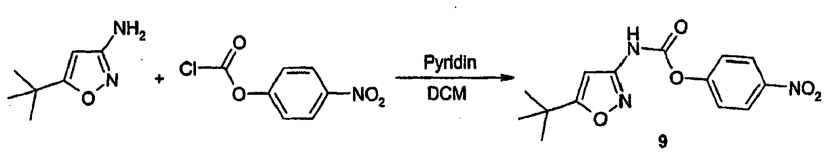

2.91 g (20.76 mmol) of 3-amino-5-tert-butylisoxazole and 1.84 ml (22.83 mmol) of pyridine are dissolved in dichloromethane, and 4.18 g (20.76 mmol) of 4-nitrophenyl chloroformate are added at room temperature. After the reaction mixture has been stirred at room temperature for 2.5 hours, it is evaporated, and the residue is digested with diethyl ether, filtered off with suction and subsequently dried under reduced pressure.
Yield: 5.68 g (90%) of **9**, colourless solid

### 1-(5-t-Butyl-3-isoxazolyl)-3-[4-(4-pyridinyloxy)benzyl]urea

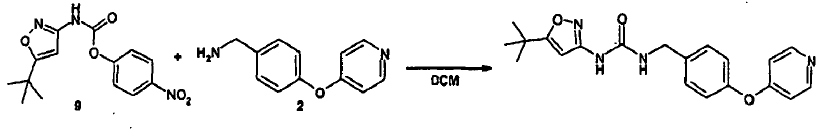

100 mg (0.33 mmol) of **9** and 79 mg (0.39 mmol) of **2** are dissolved in dichloromethane, and the solution is stirred at room temperature for 4 hours. The reaction mixture is evaporated, and the residue is purified by column chromatography (5 g of silica gel, eluent: ethyl acetate).
Yield: 67 mg (56%), colourless solid

### 1-(5-t-Butyl-3-isoxazolyl)-3-[3-(4-pyridinyloxy)benzyl]urea

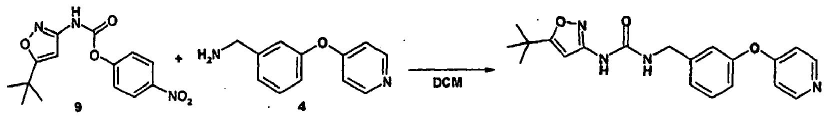

100 mg (0.33 mmol) of **9** and 79 mg (0.39 mmol) of **4** are dissolved in dichloromethane, and the solution is stirred at room temperature for 4 hours. The reaction mixture is evaporated, and the residue is purified by column chromatography (5 g of silica gel, eluent: ethyl acetate).
Yield: 33 mg (27.5%), colourless oil

### Synthesis of the substituted benzyl amino building blocks

### 4-[(4-Aminomethyl)phenoxyl-2-pyridine carbonic acid, methylamide

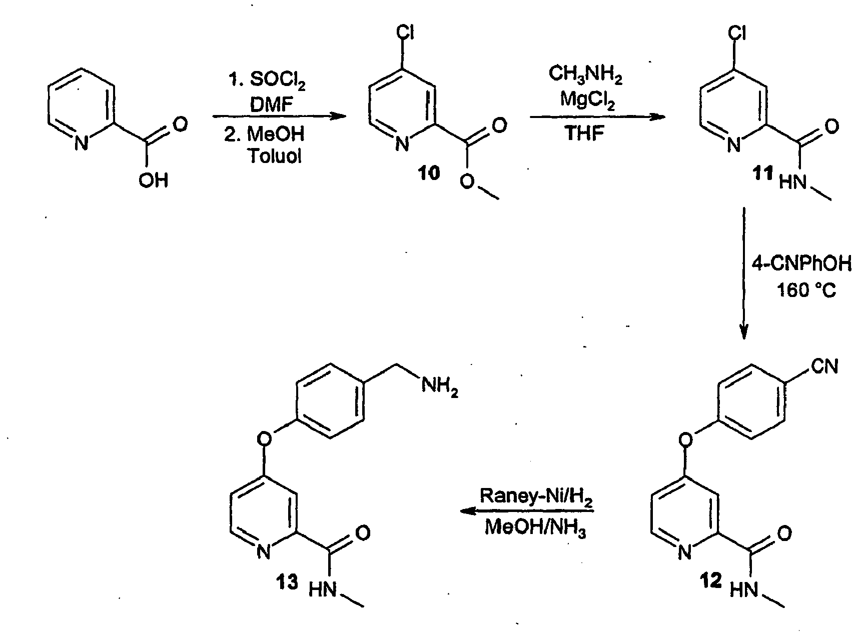

60 ml Thionylchloride are heated to a temperature of 45°C under a nitrogen atmosphere and 1.83 ml Dimethylformamide is added slowly. 20 g Pyridin-2-carbonic acid is added to the solution in portions, the reaction mixture is stirred another 15 min at 45°C and then heated to 80 °C for 24 hrs. The reaction mixture is evaporated and the resulting residue treated with dry toluene as a carrier and then evaporated. This procedure is repeated several times. The resulting oil is dissolved in toluene, cooled to 0 °C, slowly treated with methanol and stirred for one hour. The resulting precipitate is filtered by suction, washed with toluene and recrystallised from acetone.
Yield: 15 g (44 %) **10,** colourless crystals

13 g (62.5 mmol) **10** are dissolved together with 2.98 g (31.24 mmol) dry Magnesiumchloride in THF. After 5 min 110 ml methyl amine solution (2M in THF) are added dropwise within 10 min and the suspension stirred for 2 h at room temperature. 120 ml water und 63 mL 1 M HCl- solution are added and the mixture is extracted three times with ethyl acetate. The combined organic phases are washed with brine, dried with Na₂SO₄, filtered and evaporated.
Yield: 10.5 g (98.5 %) **11,** colourless oil.

4.15 g (24.32 mmol) **11** are heated to a temperature of 160°C together with 5.8 g (48.65 mmol) 4-Cyanophenol for 18 hrs in an argon atmosphere. The reaction mixture is cooled down, diluted with ethyl acetate, washed consecutively twice with 30 ml 2N NaOH- solution, twice with 30 ml Wasser and once with 30 ml brine, dried over Na₂SO₄, filtered and evaporated. The residue is digested with diethyl ether, filtered by suction, washed with diethyl ether:petrol ether = 1:1 and dried in vacuo.
Yield: 3.27 g (52 %) **12**, brownish solid.

3.27 g (12.65 mmol) **12** are hydrogenated in methanolic ammonia solution in the presence of Raney nickel at 45°C and 5 bar pressure. The reaction mixture is filtered over kieselguhr, washed with MeOH and the filtrate is evaporated. The residue is purified by chromatography (120 g kieselgel, eluent: CH₂Cl₂/ methanol/ NH₃ (9:1 + 0,1%).
Yield: 2.55 g (78 %) **13**, yellow solid

### 4-[(3-Aminomethyl)phenoxyl-2-pyridincarbonsilure, methylamid

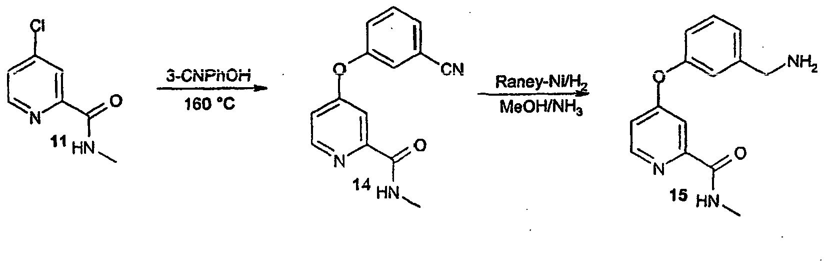

5 g (29.31 mmol) **11** are heated together with 6.98 g (58.62 mmol) 3-Cyanophenol at a temperature of 160°C. in an argon atmosphere. After 18 hrs, further 3.49 g (29.30 mmol) 3-Cyanophenol are added and heating is continued for 6 hrs at 160°C. The reaction mixture is cooled, diluted with ethyl acetate, washed consecutively twice with 40 ml 2N NaOH- solution, twice with 35 ml water and once with 30 ml brine, dried over Na₂SO₄, filtered and evaporated. The residue is digested with diethyl ether, filtered by suction, washed with diethyl ether:petrol ether = 2:1 and dried in vacuo. Another portion of product is obtained by chromatography of the mother liquor (95 g silica gel, eluent: ethyl acetat:petrol ether = 7:3).
Yield: 3.58 g (46 %) **14,** brownish solid

3.16 g (12.65 mmol) **14** are hydrogenated in methanolic ammonia solution in the presence of Raney nickel at 45°C and 5 bar pressure. The reaction mixture is filtered over kieselguhr, washed with MeOH and the filtrate is evaporated. The residue is purified by chromatography (120 g silica gel, eluent: CH₂Cl₂/ methanol/ NH₃ (9:1 + 0,1 %).
Yield: 2.67 g (86 %) **15,** light brownish oil

### Synthesis of benzyl ureas

### Variant A

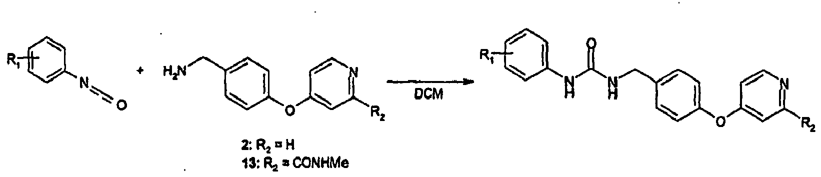

A solution of 0.16 mmol isocyanate and 0.16 mmol benzyl amine **2** or **13** in dichlormethane is stirred 16 h at room temperature. Depending to the respective reaction course, the working up of the reaction mixtures is done according to one of the variations given below:
Variant A: The resulting precipitate is filtered by suction, washed consecutively with dichlormethane, ethyl acetate and diethyl ether and dried in vacuo at 40 °C.
Variant B: The reaction mixture is evaporated and the residue is treated with 0.5 ml acetonitrile. The resulting precipitate is filtered by suction, washed with acetonitrile and diethyl ether and dried in vacuo at 40 °C.
Variant C: The reaction mixture is evaporated, the oily residue taken up in 2 ml acetonitrile: water = 1:1, frozen and then freeze dried for 16 hrs.
Variant D: The reaction mixture is evaporated to dryness. The residue is dried in vacuo at 40 °C.
Variant E: The reaction mixture is evaporated to dryness. The residue is purified by chromatography (4 g silica gel, eluent: dichlormethane:methanol = 98:2 to 95:5). The obtained crude product is taken up in 1.2 ml acetonitrile:water = 2:1, frozen and freeze dried for 16 hrs.
Variant F: The reaction mixture is evaporated to dryness, the residue dissolved in 15 ml ethyl acetate and extracted once with 10 ml 25 % hydrochloric acid. The water phase is separated, made alkaline with 32 % sodium hydroxide solution (pH= 9-10) and extracted three times, each time with 10 ml ethyl acetate. The combined organic phases are washed with 15 ml brine, dried over Na₂SO₄, filtered and the filtrate evaporated. The residue is dried at 40°C overnight.

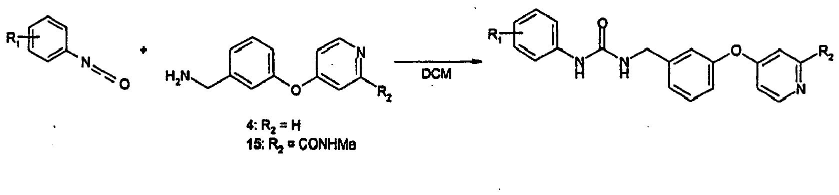

0.16 mmol isocyanate and 0.16 mmol benzyl amine **4** or **15** are dissolved in dichlormethane gelöst and stirred for 16 hrs at room temperature. Depending to the respective reaction course, the working up of the reaction mixtures is done according to one of the variations given below:
Variant A: The resulting precipitate is filtered by suction, washed consecutively with dichlormethane, ethyl acetate and diethyl ether and dried in vacuo at 40 °C.
Variant B: The reaction mixture is evaporated. The residue is dried in vacuo at 40 °C.
Variant C: The reaction mixture is evaporated to dryness. The residue is purified by chromatography (4 g silica gel, eluent: dichloromethane:methanol = 98:2 bis 95:5). The obtained crude product is taken up in 1.2 ml acetonitrile:water = 2:1, frozen and freeze dried for 16 hrs.
Variant D: The reaction mixture is evaporated, the oily residue taken up in 2 ml acetonitrile:water = 1:1, frozen and freeze dried for 16 hrs.
Variant E: The reaction mixture is evaporated, the residue digested with 1 ml ethyl acetate:diethyl ether = 2:1, filtered by suction, washed with diethyl ether and dried in vacuo at 40 °C.
Variant F: The reaction mixture is evaporated and the residue is treated with 0.5 ml acetonitrile. The resulting precipitate is filtered by suction, washed with acetonitrile and diethyl ether and dried in vacuo at 40 °C.
Variante G: The reaction mixture is evaporated to dryness, the residue dissolved in 15 ml ethyl acetate and extracted once with 10 ml 25 % hydrochloric acid. The water phase is separated, made alkaline with 32 % sodium hydroxide solution (pH= 9-10) and extracted three times, each time with 10 ml ethyl acetate. The combined organic phases are washed with 15 ml brine, dried over Na₂SO₄, filtered and the filtrate evaporated. The residue is dried at 40°C overnight.

### Variant B

### b) synthesis of the trichloro aceto anilides

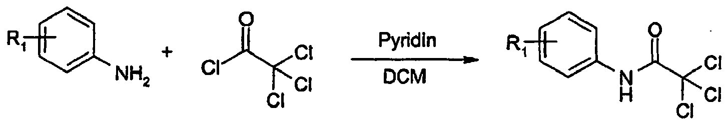

1 g of the substituted aniline and 1.3 eq. pyridine are dissolved in dichloromethane, cooled to 0 °C and 1.1 eq. trichloro acetic acid chloride is slowly added. After the addition is completed, the reaction mixture is allowed warm up to room temperature and stirring is continued for 1 h. Then the reaction mixture is extracted 1N hydrochloric acid and water consecutively and the organic phase is dried over Na₂SO₄, filtered and evaporated.
R₁ = 2-OMe, 5-CF₃; colourless solid, yield: 93 %
R₁ = 3-CF₃, 4-Br; colourless solid, yield: 100 %
R₁ = 3-OCF₃; yellow solid, yield: 82 %
R₁ = 2-OMe, 4-Me, 5-Cl; beige solid, yield: 84 %
R₁ = 2-OMe, 4-Cl, 5-CF₃; yellow oil, either: 85 %
R₁ = 2-SMe, 5-CF₃; yellow solid, yield: 92 %
R₁ = 2-OMe, 5-Me; beige solid, yield: 99 %
R₁ = 3-Me, 4-Cl; colourless solid, yield: 97 %

### b) synthesis of the final products

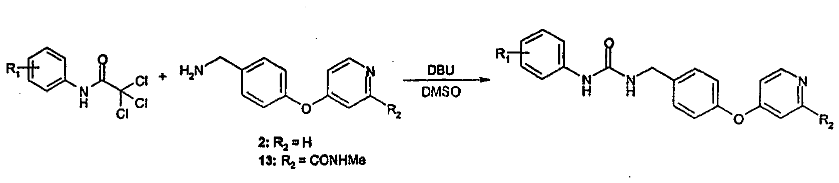

0.15 mmol of the substituted trichloro aceto anilide and 0.15 mmol benzyl amine **2** or **13** are dissolved in DMSO, 0.15 mmol DBU are added and the mixture heated to 80°C for 2.5 - 5.5 hrs. Depending to the respective reaction course, the working up of the reaction mixtures is done according to one of the variations given below:
Variant A: The reaction mixture is cooled, diluted with dichloromethane and extracted with 1 N hydrochloric acid. The water phase is made alkaline with 2N sodium hydroxide solution and extracted several times with ethyl acetate. The combined organic phases are dried over Na₂SO₄, filtered and evaporated.
Variant B: The reaction mixture is cooled, diluted with dichloromethane and extracted with 1 N hydrochloric acid. The water phase is made alkaline with 2N sodium hydroxide solution and extracted several times with ethyl acetate. The combined organic phases are dried over Na₂SO₄, filtered and evaporated. The residue is purified by chromatography (5 g silica gel, eluent:dichloromethane:methanol = 98:2 to 95:5).
Variante C: The reaction mixture is cooled, diluted with dichloromethane and washed with water. The organic phases what dried over Na₂SO₄, filtered and evaporated. The residue is digested with a small amount of water, the resulting precipitate filtered by suction and dried in vacuo at 40 °C.
Variante D: The reaction mixture is cooled, diluted with dichloromethane, extracted consecutively twice with 1 N hydrochloric acid and with water, dried over Na₂S0₄, filtered and evaporated.

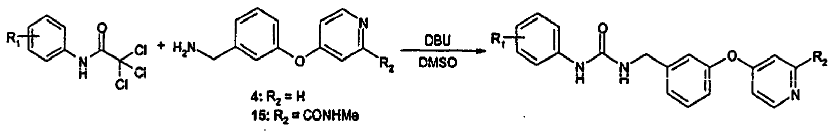

0.15 mmol of the substituted trichloro aceto anilide and 0.15 mmol benzyl amine **4** or **15** are dissolved in DMSO, 0.15 mmol DBU are added and the mixture heated to 80°C for 2.5 - 5.5 hrs. Depending to the respective reaction course, the working up of the reaction mixtures is done according to one of the variations given below:
Variant A: The reaction mixture is cooled, diluted with dichloromethane and extracted with 1 N hydrochloric acid. The water phase is made alkaline with 2N sodium hydroxide solution and extracted several times with ethyl acetate. The combined organic phases are dried over Na₂SO₄, filtered and evaporated.
Variant B: The reaction mixture is cooled, diluted with dichloromethane and extracted with 1 N hydrochloric acid. The water phase is made alkaline with 2N sodium hydroxide solution and extracted several times with ethyl acetate. The combined organic phases are dried over Na₂SO₄, filtered and evaporated. The residue is purified by chromatography (5 g silica gel, eluent:dichloromethane:methanol = 98:2 to 95:5).
Variant C: The reaction mixture is cooled, diluted with dichloromethane and washed with water. The organic phases whas dried over Na₂SO₄, filtered and evaporated. The residue is digested with a small amount of water, the resulting precipitate filtered by suction and dried in vacuo at 40 °C.

### Variante C

### a) Synthesis of the anilines

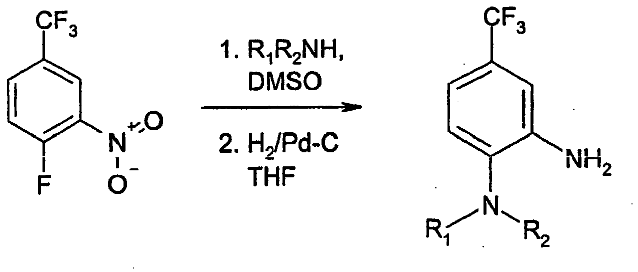

2 mmol 4-Fluoro-3-nitrobenzotrifluoride are dissolved in dimethyl sulfoxide (DMSO), treated with 2-2.4 mmol amine and stirred at 50°C overnight. The reaction mixture is diluted with dichloromethane and extracted twice with water. The organic phase is dried over Na₂SO₄, filtered and evaporated in vacuo. The obtained crude product is employed in the next synthesis step without further purification.
R₁, R₂ = Me; orange oil, yield: 96 %
R₁, R₂ = Et; orange oil, yield: 97 %
R₁ = Me, R₂ = (CH₂)₂OCH₃; orange oil, yield: 91.5 %
R₁ = Me, R₂ = (CH₂)₂N(CH₃)₂; orange oil, yield: 85 %

The accordingly obtained nitro compounds are hydrogenated in THF in the presence of H₂ and Pd/C (5%) at room temperature overnight. Then the catalyst is separated by filtration and the filtrate evaporated to yield the respective aniline.
R₁, R₂ = Me; yellow oil, yield: 92 %
R₁, R₂ = Et; yellow oil, yield: 92 %
R₁ = Me, R₂ = (CH₂)₂OCH₃; red oil, yield: 99 %
R₁ = Me, R₂ = (CH₂)₂N(CH₃)₂: yellow oil, yield 98.5 %
R₁ = Me, R₂ = (CH₂)₂N(CH₃)₂; yellow oil, yield: 98.5 %

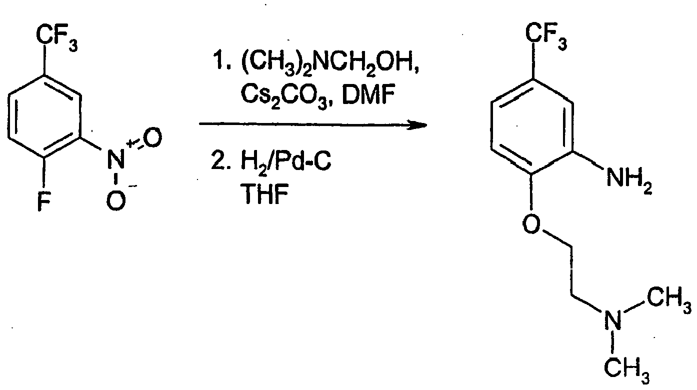

2 mmol 4-Fluor-3-nitrobenzotrifluorid are dissolved in dimethylformamide, treated with 2.2 mmol 2-(Dimethylamino)ethanol and 4.6 mmol cesium carbonate and stirred at room temperature. After 48 h, additional 1 mmol 2-(Dimethylamino)ethanol are added and at the reaction mixture is stirred at 40°C overnight. The reaction mixture is diluted with ethyl acetate and the resulting solution washed twice with water. The organic phase is dried over Na₂SO₄, filtered, treated with dry toluene as a carrier and then evaporated several times using a Rotavapor and then evaporated for dryness. The obtained residue is purified by chromatography (35 g silica gel, eluent: dichloromethane:acetone = 100:0 to 90:10).
Yield: 43 %, yellow oil
The accordingly obtained nitro compound is hydrogenated in THF in the presence of H₂ and Pd/C (5%) at room temperature overnight. Then the catalyst is separated by filtration and the filtrate evaporated to yield the respective aniline.
Yield: 97 %, yellow crystals

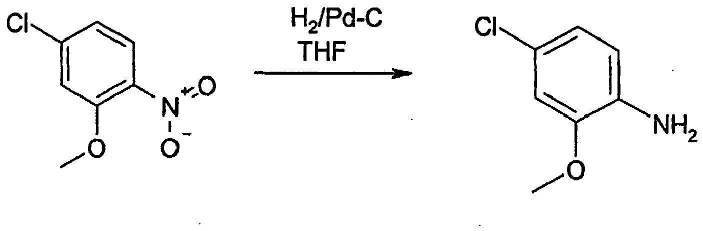

5-Chlor-2-nitroanisol in THF are hydrogenated in the presence of H₂ and Raney nickel at room temperature overnight. The catalyst is separated by filtration and the filtrate evaporated to dryness. The residue is purified by chromatography (35 g silica gel, eluent: dichloromethane:methanol = 99:1).
Yield: 69.5 %, brown oil

### b) synthesis of the final products

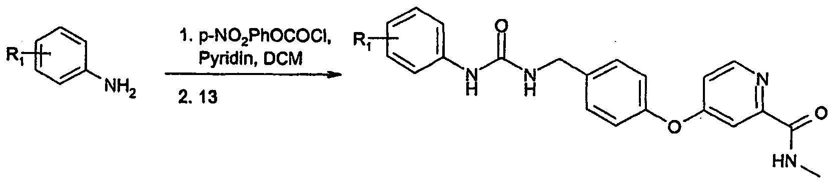

0.3 mmol aniline are dissolved in dichloromethane, consecutively treated with 0.33 mmol pyridine and 0.3 mmol of the p-Nitrophenylester of chloro formic acid and stirred at room temperature. After the reaction is finished, 0.33 mmol **13,** suspended in dichloromethane, and 0.3 mmol DIPEA are added und and the resulting solution stirred at room temperature. Depending to the respective reaction course, the working up of the reaction mixtures is done according to one of the variations given below:
Variante A: The reaction mixture is diluted with dichloromethane and washed three times with water. The organic phase is dried over Na₂SO₄, filtered and evaporated. The residue is recrystalized from ethylacetate.
Variant B: The reaction mixture is diluted with dichloromethane and washed three times with water. The organic phase is dried over Na₂SO₄, filtered and evaporated. The residue is purified by chromatography (10 g silica gel, eluent:petrol ether:ethyl acetate = 100:0 to 50:50).
Variant C: The reaction mixture is diluted with dichloromethane and washed three times with water. The organic phase is dried over Na₂SO₄, filtered and evaporated. The residue is purified by chromatography (10 g silica gel, eluent: dichlormethane:methanol = 100:0 to 95:5).
Variante D: The reaction mixture is treated with water, stirred and then the water phase discarded. The organic phase is washed once with water, once with brine, dried over Na₂SO₄, filtered and evaporated. The residue is taken up in hot acetonitrile. On cooling, a white precipitate is obtained which is filtered by suction and washed with diethylether. After some time, and additional portion of product precipitated from the mother liquor which is washed with diethylether/acetonitril (9:1). The combined products are dried in vaccuo at 40 °C.

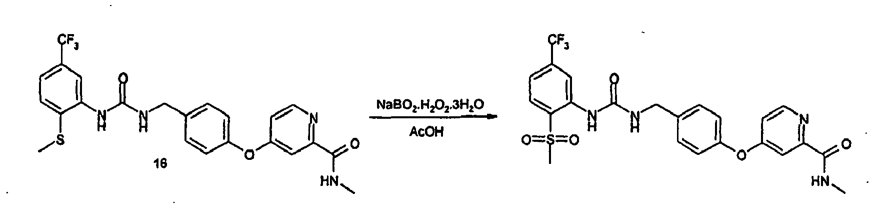

A solution of **16** in acetic acid is treated with 3 eq. sodium perborate trihydrate and heated to 55°C. After stirring for 2 hrs at 55°C the reaction mixture is allowed to stand overnight. Then, additional 1.5 eq. Sodium perborate trihydrate is added and the reaction mixture is stirred for another 4 hrs at 55°C. Then the reaction mixture is cooled and poured onto ice. After multiple extraction of the reaction mixture with ethyl acetate, the combined organic phases are washed twice with 2 N sodium hydroxide solution, once with water, dried over Na₂SO₄, filtered and evaporated. The residue is purified by chromatography (4 g silica gel, eluent: ethyl acetate:n-heptane = 4:6 to 8:2).
Yield: 58 %, colourless crystals

### Synthesis of methylene urea derivatives substituted on the methylene moiety

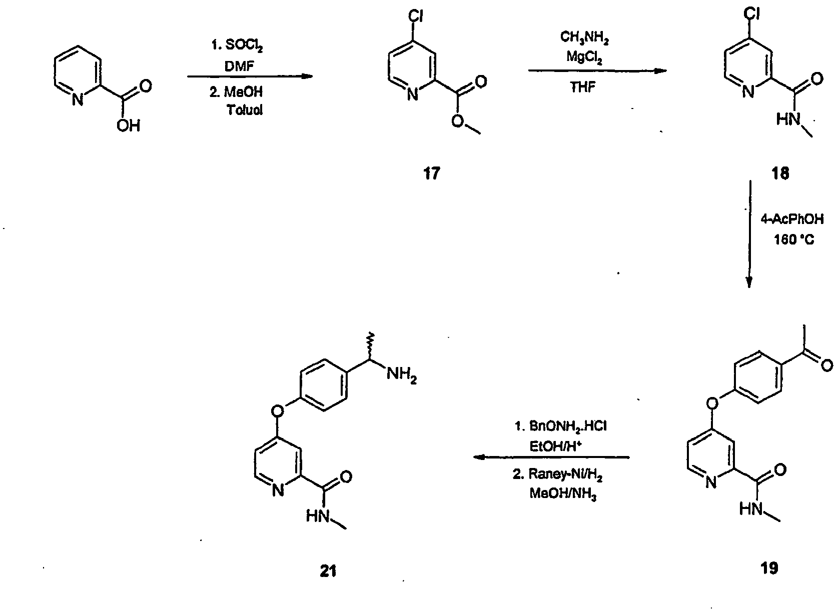

60 ml thionyl chloride are heated to 45°C under a nitrogen atmosphere and treated slowly with 1.83 ml dimethylformamide. To this solution, 20 g pyridine-to-carbonic acid are added in portions and the reaction mixture stirred another 45 minutes at the same temperature and then heated to 80 °C for 24 hours. The reaction mixture is evaporated and the resulting residue treated with dry toluene as a carrier and then evaporated. This procedure is repeated several times. The resulting oil is dissolved in toluene, cooled to 0 °C, slowly treated with methanol and stirred for one hour. The resulting precipitate is filtered by suction, washed with toluene and recrystallised from acetone.
Yield: 15 g (45%) of compound **17,** colourless crystals

13 g (62.5 mmol) of compound **17** are dissolved in THF together with 2.98 g (31.24 mmol) dry MgCl₂. After five minutes, 110 ml methyl amine-solution (2M in THF) are added within ten minutes and the resulting suspension stirred for two hours at room temperature. The reaction mixture is treated with 120 ml water and 63 ml 1 N hydrochloric acid and extracted three times with ethyl acetate. The combined organic phases are washed with brine, dried over Na₂SO₄, filtered and evaporated.
Yield: 10.5 g (98.5 %) of compound **18,** colourless oil.

440 mg (2.58 mmol) of compound **18** are heated up together with 1.05 g (7.74 mmol) 4-Hydroxy-acetophenon in an Argon atmosphere for 18 hours. The reaction mixture is cooled, diluted with ethyl acetate, washed three times with with 2N sodium hydroxide solution and twice with water, dried over Na₂SO₄, filtered and evaporated. The residue is purified by chromatography (10 g silica gel, eluent: ethyl acetate/n-heptane = 4:6 to 7:3). Further product is isolated by crystallising the mixed fractions with petrol ether/diethyl ether.
Yield: 344 mg (49%) of compound **19,** colourless solid

1 mg (0.37 mmol) of compound **19** is suspended in 2 ml ethanol together with 59 mg (0.37 mmol) O-Benzyl-hydroxylamine and treated with one drop concentrated sulphuric acid. The resulting clear solution is heated under reflux for 16 hours and then evaporated to dryness. The yellowish residue is treated with water, made alkaline with 1 N sodium hydroxide solution (pH = 8-9) and extracted twice with ethyl acetate. The combined organic phases are dried over Na₂SO₄, filtered and evaporated.
Yield: 151 mg (99%) of compound **20,** slightly yellow oil

3.46 g (9.22 mmol) of compound **20** are hydrogenated at room temperature and a pressure of 5 bar in methanolic ammonium solution in the presence of Raney nickel. The reaction mixture is filtered over kieselguhr, washed with methanol and the filtrate is evaporated. The residue is purified by chromatography (120 g silica gel, eluent: CH₂Cl₂/methanol/NH₃ = 94:6:0.1).
Yield: 2.29g (91%) of compound **21,** slightly yellow solid

### Synthesis of the methylene urea derivatives substituted on the methylene moiety:

### Method A

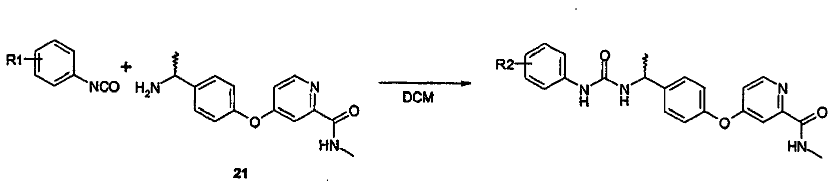

0.16 g isocyanate and 0.16 mmol benzyl amine are diluted in dichloromethane and stirred overnight at room temperature. Depending to the respective reaction course, the working up of the reaction mixtures is done according to one of the variations given below:
Variant A: The resulting precipitate is filtered by suction, washed with little dichloromethane and dried in vacuo at 40 °C.
Variant B: The reaction mixture is evaporated and the oily residue taken up in acetonitrile:water = 2:1, frozen and freeze dried overnight.
Variant C: The reaction mixture is evaporated to dryness and the residue dried in vacuo at 40 °C.

### Method B

### a) Synthesis of the trichloro aceto anilides

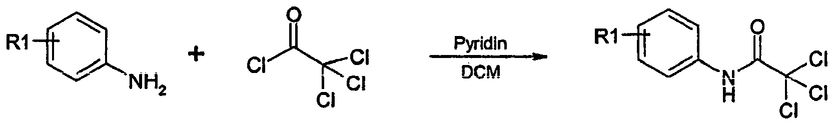

1 g substituted aniline and 1.3 eq. pyridine are dissolved in dichloromethane, cooled to 0°C and 1.1 eq. trichloro acetic acid chloride is slowly added. After the addition is completed, the reaction mixture is allowed warm up to room temperature and stirring is continued for 1 h. Then the reaction mixture is extracted 1 M hydrochloric acid and water consecutively and the organic phase is dried over Na₂SO₄, filtered and evaporated.
R₁ = 2-OMe, 5-CF₃; colourless solid, yield: 93 %
R₁ = 3-CF₃, 4-Br; colourless solid, yield: 100 %
R₁ = 3-OCF₃; yellow solid, yield: 82 %

### b) synthesis of the final products

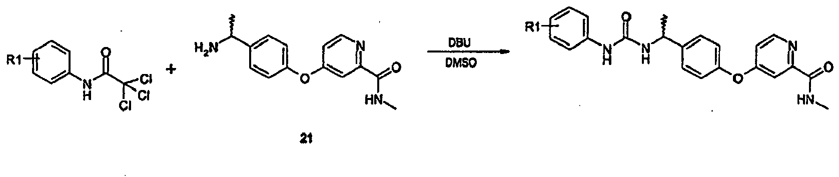

0.15 mmol of the substituted trichloro aceto anilide and 0.15 mmol benzyl amine 5 are dissolved in DMSO, 0.15 mmol DBU are added and the mixture heated to 80 °C for 2.5-34 hrs. Depending to the respective reaction course, the working up of the reaction mixtures is done according to one of the variations given below:
Variante A: The reaction mixture is cooled, diluted with dichloromethane and washed consecutively twice with 1 N hydrochloric acid and with water, dried over Na₂SO₄, filtered and evaporated. The residue is digested with a small amount of water, the resulting precipitate filtered by suction, washed with diethyl ether and dried in vacuo at 40 °C.
Variante B: The reaction mixture is cooled, diluted with dichloromethane, extracted consecutively twice with 1N hydrochloric acid and with water, dried over Na₂SO₄, filtered and evaporated. The residue is purified by chromatography (5 g silica gel, eluent: ethyl acetate/n-heptane = 4:6 to 6:4, taken up in acetonitrile: water = 2:1, frozen and freeze dried overnight.

HPLC method:
   Gradient: 5.5 min; flow rate: 2.75 ml/min from 90:10 to 0:100 H₂O/ACN Water + TFA (0.01 % by vol.); acetonitrile + TFA (0.01 % by vol.)
   Column: Chromolith SpeedROD RP 18e 50-4.6
   Wavelength: 220 nm, Rt=Retention time.
^{a}HPLC method:
   Gradient: 9 min; flow rate: 1.5 ml/min from 80:20 to 0:100 H₂O/ACN Water + TFA (0.01 % by vol.); acetonitrile + TFA (0.01 % by vol.)
   Column: Lichrospher RP-select-B (5 µm/125 mm)
   Wavelength: 220 nm; Rt=Retention time.

The compounds (1) to (224) as described above can preferably be produced according to the procedures described herein or in an analogous manner thereof.

The compounds (225) to (449) as described above can preferably be produced according to the procedures described herein, especially according to the ones for producing methylene urea derivatives beeing substituted on the methylene moiety, or in an analogous manner thereof.

The compounds (450) to (672) as described above can preferably be produced according to the procedures described herein, especially according to the ones for producing methylene urea derivatives beeing substituted on the methylene moiety, or in an analogous manner thereof.

### Example A: Injection vials

A solution of 100 g of an active compound of the formula I and 5 g of disodium hydrogenphosphate is adjusted to pH 6.5 in 3 l of double-distilled water using 2N hydrochloric acid, sterile-filtered, dispensed into injection vials, lyophilized under sterile conditions and aseptically sealed. Each injection vial contains 5 mg of active compound.

### Example B: Suppositories

A mixture of 20 g of an active compound of the formula I is fused with 100 g of soya lecithin and 1400 g of cocoa butter, poured into moulds and allowed to cool. Each suppository contains 20 mg of active compound.

### Example C: Solution

A solution of 1 g of an active compound of the formula I, 9.38 g of NaH₂PO₄ - 2 H₂O, 28.48 g of Na₂HPO₄ - 12 H₂O and 0.1 g of benzalkonium chloride in 940 ml of double-distilled water is prepared. It is adjusted to pH 6.8, made up to 1 l and sterilized by irradiation. This solution can be used in the form of eye drops.

### Example D: Ointment

500 mg of an active compound of the formula I is mixed with 99.5 g of petroleum jelly under aseptic conditions.

### Example E: Tablets

A mixture of 1 kg of active compound of the formula I, 4 kg of lactose, 1.2 kg of potato starch, 0.2 kg of talc and 0.1 kg of magnesium stearate is compressed to give tablets in a customary manner such that each tablet contains 10 mg of active compound.

### Example F: Coated tablets

Analogously to Example E, tablets are pressed and are then coated in a customary manner using a coating of sucrose, potato starch, talc, tragacanth and colourant.

### Example G: Capsules

2 kg of active compound of the formula I are dispensed into hard gelatin capsules in a customary manner such that each capsule contains 20 mg of the active compound.

### Example H: Ampoules

A solution of 1 kg of active compound of the formula I in 60 l of double-distilled water is sterile-filtered, dispensed into ampoules, lyophilized under sterile conditions and aseptically sealed. Each ampoule contains 10 mg of active compound.

## Claims

1. Methylene urea derivative, selected from the compounds of formula II, 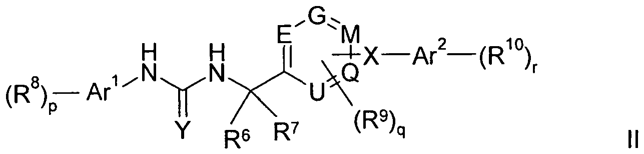 wherein
Ar¹, Ar² are selected independently from one another from ethylenical unsaturated or aromatic heterocyclic residues containing 3 to 10 carbon atoms and one or two hetero atoms, independently selected from N, O und S,
R⁶, R⁷ are independently selected from a the meanings given for R⁸, R⁹ and R¹⁰, or R⁶ and R⁷ together form a carbocyclic residue comprising 3 to 7 carbon atoms or a heterocyclic residue comprising 1, 2 or 3 hetero atoms, selected from the group consisting of O, N and S, and 2 to 6 carbon atoms, said carbocyclic or heterocyclic residue being unsubstituted or comprising 1, 2 or 3 substituents, selected from the meanings given for R⁸, R⁹ and R¹⁰,
E, G, M, Q and U are selected, independently from one another, from carbon atoms and nitrogen atoms, with the proviso that one or more of E, G, M, Q and U are carbon atoms and that X is bonded to a carbon atom,
R⁸, R⁹ and R¹⁰ are independently selected from a group consisting of H, A, cycloalkyl comprising 3 to 7 carbon atoms, Hal, CH₂Hal, CH(Hal)₂, C(Hal)₃, NO₂, (CH₂)ₙCN, (CH₂)ₙNR¹¹R¹², (CH₂)ₙO(CH₂)ₖNR¹¹R¹², (CH₂)ₙNR¹¹(CH₂)ₖNR¹¹R¹², (CH₂)ₙO(CH₂)ₖOR¹¹, (CH₂)ₙNR¹¹(CH₂)ₖOR¹², (CH₂)ₙCOOR¹³, (CH₂)ₙCOR¹³, (CH₂)ₙCONR¹¹R¹², (CH₂)ₙNR¹¹COR¹³, (CH₂)ₙNR⁸CONR¹¹R¹², (CH₂)ₙNR¹¹SO₂A, (CH₂)ₙSO₂NR¹¹R¹², (CH₂)ₙS(O)ᵤR¹³, (CH₂)ₙOC(O)R¹³, (CH₂)ₙCOR¹³, (CH₂)ₙSR¹¹, CH=N-OA, CH₂CH=N-OA, (CH₂)ₙNHOA, (CH₂)ₙCH=N-R¹¹, (CH₂)ₙOC(O)NR¹¹R¹², (CH₂)ₙNR¹¹COOR¹³, (CH₂)ₙN(R¹¹)CH₂CH₂OR¹³, (CH₂)ₙN(R¹¹)CH₂CH₂OCF₃, (CH₂)ₙN(R¹¹)C(R¹³)HCOOR⁸, (CH₂)ₙN(R¹¹), C(R¹³)HCOR⁸, (CH₂)ₙN(R¹¹)CH₂CH₂N(R¹²)CH₂COOR⁸, (CH₂)ₙN(R⁸)CH₂CH₂NR¹²R⁸, CH=CHCOOR¹³, CH=CHCH₂NR¹¹R¹², CH=CHCH₂NR¹¹R¹², CH=CHCH₂OR¹³, (CH₂)ₙN(COOR¹³)COOR¹⁴, (CH₂)ₙN(CONH₂)COOR¹³, (CH₂)ₙN(CONH₂)CONH₂, (CH₂)ₙN(CH₂COOR¹³)COOR¹⁴, (CH₂)ₙN(CH₂CONH₂)COOR¹³, (CH₂)ₙN(CH₂CONH₂)CONH₂, (CH₂)ₙCHR¹³COR¹⁴, (CH₂)ₙCHR¹³COOR¹⁴, (CH₂)ₙCHR¹³CH₂OR¹⁴, (CH₂)ₙOCN and (CH₂)ₙNCO, wherein
R¹¹, R¹² are independently selected from a group consisting of H, A, (CH₂)ₘAr³ and (CH₂)ₘHet, or in NR¹¹R¹²,
R¹¹ and R¹² form, together with the N-atom they are bound to, a 5-, 6- or 7- membered heterocyclus which optionally contains 1 or 2 additional hetero atoms, selected from N, O and S,
R¹³, R¹⁴ are independently selected from a group consisting of H, Hal, A, (CH₂)ₘAr⁴ and (CH₂)ₘHet,
A is selected from the group consisting of alkyl, alkenyl, cycloalkyl, alkylenecycloalkyl, alkoxy, alkoxyalkyl and saturated heterocyclyl,
Ar³, Ar⁴ are independently from one another aromatic hydrocarbon residues comprising 5 to 12 and preferably 5 to 10 carbon atoms which are optionally substituted by one or more substituents, selected from a group consisting of A, Hal, NO₂, CN, OR¹⁵, NR¹⁵R¹⁶, COOR¹⁵, CONR¹⁵R¹⁶, NR¹⁵COR¹⁶ , NR¹⁵ CONR¹⁵R¹⁶, NR¹⁶SO₂A, COR¹⁵, SO₂R¹⁵R¹⁶, S(O)ᵤA and OOCR¹⁵,
Het is a saturated, unsaturated or aromatic heterocyclic residue which is optionally substituted by one or more substituents, selected from a group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOCR¹⁵,
R¹⁵, R¹⁶ are independently selected from a group consisting of H, A, and (CH₂)ₘAr⁶, wherein
Ar⁶ is a 5- or 6-membered aromatic hydrocarbon which is optionally substituted by one or more substituents selected from a group consisting of methyl, ethyl, propyl, 2-propyl, tert.-butyl, Hal, CN, OH, NH₂ and CF₃,
k, n and m are independently of one another 0, 1, 2, 3, 4, or 5;
X represents a bond or is (CR¹¹R¹²)ₕ, or (CHR¹¹)ₕ-Q-(CHR¹²)ᵢ, wherein
Q is selected from a group consisting of O, S, N-R¹⁵, (CHal₂)ⱼ, (O-CHR¹⁸)ⱼ, (CHR¹⁸-O)_{j,} CR¹⁸=CR¹⁹, (O-CHR¹⁸CHR¹⁹)ⱼ, CHR¹⁸CHR¹⁹-O)ⱼ, C=O, C=S, C=_{NR} ¹5, CH(OR¹⁵), C(OR¹⁵)(OR²⁰), C(=O)O, OC(=O), OC(=O)O, C(=)N(R¹⁵), N(R¹⁵)C(=O), OC(=O)N(R¹⁵), N(R¹⁵)C(=O)O, CH=N-O, CH=N-NR¹⁵, OC(O)NR¹⁵, NR¹⁵C(O)O, S=O, SO₂, SO₂NR¹⁵ und NR¹⁵SO₂, wherein
h, i are independently from each other 0, 1, 2, 3, 4, 5 or 6, and
j is 1, 2, 3, 4, 5 or 6,
Y is selected from O, S, NR²¹, C(R²²)-NO₂, C(R²²)-CN and C(CN)₂, wherein
R²¹ is independently selected from the meanings given for R¹³, R¹⁴, and
R²² is independently selected from the meanings given for R¹¹, R¹²,
p, r are independently from one another 0, 1, 2, 3, 4 or 5,
q is 0, 1, 2, 3 or 4, preferably 0, 1 or 2,
u is 0, 1, 2 or 3, preferably 0, 1 or 2,
and
Hal is independently selected from a group consisting of F, Cl, Br and I;
and the pharmaceutically acceptable derivatives, salts and solvates thereof.

2. Methylene urea derivative according to claim 1, selected from the compounds of formula IIc, IId, IIk, IIL, IIm, IIn, IIo, IIp, IIq, IIr, IIs, IIt, IIu, IIv, IIw and IIx, 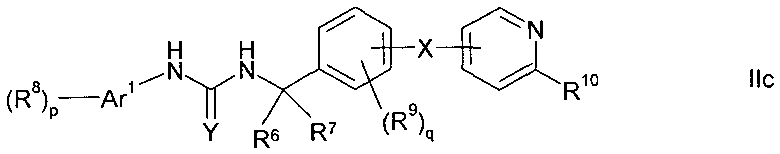 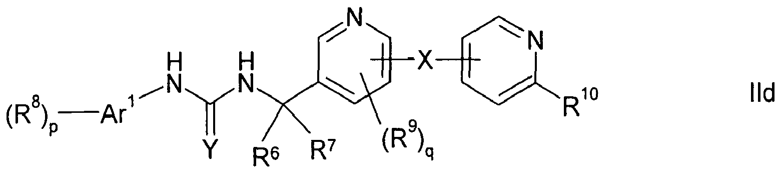 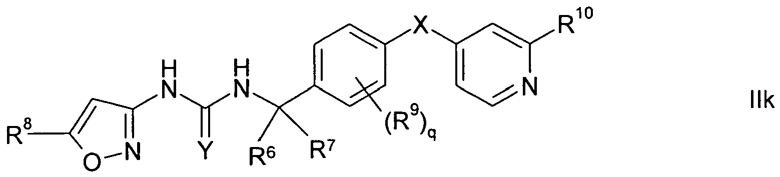 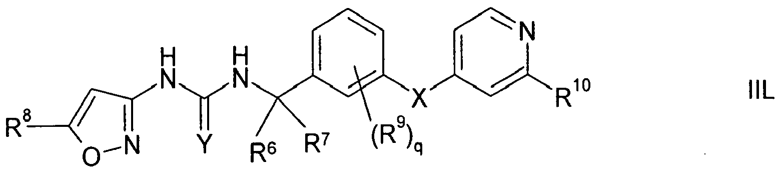 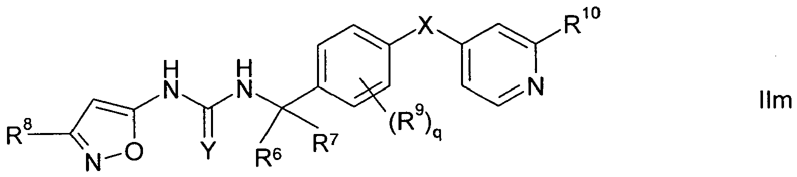 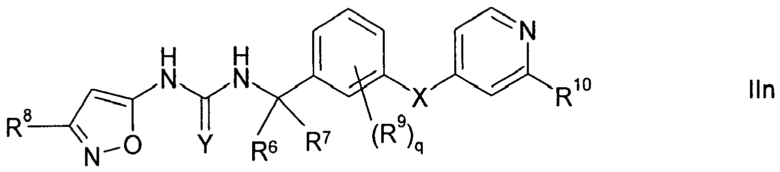 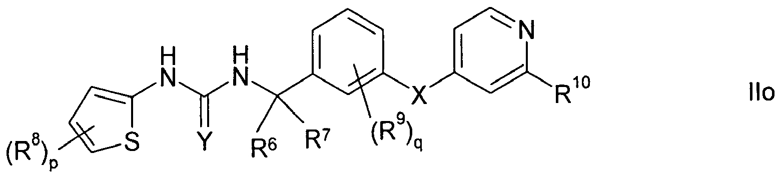 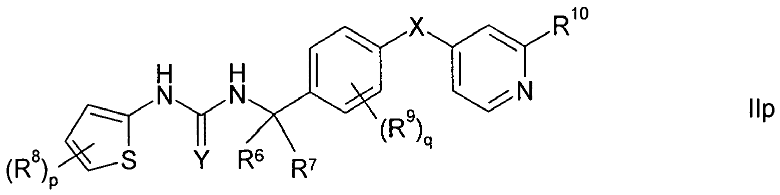 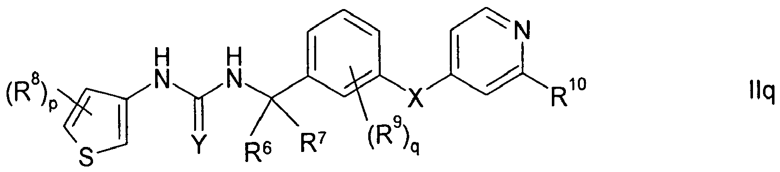 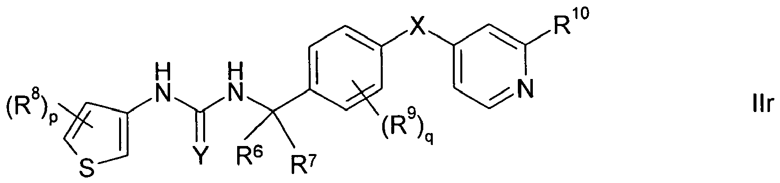 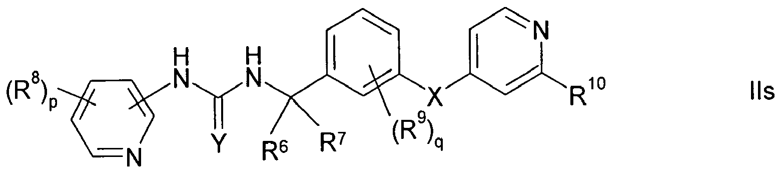 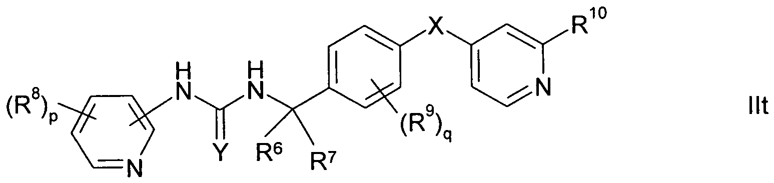 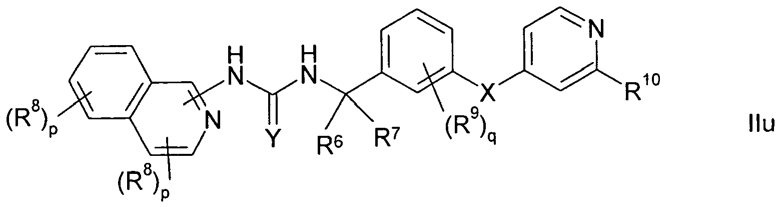 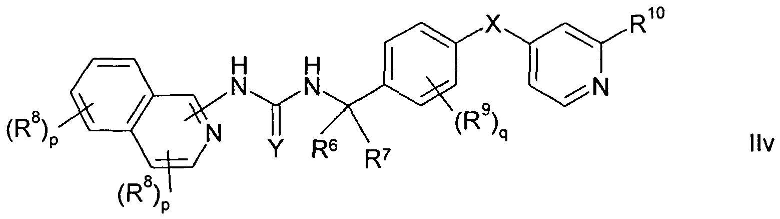 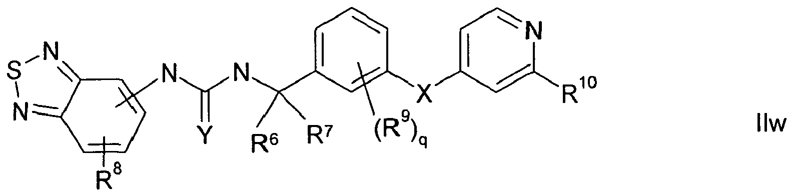 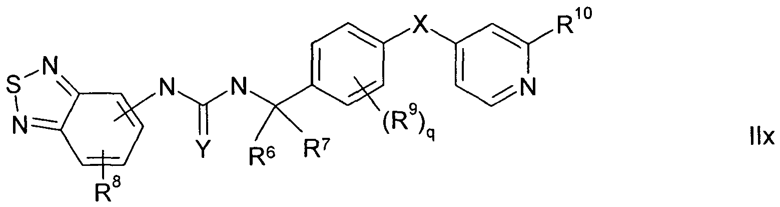 wherein R⁶, R⁷, R⁸, p, Ar¹, Y, X, R⁹ and q are as defined in claim 1, R¹⁰ is H or as defined in claim 3; and the pharmaceutically acceptable derivatives, salts and solvates thereof.

3. Methylene urea derivative according to claim 1 or 2, selected from the compounds (1) to (224) of table 1, the compounds (225) to (449) of table 2 and/or the compounds (450) to (672) of table 3, and the pharmaceutically acceptable derivatives, salts and solvates thereof.

4. Methylene urea derivative according to one of the claims 1, 2 or 3, selected from the compounds (673) to (758), the compounds (759) to (825) and/or the compounds (826) to (874), and the pharmaceutically acceptable derivatives, salts and solvates thereof.

5. Methylene urea derivative according to one of the claims 1 to 4 as a medicament.

6. Methylene urea derivative according to one of the claims 1 to 4 as a kinase inhibitor.

7. Methylene urea derivative according to claim 6, **characterized in that** the kinases are selected from raf-kinases.

8. Pharmaceutical composition, **characterised in that** it contains one or more compounds according to one of the claims 1 to 4.

9. Pharmaceutical composition according to claim 8, **characterised in that** it contains one or more additional compounds, selected from the group consisting of physiologically acceptable excipients, auxiliaries, adjuvants, carriers and pharmaceutical active ingredients other than the compounds according to one of the claims 1 to 4.

10. Process for the manufacture of a pharmaceutical composition, **characterised in that** one or more compounds according to one of the claims 1 to 4 and one or more compounds, selected from the group consisting of carriers, excipients, auxiliaries and pharmaceutical active ingredients other than the compounds according to one of the claims 1 to 4, is processed by mechanical means into a pharmaceutical composition that is suitable as dosageform for application and/or administration to a patient.

11. Use of a compound according to one of the claims 1 to 4 as a pharmaceutical.

12. Use of a compound according to one of the claims 1 to 4 in the treatment and/or prophylaxis of disorders.

13. Use of a compound according to one of the claims 1 to 4 for producing a pharmaceutical composition for the treatment and/or prophylaxis of disorders.

14. Use according to claim 12 or 13, **characterised in that** the disorders are caused, mediated and/or propagated by raf-kinases.

15. Use according to claim 12, 13 or 14, **characterised in that** the disorders are selected from the group consisting of hyperproliferative and nonhyperproliferative disorders.

16. Use according to claim 12, 13, 14 or 15, **characterised in that** the disorder is cancer.

17. Use according to claim 12, 13, 14 or 15, **characterised in that** the disorder is noncancerous.

18. Use according to claim 12, 13, 14, 15 or 17, **characterised in that** the disorders are selected from the group consisting of psioarsis, arthritis, inflammation, endometriosis, scarring, Helicobacter pylori infection, Influenza A, begnin prostatic hyperplasia, immunological diseases, autoimmune diseases and immunodeficiency diseases.

19. Use according to one of the claims 12 to 16, **characterised in that** the disorders are selected from the group consisting of melanoma, brain cancer, lung cancer, squamous cell cancer, bladder cancer, gastric cancer, pancreatic cancer, hepatic cancer, renal cancer, colorectal cancer, breast cancer, head cancer, neck cancer, oesophageal cancer, gynaecological cancer, ovarian cancar, ovary cancer, uterine cancer, prostate cancer, thyroid cancer, lymphoma, chronic leukaemia and acute leukaemia.

20. Use according to one of the claims 12 to 17, **characterised in that** the disorders are selected from the group consisting of arthritis, restenosis; fibrotic disorders; mesangial cell proliferative disorders, diabetic nephropathy, malignant nephrosclerosis, thrombotic microangiopathy syndromes, organ transplant rejection, glomerulopathies, metabolic disorders, inflammation, solid tumors, rheumatic arthritis, diabetic retinopathy, and neurodegenerative diseases.

21. Use according to one of the claims 12 to 15, **characterised in that** the disorders are selected from the group consisting of rheumatoid arthritis, inflammation, autoimmune disease, chronic obstructive pulmonary disease, asthma, inflammatory bowel disease, fibrosis, atherosclerosis, restenosis, vascular disease, cardiovascular disease, inflammation, renal disease and angiogenesis disorders.

22. Use of a compound according to one of the claims 1 to 4 as a raf-kinase inhibitor.

23. Use according to claim 22, **characterised in that** the raf-kinase is selected from the group consisting of A-Raf, B-Raf and c-Raf1.

24. Method for the treatment and/or prophylaxis of disorders, **characterised in that** one or more compounds according to one of the claims 1 to 4 is administered to a patient in need of such a treatment.

25. Method according to claim 24, **characterised in that** the one or more compounds according to one of the claims claim 1 to 4 are administered as a pharmaceutical composition according to claim 8 or 9.

26. Method for the treatment and/or prophylaxis of disorders according to claim 25, **characterised in that** the disorders are as defined in one of the claims 14 to 21.

27. Method for the treatment according to claim 26, **characterised in that** the disorder is cancerous cell growth mediated by raf-kinase.

28. Method for producing compounds of formula II, **characterised in that**
a) a compound of formula III 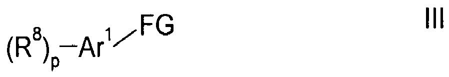 wherein
FG is a functional group, selected from
-N=C=Y and -NH-(C=Y)-LG,
wherein Y is as defined as in claim 3 and LG is a leaving group,
is reacted
b) with a compound of IV, 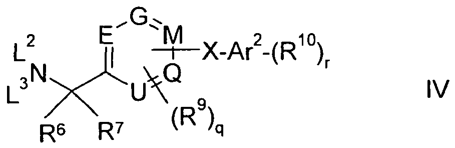 wherein
L², L³ are independently from one another H or a metal ion, and R⁶, R⁷, E, G, M, Q, U, R⁹, q, X, Ar², R¹⁰ and r are as defined in claim 3,
and optionally
c) isolating and/or treating the compound of formula II obtained by said reaction with an acid, to obtain the salt thereof.

29. Use of a compound of formula III, 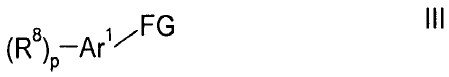 wherein
FG is a functional group, selected from
-N=C=Y and -NH-(C=Y)-LG,
wherein Y is as defined as in claim 3 and LG is a leaving group,
for the manufacture of a methylene urea derivative of formula II according to claim 1.

30. Use of a compound of formula IV, 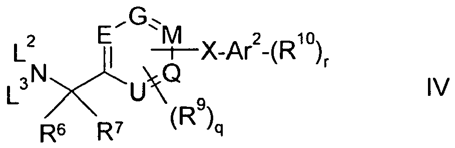 wherein
L², L³ are independently from one another H or a metal ion, and R⁶, R⁷, E, G, M, Q, U, R⁹, q, X, Ar², R¹⁰ and r are as defined in claim 3,
for the manufacture of a methylene urea derivative of formula II according to claim 1.
